# EUROPEAN PATENT SPECIFICATION

(11) **EP 0 771 794 B1**
(45) Date of publication and mention of the grant of the patent: **03.05.2006**
(21) Application number: 96915194.3
(22) Date of filing: 20.05.1996
(51) Int. Cl.: C07D 307/80, C07D 307/94, C07D 405/06, C07D 405/10, C07D 405/12, A61K 31/44, A61K 31/34

(54) **OXYGEN-CONTAINING HETEROCYCLIC COMPOUNDS**
SAUERSTOFF ENTHALTENDE HETEROCYCLISCHE VERBINDUNGEN
COMPOSES HETEROCYCLIQUES CONTENANT DE L'OXYGENE

(30) Priority: 19.05.1995 JP 12153795; 05.10.1995 JP 25865195
(43) Date of publication of application: 07.05.1997
(73) Proprietor: KYOWA HAKKO KOGYO CO., LTD., Chiyoda-ku, Tokyo 100 (JP)
(72) Inventor: KAWAKITA, Takashi, Sunto-gun Shizuoka 411 (JP); OHSHIMA, Etsuo, Sunto-gun Shizuoka 411 (JP); YANAGAWA, Koji, Sunto-gun Shizuoka 411 (JP); IIDA, Kyoichiro, Sunto-gun Shizuoka 411 (JP); KOIKE, Rie, Sunto-gun Shizuoka 411 (JP); ICHIMURA, Michio, Shizuoka 411 (JP); MANABE, Haruhiko, Sunto-gun Shizuoka 411 (JP); OHMORI, Kenji, Shizuoka 411 (JP); SUZUKI, Fumio, Shizuoka 411 (JP); NAKASATO, Yoshisuke, Sunto-gun Shizuoka 411 (JP)
(74) Representative: Casalonga, Axel
(86) International application number: PCT/JP1996/001327
(87) International publication number: WO 1996/036624

(56) References cited:
- WO-A-93/25517
- WO-A-96/03399
- WO-A-96/36625
- FR-A- 2 507 604
- JP-A- 1 110 684
- JP-A- 1 207 267
- JP-A- 60 169 473
- JP-A- 62 234 083
- JP-T- 6 503 829
- PHYTOCHEMISTRY, 26(6), (1987), BANERJEE SUNIL K. et al., "Sesebrinic Acid, a Cinnamic acid Derivative from Seseli Sibiricum", pages 1817-20.
- SCI. SIN., SER. B, (Engl. Ed.), 26(12), XIE JINGXI et al., "Studies on Antihepatitic Drugs - Total Synthesis of (.+-.)-Schizandrin C and Its Analogs", pages 1291-1303.
- J. CHEM. RES., SYNOP., (6), (1982), BISHOP DAVID et al., "3-Aminoalkylidene-3H-Indoles. Part 3. Reaction of 3-(1-Methylpyrrolidin-2-Ylidene)-3H-Indole with Diethyl Malonate", page 159.
- Z. NATURFORSCH., C: BIOSCI., 33C(7-8), (1978), DALLACKER FRANZ et al., "Derivatives of 1,3-Benzodioxoles. 43. 1,3-Benzodioxolecarboxylic Acids", pages 465-71.
- AUST. J. CHEM., 30(8), (1977), BERRY ROBERT C. et al., "Extractives of Australian Timbers. XVII. The Isolation, Structure and Synthesis of Koparin (7,2',3'-Tirhydroxy-4'-Methoxyisoflavone)", pages 1827-35.
- PHYTOCHEMISTRY, 16(8), (1977), GUIOTTO A. et al., "Coumarins from Unripe Fruits of Poncirus Trifoliata", pages 1257-60.
- ANN. CHIM., (Rome), 59(5), (1969), VENTURELLA PIETRO et al., "Structure of Trans-Meranzinic Acid (Trans-Auraptenic Acid)", pages 428-33.
- CHEM. BER., 102(8), (1969), DALLACKER FRANZ, "Derivatives of Methylene-Dioxybenzene. XXVII. Synthesis of Allyldimethoxy(Methylenedioxy)Benzenes", pages 2663-76.
- BULL. CHEM. SOC. JAP., 41(5), (1968), KAWASE YOSHIYUKI et al., "Synthesis of 4-Hydroxyfuro(2',3':7,8)Coumarins and 5H-Benzofuro(3,2-c) Furo(2,3h) (1)Benzopyran-5-one", pages 1201-8.
- J. CHEM. SOC., C. ORG., (8), (1966), S.F. DYKE et al., "Synthesis of Iso-flavones. IV. Munetone", pages 749-53.
- AGR. BIOL. CHEM., 25, (1961), MASATERU MIYANO et al., "Synthesis and Configurational Analysis of Rotenoids. XIX. The Total Synthesis of Natural Rotenone", pages 673-7.
- PHYTOCHEMISTRY, 4(2), (1965), T.R. SESHADRI et al., "Polyphenoles of the Stem Bark of Psidium Guava - a New Allagic Acid Glycoside (Amiritoside)", pages 317-26.
- BIOORG. MED. CHEM. LETT., 5(5), (1995), KAUFMAN TEODORO S. et al., "The Design, Synthesis and Evaluation of A,C,D-Ring Analogs of the Fungal Metabolite K-76 as Complement Inhibitors: A Potential Probe for the Absolute Stereochemistry Position 2", pages 501-6.
- CHEM. PHARM. BULL., 40(8), (1992), WADA HIROSHI et al., "Chemical and Chemtaxonomical Studies of Ferns. LXXXI. Characteristic Lignans of Blechnaceous Ferns", p. 2099-2101.
- CHEM. PHARM. BULL., 37(2), (1989), TANAKA TAKASHI et al., "Tannis and Related Compounds. Part LXXIII. Magnesium and Ammonium-Potassium Lithospermates B, the Active Principles Having a Uremia-Preventives Effect from Salvia Miltiorrhiza", pages 340-4.
- J. NAT. PROD., 51(1), (1988), AI CHUNBO et al., "Stereostructure of Salvianolic Acid C from Salvia Miltiorrhiza", pages 145-9.
- TETRAHEDRON LETT., 27(25), (1986), PARKER KATHLYN A. et al., "Aryl Radical-Initiated Cyclization: Effect of Aryl Substituents on Ring-Size", pages 2833-6.
- CHEM. PHARM. BULL., 34(5), (1986), AKASHI TOSHIHIRO et al., "Syntheses of Ring-Hydroxylated Nipradilols and Their Denitro Derivatives", pages 2024-36.
- J. CHEM. SOC., PERKIN TRANS. 1, (7), (1982), CROMBIE LESLIE et al., "Dihydrostilbenes of Cannabis. Synthesis of Canniprene", pages 1467-75.
- J. CHEM. SOC., PERKIN TRANS. 1, (7), (1982), CROMBIE LESLIE et al., "Natural Products of Thailand High. Delta, 1-THC-Strain Cannabis. The Bibenzyl-Spiran-Dihydrophenanthrere Group: Relations with Cannabinoids and Canniflavones", pages 1455-66.
- J. CHEM. SOC., PERKIN TRANS. 1, (12), (1981), LEE HIOK-HUANG, "Synthesis of the Mangostins", pages 3205-13.
- TETRAHEDRON LETT., (7), (1979), CROMBIE LESLIE et al., "Isolation of Cannabispiradienone and Cannabidihydrophenanthrene. Biosynthetic Relationships Between the Spirans and Dihydrostilbenes of Thailand Cannabis", pages 661-4.
- TETRAHEDRON LETT., (47), (1978), CROMBIE LESLIE et al., "Dihydrostilbenes of Thailand Cannabis", pages 4711-14.
- TETRAHEDRON LETT., (32), (1975), CANNON J.R. et al., "Structures of Nine Quinones Isolated from Two Conospermum Species", pages 2795-8.
- J. ORG. CHEM., 60(1), (1995), CRICH DAVID et al., "Inhibition of Rearrangements in Stannane-Mediated Radical Reduction Reactions by Catalytic Quantities of Diphenyl Deslenide. An Example of Polarity Reversal Catalysis", pages 84-8.

## Description

The present invention relates to oxygen-containing heterocyclic compounds which exhibit phosphodiesterase (PDE) IV inhibitory activity and which are useful as therapeutic agents for inflammatory allergic diseases such as bronchial asthma, allergic rhinitis, and nephritis; autoimmune diseases such as rheumatoid arthritis, multiple sclerosis, Crohn's disease, psoriasis, and systemic lupus erythematosus; diseases of the central nervous system such as depression, amnesia, and dementia; organopathy associated with ischemic reflux caused by cardiac failure, shock, and cerebrovascular diseases, and the like; insulin-resistant diabetes; wounds; AIDS, and the like.

Heretofore, it is known that the functions of numerous hormones and neurotransmitters are expressed by an increase in the concentration of adenosine 3',5'-cyclic monophosphate (cAMP) or guanosine 3',5'-cyclic monophosphate (cGMP), both of which are the secondary messengers in cells. The cellular concentrations of cAMP and cGMP are controlled by the generation and decomposition thereof, and their decomposition is carried out by PDE. Therefore, when PDE is inhibited, the concentrations of these secondary cellular messengers increase. Up to the present, 7 kinds of PDE isozymes have been found, and the isozyme-selective PDE inhibitors are expected to exhibit pharmacological effect based on their physiological significance and distribution in vivo (TiPS, 1990, 11, 150, TiPS, 1991, 12, 19).

It is known that the activation of inflammatory leukocytes can be suppressed by increasing the concentration of the cellular cAMP. The activation of leukocytes causes secretion of inflammatory cytokines such as tumor necrosis factor (THE), and expression of the cellular adhesion molecules such as intercellular adhesion molecules (ICAM), followed by cellular infiltration [J. Mol . Cell. Cardiol., 1989, 12, (Suppl. II), S61].

It is known that the contraction of a respiratory smooth muscle can be suppressed by increasing the concentration of the cellular cAMP (T. J. Torphy in Directions for New Anti-Asthma Drugs, eds S. R. O'Donell and C. G. A. Persson, 1988, 37, Birkhauser-Verlag). The contraction of a respiratory smooth muscle is a main symptom of bronchial asthma. Inflammatory-leukocyte infiltration of neutrophils and the like is observed in lesions of organopathy associated with ischemic reflux such as myocardial ischemia. It has been found that the IV type PDE (PDE IV) mainly participates in the decomposition of cAMP in these inflammatory cells and tracheal smooth muscle cells. Therefore, the inhibitors selective for PDE IV are expected to have therapeutic and/or preventive effect on inflammatory diseases, respiratory obstructive diseases, and ischemic diseases.

Further, the PDE IV inhibitors are expected to prevent the progress and spread of the inflammatory reaction transmitted by inflammatory cytokines such as TNFα and interleukin (IL)-8, because the PDE IV inhibitors suppress the secretion of these cytokines by increasing the concentration of cAMP. For example, TNFα is reported to be a factor of insulin-resistant diabetes because it declines the phosphorylating mechanism of insulin receptors of muscle and fat cells (J. Clin. Invest., 1994, 94, 1543-1549). Similarly, it is suggested that TNFα participates in the onset and progress of autoimmune diseases such as rheumatoid arthritis, multiple sclerosis, and Crohn's disease, and that the PDE IV inhibitors are useful for these diseases (Nature Medicine, 1995, 1, 211-214 and 244-248).

Drugs which increase cAMP are reported to enhance the healing of wounds [Nippon Yakuri-gakkai, the 68th annual meeting (Magoya), P3-116, 1995].

PDE IV-selective inhibitors having catechol structures are disclosed in WO96-00218, WO96-00215, WO95-35285, WO95-35284, WO95-35283, WO95-35281, WO95-28926, WO95-27692, WO95-24381, W095-22520, WO95-20578, WO95-17399, WO95-17392, WO95-14681, WO95-14680, WO95-14667, WO95-09837, WO95-09836, WO95-09627, WO95-09624, WO95-09623, WO95-08534, WO95-04046, WO95-04045, WO95-03794, WO95-01338, WO95-00516, WO95-00139, US5461056, EP0685479, EP0685475, EP0685474, EP0671389, WO93-25517, WO94-25437, EP0623607, WO94-20446, WO94-20455, WO94-14800, WO94-14742, WO94-12461, WO94-10118, WO94-02465, WO93-19751, WO93-19750, WO93-19749, WO93-19748, WO93-19747, WO93-18024, WO93-15048, WO93-07141, Japanese Published Unexamined Patent Application No. 117239/93, WO92-19594, and EP0497564.

Compounds which have a benzofuran structure and PDE IV-inhibitory activity are reported (Bioorganic Med. Chem. Lett., 1994, 14, 1855-1860, EP-0685479, WO96-03399).

Heretofore, benzofuran derivatives are industrially useful and are disclosed in patents of intermediates of product materials, light emitting elements, agricultural chemicals, anthelminthics, drugs, and the like.

Benzofuran, benzopyran, and benzodioxole derivatives which have a carboxyl group or a tetrazolyl group are disclosed in J. Med. Chem., 1988, 31, 84-91, and Japanese Published Unexamined Patent Application Nos. 50977/86, 126061/86, 143371/86, and 230760/87, and are described to exhibit leukotriene antagonism, phospholipase inhibitory activity, 5α reductase inhibitory activity, aldose-reductase inhibitory activity, and the like.

WO92-01681 and WO92-12144 disclose benzofuran and benzopyran derivatives which exhibit acyl-CoA acetyltransferase (ACAT) inhibitory activity.

WO93-01169 discloses benzofuran derivatives which exhibit tachykinin antagonism.

EP307172 and US4910193 disclose benzofuran derivatives which exhibit antagonistic activity against serotonin (5HT)₃ receptors.

WO 92/10096 discloses compounds that inhibit complement and/or supress immune activity. Pyridine derivatives for treatment and prevention of liver damage are taught by EP 0 285 267. EP 0 234 872 is directed to carboxamides useful as entiemetic or antipsychotic agents. EP 0 147 044 has for object benzofurancarboxamides and pharmaceutical preparations containing them.

FR 2507604 is directed to trimethoxy-3,4,5cinnamoyl piparazine compounds. Phytochemistry 26, (6)(1987) 1817-1820 discloses Sesebrinic acid, a cinnamic acid derivative from Seseli sibiricum. The Article J. Chem. Research (1982), 159 concerns 3-aminoalkylidene-3H-indoles. Document Aust. J. Chem. 30 (1977) 1827-1835 discloses the isolation structure and Synthesis of Koparin (7,2',3'-trihydroxy-4'-methoxyisoflavone).

The Article phytochemistry, 16(8) (1977) 1257-1260 concerns Coumarins from unripe fruits of Poncirus trifoliata. The Article Annali Di Chimica 59, (1969), 428-433, demonstrates the dihydrobenzofuranic structure of trans-meranzinic acid. Bull. Chem. Soc. Jpn. 41 (5) (1968) 1201-1208 concerns the synthesis of 4-hydroxyfuro[2',3':7,8]coumarins and 5H-benzofuro [3,2-c]furo[2,3-h] [1] benzopyran-5-one.

J. Chem. Soc. (C) (1966), 749-753 is directed to the synthesis of Isoflavones. Agr. Biol. Chem. 25 (9) (1961) 673-677 discloses the syntheses and configuration analysis of retinoids.

Biorganic and Medicinal Chemistry Letters 5(5), (1995), 501-506, concerns the design, synthesis and evaluation of A,C,D-ring analogs of the fungal metabolite K-76 as complement inhibitors.

Chem. Pharm. Bull. 40(8) (1992) 2099-2101 concerns chemical and chemotaxonomical studies of ferns. Chem. Pharm. Bull. 37(2) (1989) 340-344, is directed to the magnesium and ammonium-potassium Lithospermates B. Journal of Natural Product 51(1) (1988) 145-149 discloses the stereostructure of salvianolic acid B.

Tetrahedron Letters 27(25) (1986) 2833-2836 is directed to aryl radical-initiated cyclizations. The Articles J. Chem. Soc. Perkin Trans. I, (1982) 1467-1475, J. Chem. Soc. Perkin Trans. I (1982) 1455-1466, J. Chem. Soc. Perkin Trans. I (1981) 3205-3213 are directed to dihydrostilbenes of Cannabis, to natural products of Thailand High Δ¹⁻THC- strain Cannabis and to the synthesis of the mangostins.

Tetrahedron Letters (7) (1979), 661-664, and Tetrahedron Letters (47) (1978), 4711-4714, are directed to the isolation of cannabispiradienone and cannabidihydrophenanthrene and to the dihydrostylbenes of Thailand Cannabis.

Z. Naturforsch. 33C (7/8), (1978), 465-471, is a study of the preparation of 1,3-benzodioxolecarboxylic acids. Chem. Ber. 102 (1969) 2663-2676 is directed to the synthesis of dimethoxy-methylenedioxy-allylbenzol. J. Org. Chem. 60 (1995), 84-88, concerns the inhibition of rearrangements in Stannane-mediated radical reduction reactions by catalytic quantities of diphenyl Diselenide.

Chem. Pharm. Bull. 34 (5) (1986), 2024-2036, concerns the syntheses of ring-hydroxylated Nipradilols and their denitro derivatives.

WO 96/036625 has for object Phenyl Dihydrobenzofurane and WO 93/25517 concerns tri substituted phenyl derivatives as selective phosphordiesterase IV-inhibitors.

### Disclosure of the Invention

The present invention relates to the use of oxygen-containing heterocyclic compounds represented by following Formula (I): 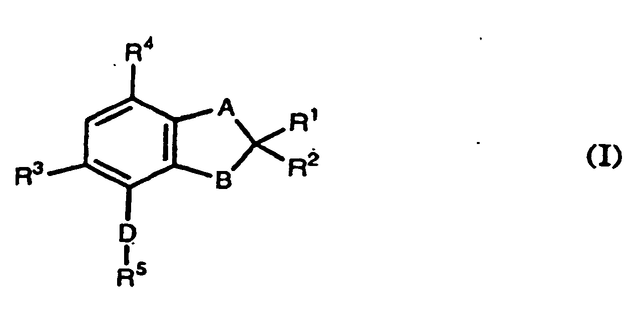 wherein R¹ and R² independently represent hydrogen, substituted or unsubstituted lower alkyl, cycloalkyl, polycycloalkyl, lower alkenyl, cycloalkenyl, substituted or unsubstituted aryl, a substituted or unsubstituted aromatic heterocyclic group, aralkyl, cyano, or -(CH₂)ₙ-E¹-CO-G¹ [wherein E¹ represents a bond, O, or NH; and G¹ represents hydrogen, substituted or unsubstituted lower alkyl, cycloalkyl, polycycloalkyl, substituted or unsubstituted aryl, a substituted or unsubstituted aromatic heterocyclic group, aralkyl, OR⁶ (wherein R⁶ represents hydrogen, lower alkyl, cycloalkyl, polycycloalkyl, substituted or unsubstituted aryl, a substituted or unsubstituted aromatic heterocyclic group, or aralkyl), or NR⁷R⁸ (wherein R⁷ and R⁸ independently represent hydrogen, lower alkyl, cycloalkyl, polycycloalkyl, substituted or unsubstituted aryl, a substituted or unsubstituted aromatic heterocyclic group, substituted or unsubstituted aralkyl, or heteroarylalkyl; or R⁷ and R⁸ are combined to represent a substituted or unsubstituted heterocyclic group containing a nitrogen atom); and n represents an integer of 0 to 4]; R¹ and R² are combined to represent a saturated carbon ring together with a carbon atom adjacent thereto; or R², and R¹¹ or R¹³ described below are combined to form a single bond; R³ represents hydrogen, phenyl, or halogen; R⁴ represents hydroxy or substituted or unsubstituted lower alkoxy; A represents -C(R⁹)(R¹⁰)- (wherein R⁹ and R¹⁰ independently represent hydrogen, substituted or unsubstituted lower alkyl, cycloalkyl, or polycycloalkyl) or O; B represents O, NR¹¹ [wherein R¹¹ represents hydrogen, lower alkyl, cycloalkyl, polycycloalkyl, lower alkenyl, cycloalkenyl, substituted or unsubstituted aryl, a substituted or unsubstituted aromatic heterocyclic group, aralkyl, or -(CH₂)ₘ-E²-CO-G² (wherein E², G², and m have the same meanings as the above-described E¹, G¹, and n, respectively); or R¹¹ and R² are combined to form a single bond], -C(R¹²)(R¹³)- [wherein R¹² and R¹³ independently represent hydrogen, substituted or unsubstituted lower alkyl, cycloalkyl, polycycloalkyl, lower alkenyl, cycloalkenyl, substituted or unsubstituted aryl, a substituted or unsubstituted aromatic heterocyclic group, aralkyl, cyano, or -(CH₂)ₚ-E³-CO-G³ (wherein E³, G³, and p have the same meanings as the above-described E¹, G¹, and n, respectively); R¹³ and R² are combined to form a single bond; or R¹³ and R² are combined to form a saturated carbon ring together with two carbon atoms adjacent thereto]; or -C(R¹⁴) (R¹⁵)-C(R¹⁶) (R¹⁷)- [wherein R¹⁴ and R¹⁵ independently represent hydrogen, substituted or unsubstituted lower alkyl, cycloalkyl, aralkyl, substituted or unsubstituted aryl, or a substituted or unsubstituted aromatic heterocyclic group; or R¹⁴ and R¹⁵ are combined to form O; and R¹⁶ and R¹⁷ independently represent hydrogen, substituted or unsubstituted lower alkyl, cycloalkyl, aralkyl, substituted or unsubstituted aryl, or a substituted or unsubstituted aromatic heterocyclic group; or R¹⁷ and R¹⁵ are combined to form a single bond; or R¹⁷ and R¹⁵ are combined to form a saturated carbon ring together with two carbon atoms adjacent thereto]; D represents (i) -C(R¹⁸)(R¹⁹)-X- [wherein R¹⁸ represents hydrogen, substituted or unsubstituted lower alkyl, cycloalkyl, polycycloalkyl, lower alkenyl, cycloalkenyl, substituted or unsubstituted aryl, a substituted or unsubstituted aromatic heterocyclic group, hydroxy, substituted or unsubstituted lower alkoxy, or lower alkanoyloxy; and R¹⁹ represents hydrogen, substituted or unsubstituted lower alkyl, cycloalkyl, polycycloalkyl, lower alkenyl, cycloalkenyl, substituted or unsubstituted aryl, a substituted or unsubstituted aromatic heterocyclic group, hydroxy, substituted or unsubstituted lower alkoxy, lower alkanoyloxy, lower alkanoyl, cycloalkanoyl, lower alkoxycarbonyl, or cyano; or R¹⁸ and R¹⁹ are combined to form O, S, or NR²⁰ (wherein R²⁰ represents hydrogen, substituted or unsubstituted lower alkyl, cycloalkyl, polycycloalkyl, lower alkenyl, cycloalkenyl, substituted or unsubstituted aryl, a substituted or unsubstituted aromatic heterocyclic group, hydroxy, substituted or unsubstituted lower alkoxy, or lower alkanoyloxy); X represents -C(R²¹)(R²²)- (wherein R²¹ and R²² independently represent hydrogen, substituted or unsubstituted lower alkyl, cycloalkyl, polycycloalkyl, lower alkenyl, cycloalkenyl, substituted or unsubstituted aryl, a substituted or unsubstituted aromatic heterocyclic group, lower alkanoyl, cycloalkanoyl, lower alkoxycarbonyl, or cyano) or S; or X represents NR²³ (wherein R²³ represents hydrogen, lower alkyl, cycloalkyl, substituted or unsubstituted aryl, a substituted or unsubstituted aromatic heterocyclic group, or aralkyl) unless R¹ and R² simultaneously represent substituted or unsubstituted lower alkyl, cycloalkyl, polycycloalkyl, lower alkenyl, or cycloalkenyl included in the above definition], (ii) -C(R^{19a})=Y- [wherein R^{19a} represents hydrogen, substituted or unsubstituted lower alkyl, cycloalkyl, polycycloalkyl, lower alkenyl, cycloalkenyl, substituted or unsubstituted aryl, a substituted or unsubstituted aromatic heterocyclic group, hydroxy, substituted or unsubstituted lower alkoxy, lower alkanoyloxy, lower alkanoyl, cycloalkanoyl, lower alkoxycarbonyl, or cyano; and Y represents -C(R²⁴)-Z-(wherein R²⁴ represents hydrogen, substituted or unsubstituted lower alkyl, cycloalkyl, polycycloalkyl, lower alkenyl, cycloalkenyl, substituted or unsubstituted aryl, a substituted or unsubstituted aromatic heterocyclic group, lower alkanoyl, cycloalkanoyl, lower alkoxycarbonyl, or cyano; or R²⁴ and R^{19a} are combined to form a single bond; and Z represents CONH, CONHCH₂, or a bond) or N], or (iii) a bond; and R⁵ represents substituted or unsubstituted aryl, a substituted or unsubstituted aromatic heterocyclic group, cycloalkyl, pyridine-N-oxide, cyano, or lower alkoxycarbonyl; or pharmaceutically acceptable salts thereof for the preparation of a phosphodiesterase IV inhibitor.

The present invention relates also to the use of an oxygen-containing heterocyclic compound represented by following Formula (ZA): 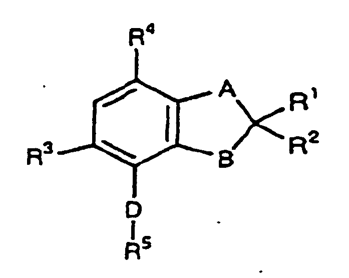 wherein R¹ and R² independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, aralkyl, cyano, or -(CH₂)ₙ-E¹-CO-G¹ {wherein E¹ represents a bond, O, or NH; and G¹ represents hydrogen, substituted or unsubstituted C₁₋₈ alkyl, cycloalkyl, poly-cycloalkyl, substituted or unsubstituted aryl, a substituted or unsubstituted aromatic heterocyclic group, aralkyl, OR⁶ (wherein R⁶ represents hydrogen, C₁₋₈ alkyl, cycloalkyl, polycycloalkyl, substituted or unsubstituted aryl, a substituted or unsubstituted aromatic heterocyclic group, or aralkyl), or NR⁷R⁸ (wherein R⁷ and R⁸ independently represent hydrogen, C₁₋₈alkyl, cycloalkyl, polycycloalkyl, substituted or unsubstituted aryl, a substituted or unsubstituted aromatic heterocyclic group, substituted or unsubstituted aralkyl, or heteroarylalkyl; or R⁷ and R⁸ are combined to represent a substituted or unsubstituted heterocyclic group containing a nitrogen atom adjacent thereto): and n represents an integer of 0 to 4}; R¹ and R² are combined to represent a saturated carbon ring together with a carbon atom adjacent thereto; or R², and R¹³ described below are combined to form a single bond;
R³ represents hydrogen, phenyl, or halogen;
R⁴ represents hydroxy or substituted or unsubstituted C₁₋₈ alkoxy;
R⁵ represents substituted or unsubstituted aryl, a substituted or unsubstituted aromatic heterocyclic group (said substituted aromaticheterocyclic group has 1 to 3 substituents), cycloalkyl, pyxidine-N-oxide, cyano, or C₁₋₈ alkoxycarbonyl;
A represents -CCR⁹)(R¹⁰)- (wherein R⁹ and R¹⁰ independently represent hydrogen, substituted or unsubstituted C₁₋₈ alkyl, cycloalkyl, or polycycloalkyl) or O;
B represents O.
-C(R¹²)(R¹³)- {wherein R¹² and R¹³ Independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, aralkyl, cyano, or -(CH₂)ₚ-E³-CO-G³ (wherein E³, G³, and p have the same meanings as the above-described E¹, G¹, and n, respectively); R¹³ and R² are combined to form a single bond; or R¹³ and R² are combined to form a saturated carbon ring together with two carbon atoms adjacent thereto}; or
-C(R¹⁴)(R¹⁵)-C(R¹⁶)(R¹⁷)- {wherein R¹⁴ and R¹⁵ independently represent hydrogen, substituted or unsubstituted C₁₋₈ alkyl, cycloalkyl, aralkyl, substituted or unsubstituted aryl, or a substituted or unsubstituted aromatic heterocyclic group; or R¹⁴ and R¹⁵ are combined to form O; and R¹⁶ and R¹⁷ independently represent hydrogen, substituted or unsubstituted C₁₋₈ alkyl, cycloalkyl, aralkyl, substituted or unsubstituted aryl, or a substituted or unsubstituted aromatic heterocyclic group; or R¹⁷ and R¹⁵ are combined to form a single bond; or R¹⁷ and R¹⁵ are combined to form a saturated carbon ring together with two carbon atoms adjacent thereto}; and
D represents (i) -C(R¹⁸)(R¹⁹)-X- {wherein R¹⁸ represents hydrogen, substituted or unsubstituted C₁₋₈ alkyl, cycloalkyl, polycyploalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy; and R¹⁹ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano; or R¹⁸ and R¹⁹ are combined to form O, S, or NR²⁰ (wherein R²⁰ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy); X represents (a) -C(R²¹)(R²²)- (wherein R²¹ and R²² independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, C₂₋₉ alkanoyl, cycloalkanoyl, C₁₋₈ alkoxycarbonyl, or cyano) or (b) S; or X represents (c) NR²³ (wherein R²³ represents hydrogen. C₁₋₈ alkyl, cycloalkyl, substituted or unsubstituted aryl, a substituted or unsubstituted aromatic heterocyclic group, or aralkyl) unless R¹ and R² simultaneously represent substituted or unsubstituted C₁₋₈ alkyl, cycloalkyl, polycycloalkyl, C₂₋₈ alkenyl, or cycloalkenyl in the above definition or unless R¹ and R² are combined to represent a saturated carbon ring together with a carbon atom adjacent thereto and R⁵ represents substituted or unsubstituted phenyl or substituted or substituted pyridyl}, or
(ii) -C(R^{19a})=Y- {wherein R^{19a} represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano; and Y represents -C(R²⁴)-Z- (wherein R²⁴ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, C₂₋₉ alkanoyl, cycloalkanoyl, C₁₋₈ alkoxycarbonyl, or cyano; or R²⁴ and R^{19a} are combined to form a single bond; and Z represents CONH, CONHCH₂, or a bond) or N}, orD represents (iii) a bond unless R⁵ represents substituted or unsubstituted phenyl, cyano, or C₁₋₈ alkoxycarbonyl,
or a pharmaceutically acceptable salt thereof for the preparation of a pbarmaceutlcal composition which is useful for the treatment of inflammatory allergic diseases. Hereinafter, the compounds represented by Formula (I) are referred to as Compounds (I). The same applies to the compounds of other formula numbers.

In the definitions of the groups in Formula (I), the lower alkyl and the lower alkyl moiety of the lower alkoxy, the lower alkanoyloxy, the lower alkanoyl, the lower alkoxycarbonyl, and the heteroarylalkyl include straight-chain or branched alkyl groups having 1 to 8 carbon atoms, such as methyl, ethyl, propyl, isopropyl, butyl, isobutyl, sec-butyl, tert-butyl, pentyl, hexyl, heptyl, and octyl; the cycloalkyl and the cycloalkyl moiety of the cycloalkanoyl include cycloalkyl groups having 3 to 10 carbon atoms, such as cyclopropyl, cyclobutyl, cyclopentyl, cyclohexyl, cycloheptyl, cyclooctyl, cyclononyl, and cyclodecyl; and the polycycloalkyl includes polycycloalkyl groups having 4 to 12 carbon atoms, such as bicyclo[3.2.1]octyl, bicyclo[4.3.2]undecyl, adamantyl, and noradamantyl. The lower alkenyl includes straight-chain or branched alkenyl groups having 2 to 8 carbon atoms, such as vinyl, 1-propenyl, allyl, methacryl, 1-butenyl, crotyl, pentenyl, isoprenyl., hexenyl, heptenyl, and octenyl; and the cycloalkenyl includes cycloalkenyl groups having 4 to 10 carbon atoms, such as cyclobutenyl, cyclopentenyl,
cyclohexenyl, cycloheptenyl, cyclooctenyl, cyclononenyl, and cyclodecenyl. The aryl includes phenyl and naphthyl; and the aralkyl includes aralkyl groups having 7 to 15 carbon atoms, such as benzyl, phenethyl, benzhydryl, and naphthylmethyl. The aromatic heterocyclic group and the heteroaryl moiety of the heteroarylalkyl include pyridyl, pyrazinyl, pyrimidinyl, pyridazinyl, quinolinyl, isoquinolinyl, phthalazinyl, quinazolinyl, quinoxalinyl, naphthyridinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl, and purinyl. The heterocyclic group containing a nitrogen atom includes pyrrolidinyl, piperidino, piperazinyl, morpholino, thiomorpholino, homopiperidino, homopiperazinyl, tetrahydropyridinyl, tetrahydroquinolinyl, and tetrahydroisoquinolinyl; and the saturated carbon ring together with two adjacent carbon atoms includes groups having 3 to 10 carbon atoms, such as cyclopropane, cyclobutane, cyclopentane, cyclohexane, cycloheptane, cyclooctane, cyclononane, and cyclodecane. The halogen includes a fluorine, chlorine, bromine, and iodine atom.

The substituted lower alkyl has the same or different 1 to 2 substituents such as cycloalkyl, which has the same meaning as defined above.

The substituted aryl, substituted aromatic heterocyclic group, and substituted aralkyl each has the same or different 1 to 3 substituents such as lower alkyl, hydroxy, lower alkoxy, lower alkanoyl, lower alkoxycarbonyl, carboxyl, aminocarbonyl, trifluoromethyl, amino, cyano, nitro, and halogen. The lower alkyl, lower alkoxy, lower alkanoyl, lower alkoxycarbonyl, and halogen each has the same meaning as defined above.

The substituted heterocyclic group containing a nitrogen atom has the same or different 1 to 3 substituents such as lower alkyl, cycloalkyl, aryl, and aralkyl. The lower alkyl, cycloalkyl, aryl, and aralkyl each has the same meaning as defined above.

The substituted lower alkoxy has the same or different 1 to 3 substituents such as halogen, which has the same meaning as defined above.

The pharmaceutically acceptable salts of Compounds (I) include pharmaceutically acceptable acid addition salts, metal salts, ammonium salts, and organic amine addition salts.

The pharmaceutically acceptable acid addition salts of Compounds (I) include inorganic acid addition salts such as hydrochloride, sulfate, nitrate, and phosphate, and organic acid addition salts such as acetate, maleate, fumarate, and citrate; the pharmaceutically acceptable metal salts include alkali metal salts such as sodium salt and potassium salt, alkaline earth metal salts such as magnesium salt and calcium salt, aluminium salt; and zinc salt; the pharmaceutically acceptable ammonium salts include ammonium and tetramethylammonium; and the pharmaceutically acceptable organic amine addition salts include addition salts with morpholine and piperidine.

The present invention relates also to oxygen-containing heterocyclic compounds represented by following Formula (I): 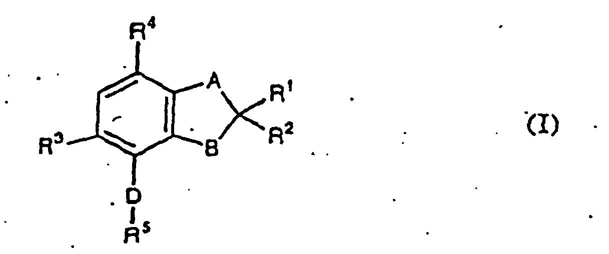 wherein R¹ and R² independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, aralkyl, cyano, or -(CH₂)ₙ-E¹-CO-G¹ {wherein E¹ represents a bond, O, or NH; and G¹ represents hydrogen, substituted or unsubstituted C₁₋₈ alkyl, cycloalkyl, polycycloalkyl, substituted or unsubstituted aryl, a substituted or unsubstituted aromatic heterocyclic group, aralkyl, OR⁶ (wherein R⁶ represents hydrogen, C₁₋₈ alkyl, cycloalkyl, polycycloalkyl, substituted or unsubstituted aryl, a substituted or unsubstituted aromatic heterocyclic group, or aralkyl), or NR⁷R⁸ (wherein R⁷ and R⁸ independently represent hydrogen, C₁₋₈ alkyl, cycloalkyl, polycycloalkyl, substituted or unsubstituted aryl, a substituted or unsubstituted aromatic heterocyclic group, substituted or unsubstituted aralkyl, or heteroarylalkyl; or R⁷ and R⁸ are combined to represent a substituted or unsubstituted heterocyclic group containing a nitrogen atom adjacent thereto); and n represents an integer of 0 to 4); R¹ and R² are combined to represent a saturated carbon ring together with a carbon atom adjacent thereto; or R², and R¹³ described below are combined to form a single bond;
R³ represents hydrogen, phenyl, or halogen;
R⁴ represents hydroxy or substituted or unsubstituted C₁₋₈ alkoxy;
R⁵ represents substituted or unsubstituted aryl, a substituted or unsubstituted aromatic heterocyclic group (said substituted aromatic heterocyclic group has 1 to 3 substituents), cycloalkyl, pyridine-N-oxide, cyano, or C₁₋₈ alkoxycarbonyl;
A represents -C(R⁹) (R¹⁰)- (wherein R⁹ and R¹⁰ independently represent hydrogen, substituted or unsubstituted C₁₋₈ alkyl, cycloalkyl, or polycycloalkyl) or O;
B represents O,
-C(R¹²) (R¹³)- {wherein R¹² and R¹³ independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, aralkyl, cyano, or -(CH₂)ₚ-E³-CO-G³ (wherein E³, G³, and p have the same meanings as the above-described E¹, G¹, and n, respectively); R¹³ and R² are combined to form a single bond; or R¹³ and R² are combined to form a saturated carbon ring together with two carbon atoms adjacent thereto}; or
-C(R¹⁴)(R¹⁵)-C(R¹⁶)(R¹⁷)- {wherein R¹⁴ and R¹⁵ independently represent hydrogen, substituted or unsubstituted C₁₋₈ alkyl, cycloalkyl, aralkyl, substituted or unsubstituted aryl, or a substituted or unsubstituted aromatic heterocyclic group; or
R¹⁴ and R¹⁵ are combined to form O; and R¹⁶ and R¹⁷ independently represent hydrogen, substituted or unsubstituted C₁₋₈ alkyl, cycloalkyl, aralkyl, substituted or unsubstituted aryl, or a substituted or unsubstituted aromatic heterocyclic group; or
R¹⁷ and R¹⁵ are combined to form a single bond; or R¹⁷ and R¹⁵ are combined to form a saturated carbon ring together with two carbon atoms adjacent thereto},
with the proviso that when A represents -C(R⁹)(R¹⁰)-, B does not represent O; and
D represents (i) -C(R¹⁸)(R¹⁹)-X- {wherein R¹⁸ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy; and R¹⁹ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano; or R¹⁸ and R¹⁹ are combined to form O, S, or NR²⁰ (wherein R²⁰ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy); X represents (a) -C(R²¹)(R²²)- (wherein R²¹ and R²² independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, C₂₋₉ alkanoyl, cycloalkanoyl, C₁₋₈ alkoxycarbonyl, or cyano) or (b) S; or X represents (c) NR²³ (wherein R²³ represents hydrogen, C₁₋₈ alkyl, cycloalkyl, substituted or unsubstituted aryl, a substituted or unsubstituted aromatic heterocyclic group, or aralkyl) unless R¹ and R² simultaneously represent substituted or unsubstituted C₁₋₈ alkyl, cycloalkyl, polycycloalkyl, C₂₋₈ alkenyl, or cycloalkenyl in the above definition or unless R¹ and R² are combined to represent a saturated carbon ring together with a carbon atom adjacent thereto and R⁵ represents substituted or unsubstituted phenyl or substituted or unsubstituted pyridyl}, or
(ii) -C(R^{19a})=Y- {wherein R^{19a} represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano; and Y represents -C(R²⁴)-Z-(wherein R²⁴ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, C₂₋₉ alkanoyl, cycloalkanoyl, C₁₋₈ alkoxycarbonyl, or cyano; or R²⁴ and R^{19a} are combined to form a single bond; and Z represents CONH, CONHCH₂, or a bond) or N), or D represents
(iii) a bond unless R⁵ represents substituted or unsubstituted phenyl, cyano, or C₁₋₈ alkoxycarbonyl,
with the proviso that when R⁵ represents substituted or unsubstituted phenyl or C₁₋₈ alkoxycarbonyl, D does not represent -CH₂CH₂- or -CH=CH-;
or a pharmaceutically acceptable salt thereof.

An other object of the invention is the use of an oxygen-containing heterocyclic compound represented by following Formula 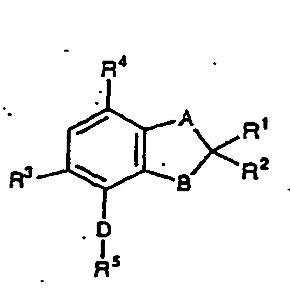 wherein R¹ and R² independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, aralkyl, cyano, or -(CH₂)ₙ-E¹-CO-G¹ {wherein E¹ represents a bond, O, or NH; and G¹ represents hydrogen, substituted or unsubstituted C₁₋₈ alkyl, cycloalkyl, polycycloalkyl, substituted or unsubstituted aryl, a substituted or unsubstituted aromatic heterocyclic group, aralkyl, OR⁶ (wherein R⁶ represents hydrogen, C₁₋₈ alkyl, cycloalkyl, polycycloalkyl, substituted or unsubstitutedaryl, a substituted or unsubstituted aromatic heterocyclic group, or aralkyl), or NR⁷R⁸ (wherein R⁷ and R⁸ independently represent hydrogen, C₁₋₈ alkyl, cycloalkyl, polycycloalkyl, substituted or unsubstituted aryl, a substituted or unsubstituted aromatic heterocyclic group, substituted or unsubstituted aralkyl, or heteroarylalkyl; or R⁷ and R⁸ are combined to represent a substituted or unsubstituted heterocyclic group containing a nitrogen atom adjacent thereto); and n represents an integer of 0 to 4}; R¹ and R² are combined to represent a saturated carbon ring together with a carbon atom adjacent thereto; or R², and R¹³ described below are combined to form a single bond;.
R³ represents hydrogen, phenyl, or halogen;
R⁴ represents hydroxy or substituted or unsubstituted C₁₋₈ alkoxy;
R⁵ represents substituted or unsubstituted aryl, a substituted or unsubstituted aromatic heterocyclic group (said substituted aromatic heterocyclic group has 1 to 3 substituents), cycloalkyl, pyridine-N-oxide, cyano, or C₁₋₈ alkoxycarbonyl;
A represents -C(R⁹)(R¹⁰)- (wherein R⁹ and R¹⁰ independently represent hydrogen, substituted or unsubstituted C₁₋₈ alkyl, cycloalkyl, or polycycloalkyl) or O; B represents O,
-C(R¹²)(R¹³)- {wherein R¹² and R¹³ independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic groups aralkyl, cyano, or -(CH₂)ₚ-E³-CO-G³ (wherein E³, G³, and p have the same meanings as the above-described E¹, G¹, and n, respectively); R¹³ and R² are combined to form a single bond; or R¹³ and R² are combined to form a saturated carbon ring together with two carbon atoms adjacent thereto}; or
-C(R¹⁴) (R¹⁵)-C(R¹⁶) (R¹⁷)- {wherein R¹⁴ and R¹⁵ independently represent hydrogen, substituted or unsubstituted C₁₋₈ alkyl, cycloalkyl, aralkyl, substituted or unsubstituted aryl, or a substituted or unsubstituted aromatic heterocyclic group; or
R¹⁴ and R¹⁵ are combined to form O; and R¹⁶ and R¹⁷ independently represent hydrogen, substituted or unsubstituted C₁₋₈ alkyl, cycloalkyl, aralkyl, substituted or unsubstituted aryl, or a substituted or unsubstituted aromatic heterocyclic group; or
R¹⁷ and R¹⁵ are combined to form a single bond; or R¹⁷ and R¹⁵ are combined to form a saturated carbon ring together with two carbon atoms adjacent thereto},
with the proviso that when A represents -C(R⁹)(R¹⁰)-, B does not represent O; and
D represents (i) -C(R¹⁸)(R¹⁹)-X- {wherein R¹⁸ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy; and R¹⁹ represents hydrogeri, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano; or R¹⁸ and R¹⁹ are combined to form O, S, or NR²⁰ (wherein R²⁰ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy); X represents (a) -C(R²¹)(R²²)- (wherein R²¹ and R²² independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, C₂₋₉ alkanoyl, cycloalkanoyl, C₁₋₈ alkoxycarbonyl, or cyano) or (b) S; or X represents (c) NR²³ (wherein R²³ represents hydrogen, C₁₋₈ alkyl, cycloalkyl, substituted or unsubstituted aryl, a substituted or unsubstituted aromatic heterocyclic group, or aralkyl) unless R¹ and R² simultaneously represent substituted or unsubstituted C₁₋₈ alkyl, cycloalkyl, polycycloalkyl, C₂₋₈ alkenyl, or cycloalkenyl in the above definition or unless R¹ and R² are combined to represent a saturated carbon ring together with a carbon atom adjacent thereto and R⁵ represents substituted or unsubstituted phenyl or substituted or unsubstituted pyridyl}, or
(ii) -C(R^{19a})=Y- {wherein R^{19a} represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano; and Y represents -C(R²⁴)-Z-(wherein R²⁴ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic groups C₂₋₉ alkanoyl, cycloalkanoyl, C₁₋₈ alkoxycarbonyl, or cyano; or R²⁴ and R^{19a} are combined to form a single bond; and Z represents CONH, CONHCH₂, or a bond) or N}, or D represents
(iii) a bond unless R⁵ represents substituted or unsubstituted phenyl, cyano, or C₁₋₈ alkoxycarbonyl,
with the proviso that when R⁵ represents substituted or unsubstituted phenyl or C₁₋₈ alkoxycarbonyl, D does not represent -CH₂CH₂- or -CH=CH-;
or a pharmaceutically acceptable salt thereof for the preparation of a phosphodiesterase IV inhibitor.

An other object of the invention is the use of An oxygen-containing heterocyclic compound represented by following Formula 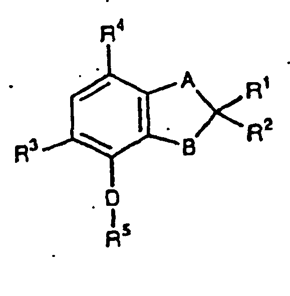 wherein R¹ and R² independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, aralkyl, cyano, or -(CH₂)ₙ-E¹-CO-G¹ {wherein E¹ represents a bond, O, or NH; and G¹ represents hydrogen, substituted or unsubstituted C₁₋₈ alkyl, cycloalkyl, polycycloalkyl, substituted or unsubstituted aryl, a substituted or unsubstituted aromatic heterocyclic group, aralkyl, OR⁶ (wherein R⁶ represents hydrogen, C₁₋₈ alkyl, cycloalkyl, polycycloalkyl, substituted or unsubstituted aryl, a substituted or unsubstituted aromatic heterocyclic group, or aralkyl), or NR⁷R⁸ (wherein R⁷ and R⁸ independently represent hydrogen, C₁₋₈ alkyl, cycloalkyl, polycycloalkyl, substituted or unsubstituted aryl, a substituted or unsubstituted aromatic heterocyclic group, substituted or unsubstituted aralkyl, or heteroarylalkyl; or R⁷ and R⁸ are combined to represent a substituted or unsubstituted heterocyclic group containing a nitrogen atom adjacent thereto); and n represents an integer of 0 to 4}; R¹ and R² are combined to represent a saturated carbon ring together with a carbon atom adjacent thereto; or R², and R¹³ described below are combined to form a single bond;
R³ represents hydrogen, phenyl, or halogen;
R⁴ represents hydroxy or substituted or unsubstituted C₁₋₈ alkoxy;
R⁵ represents substituted or unsubstituted aryl, a substituted or unsubstituted aromatic heterocyclic group (said substituted aromatic heterocyclic group has 1 to 3 substituents), cycloalkyl, pyridine-N-oxide, cyano, or C₁₋₈ alkoxycarbonyl;
A represents -C(R⁹)(R¹⁰)- (wherein R⁹ and R¹⁰ independently represent hydrogen, substituted or unsubstituted C₁₋₈ alkyl, cycloalkyl, or polycycloalkyl) or O;
B represents O, -C(R¹²)(R¹³)- {wherein R¹² and R¹³ independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, aralkyl, cyano, or -(CH₂)ₚ-E³-CO-G³
(wherein E³, G³, and p have the same meanings as the above-described E¹, G¹, and n, respectively); R¹³ and R² are combined to form a single bond; or R¹³ and R² are combined to form a saturated carbon ring together with two carbon atoms adjacent thereto}; or
-C(R¹⁴)(R¹⁵)-C(R¹⁶)(R¹⁷)- {wherein R¹⁴ and R¹⁵ independently represent hydrogen, substituted or unsubstituted C₁₋₈ alkyl, cycloalkyl, aralkyl, substituted or unsubstituted aryl, or a substituted or unsubstituted aromatic heterocyclic group; or
R¹⁴ and R¹⁵ are combined to form O; and R¹⁶ and R¹⁷ independently represent hydrogen, substituted or unsubstituted C₁₋₈ alkyl, cycloalkyl, aralkyl, substituted or unsubstituted aryl, or a substituted or unsubstituted aromatic heterocyclic group; or
R¹⁷ and R¹⁵ are combined to form a single bond; or R¹⁷ and R¹⁵ are combined to form a saturated carbon ring together with two carbon atoms adjacent thereto},
with the proviso that when A represents -C (R⁹) (R¹⁰)-, B does not represent O; and
D represents (i) -C(R¹⁸)(R¹⁹)-X- {wherein R¹⁸ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy; and R¹⁹ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic groups hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano; or R¹⁸ and R¹⁹ are combined to form O, S, or NR²⁰ (wherein R²⁰ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy); X represents
(a) -C(R²¹) (R²²)- (wherein R²¹ and R²² independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, C₂₋₉ alkanoyl, cycloalkanoyl, C₁₋₈ alkoxycarbonyl, or cyano) or (b) S; or X represents (c) NR²³ (wherein R²³ represents hydrogen, C₁₋₈ alkyl, cycloalkyl, substituted or unsubstituted aryl, a substituted or unsubstituted aromatic heterocyclic group, or aralkyl) unless R¹ and R² simultaneously represent substituted or unsubstituted C₁₋₈ alkyl, cycloalkyl, polycycloalkyl, C₂₋₈ alkenyl, or cycloalkenyl in the above definition or unless R¹ and R² are combined to represent a saturated carbon ring together with a carbon atom adjacent thereto and R⁵ represents substituted or unsubstituted phenyl or substituted or unsubstituted pyridyl}, or
(ii) -C(R^{19a})=Y- {wherein R^{19a} represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano; and Y represents -C(R²⁴)-Z-(wherein R²⁴ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, C₂₋₉ alkanoyl, cycloalkanoyl, C₁₋₈ alkoxycarbonyl, or cyano; or R²⁴ and R^{19a} are combined to form a single bond; and Z represents CONH, CONHCH₂, or a bond) or N), or D represents (iii) a bond unless R⁵ represents substituted or unsubstituted phenyl, cyano, or C₁₋₈ alkoxycarbonyl, with the proviso that when R⁵ represents substituted or unsubstituted phenyl or C₁₋₈ alkoxycarbonyl, D does not represent -CH₂CH₂- or -CH=CH-;
or a pharmaceutically acceptable salt thereof for the préparation of a pharmaceutical composition which is useful for the treatment of inflammatory allergic diseases.

Processes for preparing Compound (I) are described below.

Manufacturing method 1: Compound (Ia), which is Compound (I) in which D is (i) -C(R¹⁸)(R¹⁹)-X- and R⁵ is substituted or unsubstituted aryl or a substituted or unsubstituted aromatic heterocyclic group, can be obtained according to the following Processes 1-1 to 1-13.

Process 1-1: Compound (Iaa), which is Compound (Ia) in which X is -C(R²¹)(R²²)-, and R¹⁸ and R¹⁹ are not combined to form O, S, or NR²⁰, can be prepared according to the following reaction steps: 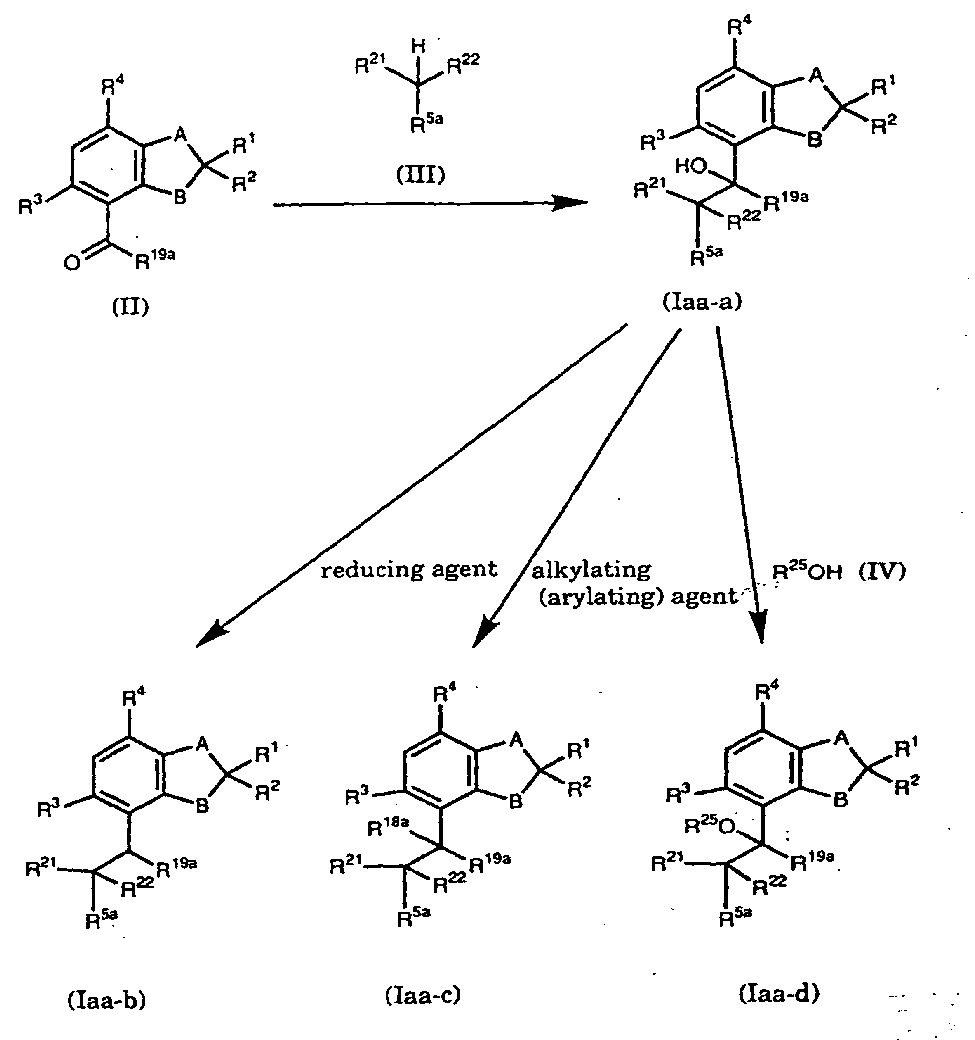 (In the formulae, R^{5a} is substituted or unsubstituted aryl or a substituted or unsubstituted aromatic heterocyclic group in the definition of R⁵; R^{18a} is a group other than hydrogen, hydroxy, substituted or unsubstituted lower alkoxy, and lower alkanoyloxy in the definition of R¹⁸, and R^{18a} and R¹⁹ are not combined to form O, S, or NR²⁰; R²⁵ is substituted or unsubstituted lower alkyl or lower alkanoyl; and A, B, R¹, R², R³, R⁴, R^{19a}, R²¹, and R²² each has the same meaning as defined above.)

The substituted or unsubstituted lower alkyl and lower alkanoyloxy in the definition of R²⁵ each has the same meaning as defined above.

The starting Compound (II) can be obtained according to the known methods (J. Org. Chem., 1987, 52, 4072, Org. Prep. Proced. Int., 1989, 21, 763, Synthesis, 1978, 886, Arzneim.-Forsch., 1971, 21, 204, WO93/18024, WO94/12461) or the methods described in Reference Examples. In addition, the starting Compound (III) is commercially available, or, if the starting Compound (III) is a picoline derivative, it can be obtained according to a known method (WO94/20455) or a similar method thereto.

Compound (Iaa-a), which is Compound (Iaa) in which R¹⁸ is hydroxy, can be obtained by treating Compound (III) with a base in an inert solvent at the temperature between -100°C and room temperature for 5 minutes to 10 hours, followed by reaction with a starting Compound (II) at the temperature between -100°C and the boiling point of the employed solvent for 5 minutes to 30 hours.

Examples of the base are sodium hydroxide, potassium hydroxide, sodium methoxide, potassium ethoxide, sodium hydride, potassium hydride, butyl lithium, lithium diisopropylamide (LDA), potassium tert-butoxide, triethylamine, diisopropylethylamine, tributylamine, dicyclohexylmethylamine, N-methylmorphorine, N-methylpiperidine, diazabicycloundecene (DBU), and diazabicyclononene (DBN).

Examples of inert solvent are tetrahydrofuran (THF), dioxane, diethyl ether, ethylene glycol, triethylene glycol, glyme, diglyme, methanol, ethanol, butanol, isopropanol, dichloromethane, chloroform, benzene, toluene, dimethylformamide (DMF), and dimethyl sulfoxide (DMSO).

Compound (Iaa-b), which is Compound (Iaa) in which R¹⁸ is hydrogen, can be obtained by treating Compound (Iaa-a) with a reducing agent in the presence or absence of a catalytic amount to a largely excess amount of an acid catalyst in an inert solvent at the temperature between -100°C and the boiling point of the employed solvent for 5 minutes to 48 hours.

Examples of the acid catalyst are p-toluenesulfonic acid, methanesulfonic acid, hydrochloric acid, trifluoroacetic acid, boron trifluoride, aluminium chloride, stannic chloride, titanium tetrachloride, zinc chloride, and ferric chloride.

Examples of the reducing agent are triethylsilane, tributylsilane, dimethylphenylsilane, and trichlorosilane.

Examples of the inert solvent are THF, dioxane, diethyl ether, ethylene glycol, triethylene glycol, glyme, diglyme, dichloromethane, chloroform, benzene, and toluene.

Compound (Iaa-ba), which is Compound (Iaa-b) in which R²² is hydrogen, can also be obtained by treating Compound (Iba) prepared by the method described below (Process 2-2) with a reducing agent in an inert solvent at the temperature between -100°C and the boiling point of the employed solvent for 5 minutes to 30 hours, or by subjecting Compound (Iba) to hydrogenation in the presence of a catalyst in an inert solvent at the temperature between room temperature and the boiling point of the employed solvent for 5 minutes to 30 hours. An example of the reducing agent is sodium borohydride; examples of the catalyst for the hydrogenation are palladium/carbon, palladium, platinum dioxide, and Raney nickel; and examples of the inert solvent are THF, dioxane, methanol, ethanol, butanol, and isopropanol.

Compound (Iaa-c), which is Compound (Iaa) in which R¹⁸ is a group other than hydrogen, hydroxy, substituted or unsubstituted lower alkoxy, and lower alkanoyloxy in the definition of R¹⁸, and R¹⁸ and R¹⁹ are not combined to form O, S, or NR²⁰, can be obtained by reacting Compound (Iaa-a) with an alkylating (arylating) agent in the presence of an acid catalyst in an inert solvent at the temperature between -100°C and the boiling point of the employed solvent for 5 minutes to 30 hours.

Examples of the alkylating (arylating) agent are various kinds of alkyl- or arylmagnesium bromides, alkyl- or arylmagnesium chlorides, alkyl- or arylmagnesium iodides, trialkylaluminium, tetraalkyltitanium, dialkyltitanium chloride, Tebbe reagent, and trialkylsilylnitrile.

Examples of the acid catalyst are boron trifluoride, aluminium chloride, stannic chloride, titanium tetrachloride, zinc chloride, and ferric chloride.

Examples of the inert solvent are THF, dioxane, diethyl ether, glyme, diglyme, dichloromethane, chloroform, benzene, and toluene.

Compound (Iaa-d), which is Compound (Iaa) in which R¹⁸ is substituted or unsubstituted lower alkoxy or lower alkanoyloxy, can be obtained by reacting Compound (Iaa-a) with Compound (IV) in the presence of an acid catalyst in an inert solvent or without a solvent at the temperature between -100°C and the boiling point of the employed solvent for 5 minutes to 48 hours.

Examples of the acid catalyst are p-toluene-sulfonic acid, methanesulfonic acid, hydrochloric acid, sulfuric acid, and trifluoroacetic acid.

Examples of the inert solvent are THF, dioxane, diethyl ether, glyme, diglyme, dichloromethane, chloroform, benzene, toluene, DMF, and DMSO.

### Process 1-2: Compound (Iab), which is Compound (Ia) in which X is 5, and R¹⁸ and R¹⁹ are not combined to form O, S, or NR²⁰, can be prepared by the following reaction steps:

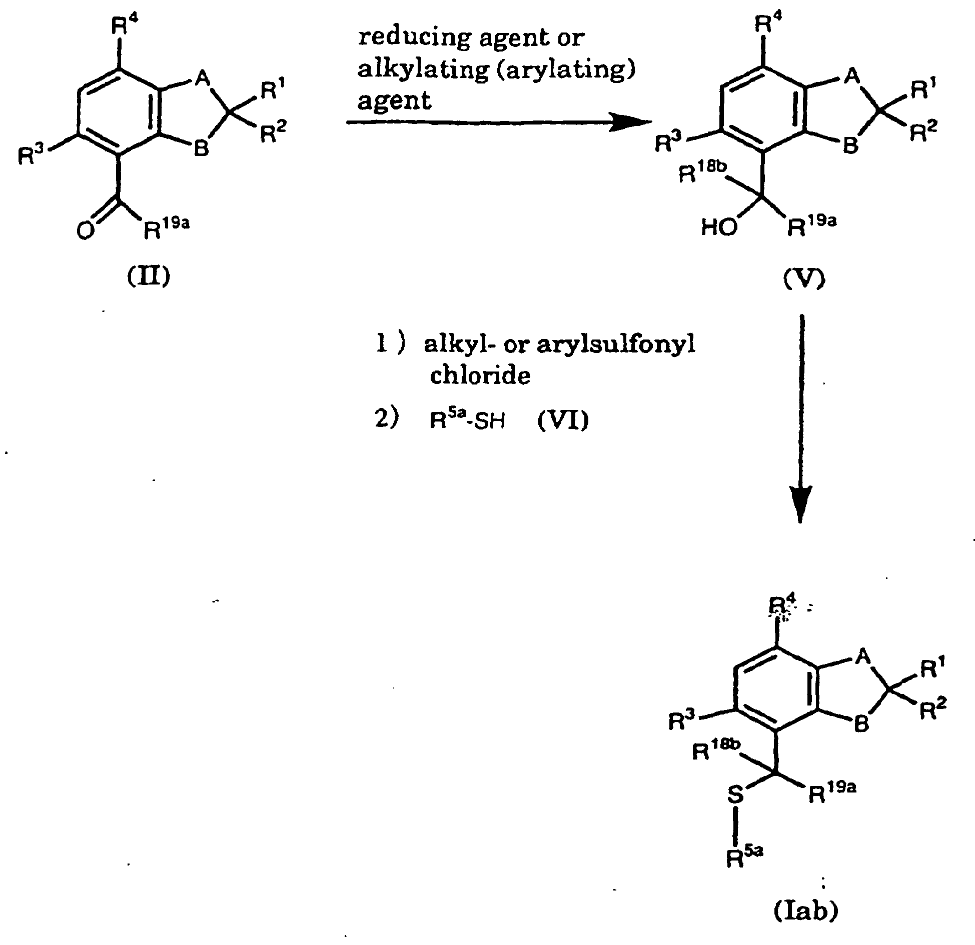

(In the formulae, R^{18b} is a group other than hydroxy, substituted or unsubstituted lower alkoxy, and lower alkanoyloxy in the definition of R¹⁸, and R^{18b} and R¹⁹ are not combined to form O, S, or NR²⁰; and A, B, R¹, R², R³, R⁴, R^{5a}, and R^{19a} each has the same meaning as defined above.)

Compound (Va), which is Compound (V) in which R^{18b} is hydrogen, can be obtained by treating Compound (II) with a reducing agent in an inert solvent at the temperature between -100°C and the boiling point of the employed solvent for 5 minutes to 30 hours.

Examples of the reducing agent are lithium aluminium hydride and sodium borohydride.

Examples of the inert solvent are THF, dioxane, diethyl ether, ethylene glycol, triethylene glycol, glyme, diglyme, methanol, ethanol, butanol, isopropanol, dichloromethane, chloroform, benzene, and toluene.

Compound (Vb), which is Compound (V) in which R^{18b} is a group other than hydrogen in the definition of R^{18b}, can be obtained by reacting Compound (II) with an alkylating (arylating) agent in an inert solvent at the temperature between -100°C and the boiling point of the employed solvent for 5 minutes to 30 hours.

Examples of the alkylating (arylating) agent are various kinds of alkyl- or arylmagnesium bromides, alkyl- or arylmagnesium chlorides, alkyl- or arylmagnesium iodides, and various kinds of alkyl or aryl lithiums.

Examples of the inert solvent are THF, dioxane, diethyl ether, glyme, diglyme, methanol, ethanol, butanol, isopropanol, dichloromethane, chloroform, benzene, and toluene.

Compound (Iab) can be obtained by reacting Compound (V) with, for example, alkyl- or arylsulfonyl chloride, in the presence of a base in an inert solvent at the temperature between -20°C and 0°C for 5 minutes to 5 hours, followed by reaction with Compound (VI) at the temperature between 0°C and the boiling point of the employed solvent for 5 minutes to 48 hours.

Examples of the base are sodium hydride, potassium hydride, butyl lithium, LDA, potassium tert-butoxide, triethylamine, diisopropylethylamine, tributylamine, dicyclohexylmethylamine, N-methylmorphorine, N-methylpiperidine, DBU, and DBN.

Examples of the alkyl- or arylsulfonyl chloride are methanesulfonyl chloride, benzenesulfonyl chloride, and p-toluenesulfonyl chloride.

Examples of the inert solvent are THF, dioxane, diethyl ether, glyme, diglyme, dichloromethane, chloroform, benzene, toluene, DMF, and DMSO.

Alternatively, Compound (Iab) can also be obtained by reacting Compound (V) with Compound (VI) in the presence of an acid catalyst in an inert solvent at the temperature between -100°C and the boiling point of the employed solvent for 5 minutes to 48 hours.

Examples of the acid catalyst are p-toluenesulfonic acid, methanesulfonic acid, hydrochloric acid, trifluoroacetic acid, boron trifluoride, aluminium chloride, stannic chloride, titanium tetrachloride, zinc chloride, and ferric chloride.

Examples of the inert solvent are THF, dioxane, diethyl ether, glyme, diglyme, dichloromethane, chloroform, benzene, and toluene.

### Process 1-3: Compound (Iac), which is Compound (Ia) in which X is NR²³, and R¹⁸ and R¹⁹ are not combined to form O, S, or NR²⁰, can be prepared by the following reaction step:

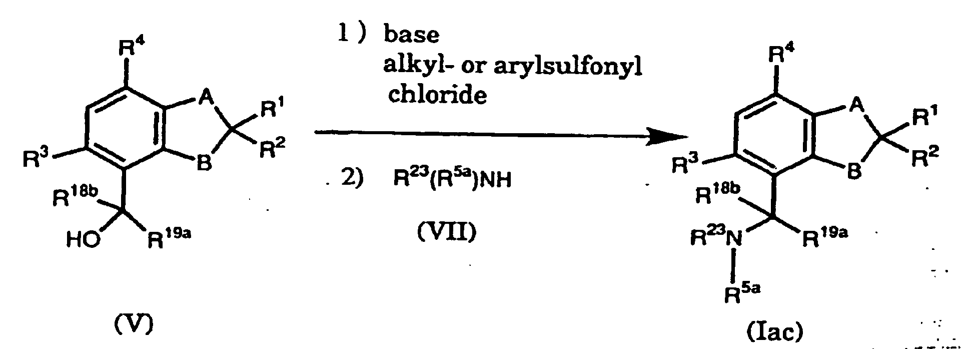

(In the formulae, A, B, R¹, R², R³, R⁴, R^{5a}, R^{18b}, R^{19a}, and R²³ each has the same meaning as defined above.)

Compound (Iac) can be obtained according to the method described in Process 1-2 in which Compound (Iab) is obtained from Compound (V) and Compound (VI), using Compound (VII) instead of Compound (VI).

### Process 1-4: Compound (Iad), which is Compound (Ia) in which D is -C(=O)-C(R²¹)(R²²)-, can be prepared by the following reaction step:

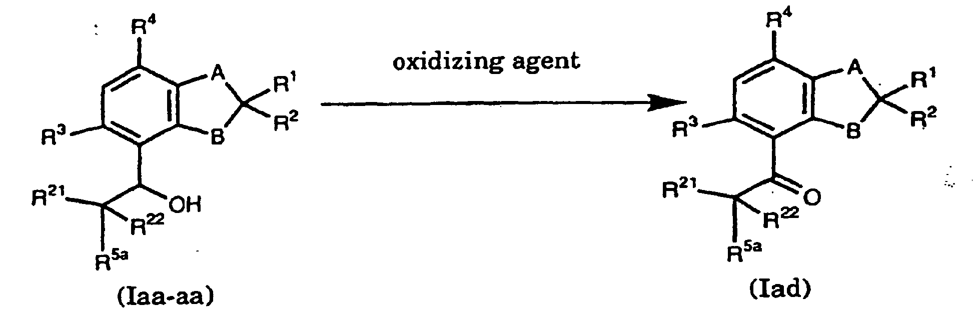

(In the formulae, R¹, R², R³, R⁴, R^{5a}, R²¹, and R²² each has the same meaning as defined above.)

Compound (Iad) can be obtained by treating Compound (Iaa-aa), which is Compound (Iaa-a) in which R^{19a} is hydrogen, with an oxidizing agent in an inert solvent containing water at the temperature between 0°C and the boiling point of the employed solvent for 5 minutes to 72 hours.

Examples of the oxidizing agent are manganese dioxide, potassium permanganate, pyridinium chlorochromate (PCC), and pyridinium dichromate (PDC).

Examples of the inert solvent are THF, dioxane, diethyl ether, ethylene glycol, triethylene glycol, glyme, diglyme, acetone, methyl vinyl ketone, dichloromethane, chloroform, benzene, toluene, DMF, and DMSO.

### Process 1-5: Compound (Iad) can also be prepared according to the following reaction step:

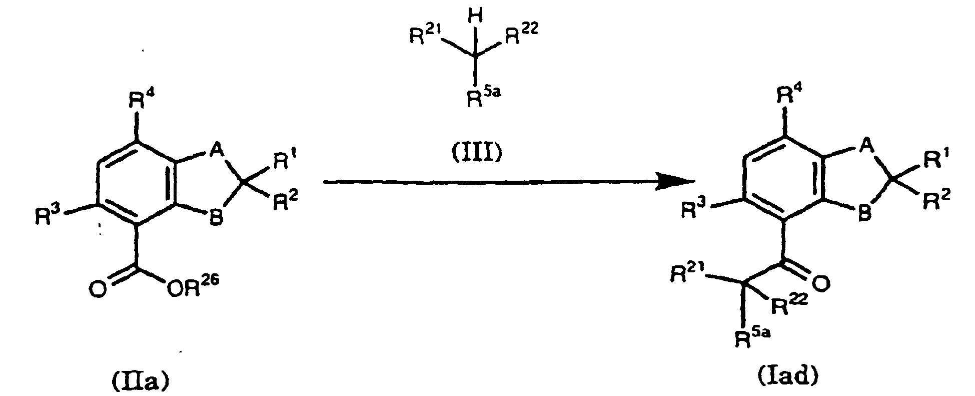

(In the formulae, R²⁶ is substituted or unsubstituted lower alkyl; and A, B, R¹, R², R³, R⁴, R^{5a}, R²¹, and R²² each has the same meaning as defined above.)

Compound (Iad) can be obtained according to the method described in Process 1-1 in which Compound (Iaa-a) is obtained from Compound (II) and Compound (III), using Compound (IIa), which is a starting Compound (II) in which R^{19a} is substituted or unsubstituted lower alkoxy.

### Process 1-6: Compound (Iad-a), which is Compound (Iad) in which R²¹ and R²² are groups other than lower alkanoyl, cycloalkanoyl, lower alkoxycarbonyl, and cyano in the definition of R²¹ and R²², can also be prepared by the following reaction step:

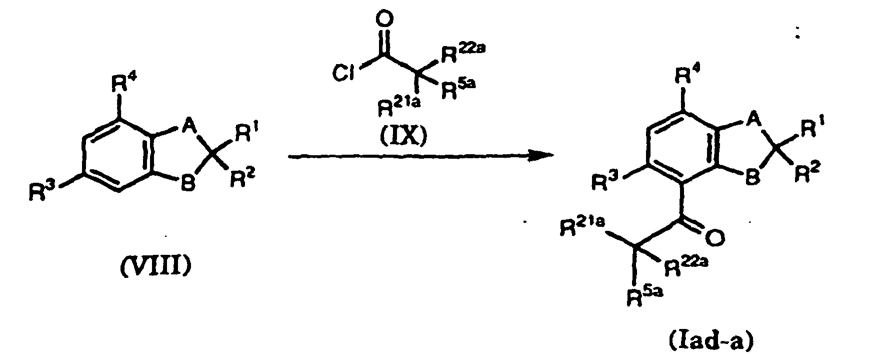

(In the formulae, R^{21a} and R^{22a} are groups other than lower alkanoyl, cycloalkanoyl, lower alkoxycarbonyl, and cyano in the definition of R²¹ and R²²; and A, B, R¹, R², R³, R⁴, and R^{5a} each has the same meaning as defined above.)

The starting Compound (VIII) can be obtained according to the methods described in Reference Examples or similar methods thereto.

Compound (Iada) can be obtained by reacting Compound (VIII) with Compound (IX) in the presence of an acid catalyst in an inert solvent at the temperature between -100°C and the boiling point of the employed solvent for 5 minutes to 30 hours.

Examples of the acid catalyst are boron trifluoride, aluminium chloride, stannic chloride, titanium tetrachloride, zinc chloride, and ferric chloride.

Examples of the inert solvent are THF, dioxane, diethyl ether, glyme, diglyme, dichloromethane, 1,2, dichloroethane, chloroform, benzene, nitrobenzene, and toluene.

### Process 1-7: Compound (Iae), which is Compound (Ia) in which D is -C(=O)-NR²³-, can be prepared by the following reaction step:

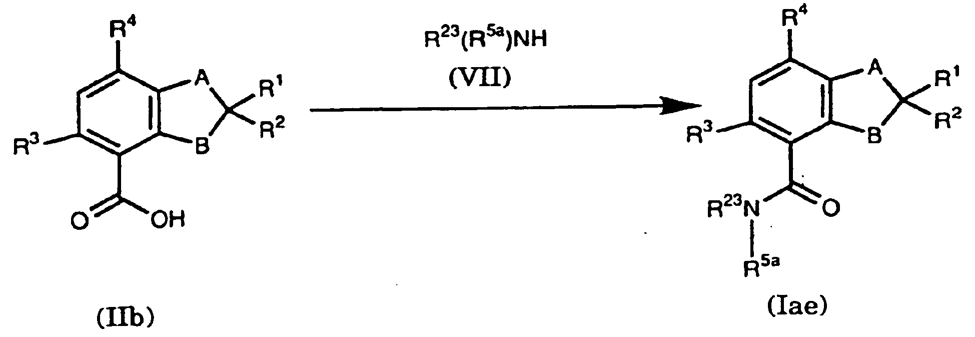

(In the formulae, A, B, R¹, R², R³, R⁴, R^{5a}, and R²³ each has the same meaning as defined above.)

The desired Compound (Iae) can be obtained by dehydrative condensation of Compound (IIb), which is a starting Compound (II) in which R^{19a} is hydroxy, and Compound (VII). For the above condensation, numerous methods are known and applicable, as described in Jikken Kagaku Koza, 22, 137-172, the 4th edition (Nippon Kagaku-Kai, 1992). For example, Compound (IIb) is treated with one equivalent to a largely excess amount of thionyl chloride, phosphorus pentachloride, oxalyl chloride, or the like, if necessary in the presence of a catalytic amount to 20 equivalents of a base, in an inert solvent at the temperature between 0°C and the boiling point of the employed solvent for 0.1 to 48 hours to give a corresponding acid chloride. Then, the desired Compound (Iae) can be obtained by reacting the obtained acid chloride with 0.5 to 50 equivalents of Compound (VII), if necessary in the presence of 0.5 equivalent to a largely excess amount of a base, in an inert solvent at the temperature between 0°C and the boiling point of the employed solvent for 0.1 to 48 hours.

Examples of the base are those which are used in the manufacturing method for Compound (Iaa-a) described in Process 1-1.

Examples of the inert solvents are dichloromethane, chloroform, benzene, toluene, THF, dioxane, DMF, and DMSO.

### Process 1-8: Compound (Iaf), which is Compound (Ia) in which D is -C(=O)-S-, can be prepared by the following reaction step:

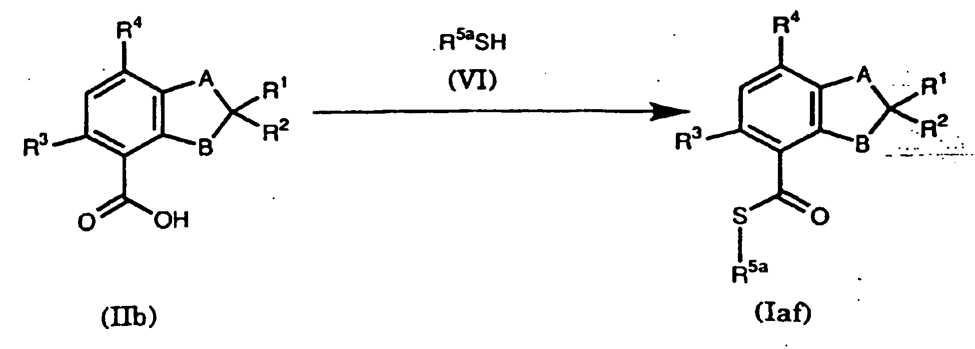

(In the formulae, A, B, R¹, R², R³, R⁴, and R^{5a} each has the same meaning as defined above.)

Compound (Iaf) can be obtained according to the method described in Process 1-7 in which Compound (Iae) is obtained from Compound (IIc) and Compound (VII), using Compound (VI) instead of Compound (VII).

### Process 1-9: Compound (Iae-a), which is Compound (Iae) in which one of R¹ and R¹¹ (or R¹³) is -(CH₂)ₙ-CO-G¹ or -(CH₂)ₘ-CO-G², can be prepared by the following reaction steps:

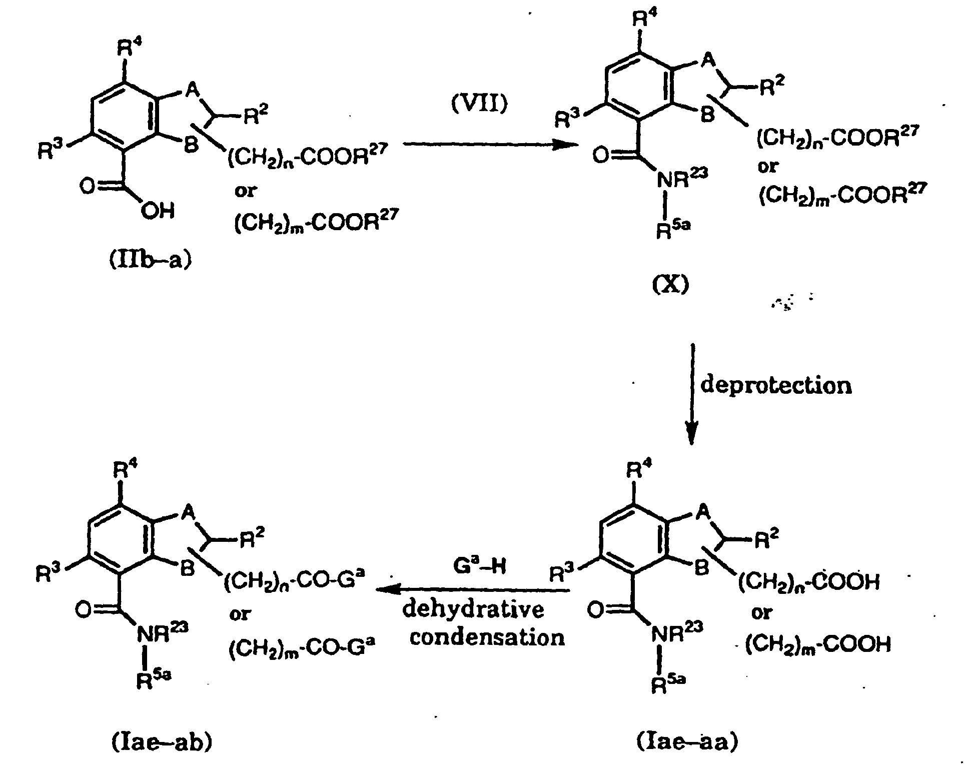

[In the formulae, G^{a} is OR⁶ (with the proviso that R⁶ is not hydrogen) or NR⁷R⁸ in the definition of G¹ (or G²) ; R²⁷ is a protective group of a carboxyl group; and A, B, R², R³, R⁴, R^{5a}, R²³, n, and m each has the same meaning as defined above.]

A protective group for a carboxyl group is generally required to be deprotected selectively compared with an amide bond for converting a protected carboxyl group to a carboxyl group, and those which are described in the fifth chapter of Protective Group in Organic Synthesis (the second edition, Green and watt, Jon Weary and Suns Incorporated, 1991) can be applied. Examples of these are esters of substituted or unsubstituted lower alkyl including methyl, ethyl, and tert-butyl, benzyl, allyl, and 2-(trimethylsilyl)ethyl.

The starting Compound (IIb-a) can be obtained according to the methods described in Reference Examples or similar methods thereto.

Compound (X) can be obtained according to the method described in Process 1-7, using Compound (IIb-a) and Compound (VII).

Compound (Iae-aa), which is Compound (Iae-a) in which G¹ (or G²) is hydroxy, can be obtained by treating Compound (X) in the presence of a catalytic to largely excess amount of a base in an inert solvent containing water at the temperature between room temperature and the boiling point of the employed solvent for 0.1 to 48 hours.

Examples of the base are those which are mentioned in Process 1-7; and examples of the inert solvent are THF, dioxane, ethylene glycol, triethylene glycol, glyme, diglyme, methanol, ethanol, butanol, and isopropanol.

Compound (Iae-ab), which is Compound (Iae-a) in which G¹ (or G²) is OR⁶ (with the proviso that R⁶ is not hydrogen) or NR⁷R⁸ in the definition of G¹ (or G²), can be obtained according to the method described in Process 1-7, using Compound (Iae-aa) and Compound G^{a}-H.

### Process 1-10: Compound (Iae-ac), which is Compound (Iae-a) in which G¹ (or G²) is substituted or unsubstituted lower alkyl, cycloalkyl, polycycloalkyl, substituted or unsubstituted aryl, a substituted or unsubstituted aromatic heterocyclic group, or aralkyl in the definition of G¹ (or G²), can be prepared by the following reaction step:

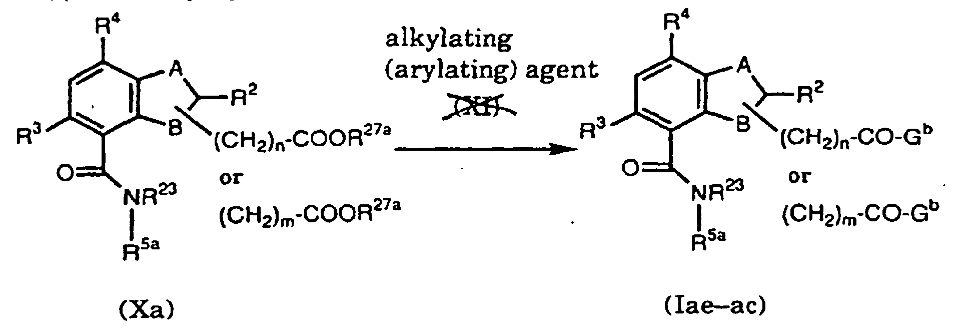

[In the formulae, R^{27a} is substituted or unsubstituted lower alkyl; G^{b} is substituted or unsubstituted lower alkyl, cycloalkyl, polycycloalkyl, substituted or unsubstituted aryl, a substituted or unsubstituted aromatic heterocyclic group, or aralkyl in the definition of G¹ (or G²); and A, B, R², R³, R⁴, R^{5a}, R²³, n, and m each has the same meaning as defined above.]

The substituted or unsubstituted lower alkyl in the definition of R^{27a} has the same meaning as defined above.

Compound (Iae-ac) can be obtained by reacting Compound (Xa), which is Compound (X) in which R²⁷ is substituted or unsubstituted lower alkyl, with an alkylating (arylating) agent in an inert solvent at the temperature between -100°C and the boiling point of the employed solvent for 5 minutes to 30 hours.

Examples of the alkylating (arylating) agent are various kinds of alkyl- or arylmagnesium bromides, alkyl- or arylmagnesium chlorides, alkyl- or arylmagnesium iodides, and various kinds of alkyl or aryl lithium.

Examples of the inert solvent are THF, dioxane, diethyl ether, glyme, diglyme, methanol, ethanol, butanol, isopropanol, dichloromethane, chloroform, benzene, and toluene.

### Process 1-11: Compound (Iae-aca), which is Compound (Iae-ac) in which one of R¹ and R¹¹ (or R¹³) is -CO-G^{b}, can also be prepared by the following reaction step:

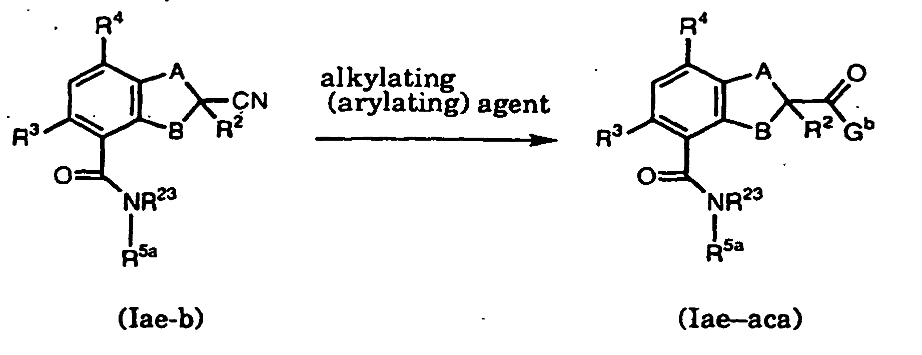

(In the formulae, A, B, R², R³, R⁴, R^{5a}, R²³, and G^{b} each has the same meaning as defined above.)

Compound (Iae-aca) can be obtained according to the method described in Process 1-10 from Compound (Iae-b), which is Compound (Iae) in which R¹ is cyano.

### Process 1-12: Compound (Iag), which is Compound (Ia) in which D is -C(=S)-X-, can be prepared by the following reaction step:

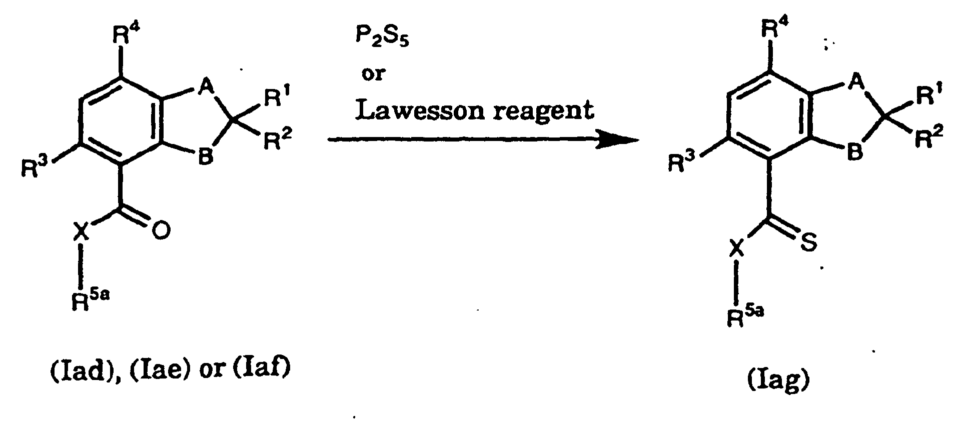

(In the formulae, A, B, R¹, R², R³, R⁴, R^{5a}, and X each has the same meaning as defined above.)

Compound (Iag) can be obtained by treating Compound (Iad), Compound (Iae), or Compound (Iaf) with phosphorus pentasulfide or Lawesson's reagent in an inert solvent at the temperature between room temperature and the boiling point of the employed solvent for 5 minutes to 72 hours.

Examples of inert solvent are pyridine, THF, dioxane, diethyl ether, ethylene glycol, triethylene glycol, glyme, diglyme, dichloromethane, chloroform, benzene, toluene, xylene, DMF, and DMSO.

### Process 1-13: Compound (Iah), which is Compound (Ia) in which D is -C(=NR²⁰)-CR²¹R²²-, can be prepared by the following reaction step:

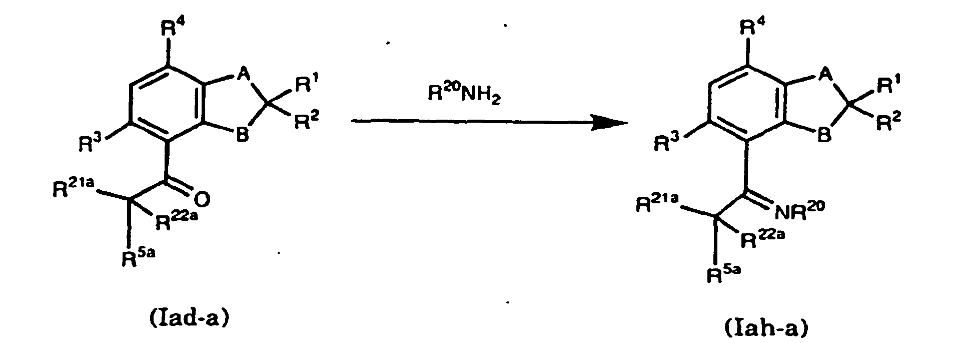

(In the formulae, A, B, R¹, R², R³, R⁴, R^{5a}, R²⁰, R^{21a}, and R^{22a} each has the same meaning as defined above.)

Compound (Iah-a), which is Compound (Iah) in which R²¹ and R²² are groups other than lower alkanoyl, cycloalkanoyl, lower alkoxycarbonyl, and cyano in the definition of R²¹ and R²², can be obtained by reacting Compound (Iad-a) with R²⁰NH₂ in the presence or absence of an acid catalyst in an inert solvent or without solvent at the temperature between room temperature and the boiling point of the employed solvent for 5 minutes to 48 hours.

Examples of the acid catalyst are p-toluenesulfonic acid, methanesulfonic acid, hydrochloric acid, sulfuric acid, acetic acid, and trifluoroacetic acid.

Examples of the inert solvent are THF, dioxane, diethyl ether, ethylene glycol, triethylene glycol, glyme, diglyme, methanol, ethanol, isopropanol, tert-butanol, dichloromethane, chloroform, benzene, toluene, DMF, DMSO, and pyridine.

### Process 1-14: Compound (Ia'), which is Compound (I) in which D is (i) -C(R¹⁸)(R¹⁹)-x- and R⁵ is pyridine-N-oxide; can be prepared by the following reaction step:

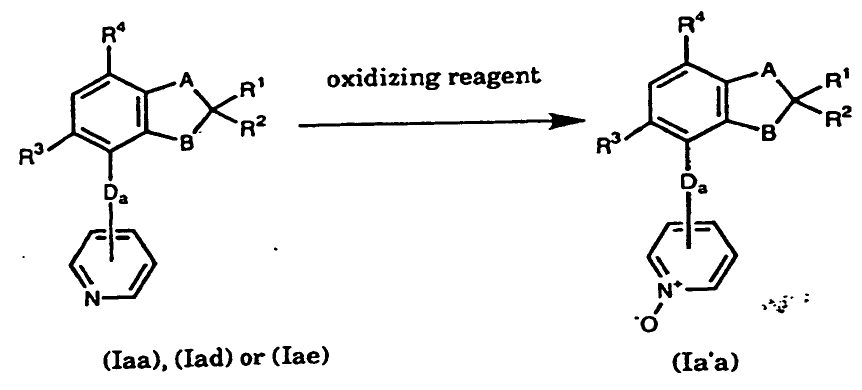

[In the formulae, D^{a} is D in Compound (Iaa), (Iad), and (Iae); and A, B, R¹, R², R³, and R⁴ each has the same meaning as defined above.]

Compound (Ia'a), which is Compound (Ia') in which D is D in Compound (Iaa), (Iad), and (Iae) in the definition of D, can be obtained by treating Compound (Iaa), (Iad), or (Iae) with an oxidizing agent in an inert solvent at the temperature between room temperature and the boiling point of the employed solvent for 5 minutes to 72 hours.

Examples of inert solvent are dichloromethane, chloroform, benzene, toluene, xylene, DMF, DMSO, and acetic acid.

Examples of the oxidizing agent are peracetic acid, trifluoroperacetic acid, metachloroperbenzoic acid, hydrogen peroxide, benzoyl peroxide, tert-butyl hydroperoxide, and tert-amyl hydroperoxide.

### Manufacturing method 2: Compound (Ib), which is Compound (I) in which D is (ii) -C(R^{19a})=Y-, can be obtained by the following Processes 2-1 to 2-5.

### Process 2-1: Compound (Iba-a), which is Compound (Ib) in which Y is -CR²⁴, R⁵ is substituted or unsubstituted aryl, or a substituted or unsubstituted aromatic heterocyclic group, and R²⁴ and R^{19a} are not combined to form a single bond, can be prepared by the following reaction steps:

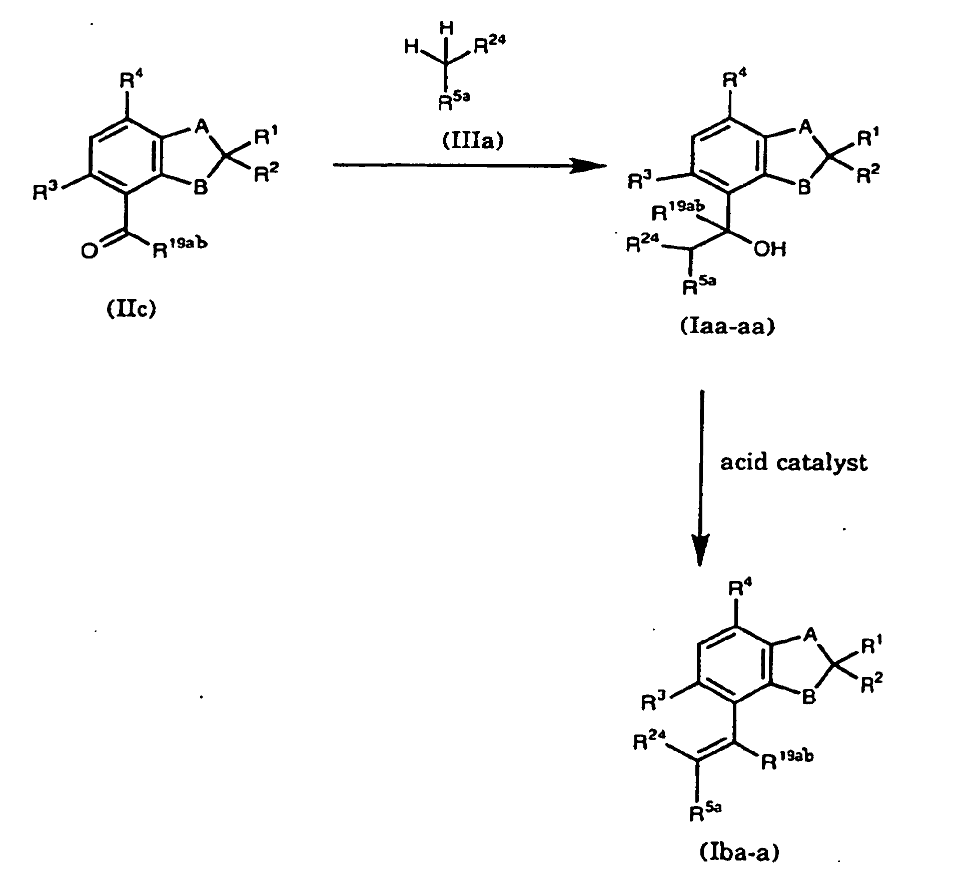

(In the formulae, R^{19ab} is a group other than hydroxy, and substituted or unsubscirutel lower alkoxy in the definition as R^{19a}; and A, B, R¹, R², R³, R⁴, R^{5a}, R^{19a}, and R²⁴ each has the same meaning as defined above.)

Compound (Iaa-aa), which is Compound (Iaa-a) in which R²² is hydrogen, can be obtained according to the method similar to the manufacturing method for Compound (Iaa-a) described in Process 1-1, using Compound (IIc) and Compound (IIIa), which is Compound (III) in which R²² is hydrogen. Compound (Iaa-aa) is directly converted to Compound (Iba-a) without isolation when R²⁴ is lower alkanoyl, cycloalkanoyl, lower alkoxycarbonyl, or cyano.

Compound (Iba-a) can be obtained by treating Compound (Iaa-aa) in the presence an acid catalyst in an inert solvent at the temperature between room temperature and the boiling point of the employed solvent for 5 minutes to 48 hours.

Examples of the acid catalyst are p-toluenesulfonic acid, methanesulfonic acid, hydrochloric acid, sulfuric acid, acetic acid, and trifluoroacetic acid.

Examples of the inert solvent are THF, dioxane, diethyl ether, ethylene glycol, triethylene glycol, glyme, diglyme, dichloromethane, chloroform, benzene, toluene, DMF, and DMSO.

### Process 2-2: Compound (Iba), which is Compound (Ib) in which Y is -CR²⁴, and R²⁴ and R^{19a} are not combined to form a single bond, can also be prepared by the following reaction step:

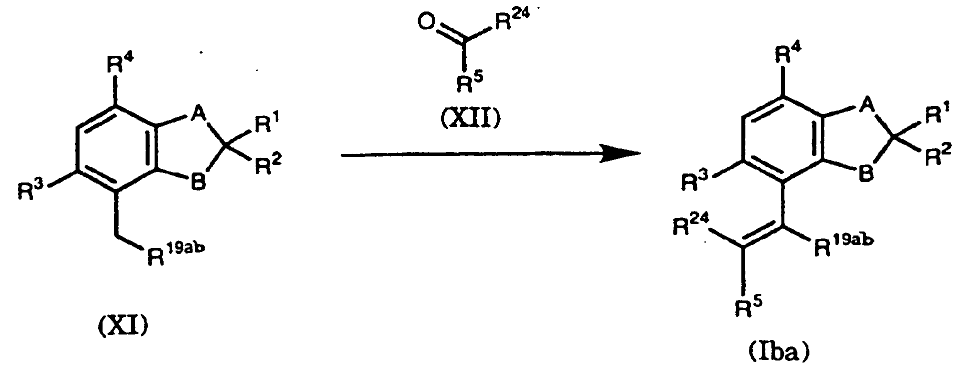

(In the formulae, A, B, R¹, R², R³, R⁴, R⁵, R^{19ab}, and R²⁴ each has the same meaning as defined above.)

The starting compound (XI) can be obtained according to the methods described in Reference Examples or similar methods thereto.

Compound (Iba) can be obtained by treating starting Compound (XI) with a base in an inert solvent at the temperature between -100°C and the boiling point of the employed solvent for 5 minutes to 10 hours, followed by reaction with Compound (XII) at the temperature between -100°C and the boiling point of the employed solvent for 5 minutes to 30 hours.

Examples of the base and the inert solvent are those used in the manufacturing method for Compound (Iaa-a) described in Process 1-1.

### Process 2-3: Compound (Ibb), which is Compound (Ib) in which Y is N, and R⁵ is substituted or unsubstituted aryl or a substituted or unsubstituted aromatic heterocyclic group, can be prepared by the following reaction step:

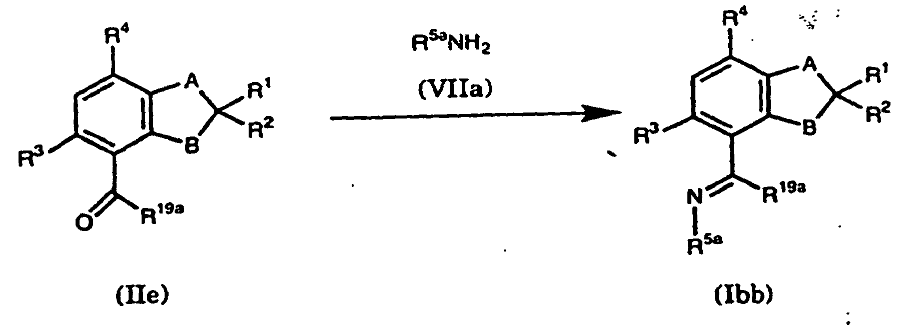

(In the formulae, A, B, R¹, R², R³, R⁴, R^{5a}, and R^{19a} each has the same meaning as defined above.)

Compound (Ibb) can be obtained by reacting Compound (IIe) with Compound (VIIa), which is Compound (VII) in which R²³ is hydrogen, in the presence of an acid catalyst in an inert solvent or without solvent at the temperature between room temperature and the boiling point of the employed solvent for 5 minutes to 48 hours.

Examples of the acid catalyst are p-toluenesulfonic acid, methanesulfonic acid, hydrochloric acid, sulfuric acid, acetic acid, and trifluoroacetic acid.

Examples of the inert solvent are THF, dioxane, diethyl ether, ethylene glycol, triethylene glycol, glyme, diglyme, methanol, ethanol, isopropanol, tert-butanol, dichloromethane, chloroform, benzene, toluene, DMF, and DMSO.

### Process 2-4: Compound (Ibc), which is Compound (Ib) in which Y is -CR²⁴-CONH-, and R⁵ is substituted or unsubstituted lower aryl or a substituted or unsubstituted aromatic heterocyclic group, can be prepared by the following reaction steps:

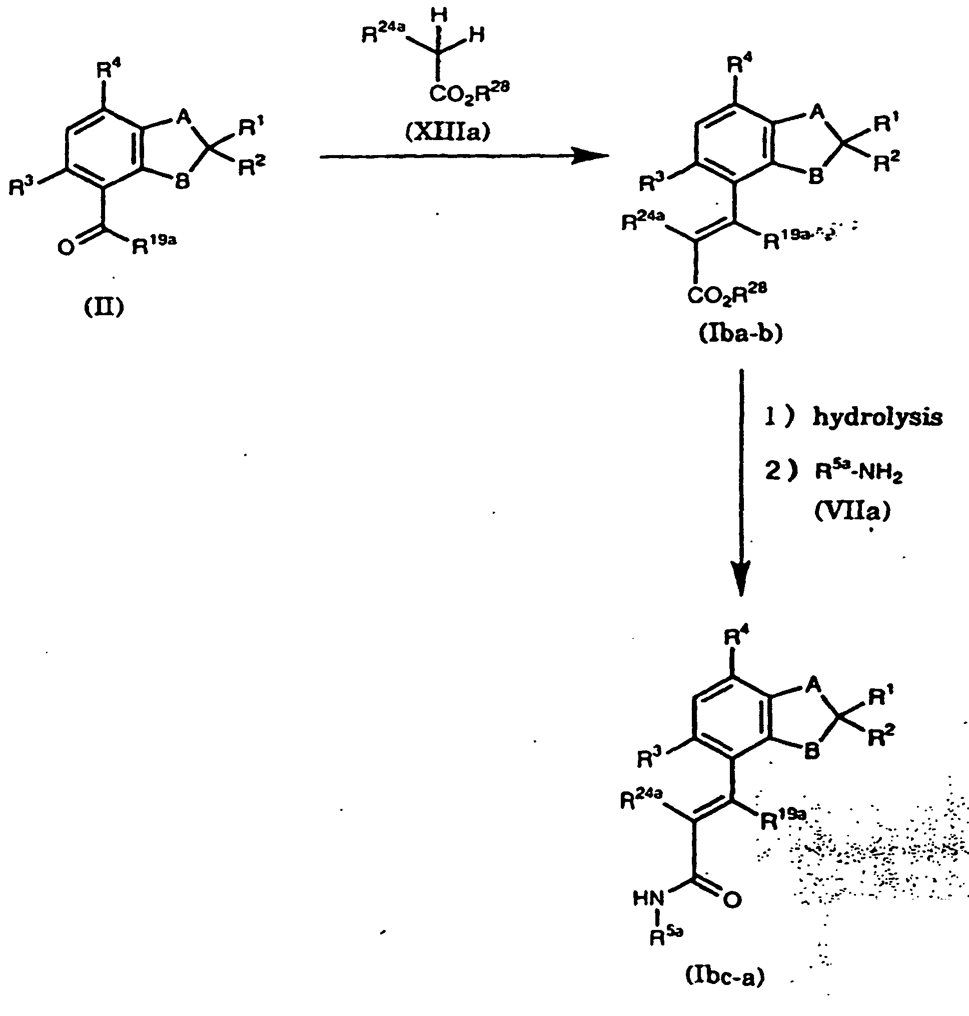

(In the formulae, R²⁸ is lower alkyl; R^{24a} is a group other than lower alkanoyl, cycloalkanoyl, lower alkoxycarbonyl, and cyano in the definition as R²⁴; and A, B, R¹, R², R³, R⁴, R^{5a}, and R^{19a} each has the same meaning as defined above.)

The lower alkyl in the definition of R²⁸ has the same meaning as defined above.

Compound (Iba-b), which is Compound (Iba) in which R⁵ is lower alkoxycarbonyl and R²⁴ is a group other than lower alkanoyl, cycloalkanoyl, lower alkoxycarbonyl, and cyano, can be obtained according to the method similar to the manufacturing method for Compound (Iba-a) described in Process 2-1, using Compound (II) and Compound (XIII). Further, Compound (Iba-b) can be obtained by reacting Compound (II) with a corresponding diester of phosphorous acid treated with a base in an inert solvent at the temperature between -100°C and the boiling point of the employed solvent for 5 minutes to 48 hours.

Examples of the inert solvent are THF, dioxane, diethyl ether, ethylene glycol, triethylene glycol, glyme, diglyme, methanol, ethanol, butanol, isopropanol, dichloromethane, chloroform, benzene, toluene, DMF, and DMSO.

Examples of the base are sodium hydroxide, potassium hydroxide, sodium methoxide, potassium ethoxide, sodium hydride, potassium hydride, butyl lithium, LDA, potassium tert-butoxide, triethylamine, diisopropylethylamine, tributylamine, dicyclohexylmethylamine, N-methylmorphorine, N-methylpiperidine, DBU, and DBN.

Compound (Ibc-a), which is Compound (Ibc) in which R²⁴ is a group other than lower alkanoyl, cycloalkanoyl, lower alkoxycarbonyl, and cyano, can be obtained according to the method described in Process 1-9 in which Compound (Iae-ab) is obtained from Compound (X), using Compound (Iba-b) and Compound (VIIa).

### Process 2-5: Compound (Ibd), which is Compound (Ib) in which Y is -CR²⁴, R²⁴ and R^{19a} are combined to form a single bond, and R⁵ is substituted or unsubstituted aryl, or a substituted or unsubstituted aromatic heterocyclic group, can be prepared by the following reaction steps:

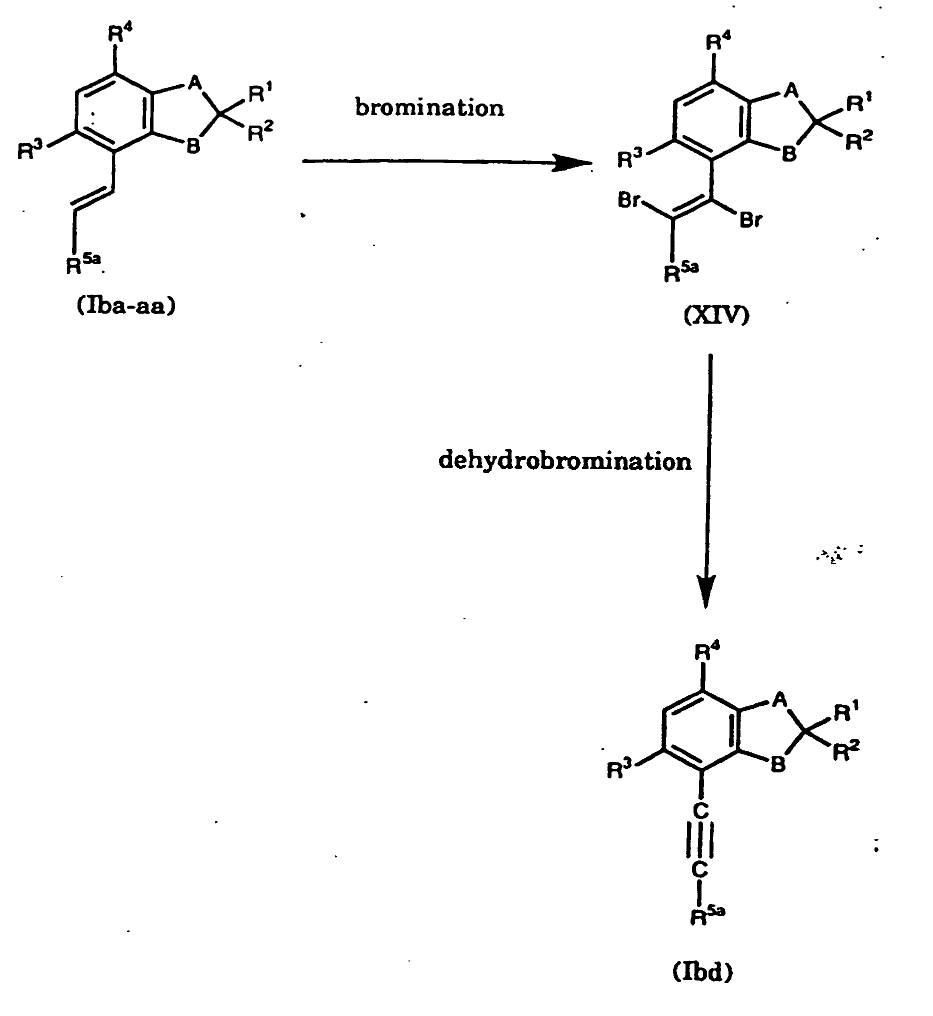

(In the formulae, A, B, R¹, R², R³, R⁴, and R^{5a} each has the same meaning as defined above.)

Compound (XIV) can be obtained by treating Compound (Iba-aa), which is Compound (Iba-a) in which R^{19a} and R²⁴ are both hydrogen, with a brominating agent in an inert solvent at the temperature between -100°C and the boiling point of the employed solvent for 5 minutes to 10 hours.

Examples of the inert solvent are THF, dioxane, diethyl ether, ethylene glycol, triethylene glycol, glyme, diglyme, methanol, ethanol, isopropanol, tert-butanol, dichloromethane, chloroform, benzene, toluene, DMF, and DMSO.

Examples of the brominating agent are bromine, tetrabutylammonium tribromide, tetramethylammonium tribromide, pyridinium tribromide, NBS, and copper bromide.

Compound (Ibd) can be obtained by treating Compound (XIV) with a base in an inert solvent at the temperature between -100°C and the boiling point of the employed solvent for 5 minutes to 10 hours.

Examples of the inert solvent are THF, dioxane, diethyl ether, ethylene glycol, triethylene glycol, glyme, diglyme, methanol, ethanol, isopropanol, tert-butanol, dichloromethane, chloroform, benzene, toluene, DMF, and DMSO.

Examples of the base are potassium hydroxide, sodium ethoxide, sodium methoxide, potassium tert-butoxide, and sodium amide.

### Manufacturing method 3: Compound (Ic), which is Compound (I) in which D is (iii) a bond, and R⁵ is substituted or unsubstituted aryl, or a substituted or unsubstituted aromatic heterocyclic group, can be obtained by the following process:

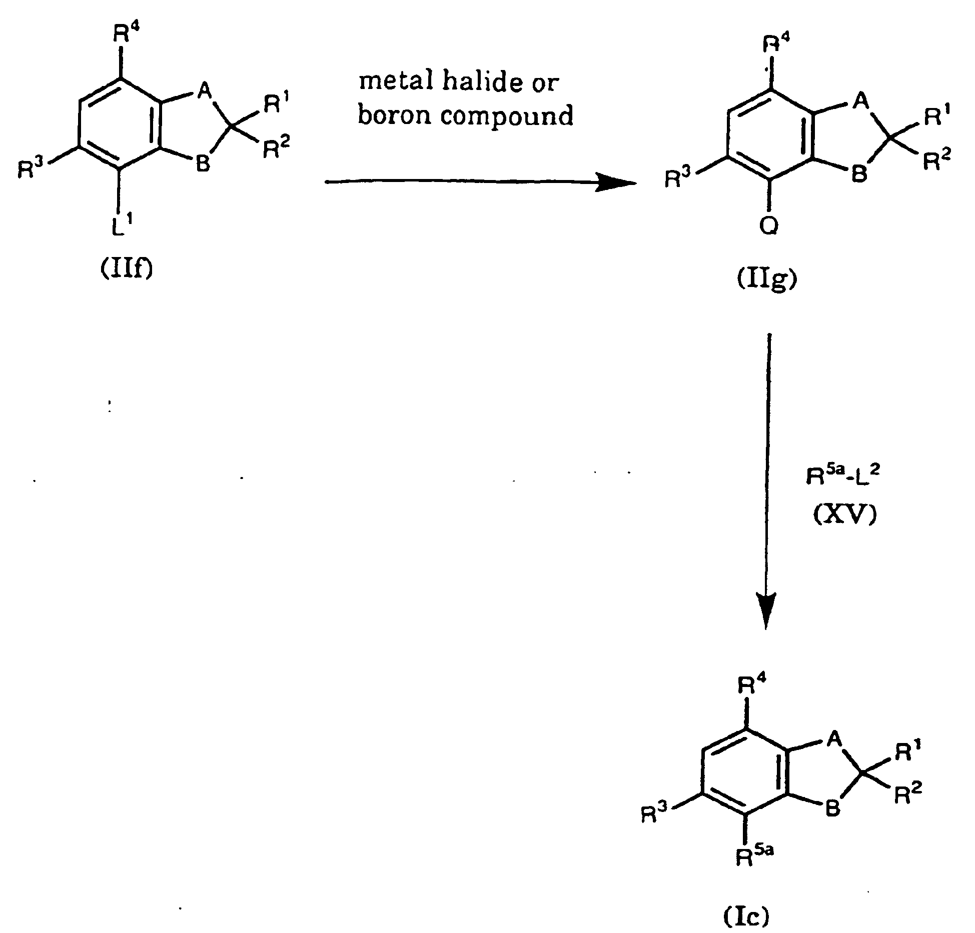

(In the formulae, L¹ and L² independently represent iodine, bromine, or chlorine; and A, B, R¹, R², R³, R⁴, and R^{5a} each has the same meaning as defined above.)

Examples of the metal halide are alkyltin halides such as tributyltin chloride and trimethyltin chloride, and zinc halides such as zinc chloride, zinc bromide, and zinc iodide; and examples of the boron compound are trimethoxy boron, phenylboric acid, and boric acid.

Compound (IIg) can be obtained by treating Compound (IIf) with a base in an inert solvent at the temperature between -100°C and room temperature for 5 minutes to 10 hours, followed by reaction with a metal halide or a boron compound at the temperature between -100°C and the boiling point of the employed solvent for 5 minutes to 30 hours.

Examples of the base are sodium hydroxide, potassium hydroxide, sodium methoxide, potassium ethoxide, sodium hydride, potassium hydride, butyl lithium, LDA, potassium tert-butoxide, triethylamine, diisopropylethylamine, tributylamine, dicyclohexylmethylamine, N-methylmorphorine, N-methylpiperidine, DBU, and DBN.

Examples of the inert solvent are THF, dioxane, diethyl ether, ethylene glycol, triethylene glycol, glyme, diglyme, methanol, ethanol, butanol, isopropanol, dichloromethane, chloroform, benzene, toluene, DMF, and DMSO.

Compound (Ic) can be obtained by reacting Compound (IIg) with Compound (XV) in the presence of a catalytic to largely excess amount of a palladium complex in an inert solvent at the temperature between room temperature and the boiling point of the employed solvent for 5 minutes to 30 hours. Moreover, a salt such as lithium chloride, or an oxidizing agent such as silver oxide may be added, if necessary.

Examples of the inert solvent are THF, dioxane, diethyl ether, dichloromethane, chloroform, benzene, toluene, dimethylacetamide (DMA), DMF, and DMSO.

The intermediates and the desired compounds in the processes described above can be isolated and purified by purification methods conventionally used in organic synthetic chemistry, for example, filtration, extraction, washing, drying, concentration, recrystallization, and various kinds of chromatography. The intermediates may also be subjected to the subsequent reaction without isolation.

Compounds (I) can exist in the form of stereoisomers such as geometrical isomers and optical isomers, and the present invention covers all isomers including these isomers and mixtures thereof.

In the case where a salt of Compound (I) is desired and it is produced in the form of the desired salt, it can be subjected to purification as such. In the case where Compound (I) is produced in the free form and its salt is desired, Compound (I) is dissolved or suspended in a suitable solvent, followed by addition of an acid or a base to form a salt, which may be isolated and purified.

Compounds (I) and pharmaceutically acceptable salts thereof may be in the form of adducts with water or various solvents, which are also within the scope of the present invention.

Examples of Compound (I) obtained in the present invention are shown in Tables 1 to 8.

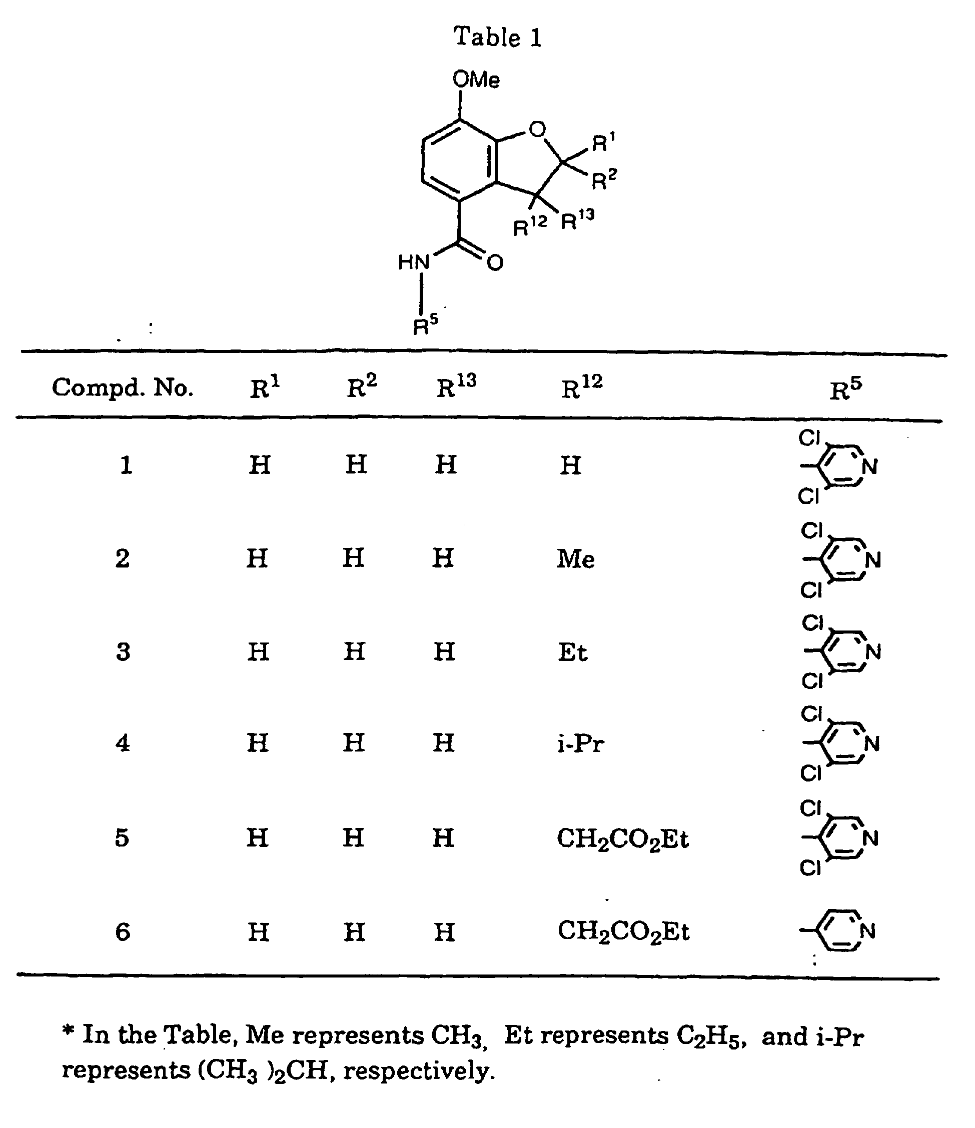

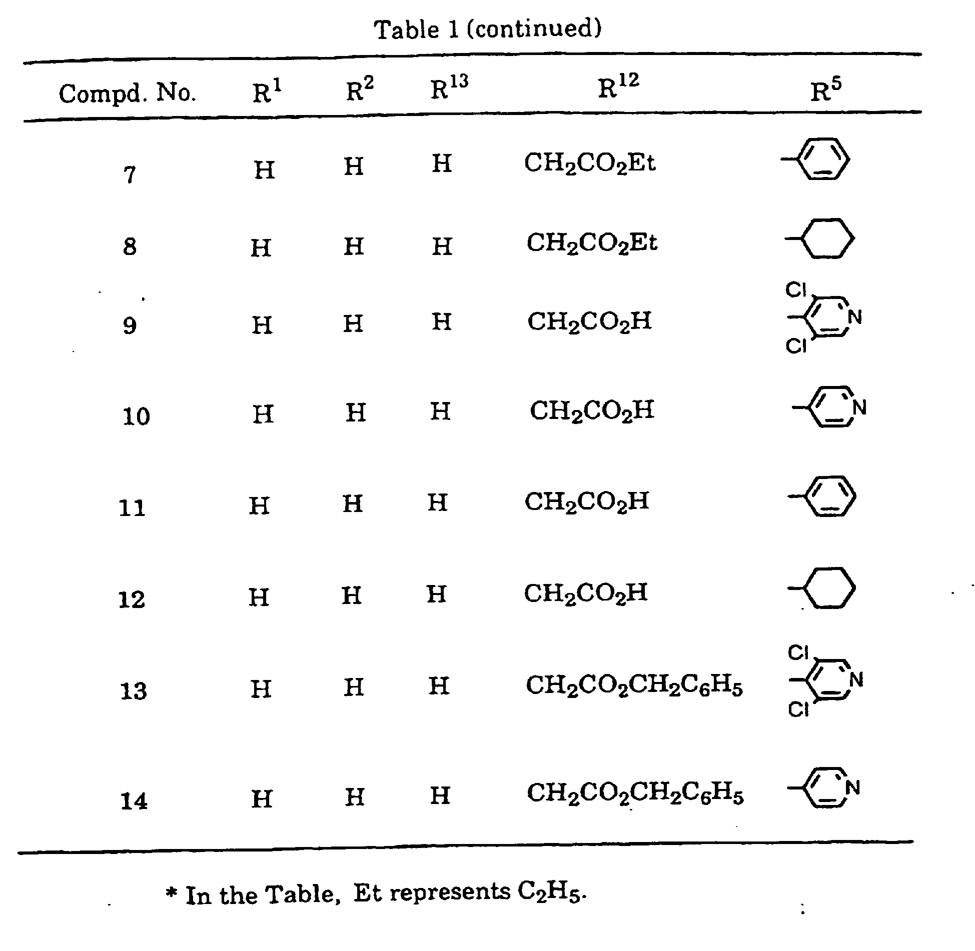

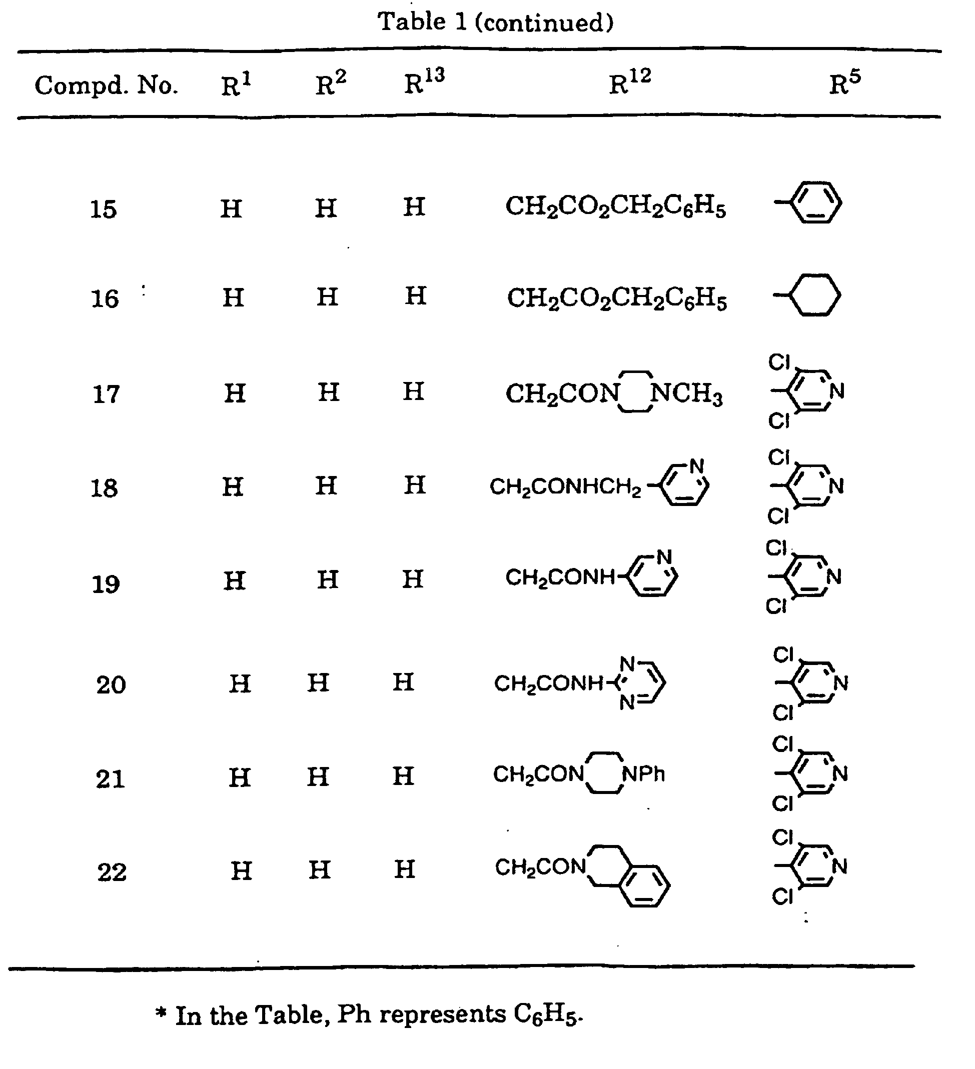

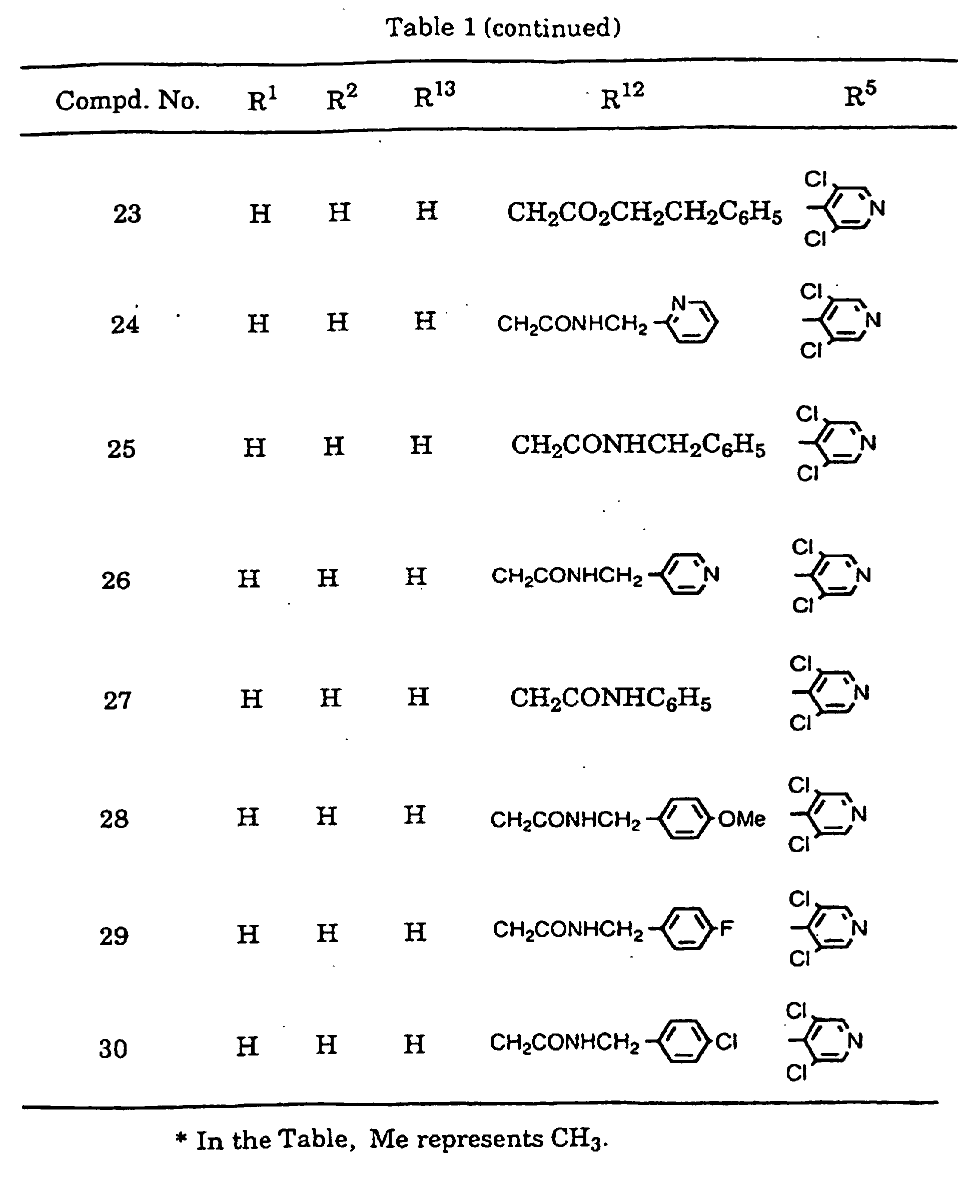

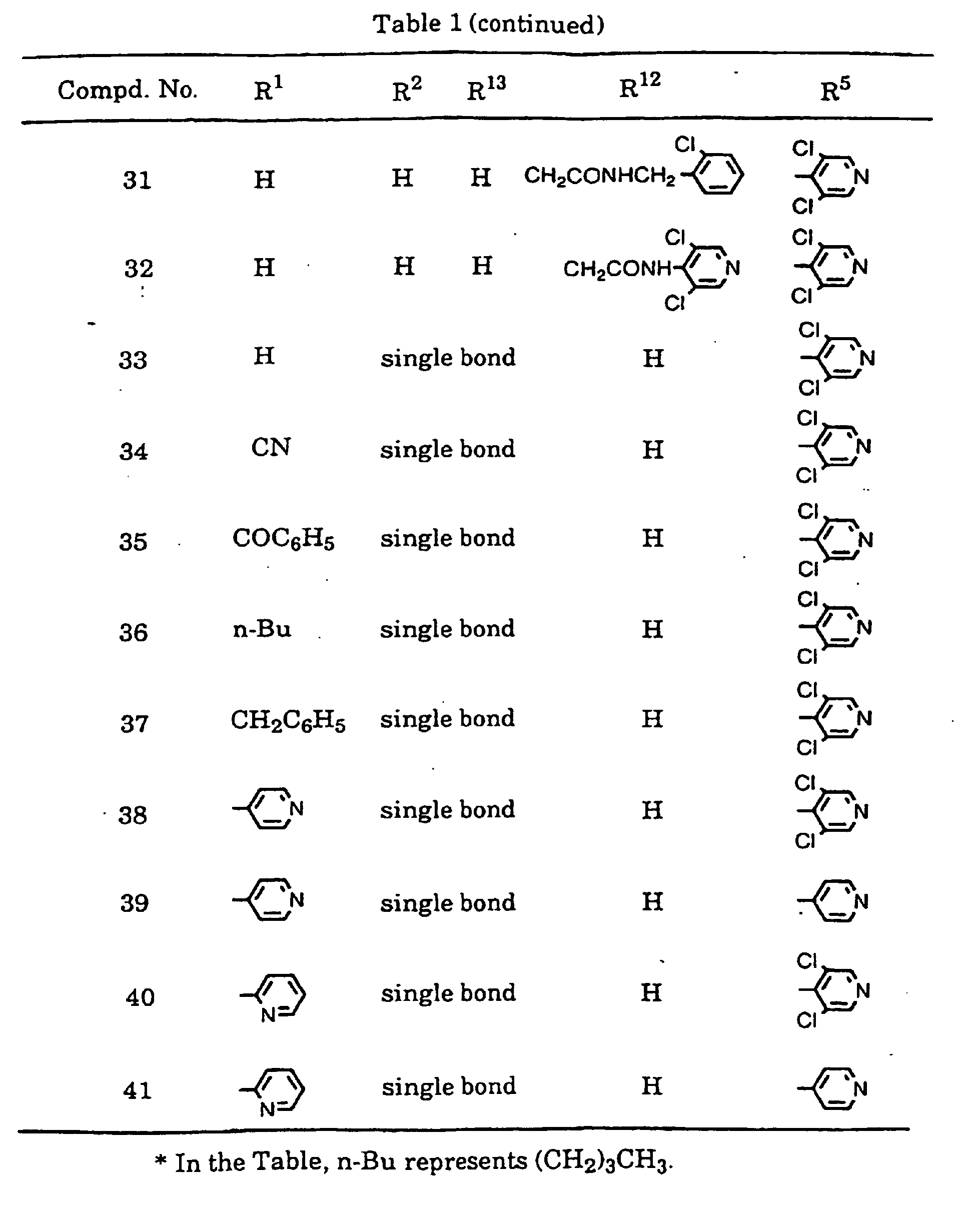

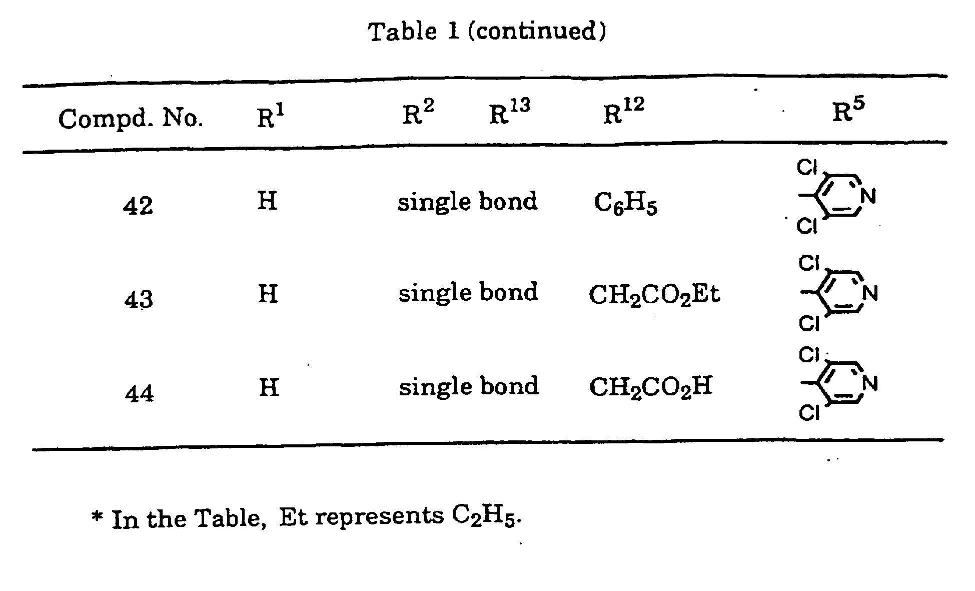

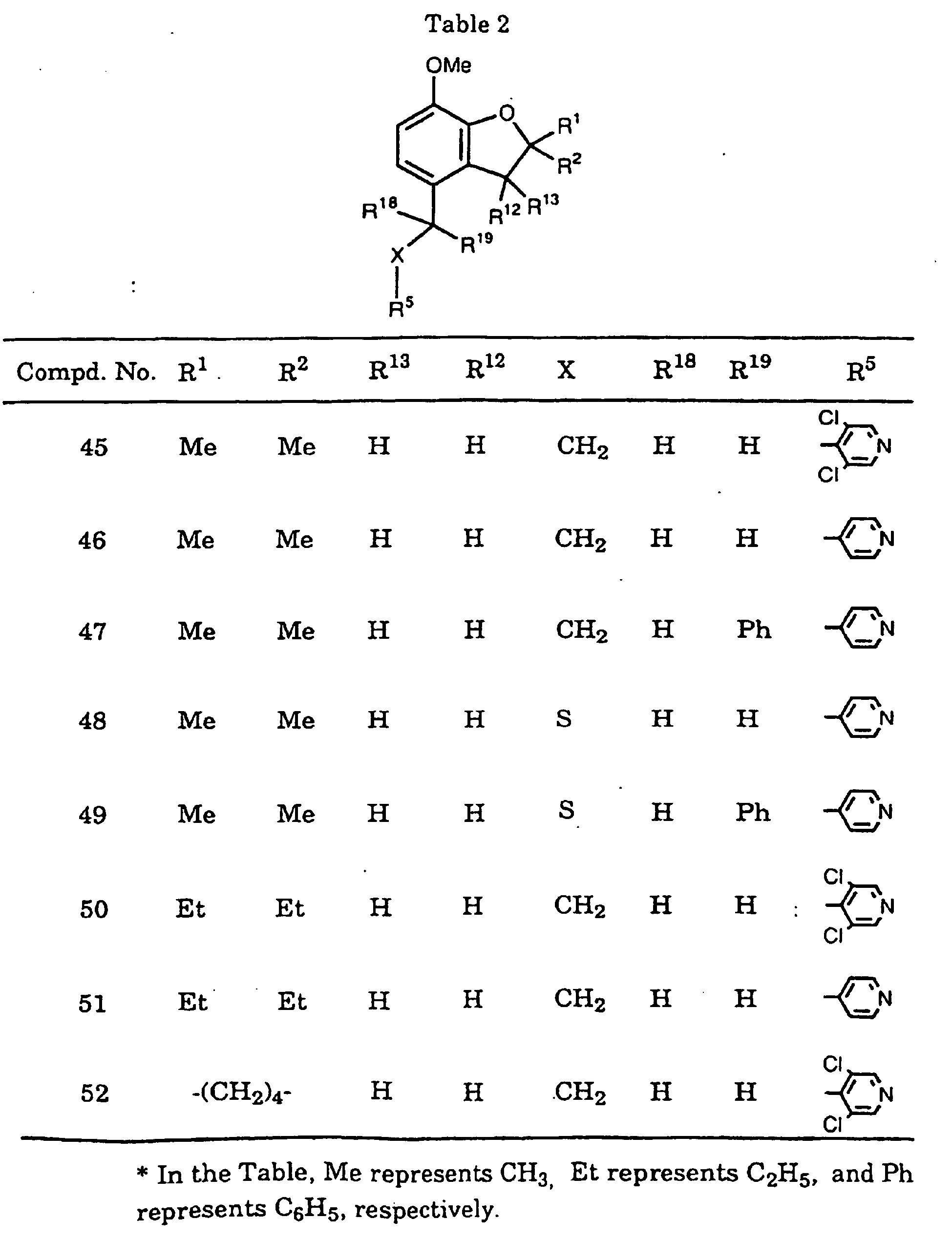

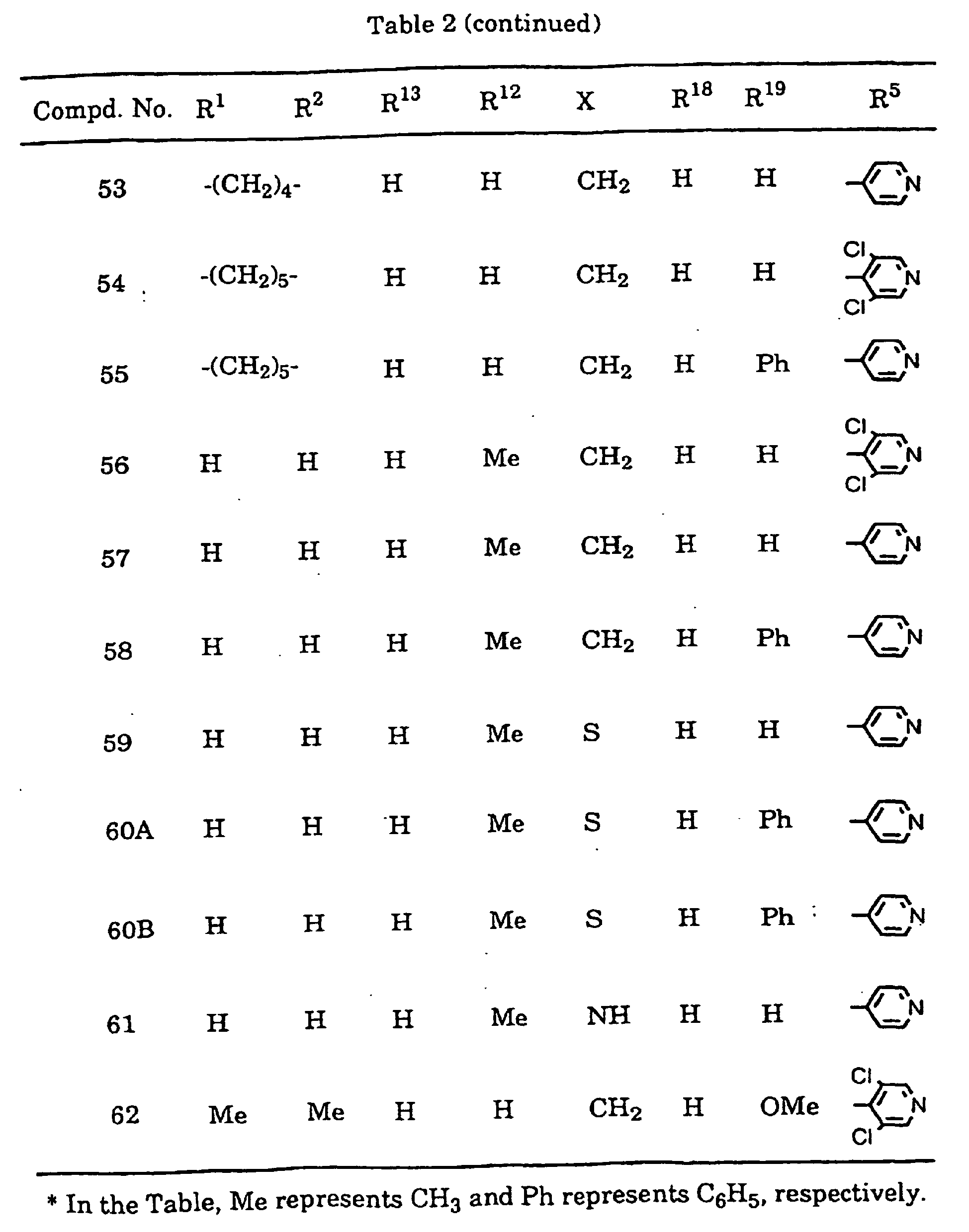

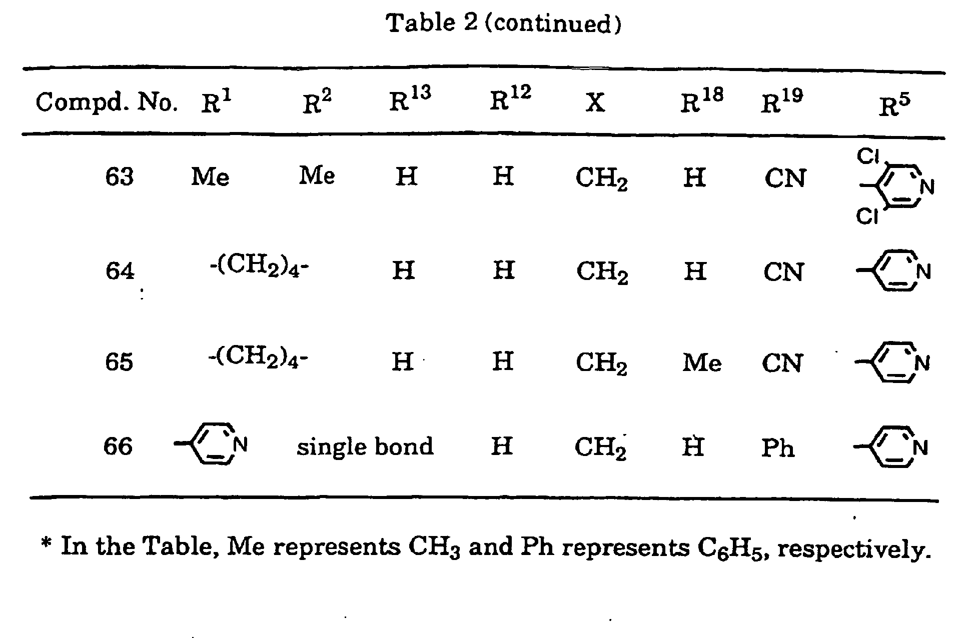

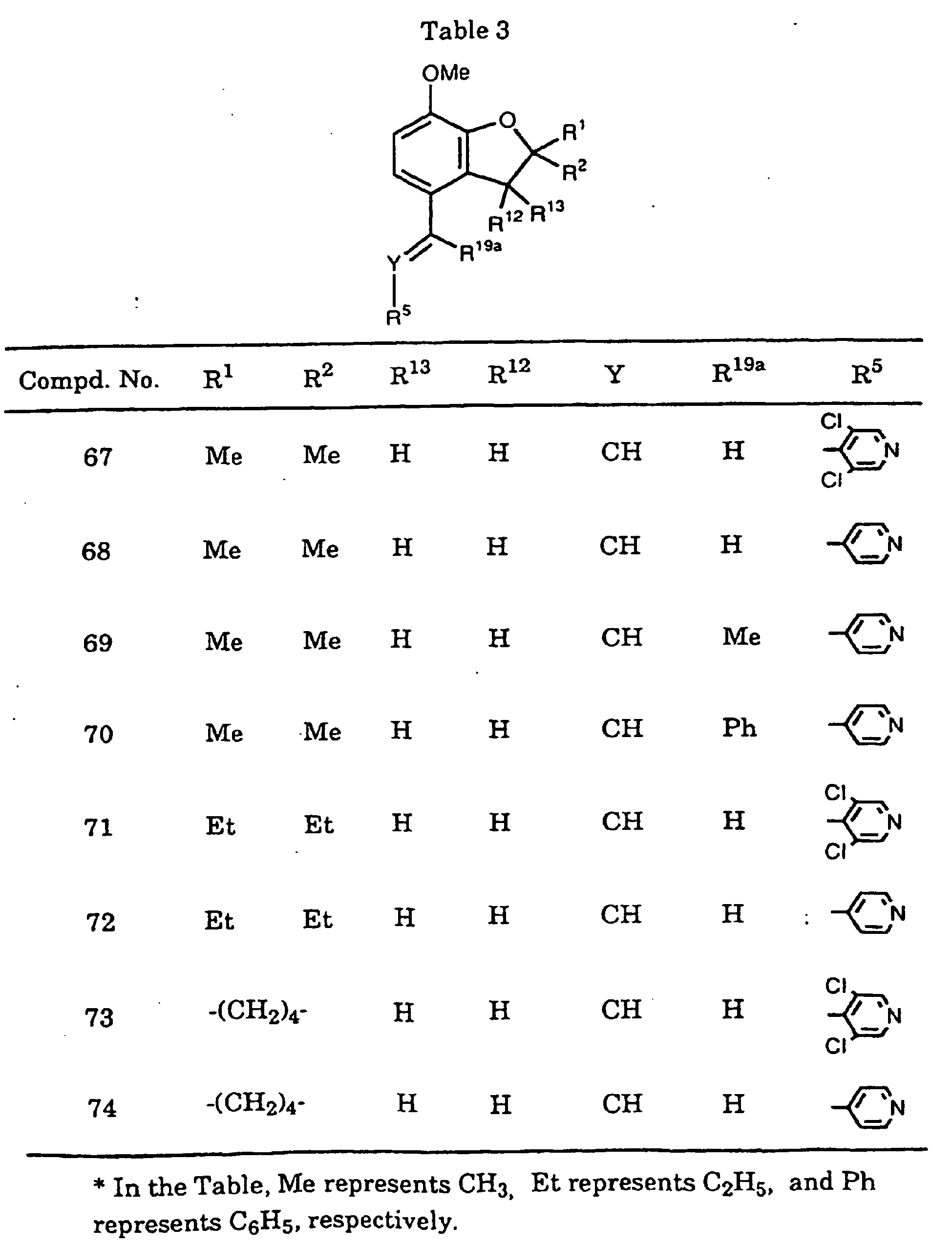

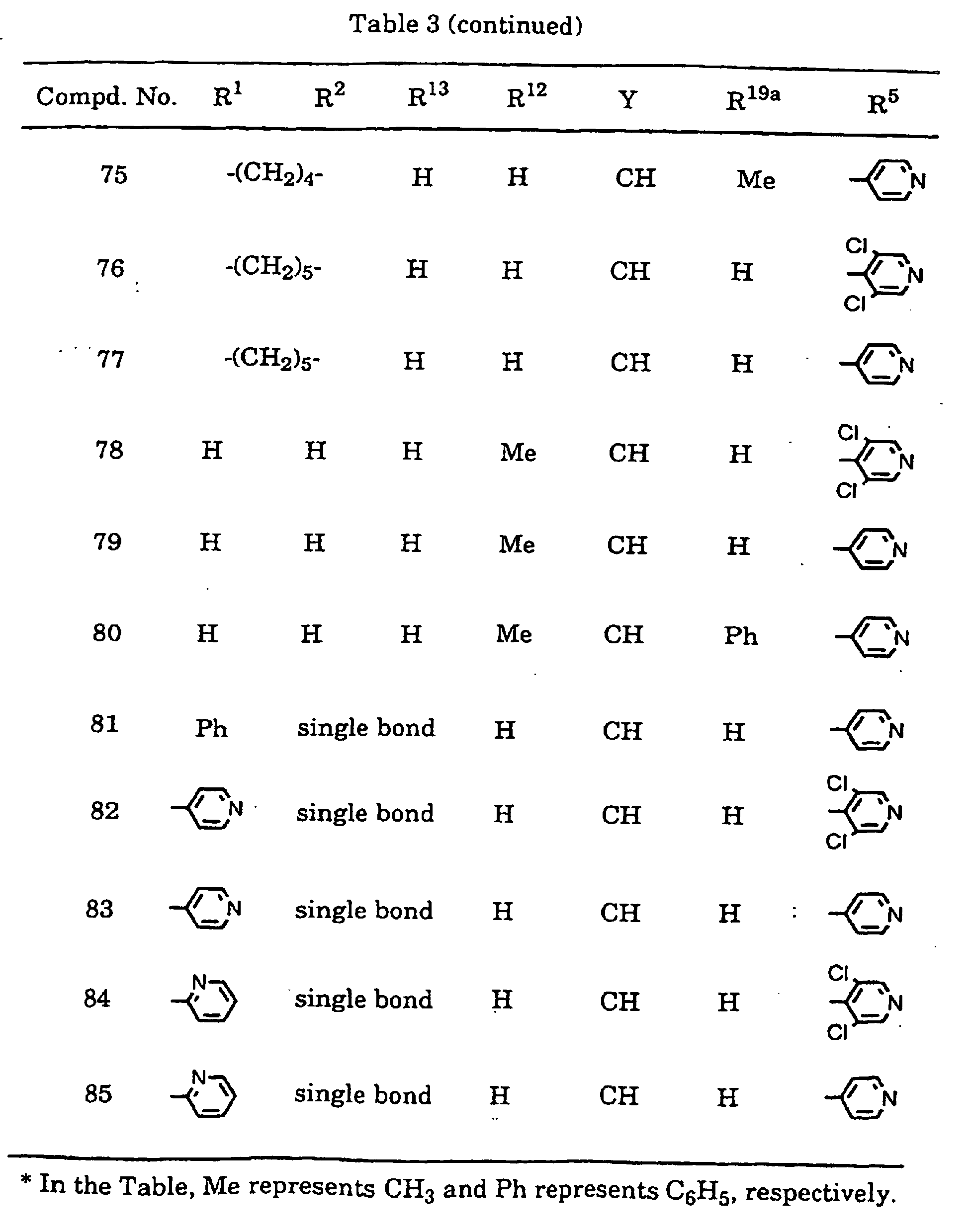

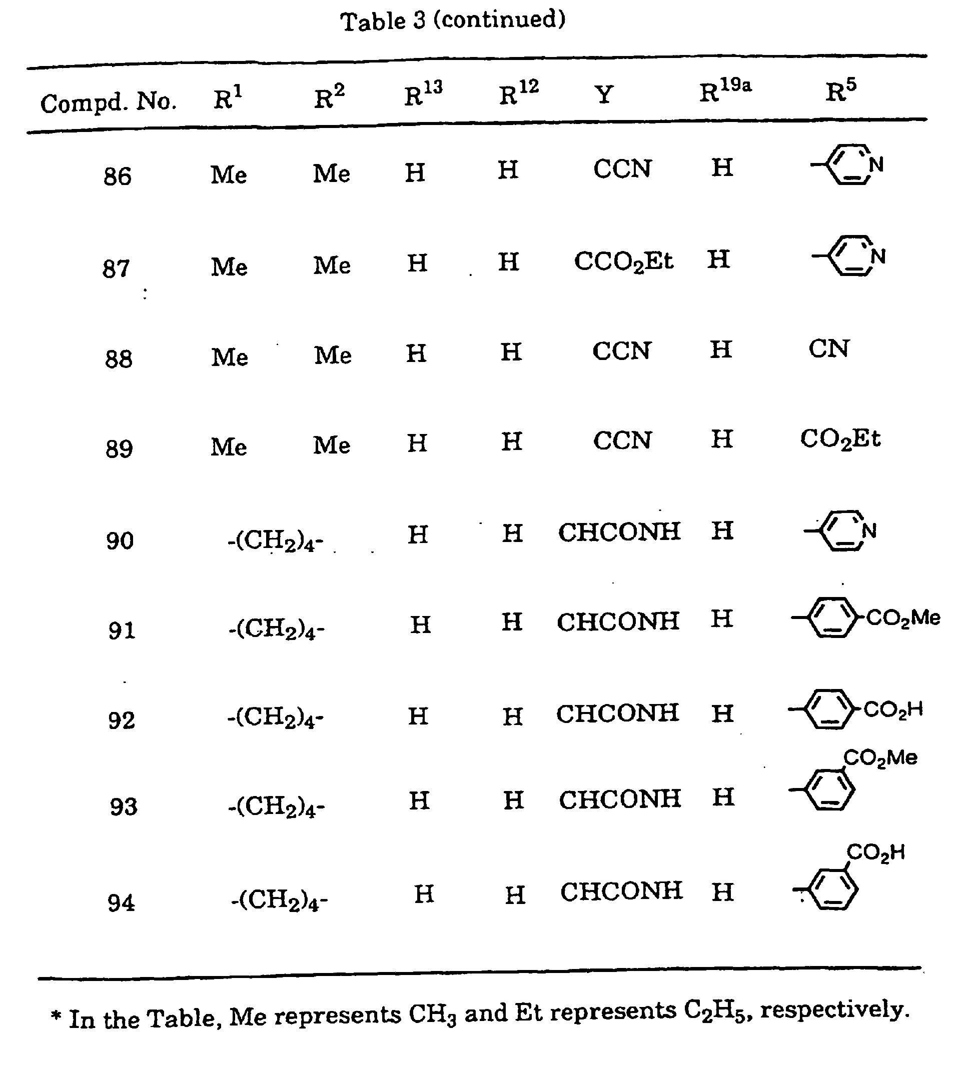

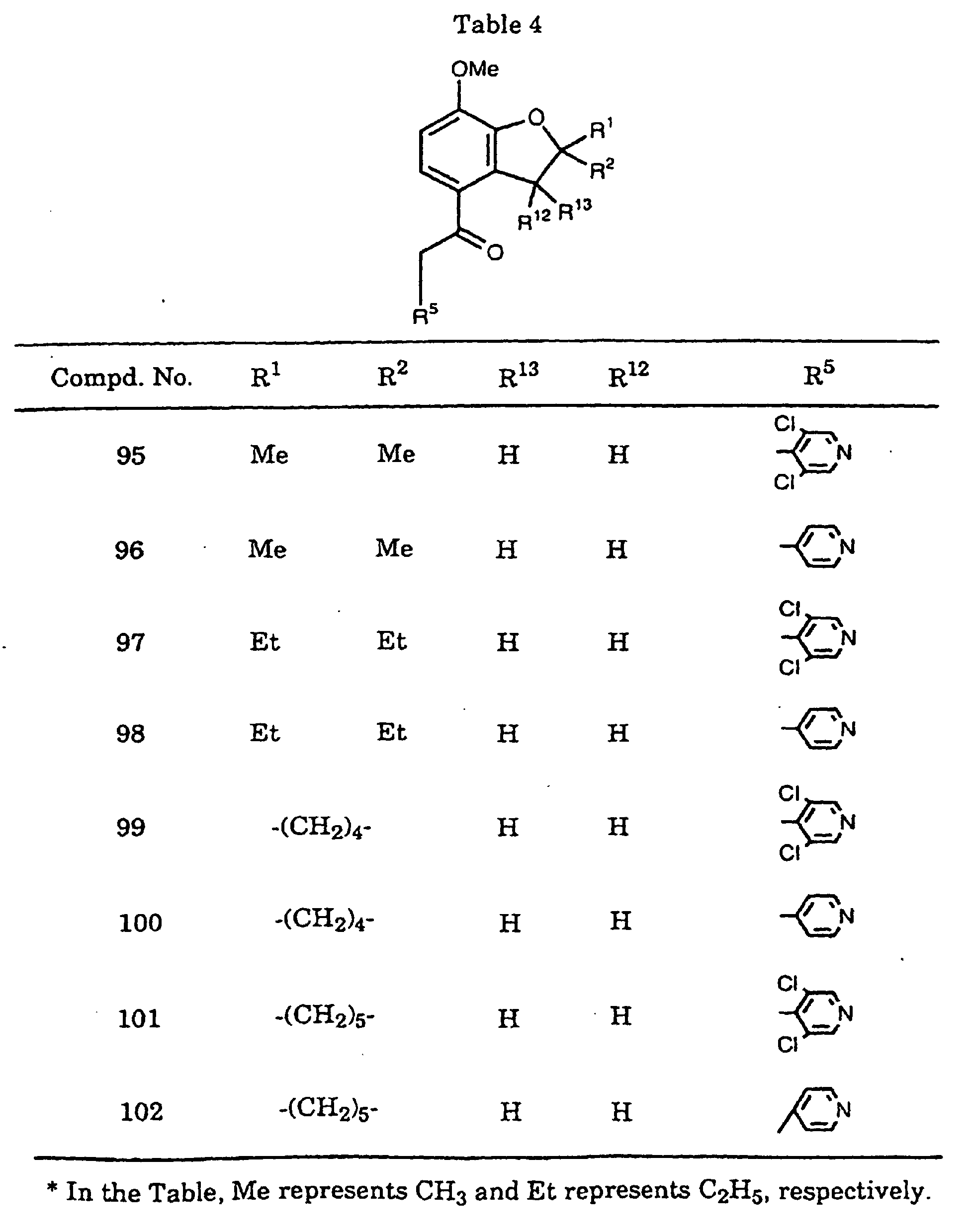

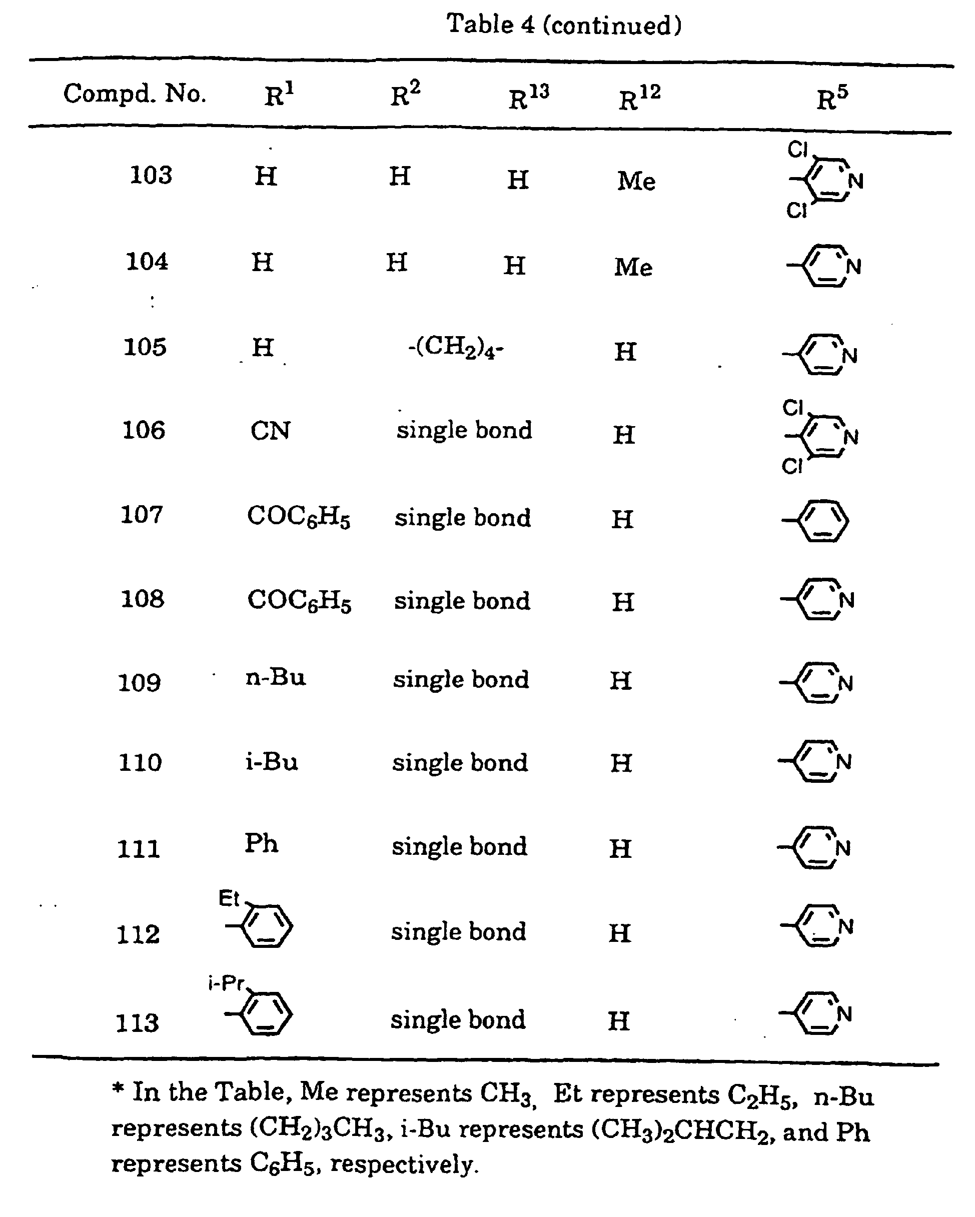

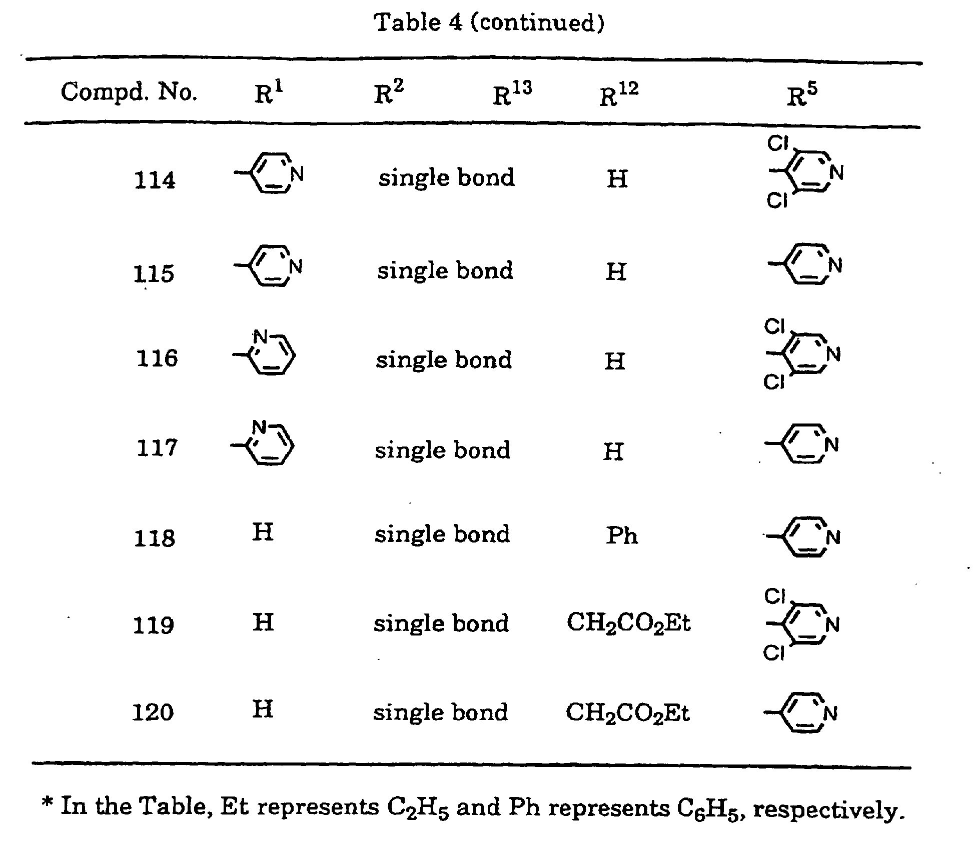

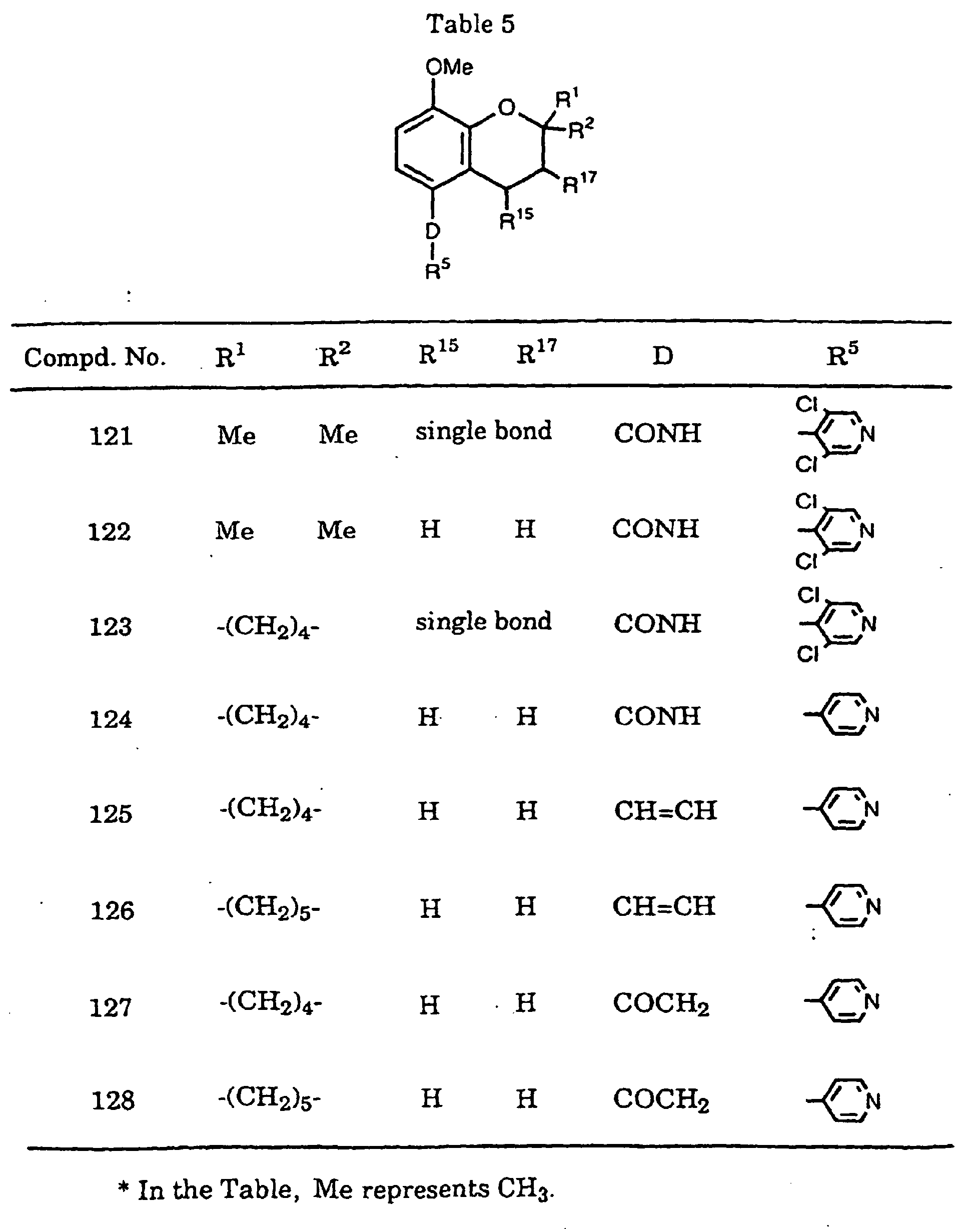

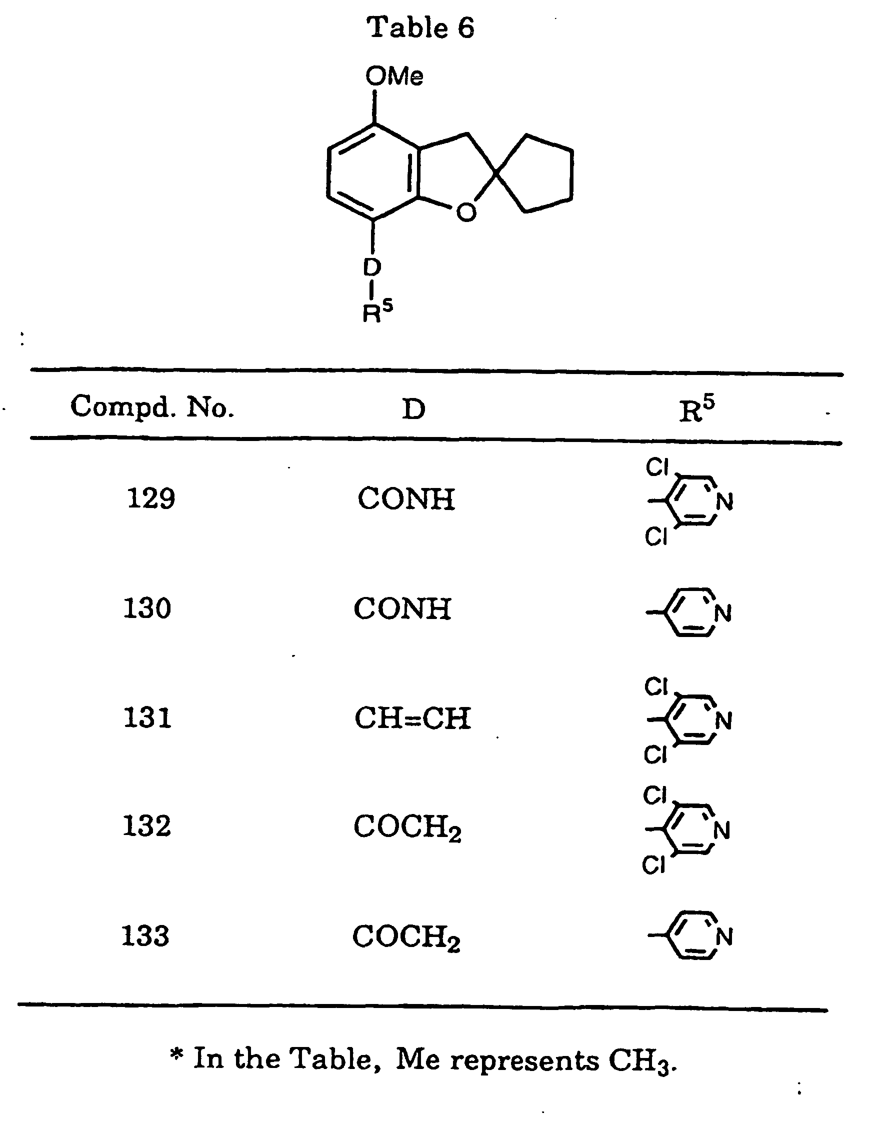

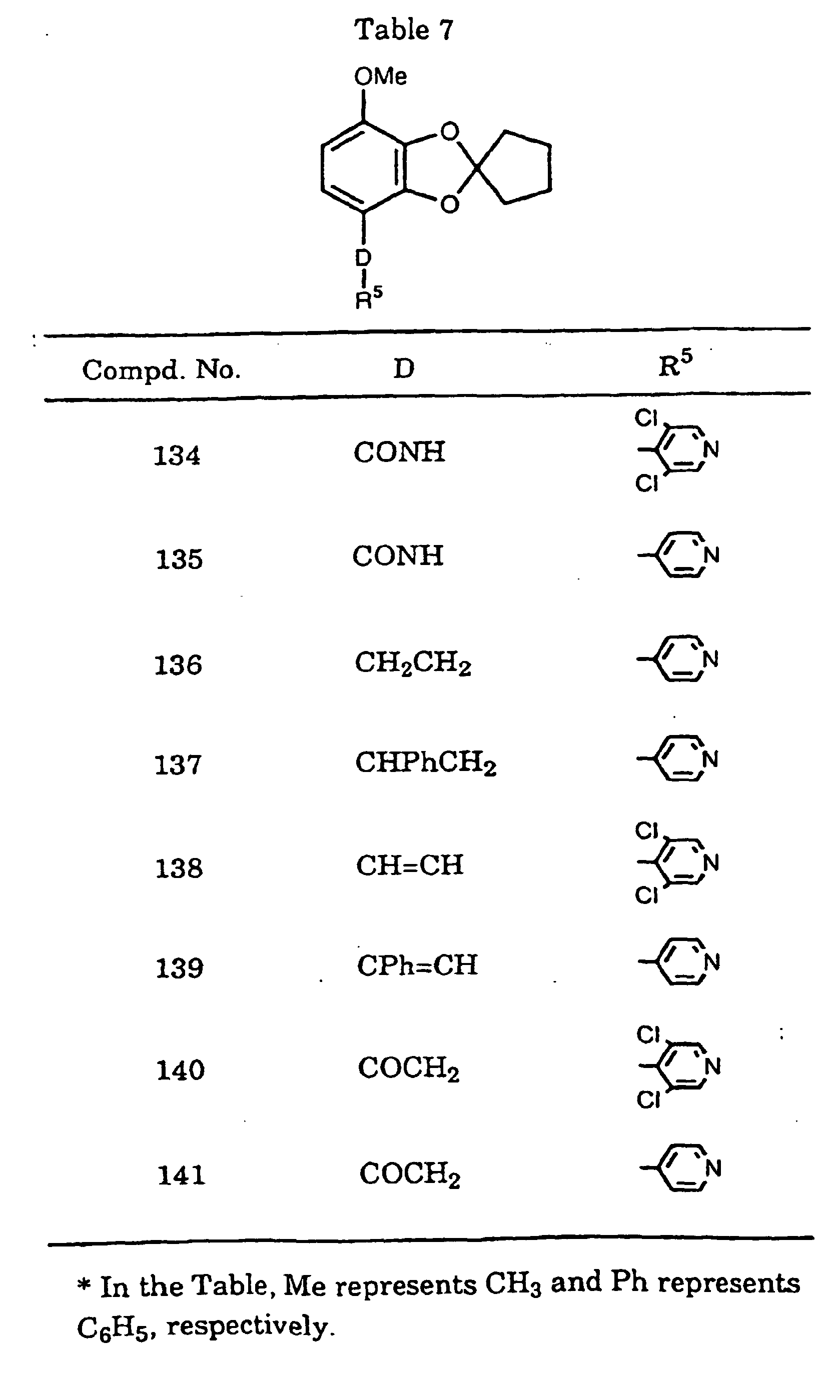

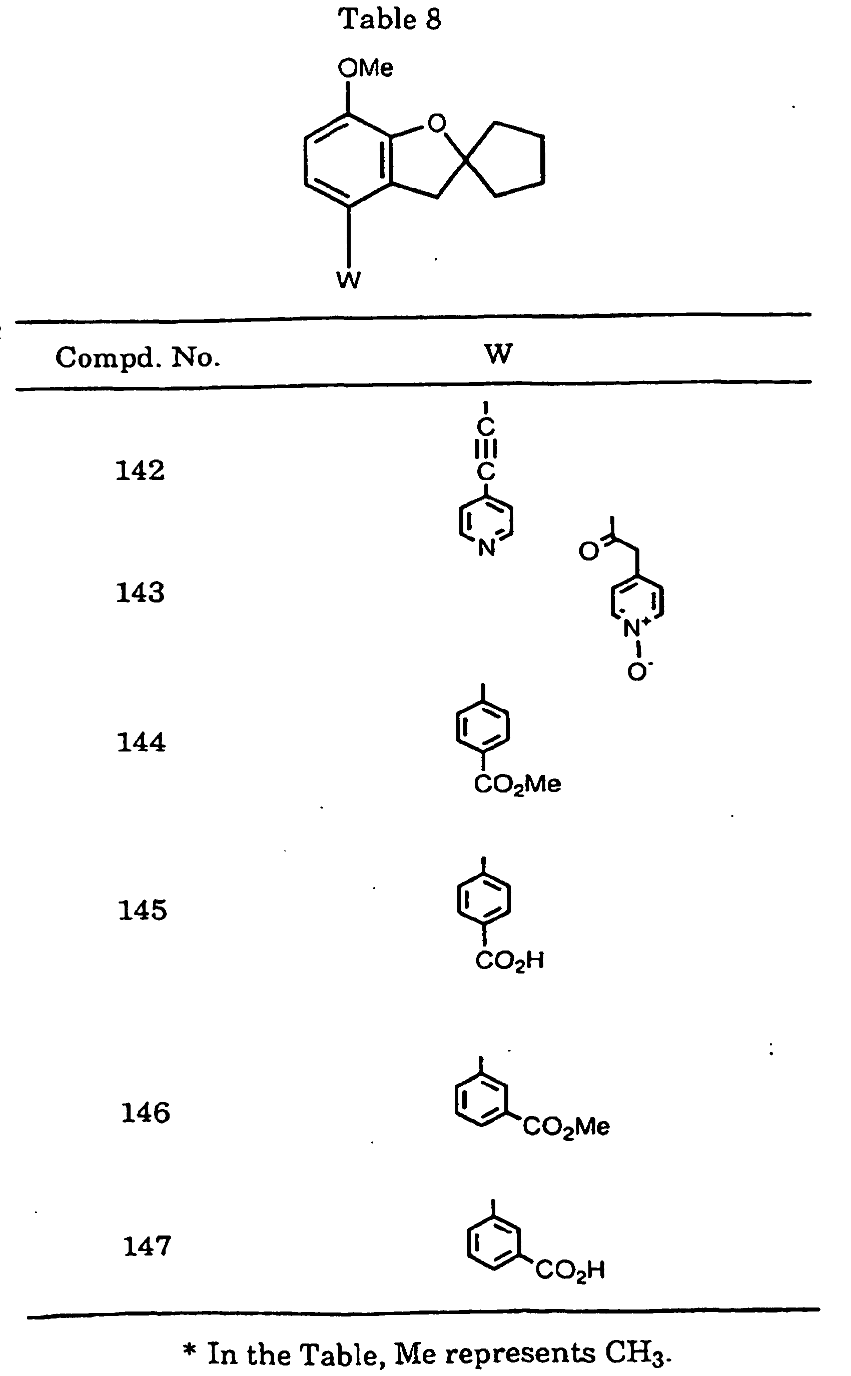

The pharmacological activities of the representative Compounds (I) are described in more detail by Test Examples.

### Test Example 1 Inhibition of the PDE IV Enzyme derived from a Dog Trachea

cAMP-specific phosphodiesterase (PDE IV) was purified from a dog tracheal smooth muscle according to the method of Torphy et al. [Molecular Pharmacol., 37, 206-214 (1990)]. The PDE activity was measured by the following two steps according to the method of Kincaid and Manganiello et al. [Method in Enzymology (J. D. Corbin and R. A. Jonson, Eds.), 199, 457-470 (1988)]. Using [³H]cAMP (at a final concentration of 1 µM) as a substrate, the reaction was carried out in a standard mixture containing N,N-bis(2-hydroxyethyl)-2-aminoethanesulfonic acid (50 mM, pH=7.2), MgCl₂ (1 mM), and soybean trypsin inhibitor (0.1 mg/ml). The reaction was initiated by adding the enzyme, followed by incubation at 30°C for 10 to 30 minutes. After stopping the reaction with hydrochloric acid, the generated 5'-AMP was completely decomposed by 5'-nucleotidase.

The resultant was subjected to chromatography on DEAE-Sephadex A-25, and radio activity of the eluted [³H] adenosine was counted using a scintillation counter. Each of the test drugs was dissolved in DMSO (final concentration 1.7%) and then added to the mixture.

The results are shown in Table 9.

**Table 9**

| Compound No. | Enzyme Inhibitory Activity (%, 10⁻⁶M) |
|---|---|
| 2 | 77 |
| 3 | 75 |
| 4 | 53 |
| 5 | 85 |
| 6 | 66 |
| 7 | 37 |
| 8 | 5 |
| 9 | 22 |
| 11 | 6 |
| 12 | 8 |
| 13 | 91 |
| 15 | 75 |
| 16 | 24 |
| 17 | 63 |
| 18 | 79 |
| 19 | 87 |
| 20 | 80 |
| 21 | 84 |
| 22 | 85 |
| 24 | 80 |
| 25 | 85 |
| 26 | 79 |
| 27 | 75 |
| 28 | 83 |
| 29 | 85 |
| 30 | 85 |
| 31 | 89 |
| 32 | 81 |
| 33 | 71 |
| 34 | 100 |
| 36 | 87 |
| 38 | 89 |
| 39 | 77 |
| 40 | 89 |
| 41 | 58 |
| 42 | 63 |
| 43 | 62 |
| 45 | 74 |
| 47 | 68 |
| 48 | 41 |
| 49 | 40 |
| 50 | 69 |
| 51 | 67 |
| 52 | 86 |
| 53 | 84 |
| 54 | 81 |
| 55 | 86 |
| 59 | 24 |
| 60A | 15 |
| 60B | 4 |
| 62 | 45 |
| 63 | 85 |
| 64 | 78 |
| 65 | 74 |
| 66 | 49 |
| 68 | 80 |
| 70 | 68 |
| 71 | 87 |
| 74 | 73 |
| 75 | 72 |
| 76 | 93 |
| 77 | 87 |
| 79 | 45 |
| 80 | 17 |
| 81 | 69 |
| 83 | 85 |
| 84 | 87 |
| 85 | 87 |
| 87 | 61 |
| 89 | 33 |
| 93 | 23 |
| 97 | 92 |
| 98 | 85 |
| 99 | 91 |
| 100 | 99 |
| 102 | 95 |
| 103 | 48 |
| 104 | 88 |
| 105 | 66 |
| 107 | 63 |
| 109 | 79 |
| 110 | 80 |
| 111 | 69 |
| 114 | 90 |
| 115 | 89 |
| 117 | 69 |
| 118 | 80 |
| 121 | 85 |
| 122 | 92 |
| 124 | 57 |
| 125 | 71 |
| 126 | 68 |
| 127 | 71 |
| 128 | 62 |
| 131 | 51 |
| 132 | 66 |
| 136 | 71 |
| 137 | 61 |
| 139 | 54 |
| 142 | 76 |

### Test Example 2 Suppression of Passive Suhults-Dale Response in Guinea Pig Bronchial Smooth Muscle

For passive sensitization, rabbit anti-ovalbumin serum prepared by the method of Kohda et al. [Nichiyakurishi, 66, 237 (1970)] was peritoneally administered to male Hartley guinea pigs weighing 350 to 500 g, and 24 hours later the tracheae thereof were removed to be used for the experiment. Zig-zag strips were prepared from the tracheae in accordance with the method of Emmerson and Mackay [J. Pharm. Pharmacol., 31, 798 (1979)], and they were suspended in a Krebs-Henseleit solution with aeration of a mixture of 95% oxygen and 5% carbon dioxide at 37°C. After stabilizing for approximately one hour, ovalbumin, as the antigen, was added to the mixture (at a final concentration of 1 µm/ml), and the constriction of the muscle was recorded by a recorder (TYPE 3066; Yokokawa Hokushin Denki) via an isotonic transducer (TD-112S; Nippon Koden). A test compound was cumulatively added to the mixture after the constriction had reached the plateau, and the relaxation ratio was determined. The concentration (IC₅₀) causing 50% relaxation was calculated by linear regression analysis. The IC₅₀ value of Compound 68 of the present invention was 1.6 µM.

### Test Example 3 Suppression of Histamine-Induced Bronchoconstriction Response in Guinea Pig

This test was carried out by a modified Konzett and Rössler method. Under anesthesia with urethane (1.2 g/kg, ip), male Hartley guinea pigs (body weight: 500 to 600 g) were fixed on plates by strings. After undertaking a tracheotomy, cannulae were inserted to the tracheae, right carotid arteries, and left cervical veins. The spontaneous respiration of the guinea pigs was stopped by the administration of gallamie (10 mg/kg) from the left cervical veins via the cannulae. The cannulae inserted into the tracheae were connected to a bronchospasm transducer (Ugo Basile) and a respirator (TB-101, Takashima-shoten, 60 to 70 strokes/minutes, output: 5 cc) and the air overflow volume was recorded by a polygraph (RM-45, Nippon Koden) to measure the amount of bronchoconstriction. For measuring blood pressure, the cannulae inserted in the right carotid arteries were connected to a blood-pressure transducer. Constant bronchoconstriction occurred when histamine (10 µM/kg, iv) was administered at 3 minutes intervals, and the induced bronchoconstriction was used as the control. A test compound was intravenously administered, and one minute later histamine (0.3 mg/kg, iv) was administered. The test compound was cumulatively administered at 5 minutes intervals, and the bronchoconstriction in control and that after the administration of the test compound was compared.

In this test, the ED₅₀ value (50% effective dose) of Compound 68 was 0.076 mg/kg in the case of intravenous administration.

### Test Example 4 Effect on Anaphylactic Bronchoconstriction Response

For passive sensitization, 1 ml of rabbit anti-ovalbumine serum was peritoneally administered to male Hartley guinea pigs, and 16 to 24 hours later, ovalbumine was intravenously administered as the antigen. The induced anaphylactic bronchoconstriction was measured by the modified Konzett and Rössler method. Each of the tracheal cannulae was completely closed at the end of the measurement and the measured constriction was defined as the maximum constriction. Changes in the constriction were measured as percentage in the maximum constriction. The area under the curve (AUC) indicating the strength of the response was calculated by an image analyzer (MCID system, Imaging Research Company). The test compound was orally administered one hour before the antigen administration, and the ED₅₀ value of each drug was calculated from the AUC suppression ratio by linear regression analysis.

In this test, the ED₅₀ value (50% effective dose) of Compound 100 was 0.53 mg/kg by oral administration.

Although Compound (I) or pharmaceutically acceptable salts thereof may be administered as they are, it is usually desirable to provide them in the form of various pharmaceutical preparations. Such pharmaceutical preparations may be used for animals and human beings.

The pharmaceutical preparations in accordance with the present invention may contain Compound (I) or a pharmaceutically acceptable salt thereof, as an active ingredient, either solely or as a mixture with other therapeutically effective components. The pharmaceutical preparations may be prepared by any means which are well known in the technical field of pharmaceutics after mixing the active ingredient with one or more pharmaceutically acceptable carriers.

It is desired to use the administration route which is the most effective in therapy such as oral route or parenteral route which includes intrabuccal, intratracheal, intrarectal, subcutaneous, intramuscular, and intravenous administrations.

Examples of the dosage form are nebulae, capsules, tablets, granules, syrups, emulsions, suppositories, injections, ointments, and tapes.

Liquid preparations suitable for oral administration such as emulsions and syrups can be prepared using water; sugars such as sucrose, sorbitol, and fructose; glycols such as polyethylene glycol and propylene glycol; oils such as sesame oil, olive oil, and soybean oil; preservatives such as p-hydroxybenzoate; flavors such as strawberry and peppermint; and the like. Capsules, tablets, powders, granules, and the like can be prepared using excipients such as lactose, glucose, sucrose, and mannitol; disintegrators such as starch and sodium alginate; lubricants such as magnesium stearate and talc; binders such as polyvinyl alcohol, hydroxypropyl cellulose, and gelatin; surfactants such as fatty acid esters; plasticizers such as glycerin; and the like.

Preparations suitable for parenteral administration comprise sterilized aqueous preparations of the active compound which are preferably isotonic to the blood of the patient. For example, a solution for injection is prepared using a carrier such as a salt solution, a glucose solution, or a mixture of a salt solution and a glucose solution. Preparations for intrarectal administration are prepared using a carrier such as cacao fat, hydrogenated fat, or a hydrogenated carboxylic acid, and provided as suppositories. Nebulae are prepared using an active compound per se or with carriers which can disperse the active compound as fine particles to facilitate absorption without stimulating oral or respiratory mucosa. Practical examples of the carrier are lactose and glycerin. Preparations such as aerosols and dry powders can be used depending upon the properties of the active compound and the employed carriers.

These parenteral preparations may also contain one or more auxiliary components selected from diluents, flavors, preservatives, excipients, disintegrators, lubricants, binders, surfactants, and plasticizers, all of which are mentioned in the above oral preparations.

The effective dose and the administration schedule of Compounds (I) or pharmaceutically acceptable salts thereof may vary depending upon the administration route, age and body weight of a patient, and the type or degree of the disease to be treated, but usually, in the case of oral administration, the effective compound is administered in a dose of 0.01 mg to 1 g, preferably, 0.05 to 50 mg/person/day at one time or in several parts. In the case of parenteral administration such as intravenous injection, the effective compound is administered in a dose of 0.001 to 100 mg, preferably, 0.01 to 10 mg/person/day at one time or in several parts. These doses should, however, vary depending upon various conditions as given above.

Certain embodiments of the present invention are illustrated in the following examples and reference examples.

### Best Mode for Carrying Out the Invention

### Example 1

### 4-(3,5-Dichloro-4-pyridylaminocarbonyl)-7-methoxy-2,3-dihydrobenzofuran (Compound 1)

A mixture of Compound IIw (0.61 g) obtained in Reference Example 23, thionyl chloride (3.6 ml), and dichloromethane (3.6 ml) was heated under reflux for 40 minutes. After being allowed to stand for cooling, the solvent was distilled off and the residue was dissolved in dry toluene. The sovent was distilled off under reduced pressure for removal of the residual thionyl chloride to give a crude acid chloride.

4-Amino-3,5-dichloropyridine (0.73 g) was dissolved in THF (7 ml) and sodium hydride (360 mg) was added thereto under ice-cooling, followed by stirring at room temperature for 15 minutes, and then the mixture was again cooled with ice. A solution of the crude acid chloride obtained above in THF (5 ml) was dropwise added to the mixture under ice-cooling, followed by stirring for one hour under ice-cooling. The reaction mixture was extracted with ether. The organic layer was washed with a saturated saline and dried over anhydrous magnesium sulfate, and the solvent was distilled off under reduced pressure. The residue was recrystallized from ethyl acetate to give Compound 1 (0.60 g, 48.0%) as a white solid.
Melting point: 196-197 °C
NMR (DMSO-d₆, δ, ppm): 3.43 (t, J=9.3Hz, 2H), 3.86(s, 3H), 4.57(t, J=9.3Hz, 2H), 7.00(d, J=8.8Hz, 1H), 7.49(d, J=8.8Hz, 1H), 8.72(s, 2H), 10.3(s, 1H)
MASS(m/e): 338(M⁺)
IR(KBr, cm⁻¹): 1650, 1490, 1280

**Elemental analysis: C₁₅H₁₂N₂O₃Cl₂**

| | | | |
|---|---|---|---|
| Found (%) | C:53.14, | H:3.50, | N:8.06 |
| Calcd.(%) | C:53.12, | H:3.57, | N:8.26 |

### Example 2

### (±)-4-(3,5-Dichloro-4-pyridylaminocarbonyl)-7-methoxy-3-methyl-2,3-dihydrobenzofuran (Compound 2)

Substantially the same procedure as in Example 1 was repeated using Compound IIx (0.116 g) obtained in Reference Example 24 to give Compound 2 (0.145 g, 74%) as a white solid.
Melting point: 198-200 °C (solidified by water)
NMR(CDCl₃, δ, ppm): 1.32(d, J=8.4Hz, 3H), 3.96(s, 3H), 3.98-4.12(m, 1H), 4.38(dd, J=9.3, 3.4Hz, 1H), 4.62-4.77(m, 1H), 6.84 (d, J=9.7Hz, 1H), 7.35(d, J=9.7Hz, 1H), 7.57-7.68(brs, 1H), 8.57(s, 2H)
IR(KBr, cm⁻¹): 1670, 1490, 1283
MASS(m/z): 353(M⁺)

**Elemental analysis: C₁₆H₁₄Cl₂N₂O₃**

| | | | |
|---|---|---|---|
| Found (%) | C:54.53, | H:3.89, | N:7.83 |
| Calcd.(%) | C:54.41, | H:4.00, | N:7.93 |

### Example 3

### (±)-4-(3,5-Dichloro-4-pyridylaminocarbonyl)-3-ethyl-7-methoxy-2,3-dihydrobenzofuran (Compound 3)

Substantially the same procedure as in Example 1 was repeated using Compound IIy (0.222 g) obtained in Reference Example 25 to give Compound 3 (0.170 g, 46.3%) as a white solid.
Melting point: 202-204 °C (ethanol)
NMR(CDCl₃, δ, ppm) : 0.91(t, J=8.0Hz, 3H), 1.47-1.88 (m, 2H), 3.85-4.05(m, 1H), 3.95(s, 3H), 4.47-4.72(m, 2H), 6.85(d, J=9.7Hz, 1H), 7.35(d, J=9.7Hz, 1H), 7.50-7.69(brs, 1H), 8.59(s, 2H)
IR(KBr, cm⁻¹) : 1668, 1488, 1280
MASS(m/z) : 367(M⁺)

**Elemental analysis: C₁₇H₁₆Cl₂N₂O₃**

| | | | |
|---|---|---|---|
| Found (%) | C:55.58, | H:4.34, | N:7.56 |
| Calcd.(%) | C:55.60, | H:4.39, | N:7.63 |

### Example 4

### (±)-4-(3,5-Dichloro-4-pyridylaminocarbonyl)-7-methoxy-3-(2-propyl)-2,3-dihydrobenzofuran (Compound 4)

Substantially the same procedure as in Example 1 was repeated using Compound IIz (0.160 g) obtained in Reference Example 26 to give Compound 4 (0.15 g, 58%) as a white solid.
Melting point: 239-241 °C
NMR(DMSO-d₆, δ, ppm): 0.60(d, J=7.5Hz, 3H), 0.89(d, J=7.1Hz, 3H), 1.98-2.15(m, 1H), 3.80-3.91(m, 1H), 3.85(s, 3H), 4.36-4.60(m, 2H), 7.01(d, J=9.4Hz, 1H), 7.40(d, J=9.4Hz, 1H), 8.75(s, 2H), 10.48(s, 1H)
IR(KBr, cm⁻¹): 1650, 1490, 1280
MASS(m/z): 381(M⁺)

**Elemental analysis: C₁₈H₁₈Cl₂N₂O₃**

| | | | |
|---|---|---|---|
| Found (%) | C:56.56, | H:4.80, | N:7.26 |
| Calcd.(%) | C:56.71, | H:4.76, | N:7.35 |

### Example 5

### (±)-4-(3,5-Dichloro-4-pyridylaminocarbonyl)-3-ethoxycarbonylmethyl-7-methoxy-2,3-dihydrobenzofuran (Compound 5)

Substantially the same procedure as in Example 1 was repeated using Compound IIaa (0.172 g) obtained in ' Reference Example 27 to give Compound 5 (0.131 g, 52%) as a white solid.
Melting point: 186-188 °C (ethanol)
NMR(CDCl₃, δ, ppm): 1.22(t, J=7.6Hz, 3H), 2.52(dd, J=16.9, 11.8Hz, 1H), 2.94-3.12 (m, 1H), 3.97(s, 3H), 4.11(q, J=7.6Hz, 2H), 4.24-4.41(m, 1H), 4.59(dd, J=10.1, 4.2Hz, 1H), 4.70-4.83(m, 1H), 6.88(d, J=9.3Hz, 1H), 7.37(d, J=9.3Hz, 1H), 7.58-7.72(brs, 1H), 8.58(s, 2H)
IR(KBr, cm⁻¹): 1722, 1662, 1493, 1285
MASS(m/z): 425(M⁺)

**Elemental analysis: C₁₉H₁₈Cl₂N₂O₅**

| | | | |
|---|---|---|---|
| Found (%) | C:53.65, | H:4.11, | N:6.59 |
| Calcd.(%) | C:53.66, | H:4.27, | N:6.59 |

### Example 6

### (±)-3-Ethoxycarbonylmethyl-7-methoxy-4-pyridylaminocarbonyl-2,3-dihydrobenzofuran (Compound 6)

Substantially the same procedure as in Example 1 was repeated using 4-aminopyridine instead of 4-amino-3,5-dichloropyridine and using Compound IIaa (4.00 g) obtained in Reference Example 27 to give Compound 6 (4.77 g, 94%) as white crystals.
Melting point: 177 °C
NMR(DMSO-d₆, δ, ppm): 1.14(t, 3H, J=7Hz), 2.56-2.46 (m, 1H), 2.79 (dd, 1H, J=3Hz, 16Hz), 3.88(s, 3H), 4.04(q, 2H, J=7Hz), 4.36-4.16(m, 1H)4.47(dd, 1H, J=4Hz, 9Hz), 4.64(t, 1H, J=9Hz), 7.08(d, 1H, J=9Hz), 7.65(d, 1H, J=9Hz), 8.35(d, 2H, J=8Hz), 8.74(d, 2H, J=7Hz), 11.64(s, 1H)
IR(KBr, cm⁻¹): 1697, 1614, 1506, 1471, 1269
MASS(m/e): 401(M⁺)

**Elemental analysis: C₁₉H₂₀N₂O₅·1HCl·0.5H₂O**

| | | | |
|---|---|---|---|
| Found (%) | C:56.79, | H:5.52, | N:6.97 |
| Calcd. (%) | C:57.05, | H:5.50, | N:6.99 |

### Example 7

### (±)-3-Ethoxycarbonylmethyl-7-methoxy-4-phenylamino-carbonyl-2,3-dihydrobenzofuran (Compound 7)

Substantially the same procedure as in Example 1 was repeated using aniline instead of 4-amino-3,5-dichloropyridine and using Compound IIaa (0.50 g) obtained in Reference Example 27 to give Compound 7 (0.59 g, 92%) as a white solid.
Melting point: 169-170 °C
NMR(COCl₃, δ, ppm): 2.22(t, 3H, J=7Hz), 2.51(dd, 1H, J=11Hz, 17Hz), 3.08(dd, 1H, J=3Hz, 17Hz), 3.93(s, 3H), 4.11(q, 2H, J=7Hz), 4.39-4.29(m, 1H), 4.55 (dd, 1H, J=3Hz, 9Hz), 4.75(t, 1H, J=9Hz), 6.82(d, 1H, J=9Hz), 7.20-7.12 (m, 3H), 7.36(d, 1H, J=9Hz), 7.40(s, 1H), 7.58(d, 2H, J=8Hz), 7.72(s, 1H)
IR(KBr, cm⁻¹): 3305, 1722, 1645, 1286, 1194
MASS(m/e): 355(M⁺)

**Elemental analysis: C₂₀H₂₁NO₅**

| | | | |
|---|---|---|---|
| Found (%) | C:67.59, | H:5.96, | N:3.94 |
| Calcd. (%) | C:67.72, | H:5.98, | N:3.95 |

### Example 8

### (±)-4-Cyclohexylaminocarbonyl-3-ethoxycarbonylmethyl-7-methoxy-2,3-dihydrobenzofuran (Compound 8)

Substantially the same procedure as in Example 1 was repeated using cyclohexylamine instead of 4-amino-3,5-dichloropyridine and using Compound IIaa (0.60 g) obtained in Reference Example 27 to give Compound 8 (0.68 g, 87%) as a white solid.
Melting point: 197-199 °C
NMR(CDCl₃, δ, ppm): 1.24(t, 3H, J=7Hz), 1.49-1.29(m, 5H), 2.17-2.00(m, 5H), 2.47(dd, 1H, J=11Hz, 17Hz), 3.07(dd, 1H, J=3Hz, 17Hz), 3.90(s, 3H), 4.13(q, 2H, J=7Hz), 4.31-4.23(m, 1H), 4.53(dd, 1H, J=3Hz, 9Hz), 4.72(t, 1H, J=9Hz), 5.87 (d, 1H, J=8Hz), 6.75(d, 1H, J=8Hz), 7.01(d, 1H, J=8Hz), 7.27(s, 1H)
IR(KBr, cm⁻¹): 3284, 1726, 1718, 1624, 1541, 1524, 1284
MASS(m/e): 361(M⁺)

**Elemental analysis: C₂₀H₂₇NO₅**

| | | | |
|---|---|---|---|
| Found (%) | C:66.46, | H:7.53, | N:3.88 |
| Calcd. (%) | C:66.38, | H:7.75, | N:4.00 |

### Example 9

### (±)-3-Carboxymethyl-4-(3,5-dichloro-4-pyridylamino-carbonyl)-7-methoxy-2,3-dihydrobenzofuran (Compound 9)

Compound 5 (0.329 g) obtained in Example 5 was mixed with a 2N aqueous solution of sodium hydroxide (6.6 ml), followed by stirring at room temperature for one hour. Under ice-cooling, the reaction mixture was adjusted to pH 2 by adding hydrochloric acid, and then the precipitated solid was collected by filtration. The obtained crude product was recrystallized from ethanol to give Compound 9 (0.302 g, 98%) as a white solid.
Melting point: 259-263 °C
NMR (DMSO-d₆, δ, ppm): 2.40(dd, J=14.5, 8.9Hz, 1H), 2.70-2.89(m, 1H), 3.86(s, 3H), 4.03-4.21(m, 1H), 4.34-4.49(m, 1H), 4.55-4.74(m, 1H), 7.04(d, J=8.4Hz, 1H), 7.49(d, J=8.4Hz, 1H), 8.75 (s, 2H), 10.51(s, 1H), 12.17-12.49(brs, 1H)
IR (KBr, cm⁻¹): 1713, 1663, 1490, 1288
MASS(m/z): 397(M⁺)

**Elemental analysis: C₁₇H₁₄Cl₂N₂ O₅·0.5C₂ H₆O·0.5H₂O**

| | | | |
|---|---|---|---|
| Found (%) | C:50.49, | H:4.37, | N:6.31 |
| Calcd.(%) | C:50.37, | H:4.23, | N:6.53 |

### Example 10

### (±)-3-Carboxymethyl-7-methoxy-4-pyridylaminocarbonyl-2,3-dihydrobenzofuran (Compound 10)

Substantially the same procedure as in Example 9 was repeated using Compound 6 (4.00 g) obtained in Example 6 to give Compound 10 (2.79 g, 76%) as a white solid.
Melting point: 227-233 °C
NMR (DMSO-d₆, δ, ppm) : 2.41(dd, 1H, J=6Hz, 17Hz), 2.72(dd, 1H, J=3Hz, 17Hz), 3.88(s, 3H), 4.20-4.10(m, 1H), 4.45(dd, 1H, J=4Hz, 9Hz), 4.64(t, 1H, J=9Hz), 7.08(d, 1H, J=9Hz), 7.42(d, 1H, J=9Hz), 8.30(d, 2H, J=7Hz), 8.72(d, 2H, J=7Hz), 11.53(s, 1H), 12.35(brs, 1H)
IR(KBr, cm⁻¹): 3300(br), 2770(br), 17.16, 1693, 1614, 1508, 1477, 1271
MASS(m/e): 390(M⁺)

**Elemental analysis: C₁₇H₁₆N₂O₅·HCl·0.2C₂H₆O·H₂O**

| | | | |
|---|---|---|---|
| Found (%) | C:53.56, | H:5.17, | N:7.18 |
| Calcd.(%) | C:53.63, | H:5.11, | N:7.11 |

### Example 11

### (±)-3-Carboxymethyl-7-methoxy-4-phenylaminocarbonyl-2,3-dihydrobenzofuran (Compound 11)

Substantially the same procedure as in Example 9 was repeated using Compound 7 (0.43 g) obtained in Example 7 to give Compound 11 (0.37 g, 94%) as a white solid.
Melting point: 248-251 °C
NMR(DMSO-d₆, δ, ppm): 2.38(dd, 1H, J=11Hz, 17Hz), 2.78(dd, 1H, J=2Hz, 17Hz), 3.84(s, 3H), 4.18-4.11(m, 1H), 4.40(dd, 1H, J=4Hz, 9Hz), 4.64(t, 1H, J=9Hz), 6.99(d, 1H, J=8Hz), 7.09(t, 1H, J=7Hz), 7.36-7.30 (m, 3H), 7.72(d, 2H, J=8Hz), 10.15(s, 1H), 12.31(brs, 1H)
IR(KBr, cm⁻¹): 2900(br), 1709, 1645, 1595, 1506, 1442, 1286
MASS (m/e) : 327(M⁺)

**Elemental analysis: C₁₈H₁₇NO₅**

| | | | |
|---|---|---|---|
| Found (%) | C:66.05, | H:5.23, | N:4.28 |
| Calcd.(%) | C:65.82, | H:5.20, | N:4.22 |

### Example 12

### (±)-3-Carboxyme<:hyl-4-cyclohe:<ylaminocarbonyl-7-methoxy-2,3-dihydrobenzofuran (Compound 12)

Substantially the same procedure as in Example 9 was repeated using Compound 8 (0.47 g) obtained in Example 8 to give Compound 12 (0.40 g, 95%) as a white solid.
Melting point: 246-247 °C
NMR(DMSO-d₆, δ, ppm): 1.36-1.06(m, 5H), 1.80-1.53(m, 5H), 2.31(dd, 1H, J=11Hz, 17Hz), 2.76(dd, 1H, J=2Hz, 17Hz), 3.75-3.69(m, 1H), 3.80(s, 3H), 4.13-4.06(m, 1H), 4.36(dd, 1H, J=4Hz, 9Hz), 4.59 (t, 1H, J=9Hz), 6.89(d, 1H, J=9Hz), 7.13(d, 1H, J=9Hz), 8.06(d, 1H, J=8Hz), 12.31(brs, 1H)
IR(KBr, cm⁻¹): 3410, 3134(br), 1727, 1546, 1282
MASS(m/e): 333(M⁺+1)

**Elemental analysis: C₁₈H₂₃NO₅**

| | | | |
|---|---|---|---|
| Found (%) | C:64.85, | H:6.95, | N:4.20 |
| Calcd.(%) | C:64.99, | H:7.08, | N:4.28 |

### Example 13

### (±)-3-Benzyloxycarbonylmethyl-4-(3,5-dichloro-4-pyridylaminocarbonyl)-7-methoxy-2,3-dihydrobenzofuran (Compound 13)

Compound 9 (0.291 g) obtained in Example 9 was dissolved in dichloromethane (2.9 ml) and thionyl chloride (1.5 ml) was added thereto, followed by stirring at room temperature for one hour. The solvent was distilled off under reduced pressure, the residue was again dissolved in toluene, and the solvent was distilled off under reduced pressure. Benzyl alcohol (2 ml) was added to the residue followed by heating under reflux for 30 minutes. The reaction solution was concentrated and the residue was recrystallized from ethanol to give Compound 13 (0.304 g, 85.2%) as a white solid.
Melting point: 198-205 °C
NMR(DMSO-d₆, δ, ppm): 2.43-2.68(m, 1H), 2.80-3.01(m, 1H), 3.85(s, 3H), 4.10-4.27(m, 1H), 4.39-4.75(m, 2H), 5.08(s, 2H), 7.05(d, J=9.5Hz, 1H), 7.24-7.43(m, 5H), 7.50(d, J=9.5Hz, 1H), 8.77(s, 2H), 10.50(s, 1H)
IR(KBr, cm⁻¹): 1722, 1668, 1490, 1288
MASS(m/z): 487(M⁺)

**Elemental analysis: C₂₄H₂₀Cl₂N₂O₅**

| | | | |
|---|---|---|---|
| Found (%) | C:59.32, | H:4.00, | M:5.72 |
| Calcd. (%) | C:59.15, | H:4.14, | N:5.75 |

### Example 14

### (±)-3-Benzyloxycarbonylmethyl-7-methoxy-4-pyridyl-aminocarbonyl-2,3-dihydrobenzofuran (Compound 14)

Substantially the same procedure as in Example 13 was repeated using Compound 10 (0.12 g) obtained in Example 10 to give Compound 14 (0.07 g, 53%) as a white solid.
Melting point: 165-166 °C
NMR(CDCl₃, δ, ppm): 2.60(dd, 1H, J=10Hz, 17Hz), 3.06 (dd, 1H, J=3Hz, 17Hz), 3.94(s, 3H), 4.40-4.33(m, 1H), 4.57(dd, 1H, J=4Hz, 10Hz), 4.74(t, 1H, J=9Hz), 5.10(s, 2H), 6.82(d, 1H, J=9Hz), 7.16(d, 2H, J=9Hz), 7.38-7.28(m, 5H), 7.52(dd, 1H, J=1Hz, 5Hz), 7.77(brs, 1H), 8.53(dd, 2H, J=1Hz, 5Hz)
IR(KBr, cm⁻¹): 3317, 1720, 1653, 1585, 1504, 1284
MASS (m/e) : 418(M⁺)

**Elemental analysis: C₂₄H₂₂N₂O₅·0.1C₂H₆O·0.4H₂O**

| | | | |
|---|---|---|---|
| Found (%) | C:67.56, | H:5.48, | N:6.51 |
| Calcd.(%) | C:67.54, | H:5.90, | N:6.47 |

### Example 15

### (±)-3-Benzyloxycarbonylmethyl-7-methoxy-4-phenylamino-carbonyl-2,3-dihydrobenzofuran (Compound 15)

Substantially the same procedure as in Example 13 was repeated using Compound 11 (0.17 g) obtained in Example 11 to give Compound 15 (0.17 g, 76%) as a white solid.
Melting point: 179-180 °C
NMR(CDCl₃, δ, ppm): 2.59(dd, 1H, J=11Hz, 17Hz), 3.13 (dd, 1H, J=3Hz, 17Hz), 3.93(s, 3H), 4.42-4.32(m, 1H), 4.55(dd, 1H, J=4Hz, 9Hz), 4.74(t, 1H, J=9Hz), 5.08(d, 1H, J=3Hz), 6.81(d, 1H, J=9Hz), 7.16(d, 1H, J=9Hz), 7.39-7.26(m, 8H), 7.55(dd, 2H, J=1Hz, 8Hz), 7.66(s, 1H)
IR(KBr, cm⁻¹): 3307, 1722, 1645, 1529, 1506, 1444, 1288
MASS(m/e): 417(M⁺)

**Elemental analysis: C₂₅H₂₃NC₅**

| | | | |
|---|---|---|---|
| Found (%) | C:71.93, | H:5.55, | N:3.36 |
| Calcd.(%) | C:71.82, | H:5.51, | N:3.36 |

### Example 16

### (±)-3-Benzyloxycarbonyl-4-cyclohexylaminocarbonyl-7-methoxy-2,3-dihydrobenzofuran (Compound 16)

Substantially the same procedure as in Example 13 was repeated using Compound 12 (0.20 g) obtained in Example 12 to give Compound 16 (0.20 g, 76%) as a white solid.
Melting point: 178-179 °C
NMR(DMSO-d₆, δ, ppm): 1.34-1.00(m, 5H), 1.86-1.66(m, 5H), 2.56-2.46(m, 1H), 2.88(dd, 1H, J=3Hz, 17Hz), 3.76-3.62(m, 1H), 3.80(s, 3H), 4.19-4.12(m, 1H), 4.37(dd, 1H, J=4Hz, 9Hz), 4.58(t, 1H, J=9Hz), 5.10(d, 1H, J=2Hz), 6.90(d, 1H, J=9Hz), 7.15(d, 1H, J=9Hz), 7.41-7.31(m, 5H), 8.06(d, 1H, J=8Hz)
IR(KBr, cm⁻¹): 3325, 1720, 1626, 1282, 1174
MASS(m/e): 423(M⁺)

**Elemental analysis: C₂₅H₂₉NO₅**

| | | | |
|---|---|---|---|
| Found (%) | C:70.90, | H:6.90, | N:3.30 |
| Calcd.(%) | C:70.90, | H:7.04, | N:3.34 |

### Example 17

### (±)-4-(3,5-Dichloro-4-pyridylaminocarbonyl)-7-methoxy-3-(4-methylpiperazine-1-ylcarbonylmethyl)-2,3-dihydrobenzofuran·hydrochloride (Compound 17)

Substantially the same procedure as in Example 13 was repeated using Compound 9 (0.354 g) obtained in Example 9 and N-methylpiperazine (0.119 ml) to give (±)-4-(3,5-dichloro-4-pyridylaminocarbonyl)-7-methoxy-3-(4-methyl-piperazin-1-ylcarbonylmethyl)-2,3-dihydrobenzofuran (0.427 g, 100%) as an oily substance. The obtained free base was dissolved in ethyl acetate (50 ml), and a saturated solution of hydrochloric acid in ethyl acetate (2 ml) was added thereto followed by stirring. The precipitated hydrochloride was collected by filtration and washed with ethyl acetate to give Compound 17 as a white solid.
Melting point: 181-187 °C
NMR(DMSO-d₆, δ, ppm): 2.40-3.52(m, 13H), 3.77-4.70(m, 3H), 3.88(s, 3H), 7.06(d, J=9.6Hz, 1H), 7.52(d, J=9.6Hz, 1H), 8.76(s, 2H), 10.55(s, 1H)
IR(KBr, cm⁻¹): 1650, 1480, 1280

**Elemental analysis: C₂₂H₂₄Cl₂N₄O₄·HCl·2.5H₂O**

| | | | |
|---|---|---|---|
| Found (%) | C:47.08, | H:5.29, | N:9.94 |
| Calcd.(%) | C:47.11, | H:5.39, | N:9.99 |

### Example 18

### (±)-4-[(3,5-Dichloro-4-pyridyl)aminocarbonyl]-7-methoxy-3-[(3-pyridylmethyl)aminocarbonyl]methyl-2,3-dihydrobenzofuran (Compound 18)

Substantially the same procedure as in Example 13 was repeated using Compound 9 (0.30 g) obtained in Example 9 and 3-pyridylmethylamine to give Compound 18 (0.07 g, 19%) as a white solid.
Melting point: 258-261 °C (decomposed)
NMR(CDCl₃, δ, ppm): 2.46(dd, 1H, J=10Hz, 14 Hz), 2.82 (dd, 1H, J=2Hz, 14Hz), 3.95(s, 3H), 4.24-4.15(m, 1H), 4.32(dd, 1H, J=6Hz, 15Hz), 4.46, 4.32(dd, 1H, J=6Hz, 15Hz), 4.67(t, 1H, J=9Hz), 4.83(dd, 1H, J=3Hz, 9Hz), 6.65-6.55(m, 1H), 6.85(d, J=8Hz, 1H), 7.26-7.21(m, 1H), 7.37(d, J=8Hz, 1H), 7.65-7.55(m, 1H), 8.57-8.38(m, 2H), 8.56(s, 2H)
IR(KBr, cm⁻¹): 3310, 3224, 1662, 1645, 1489, 1284
MASS(m/e): 486(M⁺-1)

### Example 19

### (±)-4-[(3,5-Dichloro-4-pyridyl)aminocarbonyl]-7-methoxy-3-[(3-pyridyl)aminocarbonyl]methyl-2,3-dihydrobenzofuran (Compound 19)

Substantially the same procedure as in Example 13 was repeated using Compound 9 (0.30 g) obtained in Example 9 and 3-aminopyridine to give Compound 19 (0.18 g, 51%) as a white solid.
Melting point: 267 °C (decomposed)
NMR(CDCl₃, δ, ppm): 2.55(dd, 1H, J=11Hz, 15 Hz), 3.01 (dd, 1H, J=2Hz, 15Hz), 3.94 (s, 3H), 4.30-4.21(m, 1H), 4.67(t, 1H, J=9Hz), 4.80(dd, 1H, J=3Hz, 9Hz), 6.87(d, 1H, J=9Hz), 7.24(dd, 1H, J=5Hz, 8Hz), 7.45(d, 1H, J=9Hz), 8.22-8.10(m, 2H), 8.50 (d, 1H, J=2Hz), 8.56(s, 2H)
IR(KBr, cm⁻¹): 3300(br), 1668, 1664, 1483, 1278
MASS(m/e): 472(M⁺-1)

### Example 20

### (±)-4-[(3,5-Dichloro-4-pyridyl)aminocarbonyl]-7-methoxy-3-[(2-pyrimidyl)aminocarbonyl]methyl-2,3-dihydrobenzofuran (Compound 20)

Substantially the same procedure as in Example 13 was repeated using Compound 9 (0.30 g) obtained in Example 9 and 2-aminopyrimidine to give Compound 19 (0.11 g, 31%) as a white solid.
Melting point: 259-261 °C
NMR(DMSO-d₆, δ, ppm): 2.82(dd, 1H, J=11Hz, 18Hz), 3.16-3.10(m, 1H), 3.87(s, 3H), 4.30-4.26(m, 1H), 4.38(dd, 1H, J=3Hz, 9Hz), 4.65(t, 1H, J=9Hz), 7.05(d, J=9Hz, 1H), 7.14(t, 1H, J=5Hz), 7.48(d, J=9Hz, 1H), 8.61(d, J=5Hz, 2H), 8.72(s, 2H), 10.49(s, 1H), 10.60(s, 1H)
IR(KBr, cm⁻¹): 3200(br), 1668, 1579, 1488, 1278
MASS(m/e): 474(M⁺)

### Example 21

### (±)-4-[13,5-Dichloro-4-pyridyl)aminocarbonyl]-7-methoxy-3-[(4-phenyl-1-piperazinyl)carbonyl]methyl-2,3-dihydrobenzofuran (Compound 21)

Substantially the same procedure as in Example 13 was repeated using Compound 9 (0.30 g) obtained in Example 9 and 1-phenylpiperazine to give Compound 21 (0.21 g, 51%) as a white solid.
Melting point: 224 °C
NMR(CDCl₃, δ, ppm): 2.54(dd, 1H, J=11Hz, 16 Hz), 3.21-3.11(m, 5H), 3.75-3.57(m, 4H), 3.96(s, 3H), 4.36-4.26(m, 1H), 4.66(dd, 1H, J=3Hz, 9Hz), 4.79 (t, 1H, J=9Hz), 6.92-6.86(m, 4H), 7.31-7.25(m, 3H), 7.37(d, J=9Hz, 1H), 7.68(s, 1H), 8.57(s, 2H)
IR(KBr, cm⁻¹): 3232, 1662, 1647, 1486, 1286
MASS(m/e): 542 (M⁺+1)

**Elemental analysis: C₂₇H₂₆N₄O₄Cl₂**

| | | | |
|---|---|---|---|
| Found (%) | C:59.83, | H:4.82, | N:10.20 |
| Calcd.(%) | C:59.90, | H:4.84, | N:10.35 |

### Example 22

### (±)-4-[(3,5-Dichloro-4-pyridyl)aminocarbonyl]-7-methoxy-3-[(1,2,3,4-tetrahydroisoquinolinyl)carbonyl]-methyl-2,3-dihydrobenzofuran (Compound 22)

Substantially the same procedure as in Example 13 was repeated using Compound 9 (0.30 g) obtained in Example 9 and 1,2,3,4-tetrahydroisoquinoline to give Compound 22 (0.32 g, 84%) as a white solid.
Melting point: 211-213 °C
NMR(CDCl₃, δ, ppm) : 2.63-2.51(m, 1H), 2.83(dt, 2H, J=6Hz, 5Hz), 3.22(d, J=16Hz, 1H), 3.92-3.60(m, 2H), 3.96(s, 3H), 4.37-4.28(m, 1H), 4.57(s, 1H), 4.82-4.61(m, 3H), 6.36(dd, 1H, J=3Hz, 9Hz), 7.23-7.10(m, 4H), 7.38(dd, 1H, J=2Hz, 9Hz), 7.76 (s, 1H), 8.56(s, 2H)
IR(KBr, cm⁻¹): 3188, 1659, 1635, 1487, 1282
MASS(m/e): 511(M⁺-1)

**Elemental analysis: C₂₆H₂₃N₃O₄Cl₂**

| | | | |
|---|---|---|---|
| Found (%) | C:60.95, | H:4.52, | N:8.20 |
| Calcd.(%) | C:60.67, | H:4.58, | N:8.00 |

### Example 23

### (±)-4-[(3,5-Dichloro-4-pyridyl)aminocarbonyl]-7-methoxy-3-phenethyloxycarbonyl)methyl-2,3-dihydrobenzofuran (Compound 23)

Substantially the same procedure as in Example 13 was repeated using Compound 9 (0.30 g) obtained in Example 9 and phenethyl alcohol to give Compound 23 (0.07 g, 57%) as a white solid.
Melting point: 194 °C
NMR(CDCl₃, δ, ppm): 2.50(dd, 1H, J=11Hz, 17Hz), 2.90 (t, 2H, J=7Hz), 3.03(dd, 1H, J=2Hz, 17Hz), 3.95 (s, 3H), 4.27(t, 2H, J=7Hz), 4.33-4.22(m, 1H), 4.46(dd, 1H, J=3Hz, 9Hz), 4.64(t, 1H, J=9Hz), 6.86(d, 1H, J=9Hz), 7.31-7.17(m, 5H), 7.34(d, 1H, J=8Hz), 7.62(s, 1H), 8.57(s, 2H)
IR(KBr, cm⁻¹): 3203, 1726, 1660, 1487, 1286
MASS(m/e): 50 (M⁺+1)

**Elemental analysis: C₂₅H₂₂N₂O₅Cl₂**

| | | | |
|---|---|---|---|
| Found (%) | C:59.89, | H:4.42, | N:5.59 |
| Calcd.(%) | C:59.75, | H:4.15, | N:5.48 |

### Example 24

### (±)-4-[(3,5-Dichloro-4-pyridyl)aminocarbonyl]-7-methoxy-3-[(2-pyridylmethyl)aminocarbonyl]methyl-2,3-dihydrobenzofuran (Compound 24)

Substantially the same procedure as in Example 13 was repeated using Compound 9 (0.30 g) obtained in Example 9 and 2-pyridylmethylamine to give Compound 24 (0.21 g, 56%) as a white solid.
Melting point: 255 -258 °C
NMR(DMSO-d₆, δ, ppm): 2.35 (dd, 1H, J=11Hz, 15Hz), 2.79(dd, 1H, J=3Hz, 15Hz), 3.86(s, 3H), 4.24-4.13(m, 1H), 4.36(d, 2H, J=6Hz), 4.43-4.29(m, 1H), 4.56(t, 1H, J=9Hz), 7.04(d, 1H, J=9Hz), 7.28-7.24(m, 2H), 7.47(d, 1H, J=9Hz), 7.75(dt, 1H, J=2Hz, 8Hz), 8.51-8.43(m, 2H), 8.75(s, 2H), 10.48(s, 1H)
IR(KBr, cm⁻¹): 3350, 3320, 1659, 1635, 1552, 1486, 1282
MASS(m/e): 486(M⁺)

**Elemental analysis: C₂₃H₂₀N₄O₄Cl₂**

| | | | |
|---|---|---|---|
| Found (%) | C:56.69, | H:4.14, | N:11.50 |
| Calcd.(%) | C:56.54, | H:4.02, | N:11.33 |

### Example 25

### (±)-3-(Benzylaminocarbonyl)methyl-4-[(3,5-dichloro-4-pyridyl)aminocarbonyl]-7-methoxy-2,3-dihydrobenzofuran (Compound 25)

Substantially the same procedure as in Example 13 was repeated using Compound 9 (0.10 g) obtained in Example 9 and benzylamine to give Compound 25 (0.08 g, 65%) as a white solid.
Melting point: 284-286 °C
NMR(DMSO-d₆, δ, ppm): 2.30(dd, 1H, J=11Hz, 15Hz), 2.76(dd, 1H, J=3Hz, 15Hz), 3.86(s, 3H), 4.28-4.17(m, 1H), 4.27(d, 2H, J=7Hz), 4.34(dd, 1H, J=3Hz, 9Hz), 4.57(t, 1H, J=9Hz), 7.04(d, 1H, J=9Hz), 7.35-7.20(m, 5H), 7.47(d, 1H, J=9Hz), 8.36(t, 1H, J=6Hz), 8.75(s, 2H), 10.47(brs, 1H)
IR(KBr, cm⁻¹): 3350, 3230, 1662, 1637, 1552, 1487, 1282
MASS(m/e): 487(M⁺)

**Elemental analysis: C₂₄H₂₁M₃O₄Cl₂**

| | | | |
|---|---|---|---|
| Found (%) | C:59.27, | H:4.35, | N:8.64 |
| Calcd. (%) | C:59.54, | H:4.36, | N:8.55 |

### Example 26

### (±)-4-[(3,5-Dichloro-4-pyridyl)aminocarbonyl]-7-methoxy-3-[(4-pyridylmethyl)aminocarbonyl]methyl-2,3-dihydrobenzofuran (Compound 26)

Substantially the same procedure as in Example 13 was repeated using Compound 9 (0.20 g) obtained in Example 9 and 4-pyridylmethylamine to give Compound 26 (0.04 g, 16%) as a white solid.
Melting point: 259-262 °C (decomposed)
NMR(DMSO-d₆, δ, ppm): 2.36(dd, 1H, J=11Hz, 16Hz), 2.80(dd, 1H, J=2Hz, 16Hz), 3.86(s, 3H), 4.23-4.16(m, 1H), 4.29(d, 2H, J=6Hz), 4.37(dd, 1H, J=3Hz, 9Hz), 4.57(t, 1H, J=9Hz), 7.22(d, 2H, J=9Hz), 7.47(d, 1H, J=9Hz), 8.50-8.42(m, 3H), 8.75(s, 2H), 10.48(s, 1H)
IR(KBr, cm⁻¹): 3327, 3205, 1654, 1641, 1551, 1481, 1288
MASS(m/e): 149

**Elemental analysis: C₂₃H₂₀N₄O₄Cl₂**

| | | | |
|---|---|---|---|
| Found (%) | C:56.69, | H:4.14, | N:11.50 |
| Calcd.(%) | C:56.39, | H:4.00, | N:11.39 |

### Example 27

### (±)-4-[(3,5-Dichloro-4-pyridyl)aminocarbonyl]-7-methoxy-3-(phenylaminocarbonyl)methyl-2,3-dihydrobenzofuran (Compound 27)

Substantially the same procedure as in Example 13 was repeated using Compound 9 (0.20 g) obtained in Example 9 and aniline to give Compound 27 (0.10 g, 42%) as a white solid.
Melting point: 296-300 °C (decomposed)
NMR(DMSO-d₆, δ, ppm): 2.54-2.50(m, 1H), 2.93(dd, 1H, J=2Hz, 14Hz), 3.87(s, 3H), 4.29-4.22(m, 1H), 4.43(dd, 1H, J=3Hz, 9Hz), 4.63(t, 1H, J=9Hz), 7.07-6.98(m, 2H), 7.27(d, 1H, J=8Hz), 7.30(d, 1H, J=8Hz), 7.49(d, 1H, J=8Hz), 7.55(d, 2H, J=8Hz), 8.74(s, 2H), 9.90(s, 1H), 10.50(s, 1H)
IR(KBr, cm⁻¹): 3350, 3142, 1657, 1651, 1547, 1491, 1290
MASS(m/e): 471(M⁺-1), 473(M⁺+1)

**Elemental analysis: C₂₃H₁₉N₃O₄Cl₂**

| | | | |
|---|---|---|---|
| Found (%) | C:58.49, | H:4.05, | N:8.90 |
| Calcd.(%) | C:58.14, | H:4.14, | N:8.62 |

### Example 28

### (±)-4-[(3,5-Dichloro-4-pyridyl)aminocarbonyl]-7-methoxy-3-[(4-methoxybenzyl)aminocarbonyl]methyl-2,3-dihydrobenzofuran (Compound 28)

Substantially the same procedure as in Example 13 was repeated using Compound 9 (0.20 g) obtained in Example 9 and 4-methoxybenzylamine to give Compound 28 (0.28 g, 85%) as a white solid.
Melting point: 269-271 °C
NMR(DMSO-d₆, δ, ppm): 2.27(dd, 1H, J=15Hz, 11Hz), 2.74(dd, 1H, J=3Hz, 15Hz), 3.72(s, 3H), 3.86(s, 3H), 4.19(d, 2H, J=5Hz), 4.23-4.12(m, 1H), 4.33 (dd, 1H, J=3Hz, 9Hz), 4.56(t, 1H, J=9Hz), 6.87(d, 2H, J=9Hz), 7.03(d, 1H, J=8Hz), 7.16(d, 1H, J=9Hz), 7.46(d, 1H, J=8Hz), 8.27(t, 1H, J=6Hz), 8.74(s, 2H), 10.47(s, 1H)
IR(KBr, cm⁻¹) : 3210, 1659, 1643, 1514, 1487, 1290
MASS(m/e): 515(M⁺), 517, 519

**Elemental analysis: C₂₅H₂₃N₃O₅Cl₂**

| | | | |
|---|---|---|---|
| Found (%) | C:58.15, | H:4.49, | N:8.14 |
| Calcd.(%) | C:57.97, | H:4.51, | N:8.03 |

### Example 29

### (±)-4-[(3,5-Dichloro-4-pyridyl)aminocarbonyl]-3-[(4-fluorobenzyl)aminocarbonyl]methyl-7-methoxy-2,3-dihydrobenzofuran (Compound 29)

Substantially the same procedure as in Example 13 was repeated using Compound 9 (0.20 g) obtained in Example 9 and 4-fluorobenzylamine to give Compound 29 (0.13 g, 51%) as a white solid.
Melting point: 287 °C
NMR(DMSO-d₆, δ, ppm): 2.28(dd, 1H, J=15Hz, 11Hz), 2.80(dd, 1H, J=3Hz, 15Hz), 3.85(s, 3H), 4.25-4.18(m, 1H), 4.23(d, 2H, J=6Hz), 4.33(dd, 1H, J=3Hz, 9Hz), 4.55(1, 1H, J=9Hz), 7.02(d, 1H, J=9Hz), 7.12(t, 2H, J=9Hz), 7.29-7.23(m, 2H), 7.45(d, 1H, J=9Hz), 8.40(t, 1H, J=6Hz), 8.73(s, 2H), 10.45(s, 1H)
IR(KBr, cm⁻¹): 3368, 3145, 1662, 1647, 1510, 1491, 1286
MASS(m/e): 63

**Elemental analysis: C₂₄H₂₀N₃O₄FCl₂**

| | | | |
|---|---|---|---|
| Found (%) | C:57.16, | H:4.00, | N:8.33 |
| Calcd.(%) | C:57.20, | H:4.99, | N:8.33 |

### Example 30

### (±)-3-[(4-Chlorobenzyl)aminocarbonyl)methyl-4-[(3,5-dichloro-4-pyridyl)aminocarbonyl]-7-methoxy-2,3-dihydrobenzofuran (Compound 30)

Substantially the same procedure as in Example 13 was repeated using Compound 9 (0.20 g) obtained in Example 9 and 4-chlorobenzylamine to give Compound 30 (0.15 g, 58%) as a white solid.
Melting point: 283-286 °C
NMR(DMSO-d₆, δ, ppm): 2.30(dd, 1H, J=16Hz, 12Hz), 2.76(dd, 1H, J=2Hz, 16Hz), 3.34(s, 3H), 4.26-4.20(m, 1H), 4.25(d, 2H, J=6Hz), 4.34(dd, 1H, J=3Hz, 9Hz), 4.56(t, 1H, J=9Hz), 7.04(d, 1H, J=9Hz), 7.25(d, 2H, J=8Hz), 7.37 (d, 2H, J=8Hz), 7.47(d, 1H, J=9Hz), 8.38(t, 1H, J=6Hz), 8.75(s, 2H), 10.48(s, 1H)
IR(KBr, cm⁻¹): 3307, 3296, 1660, 1647, 1489, 1286 MASS (m/e) : 520(M⁺)

**Elemental analysis: C₂₄H₂₀N₃O₄Cl₃**

| | | | |
|---|---|---|---|
| Found (%) | C:55.35, | H:3.87, | N:8.07 |
| Calcd.(%) | C:55.22, | H:3.77, | N:7.98 |

### Example 31

### (±)-3-[(2-Chlorobenzyl)aminocarbonyl]methyl-9-[(3,5-dichloro-4-pyridyl)aminocarbonyl]-7-methoxy-2,3-dihydrobenzofuran (Compound 31)

Substantially the same procedure as in Example 13 was repeated using Compound 9 (0.20 g) obtained in Example 9 and 2-chlorobenzylamine to give Compound 31 (0.15 g, 58%) as a white solid.
Melting point: 288-289 °C
NMR(DMSO-d₆, δ, ppm): 2.36(dd, 1H, J=15Hz, 12Hz), 2.80(dd, 1H, J=3Hz, 16Hz), 3.86(s, 3H), 4.22-4.17(m, 1H), 4.38-4.30(m, 3H), 4.57(t, 1H, J=9Hz), 7.39(d, 1H, J=8Hz), 7.31-7.27(m, 3H), 7.43-7.42(m, 1H), 7.47(d, 1H, J=8Hz), 8.35(brs, 1H), 8.74(s, 2H)
IR(KBr, cm⁻¹): 3350, 1660, 1651, 1547, 1493, 1286
MASS (m/e) : 519(M⁺-1), 521(M⁺+1)

**Elemental analysis: C₂₄H₂₀N₃O₄Cl₃**

| | | | |
|---|---|---|---|
| Found (%) | C:55.35, | H:3.87, | N:8.07 |
| Calcd.(%) | C:55.42, | H:3.86, | N:8.02 |

### Example 32

### (±)-4-[(3,5-Dichloro-4-pyridyl)aminocarbonyl]-3-[(3,5-dichloro-4-pyridyl)aminocarbonyl]methyl-7-methoxy-2,3-dihydrobenzofuran (Compound 32)

Substantially the same procedure as in Example 13 was repeated using Compound 9 (0.30 g) obtained in Example 9 and 4-amino-3,5-dichloropyridine to give Compound 32 (0.06 g, 17%) as a white solid.
Melting point: >300 °C
NMR(DMSO-d₆, δ, ppm): 2.61-2.55(m, 1H), 3.07-3.01(m, 1H), 3.87(s, 3H), 4.28-4.25(m, 1H), 4.40(dd, 1H, J=2Hz, 8Hz), 4.55(t, 1H, J=8Hz), 7.07 (d, 1H, J=9Hz), 7.50 (d, 1H, J=9Hz), 8.68(s, 2H), 8.75(s, 2H), 10.32(brs, 2H), 10.52(brs, 2H)
IR(KBr, cm⁻¹): 3260 (br), 1684, 1653, 1487, 1282
MASS(m/e): 542(M⁺)

**Elemental analysis: C₂₂H₁₆N₄O₄Cl₂**

| | | | |
|---|---|---|---|
| Found (%) | C:48.73, | H:2.97, | N:10.33 |
| Calcd.(%) | C:48.53, | H:2.91, | N:10.12 |

### Example 33

### 4-(3,5-Dichloro-4-pyridylaminocarbonyl)-7-methoxy-benzofuran (Compound 33)

Substantially the same procedure as in Example 1 was repeated using Compound IIac (0.22 g) obtained in Reference Example 29 to give Compound 33 (0.27 g, 68%) as a white solid.
NMR(DMSO-d₆, δ, ppm): 4.05(s, 3H), 7.10(d, J=8.7Hz, 1H), 7.31(d, J=1.5Hz, 1H), 7.99(d, J=8.7Hz, 1H), 8.10(d, J=1.5Hz, 1H), 8.77(s, 2 H), 10.5(s, 1H)
MASS(m/e): 336(M⁺)
IR(KBr, cm⁻¹): 1650, 1490, 1280

**Elemental analysis: C₁₅H₁₀N₂O₃Cl₂**

| | | | |
|---|---|---|---|
| Found (%) | C:53.31, | H:2.85, | N:8.06 |
| Calcd.(%) | C:53.44, | H:2.99, | N:8.31 |

### Example 34

### 2-Cyano-4-(3,5-dichloropyridin-4-ylaminocarbonyl)-7-methoxybenzofuran (Compound 34)

Substantially the same procedure as in Example 1 was repeated using Compound IIab (0.26 g) obtained in Reference Example 28 to give Compound 34 (0.10 g, 23.9%) as a white solid.
Melting point: 246-250 °C
NMR(DMSO-d₆, δ, ppm): 4.10(s, 3H), 7.40(d, J=8.7Hz, 1H), 8.15(d, J=8.7Hz, 1H), 8.32 (s, 1H), 8.79(s, 2H), 10.7(s, 1H)
IR(KBr, cm⁻¹): 2240, 1650, 1490, 1280
MASS(m/z): 362 (M⁺)

**Elemental analysis: C₁₆H₉Cl₂N₃O₃**

| | | | |
|---|---|---|---|
| Found (%) | C:53.31, | H:2.30, | N:11.30 |
| Calcd.(%) | C:53.06, | H:2.50, | N:11.60 |

### Example 35

### 2-Benzoyl-4-[(3,5-dichloro-4-pyridyl)aminocarbonyl)]-7-methoxybenzofuran (Compound 35)

Compound 34 (0.46 g) obtained in Example 34 was suspended in tetrahydrofuran, 1.0M phenylmagnesium bromide (28.2 g) was dropwise added thereto under stirring at 0°C, and then the temperature of the mixture was slowly raised to room temperature while stirring for 3 hours. Hydrochloric acid was added thereto at 0°C followed by stirring for one hour. The mixture was extracted with ethyl acetate, the organic layer was washed with a saturated saline and dried over magnesium sulfate, and the solvent was distilled off under reduced pressure. The residue was purified by silica gel column chromatography (toluene:ethyl acetate = 4:1) and recrystallized from ethanol to give Compound 35 (0.38 g, 67.3%) as a colorless solid.
Melting point: 217 °C
NMR (DMSO-d₆, δ, ppm) : 4.11(s, 3H), 7.37(d, 1H, J=8Nz), 7.61(d, 1H, J=7Hz), 7.65(s, 1H), 7.72(d, 1H, J=7Hz), 7.97(s, 3H), 8.01(s, 3H), 8.14(d, 1H, J=8Hz), 8.76(s, 2H), 10.70(s, 1H)
IR (KBr, cm⁻¹): 3307(br), 1647, 1487, 1286, 1271
MASS(m/e): 441(M⁺)

**Elemental analysis: C₂₂H₁₄N₂O₄Cl₂**

| | | | |
|---|---|---|---|
| Found (%) | C:59.88, | H:3.20, | N:6.35 |
| Calcd.(%) | C:59.80, | H:3.18, | N:6.28 |

### Example 36

### 2-Butyl-4-(3,5-dichloropyridin-4-ylaminocarbonyl)-7-methoxybenzofuran (Compound 36)

Substantially the same procedure as in Example 1 was repeated using Compound IIad (0.47 g) obtained in Reference Example 30 to give Compound 36 (0.25 g, 34%) as a white solid.
Melting point: 160-164 °C
NMR(DMSO-d₆, δ, ppm): 0.92(t, J=8Hz, 3H), 1.28-1.47(m, 2H), 1.59-1.78(m, 2H), 2.80 (t, J=7Hz, 2H), 4.01 (s, 3H), 7.00(s, 1H), 7.04(d, J=8Hz, 1H), 7.92(d, J=8Hz, 1H), 8.75(s, 2H), 10.4(s, 1H)
MASS(m/e): 392(M⁺)
IR(KBr, cm⁻¹): 1658, 1490, 1285

**Elemental analysis: C₁₉H₁₈Cl₂N₂O₃**

| | | | |
|---|---|---|---|
| Found (%) | C:58.08, | H:4.68, | N:7.06 |
| Calcd.(%) | C:58.03, | H:4.61, | N:7.12 |

### Example 37

### 2-Benzyl-4-[(3,5-dichloro-4-pyridyl)aminocarbonyl]-7-methoxybenzofuran (Compound 37)

Substantially the same procedure as in Example 1 was repeated using Compound IIag (0.30 g) obtained in Reference Example 33 to give Compound 37 (0.25 g, 77%).
Melting point: 141-142 °C
NMR(CDCl₃, δ, ppm): 4.17(s, 2H), 4.41(s, 3H), 6.43(s, 1H), 7.25(d, 1H, J=8Hz), 7.64-7.29(m, 5H), 8.07 (d, 1H, J=8Hz), 8.91(s, 2H), 9.97(brs, 1H)
IR(KBr, cm⁻¹): 3298(br), 1674, 1547, 1491, 1477, 1271
MASS (m/e) : 306(M⁺)

**Elemental analysis: C₂₂H₁₆N₂O₃Cl₂**

| | | | |
|---|---|---|---|
| Found (%) | C:61.84, | H:3.77, | N:6.56 |
| Calcd.(%) | C:61.79, | H:3.75, | N:6.48 |

### Example 38

### 4-(3,5-Dichloro-4-pyridylaminocarbonyl)-7-methoxy-2-(4-pyridyl)benzofuran (Compound 38)

Substantially the same procedure as in Example 1 was repeated using Compound IIae (0.21 g) obtained in Reference Example 31 to give Compound 38 (0.141 g, 50.1%) as a white solid.
Melting point: 289-290 °C
NMR(DMSO-d₆, δ, ppm): 4.10(s, 3H), 7.20(d, J=9Hz, 1H), 7.90(d, J=7Hz, 2H), 8.07(d, J=9Hz, 1H), 8.09(s, 1H), 8.69(d, J=7Hz, 2H), 8.80(s, 2H), 10.58(bs, 1H)
IR(KBr, cm⁻¹): 3300(br), 1650, 1490, 1460, 1290
MASS(m/e): 417, 415, 413(M⁺), 253, 252

**Elemental analysis: C₂₀H₁₃N₃O₃Cl₂**

| | | | |
|---|---|---|---|
| Found (%) | C:57.74, | H:3.15, | N:9.91 |
| Calcd.(%) | C:57.97, | H:3.16, | N:10.15. |

### Example 39

### 7-Methoxy-2-(4-pyridyl)-4-(4-pyridylaminocarbonyl)-benzofuran·2 hydrochloride (Compound 39)

Substantially the same procedure as in Example 6 was repeated using Compound IIae (3.0 g) obtained in Reference Example 31 to give 7-methoxy-2-(4-pyridyl)-4-(4-pyridylaminocarbonyl)benzofuran (1.45 g, 42.8%) as a white solid. Then, substantially the same procedure as in Example 17 was repeated using the above-obtained product to give Compound 39.
Melting point: 214-217 °C
NMR (DMSO-d₆, δ, ppm): 4.11(s, 3H), 7.29(d, J=9Hz, 1H), 8.39(d, J=9Hz, 1H), 8.49(d, J=7Hz, 2H), 8.52(d, J=6Hz, 2H), 8.55(s, 1H), 8.80(d, J=7Hz, 2H), 8.96(d, J=6Hz, 2H), 12.05(bs, 1H)
IR(KBr, cm⁻¹): 3400(br), 1685, 1635, 1610, 1505, 1270
MASS(m/e): 345(M⁺), 252

**Elemental analysis: C₂₀H₁₅N₃O₃·2.0HCl·3.0H₂O**

| | | | |
|---|---|---|---|
| Found (%) | C:50.87, | H:4.78, | N:8.76 |
| Calcd. (%) | C:50.86, | H:4.91, | N:8.90 |

### Example 40

### 4-(3,5-Dichloro-4-pyridylaminocarbonyl)-7-methoxy-2-(2-pyridyl)benzofuran (Compound 40)

Substantially the same procedure as in Example 1 was repeated using Compound IIaf (0.40 g) obtained in Reference Example 32 to give Compound 40 (0.162 g, 29.9%) as a white solid.
Melting point: 263-264 °C
NMR(DMSO-d₆, δ, ppm): 4.12(s, 3H), 7.20(d, J=9Hz, 1H), 7.44(ddd, J=2Hz, 5Hz, 7Hz, 1H), 7.93(s, 1H), 7.97(dd, 2Hz, 8Hz, 1H), 7.99(dd, J=7Hz, 8Hz, 1H), 8.02(d, J=9Hz, 1H), 8.70(d, J=5Hz, 1H), 8.78(s, 2H), 10.55(bs, 1H)
IR(KBr, cm⁻¹): 3200(br), 1650, 1590, 1500, 1290, 1280
MASS(m/e): 417, 415, 413(M⁺), 252

**Elemental analysis: C₂₀H₁₃N₃O₃Cl₂ ·0.1H₂O**

| | | | |
|---|---|---|---|
| Found (%) | C:57.66, | H:3.06, | N:9.91 |
| Calcd. (%) | C:57.74, | H:3.20, | N:10.10 |

### Example 41

### 7-Methoxy-2-(2-pyridyl)-4-(4-pyridylaminocarbonyl)-benzofuran·2 hydrochloride (Compound 41)

Substantially the same procedure as in Example 6 was repeated using Compound IIaf (4.87 g) obtained in Reference Example 32 to give 7-methoxy-2-(2-pyridyl)-4-(4-pyridylaminocarbonyl)benzofuran (4.24 g, 77.1%) as a white solid. Then, substantially the same procedure as in Example 17 was repeated using the above-obtained product to give Compound 41.
Melting point: 251-254 °C
NMR(DMSO-d₆/D₂O, δ, ppm): 4.17(s, 3H), 7.13(d, J=9Hz, 1H), 7.58(dd, J=5Hz, 7Hz, 1H), 7.9-8.1(m, 2H), 7.98(s, 1H), 8.12 (d, J=9Hz, 1H), 8.29(d, J=7Hz, 2H), 8.64(d, J=7Hz, 2H), 8.66(d, J=5Hz, 1H)
IR(KBr, cm⁻¹): 3400(br), 1685, 1625, 1610, 1505, 1280
MASS(m/e): 345(M⁺), 252

**Elemental analysis: C₂₀H₁₅N₃O₃·2.0HCl·1.9H₂O**

| | | | |
|---|---|---|---|
| Found (%) | C:52.99, | H:4.30, | N:9.10 |
| Calcd. (%) | C:53.09, | H:4.63, | N:9.29 |

### Example 42

### 4-(3,5-Dichloro-4-pyridylaminocarbonyl)-7-methoxy-3-phenylbenzofuran (Compound 42)

Substantially the same procedure as in Example 1 was repeated using Compound IIah (0.29 g) obtained in Reference Example 34 to give Compound 42 (0.34 g, 76%) as a white solid.
Melting point: 177-179 °C
NMR(CDCl₃, 5, ppm): 4.12(s, 3H), 6.95(d, J=9Hz, 1H), 7.17-7.43(m, 5H), 7.76(s, 1H), 7.89(d, J=9Hz, 1H), 8.44(s, 2H)
IR(KBr, cm⁻¹): 1495, 1669, 1402, 1279
MASS(m/e): 412(M⁺)

**Elemental analysis: C₂₁H₁₄N₂O₃Cl₂**

| | | | |
|---|---|---|---|
| Found (%) | C:60.99, | H:3.40, | N:6.56 |
| Calcd.(%) | C:61.03, | H:3.41, | N:6.78 |

### Example 43

### 3-Ethoxycarbonylmethyl-9-[(3,5-dichloro-4-pyridyl)-aminocarbonyl]-7-methoxybenzofuran (Compound 43)

Substantially the same procedure as in Example 1 was repeated using Compound IIai (0.80 g) obtained in Reference Example 35 to give Compound 43 (0.47 g, 39%) as a white solid.
Melting point: 216-218 °C
NMR(CDCl₃, δ, ppm): 1.10(t, J=7Hz, 3H), 3.91(s, 2H), 4.00(q, J=7Hz, 2H), 4.08(s, 3H), 6.85(d, J=8Hz, 1H), 7.66(s, 1H), 7.71(d, J=8Hz, 1H), 7.75(s, 1H), 8.56(s, 2H)

**Elemental analysis: C₁₉H₁₆Cl₂N₂O₅**

| | | | |
|---|---|---|---|
| Found (%) | C:54.01, | H:3.75, | N:6.45 |
| Calcd. (%) | C:53.92, | H:3.81, | N:6.62 |

### Example 44

### 3-Carboxymethyl-4-[(3,5-dichloro-4-pyridyl)aminocarbonyl]-7-methoaybenzofuran (Compound 44)

Substantially the same procedure as in Example 9 was repeated using Compound 43 (0.64 g) obtained in Reference Example 43 to give Compound 44 (0.27 g, 47%) as a white solid.
Melting point: 270-278 °C
NMR(DMSO-d₆, δ, ppm): 3.79(s, 2H), 4.02(s, 3H), 7.09 (d, J=9Hz, 1H), 7.77(d, J=9Hz, 2H), 7.97(s, 1H), 8.74(s, 2H), 10.6-10.7(brs, 1H), 12.0-12.1(brs, 1H)
Elemental analysis: C₁₇H₁₂Cl₂N₂O₅

| | | | |
|---|---|---|---|
| Found (%) | C:51.38, | H:2.95, | N:6.92 |
| Calcd. (%) | C:51.67, | H:3.06, | N:7.09 |

### Example 45

### 4-[2-(3,5-Dichloro-4-pyridyl)ethyl]-7-methoxy-2,2-dimethyl-2,3-dihydrobenzofuran (Compound 45)

### (Step A) (±)-4-[2-(3,5-Dichloro-4-pyridyl)-1-hydroxyethyl]-7-methoxy-2,2-dimethyl-2,3-dihydrobenzofuran (Compound 45a)

Under an argon atmosphere, a solution (20 ml) of 3,5-dichloro-4-methylpyridine (1.4 g) in THF was cooled to -78°C, and then a 1.69M solution of butyl lithium in hexane (6.3 ml) was dropwise added thereto, followed by stirring at the same temperature for one hour. A solution (10 ml) of Compound IIa (2.0 g) obtained in Reference Example 1 in THF was slowly and dropwise added to the mixture, followed by stirring at -78°C for 2 hours and then at 0°C for one hour. The reaction solution was poured into water and the mixture was extracted with ether. The organic layer was washed with a saturated saline and dried over anhydrous magnesium sulfate, and the solvent was distilled off under reduced pressure. The residue was purified by silica gel column chromatography (chloroform) to give Compound 45a (3.3 g, 93.4%) as colorless crystals.
Melting point: 100-104 °C
NMR (DMSO-d₆, δ, ppm) : 1.30(s, 3H), 1.38(s, 3H), 2.77 (d, J=15.8Hz, 1H), 3.04(d, J=15.8Hz, 1H), 3.04-3.11(m, 1H), 3.29-3.32(m, 1H), 3.71(s, 3H), 4.82-4.89 (m, 1H), 5.41(d, J=3.96Hz, 1H), 6.76(s, 2H), 8.55(s, 2H)
MASS(m/e): 369, 367(M⁺), 207
IR(KBr, cm⁻¹): 3396(br), 1625, 1507

### (Step B) (Compound 45)

Under an argon atmosphere, a solution (80 ml) of Compound 45a (3.0 g) obtained in Step A in methylene chloride was cooled to -78°C, and then boron trifluoride ether complex (2.0 ml) and triethylsilane (3.9 ml) were succcessively added thereto, followed by stirring at room temperature for 3 hours. The reaction solution was poured into a saturated aqueous solution of sodium bicarbonate and the mixture was extracted with chloroform. The organic layer was washed with a saturated saline and dried over anhydrous magnesium sulfate, and the solvent was distilled off under reduced pressure. The residue was purified by silica gel column chromatography (chloroform/methanol = 20/1) to give Compound 45 (1.57 g, 54.7%) as colorless crystals.
Melting point: 128-133 °C
NMR (DMSO-d₆, δ, ppm) : 1.38(s, 6H), 2.68 (t, J=7.25Hz, 2H), 2.91(s, 2H), 3.07(t, J=7.26Hz, 2H), 3.71(s, 3H), 6.54(d, J=8.25Hz, 1H), 6.72(d, J=8.25Hz, 1H), 8.58(s, 2H).
MASS(m/e): 353, 351(M⁺), 191
IR(cm⁻¹): 1623, 1593, 1499

**Elemental analysis: C₁₈H₁₉Cl₂NO₂**

| | | | |
|---|---|---|---|
| Found (%) | C:61.37, | H:5.41, | N:3.92 |
| Calcd.(%) | C:61.37, | H:5.44, | N:3.98 |

### Example 46

### 7-methoxy-2,2-dimethyl-4-[2-(4-pyridyl)ethyl]-2,3-dihydrobenzofuran (Compound 46)

### (Step A) 4-[1-Hydroxy-2-(4-pyridyl)ethyl]-7-methoxy-2,2-dimethyl-2,3-dihydrobenzofuran (Compound 46a)

Under an argon atmosphere, a solution (35 ml) of 4-methylpyridine (0.78 ml) in THF was cooled to -78°C, and then a 1.69M solution (5.17 ml) of butyl lithium in hexane was dropwise added thereto, followed by stirring at the same temperature for one hour. A solution (35 ml) of Compound IIa (1.5 g) obtained in Reference Example 1 in THF was slowly and dropwise added to the mixture, followed by stirring at -78°C for 2 hours and then at 0°C for one hour. The reaction solution was poured into water and the mixture was extracted with ether. The organic layer was washed with a saturated saline and dried over anhydrous magnesium sulfate, and the solvent was distilled off under reduced pressure. The residue was purified by silica gel column chromatography (chloroform/methanol = 20/1) to give Compound 46a (1.17 g, 53.8%) as a colorless oily substance.
NMR(DMSO-d₆, δ, ppm): 1.29(s, 3H), 1.35(s, 3H), 2.75 (d, J=15.8Hz, 1H), 2.81-2.94(m, 2H), 2.94(d, J=15.8Hz, 1H), 3.71(s, 3H), 4.68(m, 1H), 5.27(d, J=4.0Hz, 1H), 6.76(s, 2H), 7.12(d, J=5.9Hz, 2H), 8.39(d, J=5.9Hz, 2H)
MASS(m/z): 299(M⁺), 207

### (Step B) (Compound 46)

Under an argon atmosphere, a solution (7 ml) of Compound 46a (0.2 g) obtained in Step A in methylene chloride was cooled to -78°C, and then boron trifluoride ether complex (0.17 ml) and triethylsilane (0.33 ml) were successively added thereto, followed by stirring at 0°C for 2 hours. The reaction solution was poured into a saturated aqueous solution of sodium bicarbonate and the mixture was extracted with chloroform. The organic layer was washed with a saturated saline and dried over anhydrous magnesium sulfate, and the solvent was distilled off under reduced pressure. The residue was purified by silica gel column chromatography (chloroform/methanol = 20/1) to give Compound 46 (0.11 g, 58.1%) as a colorless oily substance.
NMR(DMSO-d₆, δ, ppm): 1.35(s, 6H), 2.70-2.82(m, 4H), 2.83(s, 2H), 3.70(s, 3H), 6.58(d, J=8.3Hz, 1H), 6.71(d, J=8.3Hz, 1H), 7.19(d, J=5.9Hz, 2H), 8.43 (d, J=5.9Hz, 2H)
IR(cm⁻¹): 1602, 1511, 1505, 1440
MASS(m/z): 283(M⁺), 191

**Elemental analysis: C₁₈H₂₁NO₂·0.3H₂O**

| | | | |
|---|---|---|---|
| Found (%) | C:74.87, | H:1.54, | N:4.85 |
| Calcd. (%) | C:75.03, | H:7.44, | N:4.89 |

### Example 47

### (±) 7-Methoxy-2,2-dimethyl-7-methoxy-4-[1-phenyl-2-(4-pyridyl)ethyl]-2,3-dihydrobenzofuran (Compound 47)

### (Step A) (±)-4-[1-Hydroxy-1-phenyl-2-(4-pyridyl)ethyl]-7-methoxy-2,2-dimethyl-2,3-dihydrobenzofuran (Compound 47a)

Under an argon atmosphere, a solution of 4-methylpyridine (0.83 ml) in THF (50 ml) was cooled to -78°C, and then a 1.69M solution (5.0 ml) of butyl lithium in hexane was dropwise added thereto, followed by stirring at the same temperature for one hour. A solution of Compound IIaj (2.0 g) obtained in Reference Example 36 in THF (20 ml) was slowly and dropwise added to the mixture, followed by stirring at 0°C for 2 hours. The reaction solution was poured into water and the mixture was extracted with ether. The organic layer was washed with a saturated saline and dried over anhydrous magnesium sulfate, and the solvent was distilled off under reduced pressure. The residue was purified by silica gel column chromatography (chloroform/methanol = 50/1) to give Compound 47a (0.87 g, 32.5%) as yellowish brown crystals.
Melting point: 78-81°C
NMR(DMSO-d₆: δ, ppm): 1.14(s, 3H), 1.19(s, 3H), 2.39 (d, J=16.1Hz, 1H), 2.67(d, J=16.1Hz, 1H), 3.51(s, 2H), 3.72(s, 3H), 5.70(s, 1H), 6.74(d, J=8.6Hz, 1H), 6.81(d, J=5.9Hz, 2H), 6.92(d, J=8.6Hz, 1H), 7.15-7.19(m, 5H), 8.23(d, J=5.6Hz, 2H)
IR(KBr, cm⁻¹): 3500-3000(br), 1606, 1506, 1446, 1427
MASS(m/z): 375(M⁺), 283

### (Step B) (Compound 47)

Under an argon atmosphere, a solution of Compound 47a (0.4 g) obtained in Step A in methylene chloride (3 ml) was cooled to -78°C, and then boron trifluoride ether complex (0.3 ml) and triethylsilane (0.52 ml) were successively added thereto, followed by stirring at 0°C for 2 hours. The reaction solution was poured into a saturated aqueous solution of sodium bicarbonate and the mixture was extracted with chloroform. The organic layer was washed with a saturated saline and dried over anhydrous magnesium sulfate, and the solvent was distilled off under reduced pressure. The residue was purified by silica gel column chromatography (chloroform/methanol = 30/1) to give Compound 47 (0.27 g, 56.7%) as a yellowish brown oily substance.
NMR(DMSO-d₆; δ, ppm): 1.23(s, 3H), 1.30(s, 3H), 2.75 (s, 2H), 3.30-3.34(m, 2H), 3.68(s, 3H), 4.22(t, J=8.3Hz, 1H), 6.74(d, J=8.6Hz, 1H), 6.81(d, J=8.6Hz, 1H), 7.13(d, J=5.9Hz, 2H), 7.15-7.26(m, 5H), 8.34(d, J=5.9Hz, 2H)
IR(cm⁻¹): 1622, 1598, 1503, 1435
MASS (m/z) : 359(M⁺), 267

### Example 48

### 7-Methoxy-2,2-dimethyl-4-(4-pyridylthiomethyl)-2,3-dihydrobenzofuran (Compound 48)

### (Step A) 4-Hydroxymethyl-2, 2-dimethyl-7-methoxy-2, 3-dihydrobenzofuran (Compound 48a)

Compound IIa (4.0 g) obtained in Reference Example 1 was added to a suspension of lithium aluminium hydride (0.52 g) in ether (20 ml), followed by stirring at room temperature for one hour. The reaction solution was poured into ice and the reaction mixture was adjusted to pH 3 by adding dropwise 1N hydrochloric acid (10 ml). The ether layer was separated, washed with a saturated saline, and dried over anhydrous magnesium sulfate, and the solvent was distilled off under reduced pressure. The residue was purified by silica gel column chromatography (chloroform/methanol = 60/1) to give Compound 48a (0.32 g, 79.2%) as a colorless oily substance.
NMR(DMSO-d₆, δ, ppm): 1.40(s, 6H), 2.97(s, 2H), 3.71 (s, 3H), 4.33(d, J=5.6Hz, 2H), 4.91(t, J=5.6Hz, 1H), 6.70 (d, J=8.3Hz, 1H), 6.75 (d, J=8.2Hz, 1H)
MASS(m/z): 208(M⁺)

### (Step B) (Compound 48)

Compound 48a (2.0 g) obtained in Step A was dissolved in methylene chloride (100 ml), and then diisopropylethylamine (5.0 ml) and methanesulfonyl chloride (0.8 ml) were added thereto, followed by stirring at room temperature for one hour. At the same temperature, 4-mercaptopyridine (1.4 g) was added to the reaction solution, and the mixture was stirred for 30 minutes. Water was added to the reaction solution and the mixture was extracted with methylene chloride. The organic layer was washed with a saturated saline and dried over anhydrous magnesium sulfate, and the solvent was distilled off under reduced pressure. The residue was purified by silica gel column chromatography (chloroform/methanol = 30/1) to give Compound 48 (1.4 g, 48.3%) as colorless crystals.
Melting point: 109-113 °C
NMR (DMSO-d₆; δ, ppm): 1.41(s, 6H), 3.06(s, 2H), 3.72 (s, 3H), 4.22(s, 2H), 6.74(d, J=8.4Hz, 1H), 6.80 (d, J=8.4Hz, 1H), 7.29(d, J=6.4Hz, 2H), 7.36(d, J=6.4Hz, 2H)
IR(KBr, cm⁻¹): 1572, 1506, 1450, 1439
MASS(m/z): 301(M⁺), 191

**Elemental analysis: C₁₇H₁₉NO₂S·0.1H₂O**

| | | | |
|---|---|---|---|
| Found (%) | C:67.34, | H:6.38, | N:4.62 |
| Calcd.(%) | C:67.30, | H:6.45, | N:4.93 |

### Example 49

### (±)-7-Methoxy-2,2-dimethyl-4-[1-phenyl-1-(4-pyridylthio)methyl]-2,3-dihydrobenzofuran (Compound 49)

Substantially the same procedure as in Step B of Example 48 was repeated using Compound IIaj-a (0.22 g) obtained in Step A of Reference Example 36 to give Compound 49 (0.20 g, 68.2%) as a pale-yellow oily substance.
NMR(DMSO-d₆, δ, ppm): 1.35(s, 3H), 1.40(s, 3H), 2.90 (d, J=15.3Hz, 1H), 3.13(d, J=15.8Hz, 1H), 3.32(s, 3H), 5.99(s, 1H), 6.77(d, J=8.4Hz, 1H), 6.83(d, J=8.4Hz, 1H), 7.18(d, J=7.0Hz, 2H), 7.26-7.48(m, 5H), 8.30(d, J=6.9Hz, 2H)
IR(cm⁻¹): 1600, 1574, 1506, 1439

### Example 50

### 4-[2-(3,5-Dichloro-4-pyridyl)ethyl]-2,2-diethyl-7-methoxy-2,3-dihydrobenzofuran·methanesulfonate (Compound 50)

### (Step A) (±)-4-[2-(3,5-Dichloro-4-pyridyl)-1-hydroxyethyl]-2,2-diethyl-7-methoxy-2,3-dihydrobenzofuran (Compound 50a)

Substantially the same procedure as in Step A of Example 45 was repeated using Compound IIb (9.0 g) obtained in Reference Example 2 to give Compound 50a (7.8 g, 51.1%) as a pale-yellow oily substance.
NMR(DMSO-d₆, δ, ppm): 0.77(t, J=6.9Hz, 3H), 0.85(t, J=7.4Hz, 3H), 1.54-1.58 (m, 2H), 1.64(q, J=7.4Hz, 2H), 2.73(d, J=15.8Hz, 1H), 3.00(d, J=16.3Hz, 1H), 3.06-3.13(m, 1H), 3.25-3.30(m, 1H), 3.72(s, 3H), 4.86-4.91(m, 1H), 5.40 (d, J=4.0Hz, 1H), 6.73(s, 2H), 8.54(s, 2H)
MASS(m/e): 397, 395(M⁺), 235

### (Step B) (Compound 50)

Substantially the same procedure as in Step B of Example 45 was repeated using Compound 50a (4.6 g) obtained in Step A to give 4-[2-(3,5-dichloro-4-pyridyl)ethyl]-2,2-diethyl-7-methoxy-2,3-dihydrobenzofuran (2.6 g, 59.6%) as a colorless oily substance. The obtained colorless oily substance was dissolved in diethyl ether and methanesulfonic acid was added thereto. The precipitated crystals were collected by filtration, washed with diethyl ether, and dried to give Compound 50.
Melting point: 87-90°C
NMR(DMSO-d₆, δ, ppm): 0.83(t, d=7.4Hz, 6H), 1.64(q, d=7.4Hz, 4H), 2.49(s, 3H), 2.70(t, J=8.4Hz, 2H), 2.87(s, 2H), 3.08(t, J=8.4Hz, 2H), 3.71(s, 3H), 6.50(d, J=8.4Hz, 1H), 6.70(d, J=8.4Hz, 1H), 8.58 (s, 2H)
MASS(m/e): 381, 379(M⁺), 219
IR(cm⁻¹): 2600-2200(br), 1506

**Elemental analysis: C₂₀H₂₃Cl₂NO₂·CH₃SO₃H**

| | | | |
|---|---|---|---|
| Found (%) | C:46.39, | H:5.54, | M:2.41 |
| Calcd. (%) | C:46.15, | H:5.46, | N:2.45 |

MASS(m/z): 377(M⁺), 267

### Example 51

### 2,2-Diethyl-7-methoxy-4-[2-(4-pyridyl)ethyl]-2,3-dihydrobenzofuran-hydrochloride (Compound 51)

### (Step A) (±)-2,2-Diethyl-4-[1-hydroxy-2-(4-pyridyl)ethyl]-7-methoxy-2,3-dihydrobenzofuran (Compound 51a)

Substantially the same procedure as in Step A of Example 46 was repeated using Compound IIb (20 g) obtained in Reference Example 2 to give Compound 51a (27.6 g, 98.7%) as a colorless oily substance.
NMR (DMSO-d₆, δ, ppm): 0.74-0.86(m, 6H), 1.51-1.66(m, 4H), 2.71(d, J=15.8Hz, 1H), 2.79-2.96(m, 3H), 3.72(s, 3H), 4.71(m, 1H), 5.27(d, J=4.45Hz, 1H), 6.74(s, 2H), 7.12(d, J=5.9Hz, 2H), 8.39(d, J=5.9Hz, 2H)
MASS(m/e): 327(M⁺), 235

### (Step B) (Compound 51)

Substantially the same procedure as in Step B of Example 46 was repeated using Compound 51a (23 g) obtained in Step A to give 2,2-diethyl-7-methoxy-4-[2-(4-pyridyl)-ethyl]-2,3-dihydrobenzofuran (8.46 g, 38.6%) as a colorless oily substance. The obtained colorless oily substance was dissolved in ethyl acetate and a hydrochloric acid-ethyl acetate solution was added thereto. The precipitated crystals were collected by filtration, washed with ethyl acetate, and dried to give Compound 51.
Melting point: 189-192 °C
NMR (DMSO-d₆, δ, ppm): 0.83 (t, J=7.4Hz, 6H), 1.63 (q, J=7.4Hz, 4H), 2.84(t, J=6.9Hz, 2H), 2.87(s, 2H), 3.13(t, J=6.9Hz, 2H), 3.71(s, 3H), 6.54(d, J=8.4Hz, 1H), 6.69(d, J=8.4Hz, 1H), 7.89(d, J=6.4Hz, 2H), 8.80(d, J=6.4Hz, 2H)
MASS(m/e): 312(M⁺), 220
IR(cm⁻¹): 2970, 1635, 1593, 1508

**Elemental analysis: C₂₀H₂₅NO₂·HCl**

| | | | |
|---|---|---|---|
| Found (%) | C:69.06, | H:7.69, | N:4.00 |
| Calcd. (%) | C:69.05, | H:7.53, | N:4.03 |

### Example 52

### 4-[2-(3,5-Dichloro-9-pyridyl)ethyl]-7-methoxy-spiro[2,3-dihydrobenzofuran-2,1'-cyclopentane] methanesulfonate (Compound 52)

### (Step A) (±)-4-[2-(3,5-Dichloro-4-pyridyl)-1-hydroxyethyl]-7-methoxy-spiro[2,3-dihydrobenzofuran-2,1'-cyclopentane] (Compound 52a)

Substantially the same procedure as in Step A of Example 45 was repeated using Compound IIc (8.0 g) obtained in Reference Example 3 to give Compound 52a (7.0 g, 51.4%) as a colorless oily substance.
NMR(DMSO-d₆, δ, ppm) : 1.57-1.91(m, 8H), 2.93(d, J=16.2Hz, 1H), 3.06-3.13(m, 1H), 3.20(d, J=16.2Hz, 1H), 3.24-3.30(m, 1H), 3.32(s, 3H), 4.84-4.90(m, 1H), 5.40(d, J=3.6Hz, 1H), 6.74(s, 2H), 8.54(s, 2H)
MASS(m/e): 395, 393(M⁺)

### (Step B) (Compound 52)

Substantially the same procedure as in Step B of Example 45 was repeated using Compound 52a (2.8 g) obtained in Step A to give 4-[2-(3,5-dichloro-9-pyridyl)ethyl]-7-methoxy-spiro[2,3-dihydrobenzofuran-2,1'-cyclopentane] (1.1 g, 40%) as a pale-yellow oily substance. The obtained colorless oily substance was dissolved in diethyl ether and methanesulfonic acid was added thereto. The precipitated crystals were collected by filtration, washed with diethyl ether, and dried to give Compound 52.
Melting point: 130-133 °C
NMR(DMSO-d₆, δ, ppm) : 1.67-1.91(m, 8H), 2.42 (s, 3H), 2.69(t, J=7.3Hz, 2H), 3.4-3.09(m, 4H), 3.71(s, 3H), 6.54(d, J=8.3Hz, 1H), 6.71(d, J=8.3Hz, 1H), 8.58(s, 2H)
MASS(m/e): 379, 377(M⁺), 217
IR(cm⁻¹): 2950(br), 1621, 1595, 1506

**Elemental analysis: C₂₀H₂₁Cl₂NO₂·CH₃SO₃H**

| | | | |
|---|---|---|---|
| Found (%) | C:53.09, | H:5.42, | N:2.92 |
| Calcd. (%) | C:53.17, | H:5.31, | N:2.95 |

### Example 53

### 7-Methoxy-4-[2-(4-pyridyl)ethyl]-spiro[2,3-dihydrobenzofuran-2,1'-cyclopentane]·hydrochloride (Compound 53)

### (Step A) (±)-4-[1-Hydroxy-2-(4-pyridyl)ethyl-7-methoxy-spiro[2,3-dihydrobenzofuran-2,1'-cyclopentane] (Compound 53a)

Substantially the same procedure as in Step A of Example 46 was repeated using Compound IIc (3.3 g) obtained in Reference Example 3 to give Compound 53a (1.3 g, 29%) as a colorless oily substance.
NMR(DMSO-d₆, δ, ppm): 1.59-1.88(m, 8H), 2.78-2.96(m, 3H), 3.10(d, J=15.8Nz, 1H), 3.71(s, 3H), 4.70(q, J=4.3Hz, 1H), 5.26(d, J=4.3Hz, 1H), 6.75(s, 2H), 7.13(d, J=5.6Hz, 2H), 8.40(d, J=5.6Hz, 2H)
MASS(m/z): 325(M⁺), 233

### (Step B) (Compound 53)

Substantially the same procedure as in Step B of Example 46 was repeated using Compound 53a (0.5 g) obtained in Step A to give 7-methoxy-4-[2-(4-pyridyl)ethyl]-spiro[2,3-dihydrobenzofuran-2,1'-cyclopentane] (0.037 g, 7.8%) as a colorless oily substance. The obtained colorless oily substance was dissolved in ethyl acetate and a hydrochloric acid-ethyl acetate solution was added thereto. The precipitated crystals were collected by filtration, washed with ethyl acetate, and dried to give Compound 53.
Melting point: 167-169 °C
NMR(DMSO-d₆, δ, ppm): 1.68-1.79(m, 6H), 1.84-1.92(m, 2H), 2.83(t, J=7.9Hz, 2H), 3.08(s, 2H), 3.11(t, J=7.9Hz, 2H), 3.70(s, 3H), 6.56(d, J=8.4Hz, 1H), 6.70 (d, J= 8.4Hz, 1H), 7.86 (d, J=6.4Hz, 2H), 8.78(d, J=6.9Hz, 2H)
MASS (m/e) : 309(M⁺), 217
IR(cm⁻¹): 1635, 1507

**Elemental analysis: C₂₀H₂₃NO₂·HCl·0.3H₂O**

| | | | |
|---|---|---|---|
| Found (%) | C:68.48, | H:6.97, | N:3.91 |
| Calcd. (%) | C:68.39, | H:7.06, | N:3.99 |

### Example 54

### 4-[2-(3,5-Dichloro-9-pyridyl)ethyl]-7-methoxy-spiro[2,3-dihydrobenzofuran-2,1'-cyclohexane]· methanesulfonate (Compound 54)

### (Step A) (±)-9-[2-(3,5-Dichloro-4-pyridyl)-1-hydroxyethyl]-7-methoxy-spiro[2,3-dihydrobenzofuran-2,1'-cyclohexane] (Compound 54a)

Substantially the same procedure as in Step A of Example 45 was repeated using Compound IId (6.0 g) obtained in Reference Example 4 to give Compound 54a (9.3 g, 85%) as a colorless oily substance.
Melting point: 104-108 °C
NMR(DMSO-d₆, δ, ppm): 1.41(brs, 5H), 1.48-1.60(m, 5H), 2.66(d, J=15.7Hz, 1H), 2.98(d, J=15.8Hz, 1H), 3.06-3.13(m, 1H), 3.25-3.30(m, 1H), 3.73(s, 3H), 4.84-4.90(m, 1H), 5.41(d, J=3.9Hz, 1H), 6.74(s, 2H), 8.54(s, 2H)
MASS(m/e): 409, 407(M⁺), 247

### (Step B) (Compound 54)

Substantially the same procedure as in Step B of Example 45 was repeated using Compound 54a (5.5 g) obtained in Step A to give 4-[2-(3,5-dichloro-4-pyridyl)ethyl]-7-methoxy-spiro[2,3-dihydrobenzofuran-2,1'-cyclohexane] (2.7 g, 51%) as a pale-yellow oily substance. The obtained colorless oily substance was dissolved in diethyl ether and methanesulfonic acid was added thereto. The precipitated crystals were collected by filtration, washed with diethyl ether, and dried to give Compound 54.
Melting point: 91-94°C
NMR (DMSO-d₆, δ, ppm) : 1.42 (brs, 4H), 1.53-1.65 (m, 6H), 1.42(s, 3H), 2.70(t, J=8.4Hz, 1H), 2.84(s, 2H), 3.08(t, J=8.4Hz, 1H), 3.72(s, 3H), 6.53(d, J=8.4Hz, 1H), 6.71(d, J=8.4Hz, 1H), 8.58(s, 2H)
MASS(m/e): 393, 391(M⁺), 231
IR(cm⁻¹): 2930(br), 1506

**Elemental analysis: C₂₁H₂₃Cl₂NO₂·1.5CH₃SO₃H**

| | | | |
|---|---|---|---|
| Found (%) | C:50.65, | H:5.53, | N:2.55 |
| Calcd. (%) | C:50.37, | H:5.45, | N:2.61 |

### Example 55

### 7-Methoxy-4-[2-(4-pyridyl)ethyl]-spiro[2,3-dihydrobenzofuran-2,1'-cyclohexane]·hydrochloride (Compound 55)

### (Step A) (±)-4-[2-Hydroxy-2-(4-pyridyl)ethyl]-7-methoxy-spiro[2,3-dihydrobenzofuran-2,1'-cyclohexane] (Compound 55a)

Substantially the same procedure as in Step A of Example 46 was repeated using Compound IId (50 g) obtained in Reference Example 4 to give Compound 55a (64.3 g, 93:3%) as a colorless oily substance.
NMR(DMSO-d₆, δ, ppm): 1.40-1.76(m, 10H), 2.65(d, J=15.8Hz, 1H), 2.77-2.96(m, 3H), 3.72(s, 3H), 4.66-4.73(m, 1H), 5.25(d, J=4.0Hz, 1H), 6.75(s, 2H), 7.11(dd, J=1.5, 4.5Hz, 2H), 8.38(dd, J=1.5, 4.5Hz, 2H)
MASS (m/e) : 339(M⁺)

### (Step B) (Compound 55)

Substantially the same procedure as in Step B of Example 46 was repeated using Compound 55a (30 g) obtained in Step A to give 7-methoxy-4-[2-(4-pyridyl)ethyl]-spiro[2,3-dihydrobenzofuran-2,1'-cyclohexane] (5.6 g, 20%) as a colorless oily substance. The obtained colorless oily substance was dissolved in ethyl acetate and a hydrochloric acid-ethyl acetate solution was added thereto. The precipitated crystals were collected by filtration, washed with ethyl acetate, and dried to give Compound 55.
Melting point: 176-179 °C
NMR(DMSO-d₆, δ, ppm): 1.43-1.53(m, 4H), 1.58-1.64(m, 6H), 2.81-2.85(m, 4H), 3.13(t, J=7.9Hz, 2H), 3.71(s, 3H), 6.55(d, J=8.4Hz, 1H), 6.70(d, J=8.4Hz, 1H), 7.89(d, J=6.4Hz, 2H), 8.81(d, J=6.9Hz, 2H)
MASS(m/e): 323(M⁺), 231
IR(cm⁻¹): 1634, 1506, 1437

**Elemental analysis: C₂₁H₂₅NO₂·HCl**

| | | | |
|---|---|---|---|
| Found (%) | C:69.97, | H:7.42, | N:3.81 |
| Calcd. (%) | C:70.08, | H:7.28, | N:3.89 |

### Example 56

### (±)-4-[2-(3,5-Dichloro-4-pyridyl)ethyl]-7-methoxy-3-methyl-2,3-dihydrobenzofuran (Compound 56)

### (Step A) 4-[2-(3,5-Dichloro-4-pyridyl)-1-hydroxyethyl]-7-methoxy-3-methyl-2,3-dihydrobenzofuran (Compound 56a) (a mixture of diastereomers)

Under an argon atmosphere, a solution (25 ml) of 3,5-dichloro-4-methylpyridine (1.1 g) in THF was cooled to -78°C, and then a 1.69M solution (4.9 ml) of butyl lithium in hexane was dropwise added to the solution, followed by stirring at the same temperature for one hour. A solution (25 ml) of Compound IIe (1.5 g) obtained in Reference Example 5 in THF was slowly and dropwise added to the mixture, followed by stirring at -78°C for one hour and then at 0°C for one hour. The reaction solution was poured into water and the mixture was extracted with ether. The organic layer was washed with a saturated saline and dried over anhydrous magnesium sulfate, and the solvent was distilled off under reduced pressure. The residue was purified by silica gel column chromatography (chloroform/methanol = 50/1) to give Compound 56a (2.35 g, 85.0%) as a colorless oily substance.
NMR(DMSO-d₆, δ, ppm): (main product) 1.22(d, J=6.93Hz, 3H), 3.10(d, J=4.95Hz, 1H), 3.24-3.32(m, 1H), 3.75(s, 3H), 4.13-4.14(m, 1H), 4.34(t, J=8.25Hz, 1H), 4.99-5.06(m, 1H), 5.39(d, J=5.28Hz, 1H), 6.86(d, J=8.35Hz, 1H), 7.00(d, J=8.58Hz, 1H), 8.57(s, 2H). (by-product) 1.22(d, J=6.93Hz, 3H), 3.05(d, J=4.95Hz, 1H), 3.24-3.32(m, 1H), 3.75(s, 3H), 4.16-4.17(m, 1H), 4.44(t, J=8.25Hz, 1H), 4.94-4.99(m, 1H), 5.28(d, J=4.29Hz, 1H), 6.82-6.88(m, 2H), 8.31(s, 2H)
IR(cm⁻¹): 1625, 1507, 1439
MASS(m/z): 355(M⁺+2), 353, 191

### (Step B) (Compound 56)

Under an argon atmosphere, a solution (28 ml) of Compound 56a (1.0 g) obtained in Step A in methylene chloride was cooled to -78°C, and then boron trifluoride ether complex (0.69 ml) and triethylsilane (1.35 ml) were successively added thereto, followed by stirring at 0°C for 2 hours. The reaction solution was poured into a saturated aqueous solution of sodium bicarbonate and the mixture was extracted with chloroform. The organic layer was washed with a saturated saline and dried over anhydrous magnesium sulfate, and the solvent was distilled off under reduced pressure. The residue was purified by silica gel column chromatography (chloroform/methanol = 50/1) to give Compound 56 (0.62 g, 64.9) as pale-yellow oily crystals.
NMR(DHSO-d₆, δ, ppm): 1.23(d, J=6.93Hz, 3H), 2.69-2.78(m, 2H), 3.08-3.15 (m, 2H), 3.46-3.52(s, 1H), 3.74(s, 3H), 4.15-4.20(m, 1H), 4.48(t, J=8.58Hz, 1H), 6.63(d, J=8.25Hz, 1H), 6.78(d, J=8.58Hz, 1H), 8.61(s, 2H)
IR(KBr, cm⁻¹): 1623, 1510, 1451, 1434
MASS(m/z): 339(M⁺+2), 337(M⁺), 177

**Elemental analysis: C₁₇H₁₇Cl₂NO₂**

| | | | |
|---|---|---|---|
| Found (%) | C:60.37, | H:5.07, | N:4.14 |
| Calcd. (%) | C:60.48, | H:5.26, | N:4.03 |

### Example 57

### (±)-7-Methoxy-3-methyl-4-[2-(4-pyridyl)ethyl]-2,3-dihydrobenzofuran (Compound 57)

### (Step A) 4-[1-Hydroxy-2-(4-pyridyl)ethyl]-7-methoxy-3-methyl-2,3-dihydrobenzofuran (Compound 57a) (a mixture of diastereomers)

Under an argon atmosphere, a solution (25 ml) of 4-methylpyridine (0.66 ml) in THF was cooled to -78°C, and then a 1.69M solution (4.9 ml) of butyl lithium in hexane was dropwise added thereto, followed by stirring at the same temperature for one hour. A solution (25 ml) of Compound IIe (1.5 g) obtained in Reference Example 5 in THF was slowly and dropwise added to the mixture, followed by stirring at -78°C for one hour and then at 0°C for one hour. The reaction solution was poured into water and the mixture was extracted with ether. The organic layer was washed with a saturated saline and dried over anhydrous magnesium sulfate, and the solvent was distilled off under reduced pressure. The residue was purified by silica gel column chromatography (chloroform/methanol = 50/1) to give Compound 57a (1.64 g, 73.6%) as colorless crystals.
Melting point: 96-100 °C
NMR(DMSO-d₆, δ, ppm): (main product) 1.18(d, J=6.93Hz, 3H), 2.83-2.97(m, 2H), 3.74(s, 3H), 4.10-4.18(m, 1H), 4.31(t, J=8.54Hz, 1H), 4.73-4.84(m, 1H), 5.25(d, J=4.62Hz, 1H), 6.81-6.94(m, 2H), 7.16(d, J=4.62Hz, 2H), 8-41(d, J=4.62Hz, 2H). (by-product) 1.10(d, J=6.93Hz, 3H), 2.83-2.97(m, 2H), 3.74(s, 3H), 4.10-4.18(m, 1H), 4.44(t, J=8.25Hz, 1H), 4.73-4.84(m, 1H), 5.31(d, J=4.62Hz, 1H), 6.81-6.94(m, 2H), 7.23(d, J=4.61Hz, 2H), 7.43(d, J=4.61Hz, 2H)
IR(KBr, cm⁻¹): 1609, 1508, 1432
MASS(m/z): 285(M⁺), 193

### (Step B) (Compound 57)

Under an argon atmosphere, a solution (17 ml) of Compound 57a (0.6 g) obtained in Step A in methylene chloride was cooled to -78°C, and then boron trifluoride ether complex (0.42 ml) and triethylsilane (0.8 ml) were successively added thereto, followed by stirring at 0°C for 2 hours. The reaction solution was poured into a saturated aqueous solution of sodium bicarbonate and the mixture was extracted with chloroform. The organic layer was washed with a saturated saline and dried over anhydrous magnesium sulfate, and the solvent was distilled off under reduced pressure. The residue was purified by silica gel column chromatography (chloroform/methanol = 30/1) to give Compound 57 (0.042 g, 9.1%) as a pale-yellow oily substance.
NMR(DMSO-d₆, δ, ppm): 1.16(d, J=6.93Hz, 3H), 2.76-2.92(m, 4H), 3.40-3.47(m, 1H), 3.72(s, 3H), 4.11-4.16(m, 1H), 4.44(t, J=8.58Hz, 1H), 6.66(d, J=8.25Hz, 1H), 6.76(d, J=8.24H=, 1H), 7.26(d, J=4.95Hz, 2H), 8.46(brs, 2H)
IR(cm⁻¹): 1602, 1510, 1435
MASS(m/z): 269(M⁺), 177

### Example 58

### 7-Methoxy-3-methyl-4-[1-phenyl-2-(4-pyridyl)ethyl]-2,3-dihydrobenzofuran (Compound 58) (a mixture of diastereomers)

### (Step A) 4-[1-Hydroxy-1-phenyl-2-(4-pyridyl)ethyl]-7-methoxy-3-methyl-2,3-dihydrobenzofuran (Compound 58a) (a mixture of diastereomers)

Under an argon atmosphere, a solution of 4-methylpyridine (0.83 ml) in THF (50 ml) was cooled to -78°C, and then a 1.69M solution (5.0 ml) of butyl lithium in hexane was added thereto, followed by stirring at the same temperature for one hour. A solution of Compound IIak (2.0 g) obtained in Reference Example 37 in THF (20 ml) was slowly and dropwise added to the mixture, followed by stirring at 0°C for 2 hours. The reaction solution was poured into water and the mixture was extracted with ether. The organic layer was washed with a saturated saline and dried over anhydrous magnesium sulfate, and the solvent was distilled off under reduced pressure to give a crude product of Compound 58a (0.87 g) as yellowish brown crystals. This crude product was subjected to a subsequent step without being purified.

### (Step B) (Compound 58)

Under an argon atmosphere, a solution of Compound 58a (0.4 g) obtained in Step A in methylene chloride (3 ml) was cooled to -78°C, and then boron trifluoride ether complex (0.3 ml) and triethylsilane (0.52 ml) were successively added thereto, followed by stirring at 0°C for 2 hours. The reaction solution was poured into a saturated aqueous solution of sodium bicarbonate and the mixture was extracted with chloroform. The organic layer was washed with a saturated saline and dried over anhydrous magnesium sulfate, and the solvent was distilled off under reduced pressure. The residue was purified by silica gel column chromatography (chloroform/methanol = 30/1) to give Compound 58 (a mixture of diastereomers) (0.27 g, 56.7%) as pale-yellow crystals.
NMR(DMSO-d₆, δ, ppm) : (main product) 0.61(d, J=6.93Hz, 3H), 3.23-3.33(m, 1H), 3.42(d, J=2.53Hz, 1H), 3.53(d, J=2.54Hz, 1H), 3.74(s, 3H), 3.97-3.99(m, 2H), 5.78(m, 1H), 6.77-6.86(m, 4H), 7.15-7.32(m, 5H), 8.24-8.26(m, 2H). (by-product) 0.77(d, J=6.93Hz, 3H), 3.27-3.55(m, 3H), 3.74(s, 3H), 3.97-3.99(m, 2H), 5.74(m, 1H), 6.77-6.86(m, 4H), 7.15-7.32(m, 5H), 8.21-8.26(m, 2H)
IR(cm⁻¹): 1605, 1506, 1447
MASS(m/z): 345(M⁺)

### Example 59

### (±)-7-Methoxy-3-methyl-4-(4-pyridylthiomethyl)-2,3-dihydrobenzofuran (Compound 59)

### (Step A) (±)-4-Hydroxymethyl-7-methoxy-3-methyl-2,3-dihydrobenzofuran (Compound 59a)

Substantially the same procedure as in Step A of Example 48 was repeated using Compound IIe (7.0 g) obtained in Reference Example 5 to give Compound 59a (6.0 g, 85.0%) as a colorless oily substance.
NMR(DMSO-d₆, δ, ppm): 1.19(d, J=6.93Hz, 3H), 3.53-3.59(m, 1H), 3.71(s, 3H), 4.14(dd, J=8.75Hz, 4.29Hz, 1H), 4.37-4.52(m, 3H), 4.99(t, J=5.61Hz, 1H), 6.77(s, 2H)
MASS(m/z): 194(M⁺)

### (Step B) (Compound 59)

Substantially the same procedure as in Step B of Example 48 was repeated using Compound 59a (1.5 g) obtained in Step A to give Compound 59 (1.5 g, 68%) as a colorless oily substance.
Melting point: 110-112 °C
NMR(DMSO-d₆, δ, ppm): 1.25(d, J=6.93Hz, 3H), 3.62-3.69(m, 1H), 3.74(s, 3H), 4.18(dd, J=3.96Hz, 8.74Hz, 1H), 4.30(s, 2H), 4.53(t, J=8.58Hz, 1H), 6.79(d, J=8.25Hz, 1H), 6.35(d, J=8.25Hz, 1H), 7.32(d, J=5.94Hz, 2H), 8.38(d, J=5.94Hz, 2H)
IR(KBr, cm⁻¹): 1618, 1575, 1506, 1439
MASS(m/z): 287(M⁺). 177

**Elemental analysis: C₁₆H₁₇NO₂S**

| | | | |
|---|---|---|---|
| Found (%) | C:66.87, | H:5.96, | N:4.87 |
| Calcd.(%) | C:66.94, | H:5.92, | N:5.08 |

### Example 60

### (±)-7-Methoxy-3-methyl-4-{1-phenyl-1-(4-pyridylthio)-methyl}-2,3-dihydrobenzofuran (Compound 60A and Compound 60B)

Substantially the same procedure as in Step B of Example 48 was repeated using Compound IIak-a (2.6 g) obtained in Step A of Reference Example 37 to give Compound 60A and Compound 60B [60A (0.11 g, 3.1%) and 60B (0.19 g, 5.4%)] each as colorless crystals.

### Compound 60A

Melting point: 59-62 °C
NMR(DMSO-d₆, δ, ppm): 1.31(d, J=6.93Hz, 3H), 3.57-3.63(m, 1H), 3.72(s, 3H), 4.20(dd, J=3.63Hz, 8.75Hz, 1H), 4.47(t, J=8.58Hz, 1H), 5.99(s, 1H), 6.82(d, J=8.58Hz, 1H), 6.90(d, J=8.24Hz, 1H), 7.17(d, J=5.94Hz, 2H), 7.23-7.36 (m, 3H), 7.51-7.54(m, 2H), 8.31(d, J=6.27Hz, 2H)
IR(KBr, cm⁻¹): 1620, 1572, 1504, 1433
MASS(m/z): 363(M⁺), 253

**Elemental analysis: C₂₂H₂₁NO₂S·0.5H₂O**

| | | | |
|---|---|---|---|
| Found (%) | C:70.94, | H:5.95, | N:3.76 |
| Calcd.(%) | C:70.85, | H:5.84, | N:3.85 |

### Compound 60B

Melting point: 84-85 °C
NMR(DMSO-d₆, δ, ppm): 1.02(d, J=6.93Hz, 3H), 3.65-3.85(m, 1H), 3.73(s, 3H), 4.19(dd, J=2.97Hz, 8.91Hz, 1H), 4.52(t, J=8.58Hz, 1H), 6.09(s, 1H), 6.84(d, J=8.58Hz, 1H), 6.94(d, J=8.25Hz, 1H), 7.16(d, J=6.27Hz, 2H), 7.25-7.39(m, 3H), 7.49-7.52(m, 2H), 8.29(d, J=5.94Hz, 2H)
IR(KBr, cm⁻¹): 1619, 1569, 1506, 1437
MASS(m/z): 363(M⁺), 253

**Elemental analysis: C₂₂H₂₁NO₂S·0.2H₂O**

| | | | |
|---|---|---|---|
| Found (%) | C:71.99, | H:5.88, | N:3.82 |
| Calcd. (%) | C:71.95, | H:5.79, | N:3.90 |

### Example 61

### (±)-7-Methoxy-3-methyl-4-(4-pyridylaminomethyl)-2,3-dihydrobenzofuran (Compound 61)

Substantially the same procedure as in Step B of Example 48 was repeated using Compound 59a obtained in Step A of Example 59 and using 4-aminopyridine instead of 4-mercaptopyridine to give Compound 61 (26.5%) as colorless crystals.
Melting point: 138-145 °C
NMR(DMSO-d₆, δ, ppm): 1.23(d, J=6.43Hz, 3H), 3.59-3.79(m, 1H), 3.73(s, 3H), 4.14-4.31(m, 3H), 4.53 (t, J=8.90Hz, 1H), 6.52(d, J=4.95Hz, 2H), 6.73(d, J=8.41Hz, 1H), 6.79(d, J=8.41Hz, 1H), 6.98(brs, 1H), 8.01(d, J=5.44Hz, 2H)
IR(KBr, cm⁻¹): 1600, 1523, 1508, 1437
MASS (m/z): 270(M⁺), 177

### Example 62

### (±)-4-[2-(3,5-Dichloro-4-pyridyl)-1-methoxyethyl]-7-methoxy-2,2-dimethyl-2,3-dihydrobenzofuran (Compound 62)

p-Toluenesulfonic acid (1.0 g) was added to a solution (50 ml) of Compound 45a (2.0 g) obtained in Step A of Example 45 in methanol at room temperature, followed by heating under reflux. The reaction solution was cooled and then the solvent was distilled off under reduced pressure. A saturated aqueous solution of sodium bicarbonate was added to the residue, followed by extraction with chloroform. The organic layer was washed with a saturated saline and dried over anhydrous magnesium sulfate and the solvent was distilled off under reduced pressure. The residue was purified by silica gel column chromatography (chloroform) to give Compound 62 (1.0 g, 48.2%) as a pale-yellow oily substance.
Melting point: 89-93 °C
NMR(DMSO-d₆, δ, ppm): 1.31(s, 3H), 1.41(s, 3H), 2.74 (d, J=15.51Hz, 1H), 3.04 (s, 3H), 3.07-3.15(m, 2H), 3.29-3.42(m, 1H), 3.73(s, 3H), 4.47(dd, J=6.59Hz, 7.59Hz, 1H), 6.64(d, J=8.58Hz, 1H), 6.79(d, J=8.25Hz, 1H), 8.56(s, 2H)
MASS(m/e): 383, 381(M⁺), 221
IR(KBr, cm⁻¹): 1622, 1506, 1436

**Elemental analysis: C₁₉H₂₁Cl₂NO₃**

| | | | |
|---|---|---|---|
| Found (%) | C:59.96, | H:5.61, | N:3.56 |
| Calcd. (%) | C:59.70, | H:5.54, | N:3.66 |

### Example 63

### (±)-4-[1-Cyano-2-(3,5-dichloro-4-pyridyl)ethyl]-2,2-dimethyl-7-methoxy-2,3-dihydrobenzofuran (Compound 63)

A solution (70 ml) obtained in Step A of Example 45 of Compound 45a (2.5 g) in methylene chloride was cooled to 0°C and then trimethylsilylcyanide (5.4 ml) and boron trifluoride ether complex (2.5 ml) were successively added thereto, followed by stirring at 0°C for 2 hours. The reaction solution was poured into a saturated aqueous solution of sodium bicarbonate and the mixture was extracted with chloroform. The organic layer was washed with a saturated saline and dried over anhydrous magnesium sulfate, and the solvent was distilled off under reduced pressure. The residue was purified by silica gel column chromatography (chloroform/methanol = 30/1) to give Compound 63 (0.61 g, 23.8%) as pale-yellow crystals.
Melting point: 158-162 °C
NMR(DMSO-d₆, δ, ppm): 1.34(s, 3H), 1.40(s, 3H), 2.83 (d, J=15.51Hz, 1H), 3.16(d, J=15.51Hz, 1H), 3.44-3.53(m, 2H), 3.74(s, 3H), 4.42(t, J=8.25, 1H), 6.80(d, J=8.25Hz, 1H), 6.87(d, J=7.92Hz, 1H), 8.66(s, 2H)
MASS(m/e): 378, 376(M⁺), 216
IR (KBr, cm⁻¹): 2248, 1622, 1506, 1437

**Elemental analysis: C₁₉H₁₈Cl₂N₂O₂**

| | | | |
|---|---|---|---|
| Found (%) | C:60.42, | H:4.93, | N:7.54 |
| Calcd. (%) | C:60.49, | H:4.81, | N:7.43 |

### Example 64

### (±)-4-[1-Cyano-2-(4-pyridyl)ethyl]-7-methoxy-spiro[2,3-dihydrobenzofuran-2,1'-cyclopentane]-hydrochloride (Compound 64)

Substantially the same procedure as in Example 63 was repeated using Compound 53a (6.6 g) obtained in Step A of Example 53 to give (±)-4-[1-cyano-2-(4-pyridyl)ethyl]-7-methoxy-spiro[2,3-dihydrobenzofuran-2,1'-cyclopentane] (2.2 g, 32%) as a pale-yellow oily substance. Then, substantially the same procedure as in Example 51 was repeated using the obtained oily substance to give Compound 64.
Melting point: 187-189 °C
NMR(DMSO-d₆, δ, ppm): 1.73(s,8H), 3.16(d, J=16.2Hz, 1H), 3.31(d, J=15.8Hz, 1H), 3.37-3.56(m, 2H), 3.74(s, 3H), 4.64(t, J=7.6Hz, 1H), 6.75(d, J=8.2Hz, 1H), 6.84(d, J=8.3Hz, 1H), 7.91(d, J=5.6Hz, 2H), 8.87(d, J=5.6Hz, 2H)
MASS(m/e): 334(M⁺),; 242
IR(KBr, cm⁻¹): 2243, 1633, 1508

**Elemental analysis: C₂₁H₂₂N₂O₂·HCl·H₂O**

| | | | |
|---|---|---|---|
| Found (%) | C:67.38, | H:6.29, | N:7.19 |
| Calcd. (%) | C:67.35, | H:6.30, | N:7.48 |

### Example 65

### (±)-4-[1-Cyano-1-methyl-2-(4-pyridyl)ethyl]-7-methoxy-spiro[2,3-dihydrobenzofuran-2,1'-cyclopentane] hydrochloride (Compound 65)

### (Step A) (±)-4-[1-Hydroxy-1-methyl-2-(4-pyridyl)ethyl]-7-methoxy-spiro[2,3-dihydrobenzofuran-2,1'-cyclopentane] (Compound 65a)

Substantially the same procedure as in Example 46 was repeated using Compound IIan (2.7 g) obtained in Reference Example 40 to give Compound 65a (2.8 g, 74.7%) as a colorless oily substance.

### (Step B) (Compound 65)

Substantially the same procedure as in Example 63 was repeated using Compound 65a (1.8 g) obtained in Step A to give (±)-4-[1-cyano-1-methyl-2-(4-pyridyl)ethyl]-7-methoxy-spiro[2,3-dihydrobenzofuran-2,1'-cyclopentane] (0.35 g, 18.9%) as a pale-yellow oily substance. Then, substantially the same procedure as in Example 51 was repeated using the obtained oily substance to give Compound 65.
Melting point: 142-144 °C
NMR(DMSO-d₆, δ, ppm): 1.74-1.94(m, 11H), 3.17(d, J=15.8Hz, 1H), 3.21(s, 2H), 3.40(d, J=16.3Hz, 1H), 3.75(s, 3H), 6.70(d, J=8.9Hz, 1H), 6.82(d, J=8.9Hz, 1H), 7.04(d, J=5.8Hz, 2H), 8.46(d, J=5.9Hz, 2H)
MASS (m/e): 348(M⁺)

### Example 66

### (±)-7-Methoxy-4-[1-phenyl-2-(4-pyridyl)ethyl]-2-(4-pyridyl)benzofuran-hydrochloride (Compound 66)

Substantially the same procedure as in Example 47 was repeated using Compound IIal (0.45 g) obtained in Reference Example 38 to give (±)-7-methoxy-4-[1-phenyl-2-(4-pyridyl)ethyl]-2-(4-pyridyl)benzofuran (0.28 g, 50%) as a pale-yellow solid. Then, substantially the same procedure as in Example 51 was repeated using the obtained crystals to give Compound 66.
Melting point: 183-185 °C
NMR(DMSO-d₆, δ, ppm): 3.88(d-like, J=8Hz, 2H), 3.96(s, 3H), 4.93(t-like, J=8Hz, 1H), 7.08(d, J=8.5Hz, 1H), 7.1-7.4(m, 3H), 7.43(d, J=8.5Hz, 1H), 7.50 (d, J=7Hz, 2H), 7.94(d, J=6Hz, 2H), 8.33(d, J=6Hz, 2H), 8.55(s, 1H), 8.75(d, J=6Hz, 2H), 8.92(d, J=6Hz, 2H)
IR(KBr, cm⁻¹): 2840, 1630, 1590, 1560, 1200
MASS(m/e): 406(M⁺), 348, 315

**Elemental analysis: C₂₇H₂₂NO₂·2.0HCl·1.7H₂O**

| | | | |
|---|---|---|---|
| Found (%) | C:63.63, | H:5.33, | N:5.23 |
| Calcd. (%) | C:63.58, | H:5.41, | N:5.49 |

### Example 67

### (E)-4-[2-(3,5-Dichloro-4-pyridyl)ethenyl]-7-methoxy-2,2-dimethyl-2,3-dihydrobenzofuran (Compound 67)

p-Toluenesulfonic acid (0.8 g) was added to a suspension of Compound 45a (1.0 g) obtained in Step A of Example 45 in toluene (27 ml), followed by heating under reflux for 30 minutes. After being allowed to stand for cooling, a saturated aqueous solution of sodium bicarbonate was added to the reaction solution for neutralization, followed by extraction with ether. The organic layer was washed with a saturated saline and dried over anhydrous magnesium sulfate, and the solvent was distilled off under reduced pressure. The resultant residue was purified by silica gel column chromatography (chloroform/methanol = 20/1) to give Compound 67 (0.59 g, 62.2%) as yellow crystals.
Melting point: 114-118 °C
NMR(DMSO-d₆, δ, ppm): 1.44(s, 6H), 3.18(s, 2H), 3.80 (s, 3H), 6.91(d, J=8.57Hz, 1H), 6.92(d, J=16.82Hz, 1H), 7.16(d, J=8.25Hz, 1H), 7.36(d, J=16.82Hz, 1H), 8.64(s, 2H)
MASS(m/e): 351, 349(M⁺)
IR(cm⁻¹): 1613, 1556, 1508

**Elemental analysis: C₁₈H₁₇Cl₂NO₂**

| | | | |
|---|---|---|---|
| Found (%) | C:61.75, | H:4.87, | N:4.00 |
| Calcd. (%) | C:61.73, | H:4.89, | N:4.00 |

### Example 68

### (E)-7-Methoxy-2,2-dimethyl-4-[2-(4-pyridyl)ethenyl]-2,3-dihydrobenzofuran (Compound 68)

Substantially the same procedure as in Example 67 was repeated using Compound 46a (0.2 g) obtained in Step A of Example 46 to give Compound 68 (0.17 g, 90.2%) as yellow crystals.
Melting point: 145-149 °C
NMR(DMSO-d₆, δ, ppm): 1.45(s, 6H), 3.24(s, 2H), 3.78 (s, 3H), 6.88(d, J=8.58Hz, 1H), 6.97(d, J=16.83Hz, 1H), 7.15(d, J=8.58Hz, 1H), 7.39(d, J=16.49Hz, 1H), 7.54(d, J=5.94Hz, 2H), 8.51(d, J=5.94Hz, 2H)
IR(KBr, cm⁻¹): 1610, 1589, 1506, 1439
MASS (m/z) : 281(M⁺), 266

**Elemental analysis: C₁₈H₁₉NO₂·0.2H₂O**

| | | | |
|---|---|---|---|
| Found (%) | C:75.87, | H:6.86, | N:4.92 |
| Calcd. (%) | C:76.10, | H:6.86, | N:5.10 |

### Example 69

### 7-Methoxy-2,2-dimethyl-4-[1-methyl-2-(4-pyridyl)-ethenyl]-2,3-dihydrobenzofuran (Compound 69)

### (Step A) (±)-4-[1-Hydroxy-1-methyl-2-(4-pyridyl)ethyl]-7-methoxy-2,2-dimethyl-2,3-dihydrobenzofuran (Compound 69a)

Substantially the same procedure as in Step A of Example 65 was repeated using Compound IIan (2.7 g) obtained in Reference Example 39 to give Compound 69a (2.8 g, 74.4%) as a pale-yellow oily substance.
NMR(DMSO-d₆, δ, ppm): 1.22(s, 3H), 1.33(s, 3H), 1.45 (s, 3H), 2.83 (d, J=16.2, 1H), 2.91(s, 2H), 3.16 (d, J=15.2Hz, 1H), 3.70(s, 3H), 6.07(s, 2H), 6.94(d, J=5.9Hz, 2H), 8.31(d, J=4.3Hz, 2H)
MASS(m/e): 313(M⁺), 221

### (Step B) (Compound 69)

Substantially the same procedure as in Example 67 was repeated using Compound 69a (0.6 g) obtained in Step A to give Compound 69 (0.52 g, 91.5%) as pale-yellow crystals.
Melting point: 85-87 °C
NMR(DMSO-d₆, δ, ppm): 1.42(s, 6H), 2.22(s, 3H), 3.15 (s, 2H), 3.77(s, 3H), 6.50(s, 1H), 6.85(s, 2H), 7.37(d, J=5.9Hz, 2H), 8.56(d, J=5.9Hz, 2H)
MASS(m/e): 295(M⁺)
IR(KBr, cm⁻¹): 1614, 1593, 1504

**Elemental analysis: C₁₉H₂₁NO₂·0.1H₂O**

| | | | |
|---|---|---|---|
| Found (%) | C:76.77, | H:7.22, | N:4.82 |
| Calcd. (%) | C:76.79, | H:7.19, | N:4.71 |

### Example 70

### 7-Methoxy-2,2-dimethyl-4-[1-phenyl-2-(4-pyridyl)-ethenyl]-2,3-dihydrobenzofuran (Compound 70) (a mixture of E/Z)

Substantially the same procedure as in Example 67 was repeated using Compound 47a (0.3 g) obtained in Step A of Example 47 to give Compound 70 (0.28 g, 98.0%) as pale-yellow crystals.
Melting point: 110-113 °C
NMR(DMSO-d₆, δ, ppm) : (main product; 76%) ; 1.29 (s, 6H), 2.56(s, 2H), 3.76(s, 3H), 6.69(d, J=8.58Hz, 1H), 6.74(s, 1H), 6.84 (d, J=8.58Hz, 1H), 6.92(d, J=5.93Hz, 2H), 7.10-7.13(m, 2H), 7.36-7.38 (m, 3H), 8.32(d, J=5.94Hz, 2H). (by-product;22%); 1.21 (s, 6H), 2.43(s, 2H), 3.80(s, 3H), 6.54(d, J=8.25Hz, 1H), 6.87(d, J=8.26Hz, 1H), 6.96(d, J=5.94Hz, 2H), 7.06(m, 1H), 7.10-7.13(m, 2H), 7.36-7.38(m, 3H), 8.37(d, J=5.94Hz, 2H)
IR(KBr, cm⁻¹): 1618, 1592, 1506, 1433
MASS(m/z): 357(M⁺)

**Elemental analysis: C₂₄H₂₃NO₂**

| | | | |
|---|---|---|---|
| Found (%) | C:80.64, | H:6.49, | N:3.92 |
| Calcd.(%) | C:80.56, | H:6.61, | N:4.00 |

### Example 71

### (E)-2,2-Diethyl-7-methoxy-4-[2-(3,5-dichloro-4-pyridyl)ethenyl]-2,3-dihydrobenzofuran·methanesulfonate (Compound 71)

Substantially the same procedure as in Example 67 was repeated using Compound 50a (3.0 g) obtained in Step A of Example 50 to give (E)-2,2-diethyl-7-methoxy-4-[2-(3,5-dichloro-4-pyridyl)ethenyl]-2,3-dihydrobenzofuran (2.5 g, 90.5%) as yellow crystals. Then, substantially the same procedure as in Example 50 was repeated using tThe obtained crystals to give Compound 71.
Melting point: 137-141 °C
NMR(DMSO-d₆, δ, ppm) : 0.87(t, d=7.4Hz, 6H), 1.71(q, d=7.4Hz, 4H), 2.36(s, 3H), 3.80(s, 3H), 6.84(d, J=8.4Hz, 1H), 6.94(d, J=16.8Hz, 1H), 7.14(d, J=8.4Hz, 1H), 7.37(d, J=16.8Hz, 1H), 8.64(s, 2H)
MASS(m/e) : 379, 377(M⁺)
IR(cm⁻¹): 1599, 1508

**Elemental analysis: C₂₀H₂₁Cl₂NO₂·CH₃SO₃H**

| | | | |
|---|---|---|---|
| Found (%) | C:52.93, | H:5.30, | N:2.88 |
| Calcd.(%) | C:53.17, | H:5.32, | N:2.95 |

### Example 72

### (E)-2,2-Diethyl-7-methoxy-4-[2-(4-pyridyl)ethenyl]-2,3-dihydrobenzofuran-hydrochloride (Compound 72)

Substantially the same procedure as in Example 67 was repeated using Compound 51a (3.0 g) obtained in Step A of Example 51 to give (E)-2,2-diethyl-7-methoxy-4-[2-(4-pyridyl)ethenyl]-2,3-dihydrobenzofuran (2.6 g, 91%) as pale-yellow crystals. Then, substantially the same procedure as in Example 51 was repeated using the obtained crystals to give Compound 72.
Melting point: 236-239 °C
NMR(DMSO-d₆, δ, ppm) : 0.90(t, J=7.4Hz, 6H), 1.72(q, J=7.4Hz, 4H), 3.27(s, 2H), 3.82(s, 3H), 6.93(d, J=8.9Hz, 1H), 7.25(d, J=8.4Hz, 1H), 7.26(d, J=14.8Hz, 1H), 7.84(d, J=16.3Hz, 1H), 8.19(d, J=6.9Hz, 2H), 8.79(d, J=6.4Hz, 2H)
MASS(m/e): 309(M⁺), 280
IR(cm⁻¹): 1603, 1571, 1507, 1437

**Elemental analysis: C₂₀H₂₃NO₂·HCl**

| | | | |
|---|---|---|---|
| Found (%) | C:69.17, | H:7.08, | N:4.00 |
| Calcd.(&) | C:69.45, | H:6.99, | N:4.05 |

### Example 73

### (E)-4-[2-(3,5-Dichloro-4-pyridyl)ethenyl]-7-methoxy-spiro[2,3-dihydrobenzofuran-2,1'-cyclopentane]-methanesulfonate (Compound 73)

Substantially the same procedure as in Example 67 was repeated using Compound 52a (4.0 g) obtained in Step A of Example 52 to give (E)-4-[2-(3,5-dichloro-4-pyridyl)ethenyl]-7-methoxy-spiro[2,3-dihydrobenzofuran-2,1'-cyclopentanel (1.8 g, 46.1%) as yellow crystals. Then, substantially the same procedure as in Example 50 was repeated using the obtained crystals to give Compound 73.
Melting point: 155-158 °C
NMR(DMSO-d₆; δ, ppm): 1.75-1.79(m, 8H), 1.99-2.10(m, 2H), 2.38(s, 3H), 3.36(s, 2H), 3.80(s, 3H), 6.90(d, J=8.9Hz, 1H), 6.94(d, J=16.8Hz, 1H), 7.16(d, J=8.4Hz, 1H), 7.37(d, J=16.8Hz, 1H), 8.64(s, 2H)
MASS(m/e): 377, 375(M⁺) 215
IR(cm⁻¹): 2935(br), 1589, 1566, 1506

**Elemental analysis: C₂₀H₁₉Cl₂NO₂·CH₃SO₃H**

| | | | |
|---|---|---|---|
| Found (%) | C:53.25, | H:4.90, | N:2.89 |
| Calcd. (%) | C:53.40, | H:4.91, | N:2.97 |

### Example 74

### (E)-7-Methoxy-4-[2-(4-pyridyl)ethenyl]-spiro[2,3-dihydrobenzofuran-2,1'-cyclopentane]·hydrochloride (Compound 74)

Substantially the same procedure as in Example 67 was repeated using Compound 53a (0.3 g) obtained in Step A of Example 53 to give (E)-7-methoxy-4-[2-(4-pyridyl)-ethenyl]-spiro[2,3-dihydrobenzofuran-2,1'-cyclopentane] (0.2 g, 72%) as pale-yellow crystals. Then, substantially the same procedure as in Example 51 was repeated using the obtained crystals to give Compound 74.
Melting point: 229-231 °C
NMR(DMSO-d₆, δ, ppm): 1.65-1.90(m, 6H), 1.90-2.15(m, 2H), 3.47(s, 2H), 3.82(s, 3H), 6.95(d, J=8.6Hz, 1H), 7.24(d, J=16.5Hz, 1H), 7.27(d, J=8.6Hz, 1H), 7.83(d, J=16.5Hz, 1H), 8.17(d, J=6.6Hz, 2H), 8.78(d, J=6.3Hz, 2H)
MASS (m/z) : 307(M⁺)
IR(cm⁻¹): 1604, 1507

**Elemental analysis: C₂₀H₂₁NO₂·HCl·H₂O**

| | | | |
|---|---|---|---|
| Found (%) | C:66.49, | H:6.69, | N:3.77 |
| Calcd. (%) | C:66.38, | H:6.68, | N:3.87 |

### Example 75

### 7-Methoxy-9-[1-methyl-2-(4-pyridyl)ethenyl]-spiro[2,3-dihydrobenzofuran-2,1'-cyclopentane] (Compound 75)

Substantially the same procedure as in Example 67 was repeated using Compound 65a (2.0 g) obtained in Step A of Example 65 to give Compound 75 (1.1 g, 57.3%) as yellow crystals.
Melting point: 85-87 °C
NMR(DMSO-d₆, δ, ppm): 1.74-1.90(m, 6H), 1.97-2.05(m, 2H), 2.36(s, 2H), 3.38(s, 3H), 3.79(s, 3H), 6.79 (s, 1H), 6.89(d, J=8.6Hz, 1H), 6.96(d, J= 8.2Hz, 1H), 8.02(d, J=6.6Hz, 2H), 8.84(d, J=6.6Hz, 2H)
MASS(m/e): 321(M⁺)
IR(KBr, cm⁻¹): 1631, 1605, 1601

**Elemental analysis: C₂₁H₂₃NO₂·HCl·0.3H₂O**

| | | | |
|---|---|---|---|
| Found (%) | C:69.45, | H:7.05, | N:3.91 |
| Calcd. (%) | C:69.43, | H:6.83. | N:3.86 |

### Example 76

### (E)-4-[2-(3,5-Dichloro-9-pyridyl)ethenyl]-7-methoxy-spiro[2,3-dihydrobenzofuran-2,1'-cyclohexane]-methanesulfonate (Compound 76)

Substantially the same procedure as in Example 67 was repeated using Compound 54a (3.5 g) obtained in Step A of Example 54 to give (E)-4-[2-(3,5-dichloro-4-pyridyl)-ethenyl]-7-methoxy-spiro[2,3-dihydrobenzofuran-2,1'-cyclohexane] (2.7 g, 81%) as pale-yellow crystals. Then, substantially the same procedure as in Example 50 was repeated using the obtained crystals to give Compound 76.
Melting point: 108-109 °C
NMR(DMSO-d₆. δ, ppm): 1.44-1.66(m, 4H), 1.70-1.76(m, 6H), 2.39(s, 3H), 3.14 (s, 2H), 3.81 (s, 3H), 6.20 (d, J=8.3Hz, 1H), 6.93 (d, J=16.8Hz, 1H), 7.15 (d, J=8.9Hz, 1H), 7.36(d, J=16.8Hz, 1H), 8.64(s, 2H)
MASS(m/e): 391, 389(M⁺)
IR(cm⁻¹): 2932, 1595, 1507

**Elemental analysis: C₂₁H₂₁Cl₂NO₂·CH₃SO₃H·1.2H₂O**

| | | | |
|---|---|---|---|
| Found (%) | C:51.99, | H:5.21, | N:2.67 |
| Calcd. (%) | C:52.01, | H:5.44, | N:2.76 |

### Example 77

### (E)-7-Methoxy-4-[2-(4-pyridyl)ethenyl]-spiro[2,3-dihydrobenzofuran-2,1'-cyclohexane]-hydrochloride (Compound 77)

Substantially the same procedure as in Example 67 was repeated using Compound 55a (4 g) obtained in Step A of Example 55 to give (E)-7-methoxy-4-[2-(4-pyridyl)ethenyl]-spiro[2,3-dihydrobenzofuran-2,1'-cyclohexane] (3.1 g, 82%) as pale-yellow crystals. Then, Substantially the same procedure as in Example 51 was repeated using the obtained crystals to give Compound 77.
Melting point: 234-239 °C
NMR(DMSO-d₆, δ, ppm): 1.47-1.68(m, 4H), 1.72-1.99(m, 6H), 3.26(s, 2H), 3.83(s, 3H), 6.94(d, J=8.4Hz, 1H), 7.26(d, J=15.3Hz, 1H), 7.27(d, J=8.9Hz, 1H), 7.83(d, J=16.3Hz, 1H), 8.19(d, J=6.9Hz, 2H), 8.78(d, J=6.4Hz, 2H)
MASS (m/e): 321(M⁺)
IR(cm⁻¹): 1600, 1511

**Elemental analysis: C₂₁H₂₃NO₂·HCl·0.3H₂O**

| | | | |
|---|---|---|---|
| Found (%) | C:69.51, | H:6.90, | N:3.84 |
| Calcd. (%) | C:69.43, | H:6.83, | N:3.86 |

### Example 78

### (E)-(±)-4-[2-(3,5-Dichloro-4-pyridyl)ethenyl]-7-methoxy-3-methyl-2,3-dihydrobenzofuran (Compound 78)

Substantially the same procedure as in Example 67 was repeated using Compound 56a (1.6 g) obtained in Step A of Example 56 to give Compound 78 (1.4 g, 92%) as yellow crystals.
Melting point: 117-118 °C
NMR(DMSO-d₆, δ, ppm): 1.23(s, J=6.93Hz, 3H), 3.68-3.74(m, 1H), 3.82(s, 3H), 4.26(dd, J=8.62Hz, 2.97Hz, 1H), 4.55(d, J=8.58Hz, 1H), 6.94(d, J=8.58Hz, 1H), 7.03(d, J=16.50Hz, 1H), 7.27(d, J=8.58Hz, 1H), 7.40(d, J=16.82Hz, 1H), 8.65(s, 2H)
MASS(m/e): 337, 335(M⁺), 300
IR(cm⁻¹): 1616, 1507

**Elemental analysis: C₁₇H₁₅Cl₂NO₂**

| | | | |
|---|---|---|---|
| Found (%) | C:60.02, | H:4.45, | N:4.14 |
| Calcd. (%) | C:60.73, | H:4.50, | N:4.17 |

### Example 79

### (E)-(±)-7-Methoxy-3-methyl-4-[2-(4-pyridyl)ethenyl]-2,3-dihydrobenzofuran (Compound 79)

Substantially the same procedure as in Example 67 was repeated using Compound 57a (0.25 g) obtained in Step A of Example 57 to give Compound 79(0.18 g, 95.3%) as yellow crystals.
Melting point: 93-95 °C
NMR(DMSO-d₆, δ, ppm): 1.21(d, J=6.93Hz, 3H), 3.80(s, 3H), 3.80-3.86(m, 1H), 4.26(dd, J=2.97Hz, 8.58Hz, 1H), 4.55(t, J=8.58Hz, 1H), 6.91(d, J=8.57Hz, 1H), 7.09(d, J=16.49Hz, 1H), 7.25(d, J=8.58Hz, 1H), 7.46(d, J=16.50Hz, 1H), 7.57(d, J=5.94Hz, 2H), 8.53(d, J=5.92Hz, 2H)
IR(KBr, cm⁻¹): 1612, 1591, 1506, 1459
MASS (m/z) : 267(M⁺)

**Elemental analysis: C₁₇H₁₇NO₁₂**

| | | | |
|---|---|---|---|
| Found (%) | C:76.38, | H:6.41, | N:5.24 |
| Calcd. (%) | C:76.50, | H:6.36, | N:5.24 |

### Example 80

### (±)-7-Methoxy-3-methyl-4-(1-phenyl-2-(9-pyridyl)-ethenyl]-2,3-dihydrobenzofuran (Compound 80) (an E/Z mixture)

Substantially the same procedure as in Example 67 was repeated using Compound 58a (1.5 g) obtained in Example 58 to give Compound 80 (1.3 g, 86.8%) as pale-yellow crystals.
Melting point: 103-105.5 °C
NMR(DMSO-d₆, δ, ppm): 1.07(d, J=6.60Hz, 3H), 2.92-3.10(m, 1H), 3.78(s, 3H), 4.08(dd, J=4.29Hz, 8.75Hz, 1H), 4.41(t, J=8.75Hz, 1H), 6.68(d, J=8.25Hz, 1H), 6.79(s, 1H), 6.86(d, J=8.25Hz, 1H), 6.95(d, J=5.28Hz, 2H), 7.13(m, 2H), 7.35 (m, 3H), 8.33(d, J=5.61Hz, 2H)
IR (KBr, cm⁻¹) : 1591, 1498, 1431
MASS (m/z) : 343 (M⁺), 251

**Elemental analysis: C₂₃H₂₁NO₂**

| | | | |
|---|---|---|---|
| Found (%) | C:79.66, | H:6.26, | N:4.07 |
| Calcd. (%) | C:79.61, | H:6.22, | N:4.04 |

### Example 81

### (E)-7-Methoxy-2-phenyl-4-[2-(4-pyridyl)ethenyl]-benzofuran·hydrochloride. (Compound 81)

### (Step A) (±)-4-[1-Hydroxy-2-(4-pyridyl)ethyl]-7-methoxy-2-phenylbenzofuran (Compound 81a)

Substantially the same procedure as in Step A of Example 46 was repeated using Compound IIh (2.6 g) obtained in Reference Example 8 to give Compound 81a (2.33 g, 65.4%) as a yellowish white solid.
NMR(CDCl₃, δ, ppm): 2.70(bs, 1H), 3.11(dd, J=6Hz, 14Hz, 1H), 3.21(dd, J=8Hz, 14Hz, 1H), 4.03(s, 3H), 5.15(dd, J=6Hz, 8Hz, 1H), 6.69(d, J=8Hz, 1H), 6.96(d, J=8Hz, 1H), 7.07(d, J=6Hz, 2H), 7.18(s, 1H), 7.37(t, J=7Hz, 1H), 7.44(dd, J=7Hz, 7Hz, 2H), 7.90(d, J=7Hz, 2H), 8.41(d, J=6Hz, 2H)
MASS(m/e): 345(M⁺), 327, 253

### (Step B) (Compound 81)

Substantially the same procedure as in Example 67 was repeated using Compound 81a (2.0 g) obtained in Step A to give (E)-7-methoxy-2-phenyl-4-[2-(4-pyridyl)ethenyl]-benzofuran (1.10 g, 58.0%) as a yellow solid. Then, substantially the same procedure as in Example 51 was repeated using the obtained crystals to give Compound 81.
Melting point: 146-148 °C
NMR(DMSO-d₆, δ, ppm) : 4.06(s, 3H), 7.11(d, J=9Hz, 1H), 7.4-7.6(m, 4H). 7.69(d, J=9Hz, 1H), 8.00(d, J=7_{Hz}, 2H), 8.16(s. 1H), 8.19(d, J=18Hz, 1H), 8.30(d, J=7Hz, 2H), 8.84(d, J=7Hz, 2H)
IR(KBr, cm⁻¹): 1600, 1510, 1290, 1100
MASS(m/e): 328, 327(M⁺)

**Elemental analysis: C₂₂H₁₇NO₂·1.0HCl·1.0H₂O**

| | | | |
|---|---|---|---|
| Found (%) | C:69.25, | H:5.20, | N:3.73 |
| Calcd. (%) | C:69.20, | H:5.28, | N:3.67 |

### Example 82

### (E)-4-[2-(3,5-Dichloro-4-pyridyl)ethenyl]-7-methoxy-2-(4-pyridyl)benzofuran (Compound 82)

### (Step A) (±)-4-[2-(3,5-Dichloro-4-pyridyl)-1-hydroxyethyl]-7-methoxy-2-(4-pyridyl)benzofuran (Compound 82a)

Substantially the same procedure as in Step A of Example 45 was repeated using Compound IIf (4.00 g) obtained in Reference Example 6 to give Compound 82a (3.91 g, 59.6%) as a yellowish white solid.
NMR(DMSO-d₆, δ, ppm): 3.23(dd, J=5Hz, 13Hz, 1H), 3.45 (dd, J=8Hz, 13Hz, 1H), 3.97 (s, 3H), 5.22(m, 1H), 5.79(d, J=4Hz, 1H), 6.95(d, J=8Hz, 1H), 7.11(d, J=8Hz, 1H), 7.69(s, 1H), 7.84(d, J=6Hz, 2H), 8.54(s, 2H), 8.69(d, 6Hz, 2H)
MASS(m/e): 416, 414(M⁺)

### (Step B) (Compound 82)

Substantially the same procedure as in Example 67 was repeated using Compound 82a (1.50 g) obtained in Step A to give Compound 82 (0.847 g, 59.1%) as a yellow solid.
Melting point: 204-206 °C
NMR(CDCl₃, δ, ppm): 4.10(s, 3H), 6.91(d, J= 8Hz, 1H), 7.16(d, J=17Hz, 1H), 7.46(d, J=8Hz, 1H), 7.50(s, 1H), 7.77(d, J=17Hz, 1H), 7.77(d, J=6Hz, 2H), 8.52(s, 2H), 8.71(d, J=6Hz, 2H)
IR(KBr, cm⁻¹): 1615, 1550, 1290, 1180
MASS(m/e): 400, 398, 396(M⁺)

**Elemental analysis: C₂₁H₁₄N₂O₂Cl₂**

| | | | |
|---|---|---|---|
| Found (%) | C:63.32, | H:3.51, | N:6.98 |
| Calcd. (%) | C:63.51, | H:3.55, | N:7.05 |

### Example 83

### (E)-7-Methoxy-2-(4-pyridyl)-4-[2-(4-pyridyl)ethenyl]-benzofuran·2 hydrochloride (Compound 83)

### (Step A) (±)-4-[1-Hydroxy-2-(4-pyridyl)ethyl]-7-methoxy-2-(4-pyridyl)benzofuran (Compound 83a)

Substantially the same procedure as in Step A of Example 46 was repeated using Compound IIf (1.0 g) obtained in Reference Example 6 to give Compound 83a (1.11 g, 81.4%) as a yellowish white solid.
NMR(DMSO-d₆, δ, ppm): 3.15(d, J=7Hz, 2H), 3.97(s, 3H), 5.17(t, J=7Hz, 1H), 5.64(bs, 1H), 6.97(d, J=8Hz, 1H), 7.16(d, J=8Hz, 1H), 7.52(d, J=6Hz, 2H), 7.91(d, J=6Hz, 2H), 8.00(s, 1H), 8.56(d, J=6Hz, 2H), 8.71(d, J=6Hz, 2H)
MASS(m/e): 346(M⁺), 328, 254

### (Step B) (Compound 83)

Substantially the same procedure as in Example 67 was repeated using Compound 83a (2.8 g) obtained in Step A to give (E)-7-methoxy-2-(4-pyridvl)-4-12-(4-pyridyl)-ethenyl]benzofuran (1.60 g, 60.4%) as a yellow solid. Then, substantially the same procedure as in Example 51 was repeated using the obtained crystals to give Compound 83.
Melting point: 200-203 °C
NMR(DMSO-d₆, δ, ppm): 4.08(s, 3H), 7.27(d, J=8Hz, 1H), 7.69(d, J=17Hz, 1H), 7.75(d, J=8Hz, 1H), 8.25(d, J=17Hz, 1H), 3.36(d, J=6Hz, 2H), 8.43(d, J=5Hz, 2H), 8.88(d, J=6Hz, 2H), 8.98(d, J=5Hz, 2H), 9.02(s, 1H)
IR(KBr, cm⁻¹): 1640, 1600, 1560, 1500
MASS(m/e): 329(M⁺ +1), 313

**Elemental analysis: C₂₁H₁₆N₂O₂·2.0HCl·1.6H₂O**

| | | | |
|---|---|---|---|
| Found (%) | C:58.61, | H:5.05, | N:6.45 |
| Calcd. (%) | C:58.64, | H:4.97, | N:6.51 |

### Example 84

### (E)-4-[2-(3,5-Dichloro-4-pyridyl)ethenyl]-7-methoxy-2-(2-pyridyl)benzofuran (Compound 84)

### (Step A) (±)-4-[2-(3,5-Dichloro-4-pyridyl)-1-hydroxyethyl]-7-methoxy-2-(2-pyridyl)benzofuran (Compound 84a)

Substantially the same procedure as in Step A of Example 45 was repeated using Compound IIg (3.40 g) obtained in Reference Example 7 to give Compound 84a (4.51 g, 80.9%) as a yellowish white solid.
NMR(DMSO-d₆, δ, ppm): 3.22(dd, J=5Hz, 14Hz, 1H), 3.45 (dd, J=9Hz, 14Hz, 1H), 3.98(s, 3H), 5.21(ddd, J=5Hz, 5Hz, 9Hz, 1H), 5.73(d, J=5Hz, 1H), 6.91(d, J=10Hz, 1H), 7.10(d, J=10Hz, 1H), 7.40(m, 1H), 7.62(s, 1H), 7.9-8.0(m, 2H), 8.55(s, 2H), 8.70 (dd, J=2Hz, 5Hz, 1H)
MASS(m/e): 416, 414(M⁺), 254

### (Step B) (Compound 84)

Substantially the same procedure as in Example 67 was repeated using Compound 84a (0.60 g) obtained in Step A to give Compound 84 (0.28 g, 49.5%) as a yellow solid.
Melting point: 157-158 °C
NMR(CDCl₃, δ, ppm): 4.10(s, 3H), 6.90(d, J= 8Hz, 1H), 7.20(d, J=17Hz, 1H), 7.27(m, 1H), 7.47(d, J=8Hz, 1H), 7.75(s, 1H), 7.82(m, 1H), 7.82(d, J=17Hz, 1H), 8.02 (d, J=8Hz, 1H), 8.51(s, 2H), 8.69(dd, J=1Hz, 4Hz, 1H).
IR(KBr, cm⁻¹): 1610, 1550, 1510, 1290
MASS(m/e): 400, 398, 396(M⁺)

**Elemental analysis: C₂₁H₁₄N₂O₂Cl₂**

| | | | |
|---|---|---|---|
| Found (%) | C:63.81, | H:3.57, | N:6.91 |
| Calcd. (%) | C:63.51, | H:3.55, | N:7.05 |

### Example 85

### (E)-7-Methoxy-2-(2-pyridyl)-4-[2-(4-pyridyl)ethenyl]-benzofuran·2 hydrochloride (Compound 85)

### (Step A) (±)-4-[1-Hydroxy-2-(4-pyridyl)ethyl]-7-methoxy-2-(2-pyridyl)benzofuran (Compound 85a)

Substantially the same procedure as in Step A of Example 46 was repeated using Compound IIg (3.0 g) obtained in Reference Example 7 to give Compound 85a (2.10 g, 51.1%) as a yellowish white solid.
NMR(DMSO-d₆, δ, ppm): 3.04(d, J=6Hz, 2H), 3.96(s, 3H), 5.15(dt, J=4Hz, 6Hz, 1H), 5.53(d, J=4Hz, 1H), 6.92(d, J=8Hz, 1H), 7.12(d, J=8Hz, 1H), 7.26(d, J=6Hz, 2H), 7.41(dd, J=5Hz, 9Hz, 1H), 7.74(s, 1H), 7.9-8.0(m, 2H), 8.41(d, J=6Hz, 2H), 8.68(d, J=5Hz, 1H)
MASS(m/e): 346(M⁺), 253, 252

### (Step B) (Compound 85)

Substantially the same procedure as in Example 67 was repeated using Compound 85a (2.1 g) obtained in Step A to give (E)-7-methoxy-2-(2-pyridyl)-4-[2-(4-pyridyl)-ethenyl]benzofuran (0.58 g, 29.2%) as a yellow solid. Then, substantially the same procedure as in Example 51 was repeated using the obtained crystals to give Compound 85.
Melting point: 192-195 °C
NMR(D₂O, δ, ppm): 4.11(s, 3H), 6.69(d, J=17Hz, 1H), 6.89(d, J=8Hz, 1H), 7.25(d, J=8Hz, 1H), 7.27(d, J=17Hz, 1H), 7.54(s, 1H), 7.72(dd, J=5Hz, 7Hz, 1H), 7.92(d, J=6Hz, 2H), 7.99(d, J=8Hz, 1H), 8.31(dd, J=7Hz, 8Hz, 1H), 8.55(d, J=5Hz, 1H), 8.72(d, J=6Hz, 2H)
IR(KBr, cm⁻¹): 1610, 1560, 1280
MASS(m/e): 328 (M⁺)

**Elemental analysis: C₂₁H₁₆N₂O₂·2.0HCl·1.4H₂O**

| | | | |
|---|---|---|---|
| Found (%) | C:59.12, | H:4.73, | N:6.51 |
| Calcd. (%) | C:59.14, | H:4.91, | N:6.57 |

### Example 86

### (E)-4-[2-Cyano-2-(4-pyridyl)ethenyl]-7-methoxy-2,2-dimethyl-2,3-dihydrobenzofuran (Compound 86)

Compound IIa (2.3 g) obtained in Reference Example 1 was suspended in glacial acetic acid, and sodium acetate (2.3 g) and 4-pyridylacetonitrile (1.6 ml) were successively added thereto, followed by stirring at 110°C for one hour. The reaction solution was poured into water and the mixture was extracted with ethyl acetate. The collected organic layer was washed with a saturated saline and dried over anhydrous magnesium sulfate. The residue was purified by silica gel column chromatography (ethyl acetate/toluene = 1/9) and recrystallized from ethanol to give Compound 86 (1.6 g, 46%) as pale-yellow crystals.
Melting point: 150-163 °C
NMR(DMSO-d₆, δ, ppm): 1.44(s, 6H), 3.33(s, 2H), 3.84 (s, 3H), 7.04(d, J=8.57Hz, 1H), 7.71(d, J=5.94Hz, 1H), 7.73 (d, J=8.25Hz, 1H), 7.98 (s, 1H), 8.67(d, J=6.27Hz, 1H)
MASS(m/e): 306(M⁺)
IR(KBr, cm⁻¹) : 2206, 1578, 1508

**Elemental analysis: C₁₉R₁₈N₂O₂**

| | | | |
|---|---|---|---|
| Found (%) | C:74.63, | H:5.95, | N:9.25 |
| Calcd. (%) | C:74.49, | H:5.92, | N:9.14 |

### Example 87

### (E)-4-[2-Ethoxycarbonyl-2-(4-pyridyl)ethenyl]-7-methoxy-2,2-dimethyl-2,3-dihydrobenzofuran (Compound 87)

Substantially the same procedure as in Example 86 was repeated using Compound IIa (2.0 g) obtained in Reference Example 1 and using ethyl ester of 4-pyridine-acetic acid instead of 4-pyridylacetonitrile to give Compound 87 (2.5 g, 73.2%) as dark brown crystals.
Melting point: 98-100 °C
NMR(DMSO-d₆, δ, ppm): 1.20(t, J=7.26Hz, 3H), 1.38(s, 6H), 3.02(s, 2H), 3.68(s, 3H), 4.19(q, J=7.26Hz, 2H), 6.15(d, J=8.57Hz, 1H), 6.60(d, J=8.57Hz, 1H), 7.23(d, J=5.93Hz, 2H), 7.71(s, 1H), 8.57(d, J=5.93Hz, 2H)
MASS(m/e): 353(M⁺), 280
IR(KBr, cm⁻¹): 1706, 1596, 1508

### Example 88

### 4-(2,2-Dicyanoethenyl)-7-methoxy-2,2-dimethyl-2,3-dihydrobenzofuran (Compound 88)

Compound IIa (2.0 g) obtained in Reference Example 1 was suspended in glacial acetic acid, and sodium acetate (1.9 g) and malonitrile (0.8 ml) were successively added thereto, followed by stirring at 110°C for one hour. The reaction solution was poured into water, and the precipitated crystals were collected by filtration, washed with water, and dried under reduced pressure. The obtained crude crystals were purified by silica gel column chromatography (chloroform) to give Compound 88 (2.4 g, 94.5%) as pale-yellow crystals.
Melting point: 198-200 °C
NMR(DMSO-d₆, δ, ppm) : 1.43(s, 6H), 3.24(s, 2H), 3.87 (s, 3H), 7.12(d, J=8.6Hz, 1H), 7.75(d, J=8.6Hz, 1H), 8.19(s, 1H)
MASS (m/e) : 254(M⁺)
IR(KBr, cm⁻¹) : 2218, 1619, 1589

**Elemental analysis: C₁₅H₁₄N₂O₂**

| | | | |
|---|---|---|---|
| Found (%) | C:70.95, | H:5.57, | N:10.96 |
| Calcd. (%) | C:70.85, | H:5.55, | N:11.02 |

### Example 89

### 4-(2-Cyano-2-ethoxycarbonylethenyl)-7-methoxy-2,2-dimethyl-2,3-dihydrobenzofuran (Compound 89)

Substantially the same procedure as in Example 88 was repeated using Compound IIa (2.0 g) obtained in Reference Example 1 and using ethyl cyanoacetate instead of malonitrile to give Compound 89 (2.8 g, 96.5%) as a dark brown oily substance.
Melting point: 112-117 °C
NMR(DMSO-d₆, δ, ppm): 1.30(1, J=6.9Hz, 3H), 1.44(s, 6H), 3.23(s, 2H), 3.86(s, 3H), 4.30(q, J=6.9Hz, 2H), 7.09(d, J=8.9Hz, 1H), 7.83(d, J=8.6Hz, 1H), 8.09 (s, 1H)
MASS(m/e): 301(M⁺)
IR(KBr, cm⁻¹): 2218, 1718, 1590

**Elemental analysis: C₁₇H₁₉NO₄**

| | | | |
|---|---|---|---|
| Found (%) | C:67.80, | H:6.41, | N:4.82 |
| Calcd. (%) | C:67.76, | H:6.35, | N:4.65 |

### Example 90

### (E)-7-Methoxy-4-[2-(4-pyridylaminocarbonyl)ethenyl]-spiro[2,3-dihydrobenzofuran-2,1'-cyclopentane] (Compound 90)

### (Step A) (E)-4-(2-Ethoxycarbonylethenyl)-7-methoxy-spiro[2,3-dihydrobenzofuran-2,1'-cyclopentane] (Compound 90a)

Triethyl phosphonoacetate (10.5 g) was suspended in THF (70 ml), and potassium t-butoxide (3.74 g) was added thereto under ice-cooling, followed by stirring at room temperature for 30 minutes. After cooling the reaction solution with ice again, a solution of Compound IIc (3.1 g) obtained in Reference Example 3 in THF (20 ml) was slowly and dropwise added thereto under ice-cooling, followed by stirring at room temperature for one hour. Water was added to the reaction solution and the mixture was extracted with ether. The collected organic layer was washed with a saturated saline and dried over anhydrous magnesium sulfate. The residue was purified by silica gel column chromatography (chloroform) to give Compound 90a (3.51 g, 87.0%) as white crystals.
Melting point: 81-91 °C
NMR(DMSO-d₆, δ, ppm): 1.25 (t, J=6.4Hz, 3H), 1.30-2.22 (m, 8H), 3.35(s, 2H), 3.79(s, 3H), 4.17(d, J=7.4Hz, 2H), 6.28(d, J=16.3Hz, 1H), 6.83(d, J=8.4Hz, 1H), 7.18(d, J=8.4Hz, 1H), 7.53(d, J=16.3Hz, 1H)
MASS(m/e): 302(M⁺), 229

### (Step B) (E)-4-(2-Carboxyethenyl)-7-methoxy-spiro[2,3-dihydrobenzofuran-2,1'-cyclopentane] (Compound 90b)

A mixture of Compound 90a (3.5 g) obtained in Step A, a 4N aqueous solution (35.0 ml) of sodium hydroxide, and ethanol (150 ml) was stirred at room temperature for 15 hours. The solvent was distilled off and the residue was dissolved in water. Concentrated hydrochloric acid was dropwise added to the solution, and a precipitate was collected by filtration, washed with water, and dried to give Compound 90b (2.38 g, 74.9%) as white crystals.
Melting point: 212-215 °C
NMR(DMSO-d₆, δ, ppm): 1.75-1.96(m, 8H), 3.33(s, 2H), 3.79(s, 3H), 6.23(d, J=15.8Hz, 1H), 6.86(d, J=8.4Hz, 1H), 7.15(d, J=8.4Hz, 1H), 7.48(d, J=16.3Hz, 1H), 12.26(brs, 1H)
MASS (m/e) : 274(M⁺)

### (Step C) (Compound 90)

Compound 90b (0.3 g) obtained in Step B was suspended in a mixed solvent of methylene chloride (6 ml) and dioxane (1 ml), and dicyclohexylcarbodiimide (DCC) (0.23 g) and 4-aminopyridine (0.11 g) were added thereto after cooling the suspension to 0°C, followed by stirring at room temperature for 6 hours. Water was added to the mixture followed by extraction with chloroform. The collected organic layer was washed with a saturated saline and dried over anhydrous magnesium sulfate. The residue was purified by silica gel column chromatography (chloroform) to give Compound 90 (0.22 g, 64.5%) as pale-yellow crystals.
Melting point: 124-128 °C
NMR(DMSO-d₆, δ, ppm): 1.77-1.90(m, 6H), 1.90-2.10(s, 2H), 3.39(s, 2H), 3.80(s, 3H), 6.60(d, J=15.8Hz, 1H), 6.91(d, J=8.4Hz, 1H), 7.09(d, J=8.4Hz, 1H), 7.55(d, J=15.8Hz, 1H), 7.57(d, J=5.7Hz, 2H), 8.45(d, J=5.9Hz, 1H), 10.47 (s, 1H)
IR(KBr, cm⁻¹): 1592, 1506
MASS(m/e): 350(M⁺), 257

**Elemental analysis: C₂₁H₂₂N₂O₃·0.4H₂O**

| | | | |
|---|---|---|---|
| Found (%) | C:70.52, | H:6.41, | N:7.60 |
| Calcd. (%) | C:70.53, | H:6.43, | N:7.83 |

### Example 91

### (E)-7-Methoxy-4-[2-[4-(methoxycarbonyl)phenyl-1-ylaminocarbonyl]ethenyl]-spiro[2,3-dihydrobenzofuran-2,1'-cyclopentane] (Compound 91)

Compound 90b (0.9 g) obtained in Step B of Example 90 was suspended in a mixed solvent of methylene chloride (18 ml) and dioxane (4 ml), and dicyclohexylcarbodiimide (DCC) (0.69 g) and ethyl 4-aminobenzoate (0.55 g) were added thereto after cooling the suspension to 0°C, followed by stirring at room temperature for 6 hours. Water was added to the mixture followed by extraction with chloroform. The collected organic layer was washed with a saturated saline and dried over anhydrous magnesium sulfate. The residue was purified by silica gel column chromatography (chloroform) to give Compound 91 (0.36 g, 26.9%) as white crystals.
Melting point: 119-123 °C
NMR (DMSO-d₆, δ, ppm) : 1.77-1.90(m, 6H) 1.90-2.10(m,
2H), 3.38(s, 2H), 3.80(s, 3H), 3.83(s, 3H), 6.67 (d, J=15.8Hz, 1H), 6. 91 (d, J=8.4Hz, 1H), 7.08(d, J=8.4Hz, 1H), 7.52(d, J=15.8Hz, 1H), 7.82(d, J=8.9Hz, 2H), 7.95(d, J=8.4Hz, 2H), 10.45(s, 1H)
IR(KBr, cm⁻¹): 1699, 1608, 1506
MASS (m/e): 407(M⁺)

**Elemental analysis: C₂₄H₂₅NO₅·0.1H₂O**

| | | | |
|---|---|---|---|
| Found (%) | C:70.43, | H:6.37, | N:3.44 |
| Calcd. (%) | C:70.43, | H:6.20, | N:3.42 |

### Example 92

### (E)-4-[2-(4-(Carboxyphenylaminocarbonyl)-ethenyl]-7-methoxy-spiro[2,3-dihydrobenzofuran-2,1'-cyclopentane] (Compound 92)

A mixture of Compound 91 (0.25 g) obtained in Example 91, a 4N aqueous solution (1.6 ml) of sodium hydroxide, and dioxane (2.5 ml) was heated under reflux for 2 hours. The reaction solution was cooled, poured into water, and the mixture was adjusted to pH 3 by 6N hydrochloric acid. The precipitated crystals were collected by filtration, washed with water, and dried to give Compound 92 (0.43 g, 17.8%) as white crystals.
Melting point: 266-269 °C
NMR(DMSO-d₆, δ, ppm): 1.65-1.90(s, 6H), 1.90-2.10(m, 2H), 3.38(s, 2H), 3.80(s, 3H), 6.63(d, J=15.8Hz, 1H), 6.91(d, J=8.4Hz, 1H), 7.09(d, J=8.4Hz, 1H), 7.52(d, J=15.3Hz, 1H), 7.80(d, J=8.9Hz, 2H), 7.92(d, J=8.9Hz, 1H), 10.43(s, 1H).
IR(KBr, cm⁻¹): 1682, 1596
MASS(m/e): 394(M⁺ +1), 257

**Elemental analysis: C₂₃H₂₃NO₅·0.1H₂O**

| | | | |
|---|---|---|---|
| Found (%) | C:69.85, | H:5.92, | N:3.54 |
| Calcd. (%) | C:69.85, | H:6.13, | N:3.52 |

### Example 93

### (E)-7-Methoxy-4-{2-[3-(methoxycarbony)phenylaminocarbonyl]ethenyl}-spiro[2,3-dihydrobenzofuran-2,1'-cyclopentane] (Compound 93)

Substantially the same procedure as in Example 91 was repeated using Compound 90b (0.9 g) obtained in Step B of Example 90 and methyl 3-aminobenzoate (0.55 g) to give Compound 93 (0.68 g, 50.8%) as white crystals.
Melting point: 88-91 °C
NMR(DMSO-d₆, δ, ppm): 1.77-1.90(s, 6H), 1.90-2.10(m, 2H), 3.39(s, 2H), 3.80(s, 3H), 3.87(s, 3H), 6.60 (d, J=15. 8Hz, 1H), 6.91(d, J=8.6Hz, 1H), 7.08(d, J=8.3Hz, 1H), 7.46-7.55(m, 2H), 7.66(d, J=7.9Hz, 1H), 7.97(d, J=7.9Hz, 1H), 8.36(s, 1H), 10.37(s, 1H)
IR(KBr, cm⁻¹): 1724, 1608
MASS(m/e): 407(M⁺), 257

**Elemental analysis: C₂₄H₂₅NO₅·0.6H₂O**

| | | | |
|---|---|---|---|
| Found (%) | C:68.69, | H:6.10, | N:3.34 |
| Calcd. (%) | C:68.92, | H:6.31, | N:3.35 |

### Example 94

### (E)-4-[2-(4-Carboxyphenylaminocarbonyl)-ethenyl]-7-methoxy-spiro[2,3-dihydrobenzofuran-2,1'-cyclopentane] (Compound 94)

Substantially the same procedure as in Example 92 was repeated using Compound 93 (0.48 g) obtained in Example 93 to give Compound 94 (0.34 g, 73.5%) as white crystals.
Melting point: >290°C
NMR(DMSO-d₆, δ, ppm): 1.77-1.90(m, 6H), 1.90-2.10(m, 2H), 3.39(s, 2H), 3.80(s, 3H), 6.60(d, J=15.8Hz, 1H), 6.91(d, J=8.6Hz, 1H), 7.08(d, J=8.2Hz, 1H), 7.43-7.63(m, 1H), 7.64(d, J=6.6Hz, 1H), 7.95(d, J=7.9Hz, 1H), 8.30(s, 1H), 10.32(s, 1H), 10.32(s, 1H), 12.98
IR(KBr, cm⁻¹): 1683, 1610
MASS(m/e): 393(M⁺), 257

**Elemental analysis: C₂₃H₂₃NO₅**

| | | | |
|---|---|---|---|
| Found (%) | C:70.23, | H:5.93, | N:3.60 |
| Calcd. (%) | C:70.21, | H:5.89, | N:3.56 |

### Example 95

### 4-[2-(3,5-Dichloro-4-pyridyl)-1-oxoethyl]-7-methoxy-2,2-dimethyl-2,3-dihydrobenzofuran (Compound 95)

Compound 45a (3.0 g) obtained in Step A of Example 45 was dissolved in methylene chloride (80 ml), and a powder of silica gel (15 g) and pyridinium chlorochromate (PCC) (2.1 g) were added thereto, followed by stirring at room temperature for 2 hours. The reaction solution was filtered and the obtained filtrate was concentrated under reduced pressure. The residue was purified by silica gel column chromatography (chloroform/methanol = 30/1) to give Compound 95 (1.3 g, 44.9%) as pale-yellow crystals.
Melting point: 127-131 °C
NMR(DMSO-d₆, δ, ppm): 1.40(s, 6H), 3.24(s, 2H), 3.87 (s, 3H), 4.71(s, 2H), 7.03(d, J=8.58Hz, 1H), 7.79(d, J=8.58Hz, 1H), 8.66(s, 2H)
MASS(m/e): 367, 365(M⁺), 205
IR(cm⁻¹): 1675, 1613, 1575

**Elemental analysis: C₁₈H₁₇Cl₂NO₃**

| | | | |
|---|---|---|---|
| Found (%) | C:58.91, | H:4.60, | N:3.73 |
| Calcd. (%) | C:59.03, | H:4.68, | N:3.82 |

### Example 96

### 7-Methoxy-2,2-dimethyl-4-[1-oxo-2-(4-pyridyl)ethyl]-2.3-dihydrobenzofuran (Compound 96)

Substantially the same procedure as in Example 95 was repeated using Compound 46a (4.5 g) obtained in Step A of Example 46 to give Compound 96 (0.7 g, 15.5%) as pale-yellow crystals.
Melting point: 107-111 °C
NMR(DMSO-d₆, δ, ppm): 1.39(s, 6H), 3.26(s, 2H), 3.85 (s, 3H), 4.37(s, 2H), 6.98(d, J=8.58Hz, 1H), 7.27(d, J=5.61Hz, 2H), 7.60(d, J=8.57Hz, 1H), 8.49(d, J=5.61Hz, 2H)
MASS(m/e): 297(M⁺), 205
IR(cm⁻¹): 1675, 1608, 1578, 1511

**Elemental analysis: C₁₈H₁₉NO₃·0.1H₂O**

| | | | |
|---|---|---|---|
| Found (%) | C:72.37, | H:6.56, | N:4.61 |
| Calcd. (%) | C:72.27, | H:6.47, | N:4.68 |

### Example 97

### 4-[2-(3,5-Dichloro-4-pyridyl)-1-oxoethyl]-2,2-diethyl-7-methoxy-2,3-dihydrobenzofuran (Compound 97)

Under an argon atmosphere, a solution (50 ml) of 3,5-dichloro-4-methylpyridine (7.8 g) in THF was cooled to -78°C, and then a 1.69M solution (29 ml) of butyl lithium in hexane was dropwise added thereto, followed by stirring at the same temperature for one hour. A solution (40 ml) of Compound IIk (4.0 g) obtained in Reference Example 11 in THF was slowly and dropwise added to the mixture, followed by stirring at 0°C for 2 hours and then at room temperature for 3 hours. The reaction solution was poured into water and the mixture was extracted with ether. The organic layer was washed with a saturated saline and dried over anhydrous magnesium sulfate, and the solvent was distilled off under reduced pressure. The residue was purified by silica gel column chromatography (chloroform) to give Compound 97 (5.0 g, 4.2%) as a white solid.
Melting point: 164-166 °C
NMR(DMSO-d₆, δ, ppm): 0.83(t, d=7.4Hz, 6H), 1.64(q, d=7.4Hz, 4H), 3.20(s, 2H), 3.88(s, 3H), 4.71(s, 2H), 7.01(d, J=8.4Hz, 1H), 7.76(d, J=8.9Hz, 1H), 8.65(s, 2H)
MASS(m/e): 395, 393(M⁺), 233
IR(cm⁻¹): 2970(br), 1677, 1615, 1574

**Elemental analysis: C₂₀H₂₁Cl₂NO₃**

| | | | |
|---|---|---|---|
| Found (%) | C:60.84, | H:5.37, | N:3.53 |
| Calcd. (%) | C:60.92, | H:5.37, | N:3.55 |

### Example 98

### 2,2-Diethyl-7-methoxy-4-[1-oxo-2-(4-pyridyl)ethyl]-2,3-dihydrobenzofuran-hydrochloride (Compound 98)

Under an argon atmosphere, a solution (50 ml) of 4-methylpyridine (4.8 ml) in THF was cooled to -78°C, and then a 1.69M solution (29 ml) of butyl lithium in hexane was dropwise added thereto, followed by stirring at the same temperature for one hour. A solution (40 ml) of Compound IIk (4.0 g) obtained in Reference Example 11 in THF was slowly and dropwise added to the mixture, followed by stirring at 0°C for 2 hours and then at room temperature for 2 hours. The reaction solution was poured into water and the mixture was extracted with ether. The organic layer was washed with a saturated saline and dried over anhydrous magnesium sulfate, and the solvent was distilled off under reduced pressure. The residue was purified by silica gel column chromatography (chloroform/methanol = 20/1) to give 2,2-diethyl-7-methoxy-9-[1-oxo-2-(4-pyridyl)-ethyl]-2,3-dihydrobenzofuran as a colorless oily substance. Then, substantially the same procedure as in Example 51 was repeated using the obtained oily substance to give Compound 98.
Melting point: 185-191 °C
NMR(DMSO-d₆, δ, ppm): 0.84(t, J=7.4Hz, 6H), 1.67(d, J=7.4Hz, 9H), 3.24(s, 2H), 3.88(s, 3H), 4.78(s, 2H), 7.02(d, J=8.4Hz, 1H), 7.67(d, J=8.4Hz, 1H), 7.95(d, J=6.4Hz, 2H), 8.86(d, J=6.4Hz, 2H)
MASS(m/e): 325(M⁺), 233
IR(cm⁻¹): 1671, 1611, 1574, 1505

**Elemental analysis: C₂₀H₂₃NO₃·HCl**

| | | | |
|---|---|---|---|
| Found (%) | C:66.36, | H:6.85, | N:3.85 |
| Calcd. (%) | C:66.38, | H:6.69, | N:3.87 |

### Example 99

### 4-[2-(3,5-Dichloro-4-pyridyl)-1-oxoethyl]-7-methoxy-spiro(2,3-dihydrobenzofuran-2,1'-cyclopentane) (Compound 99)

Substantially the same procedure as in Example 97 was repeated using Compound III (1.0 g) obtained in Reference Example 12 to give Compound 99 (0.42 g, 42.0%) as pale-yellow crystals.
Melting point: 159-162 °C
NMR(DMSO-d₆, δ, ppm): 1.70-1.78(m, 6H), 1.90-2.09(m, 2H), 3.42(s, 2H), 3.88(s, 3H), 4.71(s, 3H), 7.03 (d, J=8.9Hz, 1H), 7.78(d, J=8.4Hz, 1H), 8.65(s, 2H)
MASS(m/e): 393, 391(M⁺), 231
IR(cm⁻¹): 1675, 1612, 1576

**Elemental analysis: C₂₀H₁₉Cl₂NO₃·0.3H₂O**

| | | | |
|---|---|---|---|
| Found (%) | C:60.40, | H:4.80, | N:3.50 |
| Calcd. (%) | C:60.40, | H:4.97, | N:3.52 |

### Example 100

### 7-Methoxy-4-[1-oxo-2-(4-pyridyl)ethyl]-spiro[2,3-dihydrobenzofuran-2,1'-cyclopentane]·hydrochloride (Compound 100)

Substantially the same procedure as in Example 98 was repeated using Compound III (4.0 g) obtained in Reference Example 12 to give 7-methoxy-4-[1-oxo-2-(4-pyridyl)ethyl]-spiro[2,3-dihydrobenzofuran-2,1'-cyclopentane] (2.1 g, 42.6%) as a pale-yellow oily substance. Then, substantially the same procedure as in Example 51 was repeated using the obtained oily substance to give Compound 100.
Melting point: 215-219 °C
NMR (DMSO-d₆, δ, ppm): 1.70-1.79 (m, 6H), 1.90-1.97(m, 2H), 3.44(s, 2H), 3.87 (s, 3H), 4.77(s, 2H), 7.03(d, J=6.4Hz, 2H), 7.68(d, J=6.4Hz, 2H), 7.94 (d, J= 8.4Hz, 1H), 8.86(d, J=8.9Hz, 1H)
MASS (m/e) : 323(M⁺), 294
IR(cm⁻¹): 1670, 1610, 1510

**Elemental analysis: C₂₀H₂₁NO₃·HCl·0.2H₂O**

| | | | |
|---|---|---|---|
| Found (%) | C:66.21, | H:6.26, | N:3.79 |
| Calcd. (%) | C:66.09, | H:6.21, | N:3.85 |

### Example 101

### 4-[2-(3,5-Dichloro-4-pyridyl)-1-oxoethyl]-7-methoxy-spiro[2,3-dihydrobenzofuran-2,1'-cyclohexane] (Compound 101)

Substantially the same procedure as in Example 97 was repeated using Compound IIm (4.0 g) obtained in Reference Example 13 to give Compound 101 (4.3 g, 72.3%) as pale-yellow crystals.
Melting point: 149-151 °C
NMR(DMSO-d₆, δ, ppm): 1.43(brs, 4H), 1.62-1.72(m, 6H), 3.20(s, 2H), 3.89(s, 3H), 4.71(s, 2H), 7.02(d, J=8.4Hz, 1H), 7.78(d, J=8.9Hz, 1H), 8.65(s, 2H)
MASS(m/e) : 407, 405(M⁺), 295
IR(cm⁻¹): 2841(br), 1678, 1578

**Elemental analysis: C₂₀H₁₉Cl₂NO₃·0.2H₂O**

| | | | |
|---|---|---|---|
| Found (%) | C:60.54, | H:4.77, | N:3.56 |
| Calcd. (%) | C:60.68, | H:4.94, | N:3.54 |

### Example 102

### 7-Methoxy-4-[1-oxo-2-(4-pyridyl)ethyl]-spiro[2,3-dihydrobenzofuran-2,1'-cyclohexane]·hydrochloride (Compound 102)

Substantially the same procedure as in Example 98 was repeated using Compound IIm (3.0 g) obtained in Reference Example 13 to give 7-methoxy-4-[1-oxo-2-(4-pyridyl)ethyl]-spiro[2,3-dihydrobenzofuran-2,1'-cyclohexane] (2.0 g, 54.9%) as a pale-yellow oily substance. Then, substantially the same procedure as in Example 51 was repeated using the obtained oily substance to give Compound 102.
Melting point: 193-196 °C
NMR(DMSO-d₆, δ, ppm): 1.43(brs, 4H), 1.50-1.72(m, 6H), 3.23(s, 2H), 3.88(s, 3H), 4.80(s, 2H), 7.03(d, *J*=8.9Hz, 1H), 7.68(d, J=8.4Hz, 1H), 7.97(d, J=6.4Hz, 2H), 8.88(d, J=6.4Hz, 2H).
MASS(m/e): 338(M⁺), 245
IR(cm⁻¹): 1674, 1610, 1510

**Elemental analysis: C₂₁H₂₃NO₃·HCl·0.1H₂O**

| | | | |
|---|---|---|---|
| Found (%) | C:66.99, | H:6.58, | N:3.68 |
| Calcd. (%) | C:67.14, | H:6.49, | N:3.73 |

### Example 103

### (±)-4-[2-(3,5-Dichloro-4-pyridyl)-1-oxoethyl]-7-methoxy-3-methyl-2,3-dihydrobenzofuran (Compound 103)

Substantially the same procedure as in Example 95 was repeated using Compound 56a (1.0 g) obtained in Step A in Example 56 to give Compound 103 (0.5 g, 51.3%) as pale-yellow crystals.
Melting point: 99-104 °C
NMR(DMSO-*d*₆, δ, ppm) : 1.08(d, J=6.93Hz, 3H), 3.77-3.90(m, 1H), 3.90(s, 3H), 4.28(dd, J=2.64Hz, 8.58Hz, 1H), 4.49(t, J=8.58Hz, 1H), 4.68(d, J=17.49Hz, 1H), 4.80(d, J=17.81Hz, 1H), 7.05(d, J=8.57Hz, 1H), 7.84(d, J=8.58Hz, 1H), 8.67(s, 2H)
IR(KBr, cm⁻¹): 1684, 1612, 1579, 1506, 1433
MASS(m/z): 353(M⁺+2), 351(M⁺), 191

**Elemental analysis: C₁₇H₁₅Cl₂NO₃**

| | | | |
|---|---|---|---|
| Found (%) | C:57.97, | H:4.29, | N:3.98 |
| Calcd. (%) | C:57.93, | H:4.37, | N:3.77 |

### Example 104

### (±)-7-Methoxy-3-methyl-4-[1-oxo-2-(4-pyridyl)ethyl]-2,3-dihydrobenzofuran (Compound 104)

Substantially the same procedure as in Example 95 was repeated using Compound 57a (0.6 g) obtained in Step A in Example 57 to give Compound 104 (0.03 g, 4,2%) as pale-yellow crystals.
Melting point: 111-117 °C
NMR(DMSO-d₆, δ, ppm) : 1.08(d, J=6.93Hz, 3H), 3.77-3.86(m, 1H), 3.86(s, 3H), 4.27(dd, J=2.64Hz, 8.75Hz, 1H), 4.40(s, 2H), 4.46(t, J=8.75Hz, 1H), 7.00(d, J=8.58Hz, 1H), 7.28(d, J=4.29Hz, 2H), 7.72(d, J=8.58Hz, 1H), 8.50(d, J=4 .29Hz, 2H)
IR(KBr, cm⁻¹): 1686, 1613, 1579, 1508, 1433
MASS(m/z): 283(M⁺), 191

**Elemental analysis: C₁₇H₁₇NO₃·0.3H₂O**

| | | | |
|---|---|---|---|
| Found (%) | C:70.72, | H:6.14, | N:4.85 |
| Calcd. (%) | C:70.54, | H:6.10, | N:4.46 |

### Example 105

### (±)-cis-6-Methoxy-9-[1-oxo-2-(4-pyridyl)ethyl]-1,2,3,4,4a,9b-hexahydrodibenzofuran-hydrochloride (Compound 105)

Substantially the same procedure as in Example 98 was repeated using Compound IIn (0.4 g) obtained in Reference Example 14 to give (±)-cis-6-methoxy-9-[1-oxo-2-(4-pyridyl)ethyl]-1,2,3,4,4a,9b-hexahydrodibenzofuran (0.34 g, 68%) as a pale-yellow oily substance. Then, substantially the same procedure as in Example 51 was repeated using the obtained oily substance to give Compound 105.
Melting point: 225-233 °C
NMR(CDCl₃, δ, ppm): 0.80-1.00(m, 1H), 1.10-1.36(m, 1H), 1.40-1.85(m, 1H), 1.98-2.12(m, 1H), 2.35-2.52(m, 1H), 3.45-3.64(m, 1H), 3.99(s, 3H), 4.58 (s, 2H), 4.50-4.65(m, 1H), 6.89(d, J=9Hz, 1H), 7.51(d, J=9Hz, 1H), 7.83(d, J=7Hz, 2H), 8.73(d, J=7Hz, 2H)

**Elemental analysis: C₂₀H₂₁NO₃·HCl**

| | | | |
|---|---|---|---|
| Found (%) | C:66.59, | H:6.15, | N:4.02 |
| Calcd. (%) | C:66.76, | H:6.16, | N:3.89 |

### Example 106

### 2-Cyano-4-[2-(3,5-dichloro-4-pyridyl)-1-oxoethyl]-7-methoxybenzofuran (Compound 106)

### (Step A) 2-Cyano-4-(2-(3,5-dichloro-4-pyridyl)-1-hydroxyethyl)-7-methoxybenzofuran (Compound 106a)

Substantially the same procedure as in Step A of Example 45 was repeated using Compound IIi (2.0 g) obtained in Reference Example 9 to give Compound 106a (2.3 g, 63.2%) as pale-yellow crystals.
NMR(DMSO-d₆, δ, ppm): 3.15-3.22(m, 1H), 3.30-3.50(m, 1H), 3.94(s, 3H), 5.13-5.20(m, 1H), 5.83(d, J=4.0Hz, 1H), 7.10(d, J=8.3Hz, 1H), 7.16(d, J=7.9Hz, 1H), 8.12(s, 1H), 8.55(s, 2H)
MASS(m/e): 362(M⁺)

### (Step B) (Compound 106)

Substantially the same procedure as in Example 95 was repeated using Compound 106a (1.1 g) obtained in Step A to give Compound 106 (0.27 g, 25.0%) as white crystals.
Melting point: 197-199 °C
NMR(DMSO-d₆, δ, ppm) : 4.12(s, 3H), 4.88(s, 2H), 7.39 (d, J=8.6Hz, 1H), 8.41(s, 1H), 8.47(d, J=8.3Hz, 1H), 8.69(s, 2H).
MASS(m/e): 362, 360(M⁺), 200
IR(cm⁻¹): 1675, 1557.

**Elemental analysis: C₁₇H₁₀Cl₂N₂O₃**

| | | | |
|---|---|---|---|
| Found (%) | C:56.62, | H:2.77, | N:7.54 |
| Calcd. (%) | C:56.53, | H:2.79, | N:7.76 |

### Example 107

### 2-Benzoyl-7-methoxy-4-(1-oxo-2-phenylethyl)benzofuran (Compound 107)

Compound IIag-a (1.0 g) obtained in Step A of Reference Example 33 and phenylacetyl chloride (0.79 ml) were dissolved in dry dichloromethane (50 ml), the solution was cooled to 0°C, and titanium tetrachloride (1.3 ml) was dropwise added thereto, followed by stirring at the same temperature. After 5 minutes, the reaction was stopped by adding distilled water, and the reaction solution was extracted with diethylether. Then, the organic layer was washed with a saturated saline and dried over anhydrous magnesium sulfate, and the solvent was distilled off under reduced pressure. The residue was purified by silica gel column chromatography (hexane/ethyl acetate = 4/1) to give Compound 107 (0.94 g, 64.0%) as a pale-yellow solid.
NMR(CDCl₃, δ, ppm): 4.10(s, 3H), 4.37(s, 2H), 6.93(d, J=8.5Hz, 1H), 7.2-7.4 (m, 5H), 7.51(dd, J=7.5Hz, 8Hz, 2H), 7.61(t, J=8Hz, 1H), 7.91(d, J=8.5Hz, 1H), 8.01(d, J=7.5Hz, 2H), 8.26(s, 1H)
MASS(m/e): 370(M⁺), 279, 251

**Elemental analysis: C₂₄H₁₈O₄**

| | | |
|---|---|---|
| Found (%) | C:77.97, | H:4.94 |
| Calcd. (%) | C:77.82, | H:4.91 |

### Example 108

### 2-Benzoyl-7-methoxy-4-[1-oxo-2-(4-pyridyl)ethyl]-benzofuran (Compound 108)

Substantially the same procedure as in Example 107 was repeated using Compound IIag-a obtained in Step A of Reference Example 33 to give Compound 108 as a pale-yellow solid.
NMR(CDCl₃, δ, ppm): 4.13(s, 3H), 4.35(s, 2H), 6.98(d, J=8Hz, 1H), 7.23(d, J= 5.5Hz, 2H), 7.52(dd, J=7Hz, 8Hz, 2H), 7.63(t, J=7Hz, 1H), 7.98(d, J=8Hz, 2H), 8.03(d, J=8Hz, 1H), 8.24(s, 1H), 8.57(d, J=5.5Hz. 2H)
MASS(m/e): 371(M⁺), 279

### Example 109

### 2-Butyl-7-methoxy-4-[1-oxo-2-(4-pyridyl)ethyl]-benzofuran-hydrochloride (Compound 109)

Substantially the same procedure as in Example 98 was repeated using Compound IIo (1.3 g) obtained in Reference Example 15 to give 2-butyl-7-methoxy-4-[1-oxo-2-(4-pyridyl)ethyl]benzofuran (0.42 g, 42%) as pale-yellow crystals. Then, substantially the same procedure as in Example 51 was repeated using the obtained crystals to give Compound 109.
Melting point: 212-218 °C
NMR(CDCl₃, δ, ppm): 0.941(t, J=7Hz, 3H), 1.30-1.55(m, 2H), 1.65-1.85(m, 2H), 2.83(t, J=7Hz, 2H), 4.12 (s, 3H), 4.65(s, 2H), 6.82(d, J=9Hz, 1H), 7.12(s, 1H), 7.84(d, J=9Hz, 1H), 7.87(d, J=6Hz, 2H), 8.72(d, J=6Hz, 2H)

**Elemental analysis: C₂₀H₂₁NO₃HCl0.2H₂O**

| | | | |
|---|---|---|---|
| Found (%) | C:66.03, | H:6.09, | N:3.77 |
| Calcd. (%) | C:66.09, | H:6.21, | N:3.85 |

### Example 110

### 7-Methoxy-2-(2-methylpropyl)-4-[1-oxo-2-(4-pyridyl)-ethyl]benzofuran·hydrochloride (Compound 110)

Substantially the same procedure as in Example 98 was repeated using Compound IIp (1.8 g) obtained in Reference Example 16 to give 7-methoxy-2-(2-methylpropyl)-4-[1-oxo-2-(4-pyridyl)ethyl]benzofuran (1.2 g, 56%) as white crystals. Then, Substantially the same procedure as in Example 51 was repeated using the obtained crystals to give Compound 110.
Melting point: 193-198 °C
NMR(CDCl₃, δ, ppm): 0.970(d, J=7Hz, 6H), 2.05-2.20(m, 1H), 2.70(d, J=7Hz, 2H), 4.12(s, 3H), 4.64(s, 2H), 6.82(d, J=9Hz, 1H), 7.13(s, 1H), 7.77-7.88 (m, 3H), 8.71(d, J=7Hz, 2H)

**Elemental analysis: C₂₀H₂₁NO₃HCl**

| | | | |
|---|---|---|---|
| Found (%) | C:66.64, | H:6.16, | N:3.90 |
| Calcd. (%) | C:66.76, | H:6.16, | N:3.89 |

### Example 111

### 7-Methoxy-4-[1-oxo-2-(4-pyridyl)ethyl]-2-phenyl-benzofuran·hydrochloride (Compound 111)

Substantially the same procedure as in Example 98 was repeated using Compound IIs (2.30 g) obtained in Reference Example 19 to give 7-methoxy-4-[1-oxo-2-(4-pyridyl)ethyl]-2-phenylbenzofuran (1.30 g, 26.6%) as a white solid. Then, substantially the same procedure as in Example 51 was repeated using the obtained crystals to give Compound 111.
NMR(DMSO-d₆, δ, ppm) : 4.12(s, 3H), 4.94(s, 2H), 7.16 (d, J=8.5Hz, 1H), 7.4-7.6(m, 3H), 7.90(s, 1H), 7.97(d, J=7Hz, 2H), 8.04(d, J=5.5Hz, 2H), 8.18(d, J=8.5Hz, 1H), 8.92(d, J=5.5Hz, 2H)
MASS(m/e): 343(M⁺), 251, 223

**Elemental analysis: C₂₂H₁₇NO₃·HCl·0.1H₂O**

| | | | |
|---|---|---|---|
| Found (%) | C:69.07, | H:4.73, | N:3.80 |
| Calcd. (%) | C:69.24, | H:4.81, | N:3.67 |

### Example 112

### 2-(2-Ethylphenyl)-7-methoxy-4-[1-oxo-2-(4-pyridyl)-ethyl]benzofuran·hydrochloride (Compound 112)

Substantially the same procedure as in Example 98 was repeated using Compound IIt (3.0 g) obtained in Reference Example 20 to give 2-(2-ethylphenyl)-7-methoxy-4-[1-oxo-2-(4-pyridyl)ethyl]benzofuran (1.00 g, 27.8%) as a white solid. Then, substantially the same procedure as in Example 51 was repeated using the obtained crystals to give Compound 112.
Melting point: 186-188 °C
NMR(DMSO-d₆, δ, ppm) : 1-19(t, J=7Hz, 3H), 2.87(q, J=7Hz, 2H), 4.11(s, 3H), 4.93(s, 2H), 7.18(d, J=8.5Hz, 1H), 7.3-7.5(m, 3H), 7.61(s, 1H), 7.75 (d, J=7.5Hz, 1H), 8.02(d, J=6Hz, 2H), 8.21(d, J=8.5Hz, 1H), 8.89(d, J=6Hz, 2H)
IR(KBr, cm⁻¹): 2960, 2920, 1654, 1618, 1573
MASS (m/e) : 371(M⁺), 279

**Elemental analysis: C₂₄H₂₁NO₃·HCl**

| | | | |
|---|---|---|---|
| Found (%) | C:70.69, | H:5.45, | N:3.46 |
| Calcd. (%) | C:70.66, | H:5.45, | N:3.43 |

### Example 113

### 2-(2-Isopropylphenyl)-7-methoxy-4-[1-oxo-2-(4-pyridyl)ethyl]benzofuran·hydrochloride (Compound 113)

Substantially the same procedure as in Example 98 was repeated using Compound IIu (2.50 g) obtained in Reference Example 21 to give 2-(2-isopropylphenyl)-7-methoxy-4-[1-oxo-2-(4-pyridyl)ethyl]benzofuran (1.10 g, 37.0%) as a white solid. Then, substantially the same procedure as in Example 51 was repeated using the obtained crystals to give Compound 113.
Melting point: 184-185 °C
NMR(DMSO-d₆, δ, ppm): 1.23(d, J=6.5Hz, 6H), 3.44(sep, J=6.5Hz, 1H), 4.11(s, 3H), 4.94(s, 2H), 7.17(d, J=8.5Hz, 1H), 7.37(dd, J=5Hz, 7Hz, 1H), 7.4-7.6 (m, 2H), 7.53(s, 1H), 7.62(d, J=7Hz, 1H), 8.02(d, J=6Hz, 2H), 8.22(d, J=8.5Hz, 1H), 8.90(d, J=6Hz, 2H)
IR(KBr, cm⁻¹): 2960, 2950, 1653, 1618, 1577
MASS(m/e): 385(M⁺), 293

**Elemental analysis: C₂₅H₂₃NO₃·HCl**

| | | | |
|---|---|---|---|
| Found (%) | C:71.00, | H:5.73, | N:3.35 |
| Calcd. (%) | C:71.16, | H:5.74, | N:3.32 |

### Example 114

### 4-[2-(3,5-Dichloro-4-pyridyl)-1-oxoethyl]-7-methoxy-2-(4-pyridyl)benzofuran-2 hydrochloride (Compound 114)

Substantially the same procedure as in Example 97 was repeated using Compound IIq (2.0 g) obtained in Reference Example 17 to give 4-[2-(3,5-dichloro-4-pyridyl)-1-oxoethyl]-7-methoxy-2-(4-pyridyl)benzofuran (0.18 g, 9.1%) as a white solid. Then, substantially the same procedure as in Example 51 was repeated using the obtained crystals to give Compound 114.
Melting point: 263-266 °C
NMR(DMSO-d₆, δ, ppm): 4.16(s, 3H), 4.91(s, 2H), 7.34 (d, J=9Hz, 1H), 8.40(d, J=9Hz, 1H), 8.50(d, J=6Hz, 2H), 8.66(s, 1H), 8.70(s, 2H), 8.97(d, J=6Hz, 2H).
IR(KBr, cm⁻¹): 1675, 1630, 1585, 1350
MASS(m/e): 416, 414, 412(M⁺), 253, 252

**Elemental analysis: C₂₁H₁₄N₂O₃Cl₂·2HCl·0.8H₂O**

| | | | |
|---|---|---|---|
| Found (%) | C:50.36, | H:3.68, | N:5.45 |
| Calcd. (%) | C:50.38, | H:3.54, | N:5.59 |

### Example 115

### 7-Methoxy-4-[1-oxo-2-(4-pyridyl)ethyl]-2-(4-pyridyl)-benzofuran-2 hydrochloride (Compound 115)

Substantially the same procedure as in Example 98 was repeated using Compound IIq (2.6 g) obtained in Reference Example 17 to give 7-methoxy-4-[1-oxo-2-(4-pyridyl)ethyl]-2-(4-pyridyl)benzofuran (1.78 g, 55.9%) as a white solid. Then, substantially the same procedure as in Example 51 was repeated using the obtained crystals to give Compound 115.
Melting point: 225-228 °C
NMR(DMSO-d₆, δ, ppm): 4.13(s, 3H), 5.00(s, 2H), 7.32 (d, J=9Hz, 1H), 8.07(d, J=6Hz, 2H), 8.25(d, J=9Hz, 1H), 8.44(d, J=7Hz, 2H), 8.57(s, 1H), 8.9-9.0(m, 4H)
IR(KBr, cm⁻¹): 1665, 1635, 1610, 1520, 1350
MASS(m/e): 344(M⁺), 252

**Elemental analysis: C₂₁H₁₆N₂O₃·2.0HCl·2.0H₂O**

| | | | |
|---|---|---|---|
| Found (%) | C:55.74, | H:4.82, | N:6.10 |
| Calcd. (%) | C:55.64, | H:4.89, | N:6.18 |

### Example 116

### 4-[2-(3,5-Dichloro-4-pyridyl)-1-oxoethyl]-7-methoxy-2-(2-pyridyl)benzofuran-2 hydrochloride (Compound 116)

Substantially the same procedure as in Example 97 was repeated using Compound IIr (3.0 g) obtained in Reference Example 18 to give 4-[2-(3,5-dichloro-4-pyridyl)-1-oxoethyl]-7-methoxy-2-(2-pyridyl)benzofuran (1.89 g, 63.4%) as a yellowish white solid. Then, substantially the same procedure as in Example 51 was repeated using the - obtained crystals to give Compound 116.
Melting point: 226-227 °C
NMR(DMSO-d₆, δ, ppm): 4.14(s, 3H), 4.88(s, 2H), 7.24 (d, J=9Hz, 1H), 7.53(dd, J=5Hz, 7Hz, 1H), 8.0-8.1(m, 2H), 8.13(s, 1H), 8.34(d, J=9Hz, 1H), 8.70(s, 2H), 8.73(d, J=5Hz, 1H)
IR(KBr, cm⁻¹): 1670, 1605, 1580, 1310
MASS[FAB(pos.), m/e]: 417, 415, 413(M⁺), 252

**Elemental analysis: C₂₁H₁₄N₂O₃Cl₂·2HCl**

| | | | |
|---|---|---|---|
| Found (%) | C:51.71, | H:3.26, | N:5.62 |
| Calcd. (%) | C:51.88, | H:3.32, | N:5.76 |

### Example 117

### 7-Methoxy-4-[1-oxo-2-(4-pyridyl)ethyl]-2-(2-pyridyl)-benzofuran-2 hydrochloride (Compound 117)

Substantially the same procedure as in Example 98 was repeated using Compound IIr (4.0 g) obtained in Reference Example 18 to give 7-methoxy-4-[1-oxo-2-(4-pyridyl)ethyl]-2-(2-pyridyl)benzofuran (1.30 g, 26.6%) as a white solid. Then, substantially the same procedure as in Example 51 was repeated using the obtained crystals to give Compound 117.
Melting point: 218-220 °C
NMR(DMSO-d₆, δ, ppm): 4.13(s, 3H), 4.97(s, 2H), 7.23 (d, J=8Hz, 1H), 7.49(m, 1H), 8.0-8.1(m, 5H), 8.22(d, J=8Hz, 1H), 8.72(d, J=4Hz, 1H), 8.93(d, J=6Hz, 2H)
IR(KBr, cm⁻¹) : 1670, 1610, 1470, 1305
MASS (m/e) : 344(M⁺), 252

**Elemental analysis: C₂₁H₁₆N₂O₃·2.0HCl·0.6H₂O**

| | | | |
|---|---|---|---|
| Found (%) | C:58.86, | H:4.54, | N:6.47 |
| Calcd. (%) | C:58.92, | H:4.52, | N:6.54 |

### Example 118

### 7-Methoxy-4-[1-oxo-2-(4-pyridyl)ethyl]-3-phenyl-benzofuran-hydrochloride (Compound 118)

Substantially the same procedure as in Example 98 was repeated using Compound IIv (0.60 g) obtained in Reference Example 22 to give 7-methoxy-4-[1-oxo-2-(4-pyridyl)ethyl]-3-phenylbenzofuran (0.25 g, 35%) as a white solid. Then, substantially the same procedure as in Example 51 was repeated using the obtained crystals to give Compound 118.
Melting point: 176-178 °C
NMR(DMSO-d₆, δ, ppm): 4.08(s, 3H), 4.77(s, 2H), 7.13-7.44(m, 6H), 7.80(d, J=6Hz, 1H), 7.98(d, J=8Hz, 1H), 8.21(s, 1H), 8.84(d, J=6Hz, 1H)
IR(KBr, cm⁻¹): 1674, 1618, 1402, 1304
MASS(m/e): 343(M⁺)

**Elemental analysis: C₂₂H₁₇NO₃·HCl·0.5H₂O**

| | | | |
|---|---|---|---|
| Found (%) | C:67.85, | H:4.88, | N:3.52 |
| Calcd. (%) | C:67.95, | H:4.92, | N:3.60 |

### Example 119

### 4-[2-(3,5-Dichloro-4-pyridyl)-1-oxoethyl]-3-ethoxycarbonylmethyl-7-methoxybenzofuran (Compound 119)

### (Step A) (±)-4-[2-(3,5-Dichloro-4-pyridyl)-1-hydroxyethyl]-3-ethoxycarbonyl-7-methoxybenzofuran (Compound 119a)

Substantially the same procedure as in Step A of Example 45 was repeated using Compound IIj (0.28 g) obtained in Reference Example 10 to give Compound 119A (0.31 g, 70%) as a pale-yellow solid.
Melting point: 133-135 °C
NMR(CDCl₃, δ, ppm): 1.22(t, J=7Hz, 3H), 2.40(d, J=5Hz, 1H), 3.34 (dd, J=4, 13Hz, 1H), 3.76 (dd, J=10, 13Hz, 1H), 3.97(s, 2H), 4.02(s, 3H), 4.07-4.23(m, 2H), 5.30-5.46(m, 1H), 6.82(d, J=8Hz, 1H), 7.32 (d, J=8Hz, 1H), 7.64(s, 1H), 8.46 (s, 2H)

### (Step B) (Compound 119)

Substantially the same procedure as in Example 95 was repeated using Compound 119A (0.30 g) obtained in Step A to give Compound 119 (0.28 g, 95%) as a white solid.
Melting point: 105-115 °C
NMR (CDCl₃, δ, ppm): 1.16(t, J=7Hz, 3H), 3.88 (s, 2H), 4.00-4.15(m, 5H), 4.69(s, 2H), 6.87(d, J=8Hz, 1H), 7.65 (s, 1H), 7.95 (d, J=8Hz, 1H), 8.51 (s, 2H)

### Example 120

### 3-Ethosycarbonylmethyl-7-methoxy-4-[1-oxo-2-(4-pyridyl)ethyl]benzofuran (Compound 120)

Compound 119 (0.04 g) obtained in Example 119 was dissolved in DMF-methanol (1:1) (1.0 ml), and 10% palladium carbon (0.016 g) was added thereto, followed by hydrogenation at normal temperature and normal pressure for 6 hours. The catalyst was removed and the filtrate was concentrated. Water and a saturated aqueous solution of sodium bicarbonate were added to the residue, and a precipitate was collected by filtration and dried to give Compound 120 (0.02 g, 9%) as a white solid.
Melting point: 111-117 °C
NMR(CDCl₃, δ, ppm): 1.18(t, J=7Hz, 3H), 3.92(s, 2H), 4.03(q, J=7Hz, 2H), 4.07(s. 3H), 4.29(s, 2H), 6.82(d, J=9Hz, 1H), 7.22(d, J=6Hz, 2H), 7.69(s, 1H), 7.75(d, J=9Hz, 1H), 8.56(d, J=6Hz, 2H)

### Example 121

### 5-(3,5-Dichloro-4-pyridylaminocarbonyl)-8-methoxy-2,2-dimethylbenzopyran (Compound 121)

Substantially the same procedure as in Example 1 was repeated using Compound IIao (0.432 g) obtained in Reference Example 41 to give Compound 121 (0.229 g, 33%) as a white solid.
Melting point: 174-178 °C
NMR(CDCl₃, δ, ppm): 1.51(s, 6H), 3.92(s, 3H), 5.77(d, J=10Hz, 1H), 6.82(d, J=8.7Hz, 1H), 6.95(d, J=10Hz, 1H), 7.29(d, J=8.7Hz, 1H), 7.41-7.52(brs, 1H), 8.58(s, 2H)
MASS(m/e): 378(M⁺)
IR(KBr, cm⁻¹): 1660, 1480, 1280

**Elemental analysis: C₁₈H₁₆N₂O₃Cl₂**

| | | | |
|---|---|---|---|
| Found (%) | C:57.12, | H:4.37, | N:7.23 |
| Calcd. (%) | C:57.01, | H:4.25, | N:7.39 |

### Example 122

### 5-(3,5-Dichloro-4-pyridylaminocarbonyl)-8-methoxy-2,2-dimethyl-3,4-dihydrohenzopyran (Compound 122)

Substantially the same procedure as in Example 1 was repeated using Compound IIap (1.05 g) obtained in Reference Example 42 to give Compound 122 (0.94 g, 56%) as a white solid.
Melting point: 155-156 °C
NMR(CDCl₃, δ, ppm): 1.42(s, 6H), 1.82(t, J=7.2Hz, 2H), 3.05(t, J=7.2Hz, 2H), 3.91(s, 3H), 6.79(d, J=8.3Hz, 1H), 7.28(d, J=8.3Hz, 1H), 7.38-7.59 (brs, 1H), 8.56(s, 2H)
MASS (m/e): 380(M⁺)
IR(KBr, cm⁻¹): 1680, 1480, 1280

**Elemental analysis: C₁₈H₁₈N₂O₃Cl₂**

| | | | |
|---|---|---|---|
| Found (%) | C:56.71, | H:4.84, | N:7.22 |
| Calcd. (%) | C:56.71, | H:4.76, | N:7.35 |

### Example 123

### 5-(3,5-Dichloro-4-pyridylaminocarbonyl)-8-methoxy-spiro[benzopyran-2,1'-cyclopentane] (Compound 123)

Substantially the same procedure as in Example 1 was repeated using Compound IIaq (1.67 g) obtained in Reference Example 43 to give Compound 123 (1.44 g, 55%) as a white solid.
Melting point: 129-131 °C
NMR(CDCl₃, δ, ppm): 1.50-2.32(m, 8H), 3.90(s, 3H), 5.82(d, J=9.0Hz, 1H), 6.80(d, J=8.2Hz, 1H), 6.99 (d, J=9.0Hz, 1H), 7.28(d, J=8.2Hz, 1H), 7.39-7.51(brs, 1H), 8.55(s, 2H)
MASS (m/e) : 404(M⁺)
IR(KBr, cm⁻¹): 1670, 1480, 1270

**Elemental analysis: C₂₀H₁₈N₂O₃Cl₂**

| | | | |
|---|---|---|---|
| Found (%) | C:59.13, | H:4.54, | N:6.66 |
| Calcd. (%) | C:59.27, | H:4.48, | N:6.91 |

### Example 124

### 8-Methoxy-5-(4-pyridylaminocarbonyl)-spiro[3,4-dihydrobenzopyran-2,1'-cyclopentane]·methanesulfonate (Compound 124)

Substantially the same procedure as in Example 6 was repeated using Compound IIas (0.96 g) obtained in Reference Example 45 to give 8-methoxy-5-(4-pyridylaminocarbonyl)-spiro[3,4-dihydrobenzopyran-2,1'-cyclopentane] (1.14 g, 92%) as a white solid. Then, substantially the same procedure as in Example 50 was repeated using the obtained solid to give Compound 124.
Melting point: 231-233 °C
NMR(DMSO, δ, ppm): 1.45-1.93(m, 10H), 2.30 (s, 3H), 2.92(t, J=5Hz, 2H), 3.80(s, 3H), 6.94(d, J=8Hz, 1H), 7.21(d, J=8Hz, 1H), 8.20(d, J=7Hz, 2H), 8.72(d, J=7Hz, 2H), 11.4(s, 1H)
IR(KBr, cm⁻¹): 1690, 1510, 1270

**Elemental analysis: C₂₀H₂₂N₂O₃·CH₃SO₃H·0.1H₂O**

| | | | |
|---|---|---|---|
| Found (%) | C:57.78, | H:6.10, | N:6.15 |
| Calcd. (%) | C:57.81, | H:6.05, | N:6.42 |

### Example 125

### 8-Methoxy-5-[2-(4-pyridyl)ethenyl]-spiro[3,4-dihydrobenzopyran-2,1'-cyclopentane]·hydrochloride (Compound 125)

### (Step A) 5-[1-Hydroxy-2-(4-pyridyl)ethyl]-8-methoxy-spiro[3,4-dihydrobenzopyran-2,1'-cyclopentane] (Compound 125a)

Compound 127 (0.78 g) obtained in Example 127 was dissolved in methanol (8 ml) and sodium borohydride (0.18 g) was added thereto under ice-cooling, followed by stirring at room temperature for 2 hours. The mixture was cooled again with ice and dilute hydrochloric acid was dropwise added thereto. After the solvent was distilled off, water was added to the residue, and the mixture was extracted with ethyl acetate and washed with a saturated saline. The resultant was dried over sodium sulfate and the solvent was distilled off to give Compound 125a (0.63 g, 80%) as white crystals.
Melting point: 153-156 °C
NMR(CDCl₃, δ, ppm): 1.36-2.07(m, 10H), 2.30-2.50(m, 1H), 2.70-3.10(m, 3H), 3.83(s, 3H), 4.99-5.10(m, 1H), 6.78(d, J=8.2Hz, 1H), 7.02(d, J=8.2Hz, 1H), 7.08(d, J=6.8Hz, 2H), 8.46(d, J=6.8Hz, 2H)
MASS(m/e): 339(M⁺)

### (Step B) (Compound 125)

Substantially the same procedure as in Example 67 was repeated using Compound 125a (0.58 g) obtained in Step A to give 8-methoxy-5-(2-(4-pyridyl)ethenyl)-spiro(3,4-dihydrobenzopyran-2,1'-cyclopentane) (0.355 g, 65%) as a yellow solid. Then, substantially the same procedure as in Example 51 was repeated using the obtained solid to give Compound 125.
Melting point: 208-215 °C
NMR(CDCl₃, δ, ppm): 1.49-1.99(m, 10H), 2.95(t, J=6.8Hz, 2H), 3.90(s, 3H), 6.80 (d, J=8.5Hz, 1H), 7.00(d, J=15Hz, 1H), 7.29(d, J=8.5Hz, 1H), 7.70-7.90(m, 3H), 8.50-8.67(m, 2H)
IR (KBr, cm⁻¹): 1620, 1580, 1500

**Elemental analysis: C₂₁H₂₃NO₃·HCl·0.2H₂O**

| | | | |
|---|---|---|---|
| Found (%) | C:69.75, | H:6.74, | N:3.82 |
| Calcd. (%) | C:69.78, | H:6.80, | N:3.87 |

### Example 126

### 8-Methoxy-5-[2-(4-pyridyl)ethenyl]-spiro[3,4-dihydrobenzopyran-2,1'-cyclohexane]·hydrochloride (Compound 126)

### (Step A) 5-[1-Hydroxy-2-(4-pyridyl)ethyl]-8-methoxy-spiro[3,4-dihydrobenzopyran-2,1'-cyclohexane] (Compound 126a)

Substantially the same procedure as in Step A of Example 125 was repeated using Compound 128 (0.73 g) obtained in Example 128 to give Compound 126a (0.47 g, 64%) as a white solid.
Melting point: 123-133 °C
NMR(CDCl₃, δ, ppm): 1.20-1.90(m, 12H), 2.29-2.45(m, 1H), 2.68-3.15(m, 3H), 3.36(s, 3H), 4.98-5-12(m, 1H), 6.78(d, J=9Hz, 1H), 7.01(d, J=9Hz, 1H), 7.08 (d, J=6Hz, 2H), 8.47(d, J=6Hz, 2H)

### (Step B) (Compound 126)

Substantially the same procedure as in Example 67 was repeated using Compound 126a (0.48 g) obtained in Step A to give 8-methoxy-5-[2-(4-pyridyl)ethenyl]-spiro[3,4-dihydrobenzvpyran-2,1'-cyclohexane] (0.14 g, 31%) as yellow crystals. Then, substantially the same procedure as in Example 51 was repeated using the obtained crystals to give Compound 126.
Melting point: 222-230 °C
NMR(CDCl₃, δ, ppm): 1.25-2.00(m, 12H), 2.90(t, J=7Hz, 2H), 3.92(s, 3H), 6.80 (d, J=9Hz, 1H), 6.97(d, J=16Hz, 1H), 7.75-7.90(m, 4H), 8.59(d, J=6Hz, 2H)

**Elemental analysis: C₂₂H₂₅NO₂·HCl·0.1H₂O**

| | | | |
|---|---|---|---|
| Found (%) | C:70.68, | H:7.04, | N:3.65 |
| Calcd. (%) | C:70.71, | H:7.07, | N:3.75 |

### Example 127

### 8-Methoxy-5-[1-oxo-2-(4-pyridyl)ethyl]-spiro[3,4-dihydrobenzopyran-2,1'-cyclopentane]-hydrochloride (Compound 127)

Substantially the same procedure as in Example 98 was repeated using Compound IIar (1.83 g) obtained in Reference Example 44 to give 8-methoxy-5-[1-oxo-2-(4-pyridyl)ethyl]-spiro[3,4-dihydrobenzopyran-2,1'-cyclopentane] (1.61 g, 72%) as a pale-yellow solid. Then, substantially the same procedure as in Example 51 was repeated using the obtained solid to give Compound 127.
Melting point: 186-192 °C
NMR(CDCl₃, δ, ppm): 1.50-2.07(m, 10H), 3.06(t, J=6.8Hz, 2H), 3.91(s, 3H), 4.59(s, 2H), 6.80(d, J=8.5HZ, 1H), 7.52(d, J=8.5Hz, 1H), 7.88(d, J=6.7Hz, 2H), 8.72(d, J=6.7Hz. 2H)
IR(KBr, cm⁻¹): 1670, 1560, 1280

**Elemental analysis: C₂₁H₂₃NO₃·HCl·0.4H₂O**

| | | | |
|---|---|---|---|
| Found (%) | C:66.19, | H:6.75, | N:3.72 |
| Calcd. (%) | C:66.19, | H:6.56, | N:3.68 |

### Example 128

### 8-Methoxy-5-[1-oxo-2-(4-pyridyl)ethyl]-spiro[3,4-dihydrobenzopyran-2,1'-cyclohexane]-hydrochloride

Substantially the same procedure as in Example 98 was repeated using Compound IIat (2.1 g) obtained in Reference Example 46 to give 8-methoxy-5-[1-oxo-2-(4-pyridyl)ethyl]-spiro[3,4-dihydrobenzopyran-2,1'-cyclohexane] (1.2 g, 48%) as pale-yellow crystals. Then, substantially the same procedure as in Example 51 was repeated using the obtained crystals to give Compound 128.
Melting point: 185-194 °C
NMR(CDCl₃, δ, ppm): 1.25-1.90(m, 12H), 3.01(t, J=7Hz, 2H), 3.95(s, 3H), 4.56(s, 2H), 6.82(d, J=9Hz, 1H), 7.51(d, J=9Hz, 1H), 7.82(d, J=6Hz, 2H), 8.71(d, J=6Hz, 2H)

**Elemental analysis: C₂₂H₂₅NO₃·HCl·0.6H₂O**

| | | | |
|---|---|---|---|
| Found (%) | C:66.34, | H:6.84, | N:3.45 |
| Calcd. (%) | C:66.27, | H:6.88, | N:3.51 |

### Example 129

### 7-(3,5-Dichloro-4-pyridylaminocarbonyl)-4-methoxy-spiro[2,3-dihydrobenzofuran-2,1'-cyclopentane] (Compound 129)

Substantially the same procedure as in Example 1 was repeated using Compound IIav (1.00 g) obtained in Reference Example 48 to give Compound 129 (1.33 g, 84%) as pale-yellow crystals.
Melting point: 156-158 °C
NMR(CDCl₃, δ, ppm): 1.80-2.29(m, 8H), 3.20(s, 2H), 3.91(s, 3H), 6.58(d, J=9Hz, 1H), 7.99(d, J=9Hz, 1H), 8.54(s, 2H), 9.42(s, 1H)
IR(KBr, cm⁻¹): 1690, 1552, 1495, 1271
MASS (m/e) : 392 (M⁺)

**Elemental analysis: C₁₉H₁₈N₂O₃Cl₂**

| | | | |
|---|---|---|---|
| Found (%) | C:58.06, | H:4.56, | N:6.94 |
| Calcd. (%) | C:58.03, | H:4.61, | N:7.12 |

### Example 130

### 4-Methoxy-7-(4-pyridylaminocarbonyl)-spiro[2,3-dihydrobenzofuran-2,1'-cyclopentane]-methanesulfonate (Compound 130)

Substantially the same procedure as in Example 6 was repeated using Compound IIav (1.00 g) obtained in Reference Example 48 to give 4-methoxy-7-(4-pyridylaminocarbonyl)-spiro[2,3-dihydrobenzofuran-2,1'-cyclopentane] (0.88 g, 63%) as pale-yellow crystals. Then, substantially the same procedure as in Example 50 was repeated using the obtained crystals to give Compound 130.
Melting point: 164 °C (decomposed)
NMR(DMSO-d₆, δ, ppm): 1.75-1.88(m, 6H), 2.10-2.22(m, 2H), 2.31(s, 3H), 3.18(s, 2H), 3.89(s, 3H), 6.76 (d, J=9Hz, 1H), 7.72(d, J=9Hz, 1H), 8.13(d, J=7Hz, 1H), 8.75(d, J=7Hz, 1H), 10.5(s, 1H)
IR(KBr, cm⁻¹): 1693, 1612, 1512, 1267
MASS(m/e): 324 (M⁺)

**Elemental analysis: C₁₉H₂₀N₂O₃·CH₃SO₃H·0.3H₂O**

| | | | |
|---|---|---|---|
| Found (%) | C:56.45, | H:5.78, | N:6.52 |
| Calcd. (%) | C:58.41, | H:5.82, | N:6.58 |

### Example 131

### 7-[2-(3,5-Dichloro-4-pyridyl)ethenyl]-4-methoxy-spiro[2,3-dihydrobenzofuran-2,1'-cyclopentane] (Compound 131)

### (Step A) 7-[2-(3,5-Dichloro-4-pyridyl)-1-hydroxyethyl]-4-methoxy-spiro[2,3-dihydrobenzofuran-2,1'-cyclopentane] (Compound 131a)

Substantially the same procedure as in Step A of Example 45 was repeated using Compound IIau (1.00 g) obtained in Reference Example 47 to give Compound 131a (1.32 g, 78%) as pale-yellow crystals.
NMR(CDCl₃, δ, ppm): 1.70-2.20(m, 8H), 2.91(d, J=9Hz, 1H), 3.11(s, 2H), 3.25(dd, J=5, 13Hz, 1H), 3.61 (dd, J=9, 13Hz, 1H), 3.82(s, 3H), 4.94-5.03(m, 1H), 6.35(d, J=9Hz, 1H), 6.98(d, J=9Hz, 1H), 8.43(s, 1H)
MASS(m/e): 393(M⁺)

### (Step B) (Compound 131)

Substantially the same procedure as in Example 67 was repeated using Compound 131a (0.66 g) obtained in Step A to give Compound 131 (0.55 g, 87%) as yellow crystals.
Melting point: 99-101 °C
NMR(CDCl₃, δ, ppm): 1.65-2.20(m, 8H), 3.11(s, 2H), 3.82(s, 3H), 6.38(d, J=9Hz, 1H), 7.13(d, J=9Hz, 1H), 7.45(d, J=17Hz, 1H), 7.50(d, J=17Hz, 1H), 8.43(s, 2H)
IR(KBr, cm⁻¹): 1612, 1556, 1500, 1232
MASS (m/e) : 375(M⁺)

**Elemental analysis: C₂₀H₁₉NO₂Cl₂**

| | | | |
|---|---|---|---|
| Found (%) | C:64.14, | H:5.19, | N:3.57 |
| Calcd. (%) | C:63.84, | H:5.09, | N:3.72 |

### Example 132

### 7-[2-(3,5-Dichloro-9-pyridyl)-1-oxoethyl]-4-methoxy-spiro[2,3-dihydrobenzofuran-2,1'-cyclopentane] (Compound 132)

Substantially the same procedure as in Example 95 was repeated using Compound 131a (0.66 g) obtained in Step A in Example 131 to give Compound 132 (0.23 g, 35%) as white crystals.
Melting point: 70-72 °C
NMR(CDCl₃, δ, ppm): 1.78-2.24(m, 8H), 3.16(s, 2H), 3.90(s, 3H), 4.63(s, 2H), 6.51(d, J=9Hz, 1H), 7.82(d, J=9Hz, 1H), 8.49(s, 2H)
IR(KBr, cm⁻¹): 1668, 1427, 1297, 1093
MASS(m/e): 391(M⁺)

**Elemental analysis: C₂₀H₁₉NO₃Cl₂**

| | | | |
|---|---|---|---|
| Found (%) | C:61.30, | H:4.84, | N:3.41 |
| Calcd. (%) | C:61.24, | H:4.88, | N:3.57 |

### Example 133

### 4-Methoxy-7-[1-oxo-2-(4-pyridyl)ethyl]-spiro[2,3-dihydrobenzofuran-2,1'-cyclopentane] (Compound 133)

Substantially the same procedure as in Example 98 was repeated using Compound IIaw (0.86 g) obtained in Reference Example 49 to give Compound 133 (0.42 g, 40%) as white crystals.
Melting point: 101-103 °C
NMR(CDCl₃, δ, ppm): 1.73-2.17(m, 8H), 3.11(s, 2H), 3.88(s, 3H), 4.26(s, 2H), 6.49(d, J=9Hz, 1H), 7.17-7.19(m, 2H), 7.81(d, J=9Hz, 1H), 8.50-8.53 (m, 2H)
IR(KBr, cm⁻¹): 1680, 1612, 1430, 1248
MASS (m/e) : 323 (M⁺)

**Elemental analysis: C₂₀H₂₁NO₃**

| | | | |
|---|---|---|---|
| Found (%) | C:79.63, | H:6.68, | N:4.26 |
| Calcd. (%) | C:74.28, | H:6.54, | N:4.33 |

### Example 134

### 7-(3,5-Dichloro-4-pyridylaminocarbonyl)-4-methoxy-spiro[1,3-benzodioxole-2,1'-cyclopentane] (Compound 134)

Substantially the same procedure as in Example 1 was repeated using Compound IIaz (0.70 g) obtained in Reference Example 52 to give Compound 134 (0.73 g, 66%) as white crystals.
Melting point: 168-170 °C
NMR(CDCl₃, δ, ppm): 1.84-1.96(m, 4H), 2.24-2.31(m, 4H), 3.97(s, 3H), 6.67(d, J=9Hz, 1H), 7.60(d, J=9Hz, 1H), 8.55(s, 2H), 8.78(s, 1H)
IR(KBr, cm⁻¹): 1689, 1641, 1490, 1286
MASS(m/e): 394 (M⁺)

**Elemental analysis: C₁₈H₁₆N₂O₄Cl₂**

| | | | |
|---|---|---|---|
| Found (%) | C:54.57, | H:4.05, | N:6.95 |
| Calcd. (%) | C:54.70, | H:4.08, | N:7.09 |

### Example 135

### 4-Methoxy-7-(4-pyridylaminocarbonyl)-spiro[1,3-benzodioxole-2,1'-cyclopentane]·methanesulfonate (Compound 135)

Substantially the same procedure as in Example 6 was repeated using Compound IIaz (0.84 g) obtained in Reference Example 52 to give 4-methoxy-7-(4-pyridylaminocarbonyl)-spiro[1,3-benzodioxole-2,1'-cyclopentane] (0.34 g, 31%) as pale-yellow crystals. Then, substantially the same procedure as in Example 50 was repeated using the obtained crystals to give Compound 135.
Melting point: 133-134 °C
NMR(DMSO-d₆, δ, ppm): 1.77-1.83(m, 4H), 2.06-2.22(m, 4H), 2.31(s, 3H), 3.90(s, 3H), 6.84(d, J=9Hz, 1H), 7.36(d, J=9Hz, 1H), 8.18(d, J=7Hz, 2H), 8.73 (d, J=7Hz, 2H), 10.9(s, 1H)
IR(KBr, cm⁻¹): 1637, 1508, 1280, 1120
MASS(m/e): 326(M⁺)

**Elemental analysis: C₁₈H₁₈N₂O₄·CH₃SO₃H·0.3H₂O**

| | | | |
|---|---|---|---|
| Found (%) | C:53.34, | H:5.20, | N:6.58 |
| Calcd. (%) | C:53.34, | H:5.32, | N:6.55 |

### Example 136

### 4-Methoxy-7-[2-(4-pyridyl)ethyl]-spiro[1,3-benzodioxole-2,1'-cyclopentane]·hydrochloride (Compound 136)

Substantially the same procedure as in Example 120 was repeated using Compound 138 (0.86 g) obtained in Example 138 to give 4-methoxy-7-[2-(4-pyridyl)ethyl]-spiro[1,3-benzodioxole-2,1'-cyclopentane] (0.078 g, 99%) as pale-yellow crystals. Then, substantially the same procedure as in Example 51 was repeated using the obtained crystals to give Compound 136.
Melting point: 160-162 °C
NMR(DMSO-d₆, δ, ppm): 1.71-2.01(m, 8H), 2.89(t, J=7Hz, 2H), 3.15(t, J=7Hz, 2H), 3.75(s, 3H), 6.51(d, J=9Hz, 1H), 6.61(d, J=9Hz, 1H), 7.83(d, J=6Hz, 2H), 8.79(d, J=6Hz, 2H)
IR(KBr, cm⁻¹): 1640, 1508, 1456, 1333
MASS (m/e) : 311(M⁺)

**Elemental analysis: C₁₉H₂₁NO₃·HCl·0.2H₂O**

| | | | |
|---|---|---|---|
| Found (%) | C:64.82, | H:6.35, | N:3.82 |
| Calcd. (%) | C:64.93, | H:6.42, | N:3.99 |

### Example 137

### 4-Methoxy-7-[1-phenyl-2-(4-pyridyl)ethyl]-spiro[1,3-benzodioxole-2,1'-cyclopentane]·hydrochloride (Compound 137)

Substantially the same procedure as in Example 120 was repeated using Compound 139 (0.76 g) obtained in Example 139 to give 4-methoxy-7-[1-phenyl-2-(4-pyridyl)-ethyl]-spiro[1,3-benzodioxole-2,1'-cyclopentane] (0.75 g, 98%) as a pale-yellow oily substance. Then, substantially the same procedure as in Example 51 was repeated using the obtained crystals to give Compound 137.
Melting point: 179-182 °C
NMR(DMSO-d₆, δ, ppm): 1.75-2.00(m, 8H), 3.69-3.71(m, 2H), 3.72(s, 3H), 4.48(t, J=8Hz, 1H), 6.51(d, J=9Hz, 1H), 6.76(d, J=9Hz, 1H), 7.16-7.38(m, 5H), 7.84(d, J=5Hz, 2H), 8.75(d, J=5Hz, 2H)
IR(KBr, cm⁻¹): 1645, 1633, 1504
MASS(m/e): 387(M⁺)

**Elemental analysis: C₂₅H₂₅NO₃·HCl·0.3H₂O**

| | | | |
|---|---|---|---|
| Found (%) | C:70.07, | H:6.23, | N:3.17 |
| Calcd. (%) | C:69.94, | H:6.24, | N:3.26 |

### Example 138

### 7-[2-(3,5-Dichloro-4-pyridyl)ethenyl]-4-methoxy-spiro[1,3-benzodioxole-2,1'-cyclopentane] (Compound 138)

### (Step A) 7-[2-(3,5-Dichloro-4-pyridyl)-1-hydroxyethyl]-4-methoxy-spiro[1,3-benzodioxole-2,1'-cyclopentane] (Compound 138a)

Substantially the same procedure as in Step A of Example 45 was repeated using Compound IIax (0.47 g) obtained in Reference Example 50 to give Compound 138a (0.73 g, 92%) as pale-yellow crystals.
NMR(CDCl₃, δ, ppm): 1.75-2.15(m, 8H), 3.09(d, J=6Hz, 1H), 3.31(dd, J=6, 13Hz, 1H), 3.51(dd, J=9, 13Hz, 1H), 3.87(s, 3H), 5.09-5.15(m, 1H), 6.46(d, J=9Hz, 1H), 6.79(d, J=9Hz, 1H), 8.34(s, 2H)
MASS(m/e): 395(M⁺)

### (Step B) (Compound 138)

Substantially the same procedure as in Example 67 was repeated using Compound 138a (0.74 g) obtained in Step A to give Compound 138 (0.59 g, 80%) as yellow crystals.
Melting point: 100-101 °C
NMR(CDCl₃, δ, ppm): 1.82-1.94(m, 4H), 2.14-2.26(m, 4H), 3.91(s, 3H), 6.51(d, J=9Hz, 1H), 6.87(d, J=9Hz, 1H), 7.30(d, J=16Hz, 1H), 7.42(d, J=16Hz, 1H), 8.45(s, 2H)
IR(KBr, cm⁻¹): 1618, 1452, 1288, 1113
MASS(m/e): 377(M⁺)

**Elemental analysis: C₁₉H₁₇NO₃Cl₂**

| | | | |
|---|---|---|---|
| Found (%) | C:60.39, | H:4.49, | N:3.65 |
| Calcd. (%) | C:60.33, | H:4.53, | N:3.70 |

### Example 139

### 4-Methoxy-7-[1-phenyl-2-(4-pyridyl)ethenyl]-spiro[1,3-benzodioxole-2,1'-cyclopentane] (Compound 139)

### (Step A) 7-[1-Hydroxy-1-phenyl-2-(4-pyridyl)ethyl]-4-methoxy-spiro[1,3-benzodiozole-2,1'-cyclopentane] (Compound 139a)

Substantially the same procedure as in Step A of Example 47 was repeated using Compound IIba (4.90 g) obtained in Reference Example 53 to give Compound 139 (5.34 g, 84%) as pale-yellow crystals.
NMR(CDCl₃, δ, ppm): 1.69-2.10(m, 8H), 3.10 (s, 1H), 3.46(d, J=12Hz, 1H), 3.69(d, J=12Hz, 1H), 3.87(s, 3H), 6.44(d, J=9Hz, 1H), 6.71(d, J=9Hz, 1H), 6.93(d, J=6Hz, 2H), 7.22-7.39(m, 5H), 8.37(d, J=6Hz, 2H)
MASS(m/e): 403(M⁺)

### (Step B) (Compound 139) (an E/Z mixture)

Substantially the same procedure as in Example 67 was repeated using Compound 139a (2.0 g) obtained in Step A to give Compound 139 (0.76 g, 40%) as pale-yellow crystals.
NMR(CDCl₃, δ, ppm): 0.83-2.22(m, 8H), 3.88(s, 3H x 0.75), 3.92(s, 3H x 0.25), 6.39(s, 2H x 0.75), 6.49-6.53(m, 2H x 0.25), 6.79(d, J=6Hz, 2H x 0.75), 6.88(s, 1H x 0.25), 7.00(d, J=6Hz, 2H x 0.25), 7.20(s, 1H x 0.75), 7.15-7.38(m, 5H), 8.31(d, J=6Hz, 2H x 0.75), 8.40(d, J=6Hz, 2H x 0.25)
MASS(m/e): 385(M⁺)

### Example 140

### 7-[2-(3,5-Dichloro-4-pyridyl)-1-oxoethyl]-4-methoxy-spiro[1,3-benzodioxole-2,1'-cyclopentane] (Compound 140)

Substantially the same procedure as in Example 95 was repeated using Compound 138a (1.50 g) obtained in Step A of Example 138 to give Compound 140 (0.77 g, 52%) as white crystals.
Melting point: 110-112 °C
NMR(CDCl₃, δ, ppm): 1.83-1.96(m, 4H), 2.18-2.28(m, 4H), 3.97(s, 3H), 4.59(s, 2H), 6.61(d, J=9Hz, 1H), 7.47(d, J=9Hz, 1H), 8.50(s, 2H)
IR(KBr, cm⁻¹) : 1633, 1448, 1286, 1263
MASS(m/e): 393(M⁺)

**Elemental analysis: C₁₉H₁₇NO₄Cl₂**

| | | | |
|---|---|---|---|
| Found (%) | C:58.05, | H:4.32, | N:3.52 |
| Calcd. (%) | C:57.88, | H:4.35, | N:3.55 |

### Example 141

### 4-Methoxy-7-[1-oxo-2-(4-pyridyl)ethyl]-spiro[1,3-benzodioxole-2,1'-cyclopentane]·hydrochloride (Compound 141)

Substantially the same procedure as in Example 98 was repeated using Compound IIay (1.0 g) obtained in Reference Example 51 to give 4-methoxy-7-[1-oxo-2-(4-pyridyl)ethyl]-spiro[1,3-benzodioxole-2,1'-cyclopentane] (0.33 g, 27%) as white crystals. Then, substantially the same procedure as in Example 51 was repeated using the obtained crystals to give Compound 141.
Melting point: 110-111 °C
NMR(CDCl₃, δ, ppm): 1.75-1.88(m, 4H), 2.18-2.28(m, 4H), 3.90(s, 3H), 4.62(s, 2H), 6.82(d, J=9Hz, 1H), 7.38(d, J=9Hz, 1H), 7.92(d, J=5Hz, 2H), 8.84(d, J=5Hz, 2H)
IR(KBr, cm-¹): 1668, 1633, 1446, 1119

### Example 142

### 7-Methoxy-4-[2-(4-pyridyl)ethynyl]-spiro[2,3-dihydrobenzofuran-2,1'-cyclopentane] (Compound 142)

### (Step A) 6-Bromo-4-[1,2-dibromo-2-(4-pyridyl)ethyl]-7-methoxy-spiro[2,3-dihydrobenzofuran-2,1'-cyclopentane] (Compound 142a)

Bromine (0.1 ml) was dropwise added to a solution of (E)-7-methoxy-4-[2-(4-pyridyl)ethenyl]-spiro[2,3-dihydrobenzofuran-2,1'-cyclopentane] (0.18 g) obtained in Example 74 in dichloromethane (15 ml) at 0°C, followed by stirring at the same temperature for 30 minutes. Water was added to the reaction solution and the mixture was extracted with chloroform. The organic layer was washed with a saturated saline and dried over anhydrous magnesium sulfate, and the solvent was distilled off under reduced pressure. The residue was purified by silica gel column chromatography (ethyl acetate/n-hexane = 1/2) to give Compound 142a (0.26 g, 81.2%) as pale-yellow crystals.
NMR(DMSO-d₆, δ, ppm): 1.50-2.15(m, 8H), 3.24(d, J=15.3Hz, 1H), 3.65(d, J= 15.8Hz, 1H), 3.82(s, 3H), 5.90(d, J=11.8Hz, 1H), 6.15(d, J=12.3Hz, 1H), 7.13(s, 1H), 7.67(d, J=5.9Hz, 2H), 8.69(d, J=5.4Hz, 2H)

### (Step B) 6-Bromo-7-methoxy-4-[2-(4-pyridyl)ethynyl]-spiro[2,3-dihydrobenzofuran-2,1'-cyclopentane] (Compound 142b)

Potassium tert-butoxide (0.15 g) was added to a solution of Compound 142a (0.25 g) obtained in Step A in THF (9 ml) at 0°C, followed by stirring at room temperature for 5 hours. The reaction solution was poured into water and the mixture was extracted with diethyl ether. The organic layer was washed with a saturated saline and dried over anhydrous magnesium sulfate, and the solvent was distilled off under reduced pressure. The residue was purified by silica gel column chromatography (ethyl acetate/n-hexane = 1/1) to give Compound 142b (0.12 g, 68.2%) as pale-yellow crystals.
NMR(DMSO-d₆, δ, ppm): 1.70-1.95(m, 6H), 2.05-2.25(m, 2H), 3.32(s, 2H), 3.88(s, 3H), 6.97(s, 1H), 7.39 (d, J=5.4Hz, 2H), 8.60(d, J=5.4Hz, 2H)
MASS(m/e): 383, 385(M⁺)

### (Step C) (Compound 142)

Under an argon atmosphere, a solution (2.6 ml) of Compound 142b (0.1 g) obtained in Step B in THF was cooled to -78°C, and then a 1.7M solution (0.2 ml) of n-butyl lithium in hexane was dropwise added thereto, followed by stirring at the same temperature for one hour. The reaction solution was adjusted to pH 7 by adding droppwise 1N hydrochloric acid, followed by stirring at room temperature for one hour. A small amount of water was added to the reaction solution and the mixture was extracted with ether. The organic layer was washed with a saturated saline and dried over anhydrous magnesium sulfate, and the solvent was distilled off under reduced pressure. The residue was purified by silica gel column chromatography (ethyl acetate/n-hexane = 1/2) to give Compound 142 (0.014 g, 17.4 %) as pale-yellow crystals.
Melting point: 128-131 °C
NMR(DMSO-d₆, δ, ppm) : 1.42(s, 6H), 3.15(s, 2H), 3.80 (s, 3H), 3.87(s, 3H), 6.94(s, 2H), 7.62(d, J=8.4Hz, 2H), 7.99(d, J=8.4Hz, 2H)
IR(KBr, cm⁻¹): 2216, 1589, 1506
MASS(m/e): 305(M⁺)

### Example 143

### 7-Methoxy-4-[1-oxo-2-(N-oxo-4-pyridyl)ethyl]-spiro[2,3-dihydrobenzofuran-2,1'-cyclopentane] (Compound 143)

m-Chloroperbenzoic acid (0.72 g) was added to a solution of 7-methoxy-4-[1-oxo-2-(4-pyridyl)ethyl]-spiro[2,3-dihydrobenzofuran-2,1'-cyclopentane] (0.27 g) obtained in Example 100 in dichloromethane (8.3 ml) at 0°C, followed by stirring at room temperature for 5 hours. A saturated aqueous solution of sodium bicarbonate was added to the reaction solution and the mixture was extracted with ethyl acetate. The organic layer was washed with a saturated saline and dried over anhydrous magnesium sulfate, and the solvent was distilled off under reduced pressure. The residue was purified by silica gel column chromatography (chloroform/methanol = 15/1) to give Compound 143 (0.07 g, 24.8 %) as pale-yellow crystals.
NMR(DMSO-d₆, δ, ppm): 1.72-1.91(m, 6H), 2.10-2.16(m, 2H), 3.51(s, 2H), 3.95(s, 3H), 4.24(s, 2H), 6.81 (d, J=8.6H=, 2H), 7.18(d, J=6.9Hz, 2H), 7.45(d, J=8.6Hz, 1H), 8.20(d, J=6.9Hz, 2H)
MASS(m/e): 339(M⁺)

### Example 144

### 7-Methoxy-4-[4-(methoxycarbonyl)phenyl]-spiro[2,3-dihydrobenzofuran-2,1'-cyclopentane] (Compound 144)

### (Step A) 7-Methoxy-4-tributylstannyl-spiro[2,3-dihydrobenzofuran-2,1'-cyclopentane] (Compound 144a)

Under an argon atmosphere, a solution (80 ml) of Compound IIa-c (2.0 g) obtained in Step C of Reference Example 1 in THF was cooled to -78°C, and then a solution (5.0 ml) of 1.70M butyl lithium in hexane was dropwise added thereto, followed by stirring at the same temperature for one hour. Tributyltin chloride (2.1 ml) was dropwise added to the mixture, followed by stirring at room temperature for 2 hours and then at 60°C for one hour. The solvent was distilled off and the residue was dried under reduced pressure to give a crude desired product. This product was immediately subjected to a subsequent step without being purified.

### (Step B) (Compound 144)

A solution (30 ml) of Compound 144a obtained in Step A in DMF was added to a mixture of methyl 4-bromobenzoate (1.67 g), palladium acetate (0.18 g), sodium carbonate (2.10 g), and dimethylformamide (DMF) (70 ml), followed by stirring at 80°C for one hour. A small amount of water was added to the reaction solution and the mixture was extracted with ethyl acetate. The organic layer was washed with 1N hydrochloric acid and a saturated saline and dried over anhydrous magnesium sulfate, and the solvent was distilled off under reduced pressure. The residue was purified by silica gel column chromatography (ethyl acetate/hexane = 1/20) to give Compound 144 (1.35 g, 55.6%) as colorless crystals.
Melting point: 116-122 °C
NMR(DMSO-d₆, δ, ppm): 1.42(s, 6H), 3.15(s, 2H), 3.80 (s, 3H), 3.87(s, 3H), 6.94(s, 2H), 7.62(d, J=8.4Hz, 2H), 7.99(d, J=8.4Hz, 2H)
IR(KBr, cm⁻¹): 1720, 1606
MASS(m/e): 312(M⁺)

**Elemental analysis: C₁₉H₂₀O₄**

| | | | |
|---|---|---|---|
| Found (%) | C:73.19, | H:6.58, | N:0.12 |
| Calcd. (%) | C:73.06, | H:6.45, | N:0.00 |

### Example 145

### 4-(4-Carboxyphenyl)-7-methoxy-spiro[2,3-dihydrobenzofuran-2,1'-cyclopentane] (Compound 145)

A mixture of Compound 144 (1.0 g) obtained in Example 144, a 4N aqueous solution (8.0 ml) of sodium hydroxide, and ethanol (40 ml) was stirred at room temperature for 4 hours. The solvent was removed and the residue was dissolved in water. Concentrated hydrochloric acid was dropwise added to the solution, and the generated precipitate was collected by filtration, washed with water, and dried to give Compound 145 (0.82 g, 85.9%) as white crystals.
Melting point: 249-252 °C
NMR (DMSO-d₆, δ, ppm): 1.42(s, 6H), 3.15(s, 2H), 3.80 (s, 3H), 6.94(s, 2H), 7.59(d, J=8.4Hz, 2H), 7.98 (d, J=7.9Hz, 2H), 12.94(brs, 1H)
IR(KBr, cm⁻¹): 1681, 1606
MASS (m/e) : 298(M⁺)

**Elemental analysis: C₁₈H₁₈O₄**

| | | | |
|---|---|---|---|
| Found (%) | C:72.51, | H:6.18, | N:0.15 |
| Calcd. (%) | C:72.47, | H:6.08, | N:0.00 |

### Example 146

### 7-Methoxy-4-[3-(methoxycarbonyl)phenyl]-spiro[2,3-dihydrobenzofuran-2,1'-cyclopentane] (Compound 146)

A solution (30 ml) of Compound 144a obtained in Step A of Example 144 in DMF was added to a mixture of methyl 4-bromobenzoate (1.67 g), palladium acetate (0.18 g), sodium carbonate (2.10 g), and dimethylformamide (DMF) (70 ml), followed by stirring at 80°C for one hour. A small amount of water was added to the reaction solution and the mixture was extracted with ethyl acetate. The organic layer was washed with 1N hydrochloric acid and a saturated saline and dried over anhydrous magnesium sulfate, and the solvent was distilled off under reduced pressure. The residue was purified by silica gel column chromatography (ethyl acetate/hexane = 1/20) to give Compound 146 (1.69 g, 69.5%) as pale-yellow crystals.
Melting point: 89-91 °C
NMR(DMSO-d₆, δ, ppm): 1.42(s, 6H), 3. 12 (s, 2H), 3.80 (s, 3H), 3.88(s, 3H), 6.90(d, J=8.4Hz, 1H), 6.95 (d, J=8.4Hz, 1H), 7.58(dd, J=7.4Hz, 1H), 7.76(dd, J=7.9, 1.5Hz, 1H), 7.91(d, J=7.4Hz, 1H), 7.99(d, J=1.5Hz, 1H)
IR(KBr, cm⁻¹): 1716
MASS(m/e): 312(M⁺)

### Example 147

### 4-(3-Carboxyphenyl)-7-methoxy-spiro(2,3-dihydrobenzofuran-2,1'-cyclopentane] (Compound 147)

A mixture of Compound 146 (1.3 g) obtained in Example 146, a 4N aqueous solution (10.4 ml) of sodium hydroxide, and ethanol (50 mi) was stirred at room temperature for 3 hours. The solvent was removed and the residue was dissolved in water. Concentrated hydrochloric acid was dropwise added to the solution, and the generated precipitate was collected by filtration, washed with water, and dried to give Compound 147 (1.15 g, 92.7%) as white crystals.
Melting point: 220-225 °C
NMR (DMSO-d₆, δ, ppm) : 1.42(s, 6H), 3.12(s, 2H), 3. 79 (s, 3H), 6.90(d, J=8.4Hz, 1H), 6.95(d, J=8.4Hz, 1H), 7.55(dd, J=7.4Hz, 1H), 7.72(dd, J=6.4, 1.5Hz, 1H), 7.89(dd, J=6.4, 1.5Hz, 1H), 7.97(d, J=1.5Hz, 1H), 13.17(brs, 1H)
IR(KBr, cm⁻¹): 1683
MASS (m/e): 298(M⁺)

**Elemental analysis: C₁₈H₁₈O₄**

| | | | |
|---|---|---|---|
| Found (%) | C:72.21, | H:6.02, | N:0.05 |
| Calcd. (%) | C:72.47, | H:6.08, | N:0.00 |

### Reference Example 1

### 7-Methoxy-2,2-dimethyl-2,3-dihydrobenzofuran-4-carbaldehyde] (Compound IIa)

### (Step A) 2-(2-Methyl-2-propen-1-yloxy)-4-bromoanisole (Compound IIa-a)

A mixture of 5-bromo-2-methoxyphenol (17.8 g), 3-chloro-2-methyl-1-propene (13.0 ml), potassium carbonate (18.2 g), and DMF (150 ml) was stirred at 80°C for 2 hours. The mixture was diluted with toluene, washed with a saturated saline and dried over anhydrous magnesium sulfate, and the solvent was distilled off to give Compound IIa-a (22.2 g, 98.41) as a colorless oily substance.
NMR(DMSO-d₆, δ, ppm): 1.76(s, 3H), 3.76(s, 3H), 4.48 (s, 2H), 4.96(s, 1H), 5.05(s, 1H), 6.92(d, J=8.41Hz, 1H), 7.04-7.11(m, 2H)

### (Step B) 3-Bromo-6-methoxy-2-(2-methyl-2-propen-1-yl)phenol (Compound IIa-b)

Compound IIa-a (22.2 g) obtained in Step A was dissolved in 1-methylpyrrolidinone (50 ml) followed by stirring at 180°C for 5 hours. The mixture was extracted with ethyl acetate, washed with a saturated saline and dried over anhydrous magnesium sulfate, and the solvent was distilled off. The residue was purified by silica gel column chromatography (chloroform) to give Compound IIa-b (19.6 g, 88.5%) as a colorless oily substance.
NMR(DMSO-d₆, δ, ppm): 1.74(s, 3H), 3.37(s, 2H), 3.79 (s, 3H), 4.31(s, 1H), 4.68(s, 1H), 6.81(d, J=8.58Hz, 1H), 7.00(d, J=8.91Hz, 1H)

### (Step C) 4-Bromo-7-methoxy-2,2-dimethyl-2,3-dihydrobenzofuran (Compound IIa-c)

Compound IIa-b (19.6 g) obtained in Step B was dissolved in 88% formic acid (80 ml) followed by stirring at room temperature for 24 hours. The mixture was neutralized with an aqueous solution of sodium bicarbonate and extracted with toluene. The organic layer was washed with a saturated saline and dried over anhydrous magnesium sulfate, and the solvent was distilled off. The residue was purified by silica gel column chromatography (chloroform) to give Compound IIa-c (16.3 g, 83.3%) as an oily substance.
NMR(DMSO-d₆, δ, ppm) : 1.43(s, 6H), 2.99(s, 2H), 3.74 (s, 3H), 6.79(d, J=8.58Hz, 1H), 6.93(d, J=8.57Hz, 1H)
MASS(m/z): 258, 256(M⁺)

### (Step D) (Compound IIa)

Under an argon atmosphere, a solution (300 ml) of Compound IIa-c (20.0 g) obtained in Step C in THF was cooled to -78°C, and then a 1.69M solution (50.6 ml) of butyl lithium in hexane was dropwise added thereto. The reaction solution was gradually warmed and stirred at -20°C for one hour, and then DMF (200 ml) was dropwise added thereto, followed by stirring at room temperature for 2 hours. A small amount of water was added to the reaction solution and the mixture was extractied with ether. The organic layer was washed with a saturated saline and dried over anhydrous magnesium sulfate, and the solvent was distilled off under reduced pressure. The residue was purified by silica gel column chromatography (chloroform/hexane = 9/1) to give Compound IIa (7.11 g, 44.3%) as colorless crystals.
NMR(DMSO-d₆, δ, ppm): 1.41(s, 6H), 3.28(s, 2H), 3.84 (s, 3H), 7.04(d, J=8.25Hz, 1H), 7.39(d, J=8.24Hz, 1H), 9.85(s, 1H)
MASS (m/z) : 206, 191

### Reference Example 2

### 2,2-Diethyl-7-methoxy-2,3-dihydrobenzofuran-4-carbaldehydel (Compound IIb)

### (Step A) 4-Bromo-2-(3-oxopentan-2-yloxy)anisole (Compound IIb-a)

A mixture of 5-bromo-2-methoxyphenol (50.0 g), 2-bromo-3-pentanone (68.1 g), potassium carbonate (52.8 g), and DMF (500 ml) was stirred at 70°C for 2 hours. After being allowed to stand for cooling, water was added to the mixture followed by extraction with ether. The organic layer was washed with a saturated saline and dried over anhydrous magnesium sulfate, and the solvent was distilled off. The residue was purified by silica gel column chromatography (hexane:chloroform = 1:1) to give Compound IIb-a (86.8 g, 94.0%) as a pale-yellow oily substance.
NMR(DMSO-d₆, δ, ppm): 0.93(t, J=7.4Hz, 3H), 1.39(d, J=6.9Hz, 3H), 2.47-2.75(m, 2H), 3.77(s, 3H), 4.92(q, J=6.9Hz, 1H), 6.96(d, J=8.9Hz, 1H), 7.00 (d, J=2.7Hz, 1H), 7.10(dd, J=8.9. 2.5Hz, 1H)
MASS(m/e): 287(M⁺), 285

### (Step B) 4-Bromo-2-(3-methylenepentan-2-yloxy)anisole (Compound IIb-b)

Methyltriphenylphosphonium bromide (308.1 g) was suspended in THF (1 ℓ), and potassium t-butoxide (92.4 g) was added thereto under ice-cooling, followed by stirring for one hour under ice-cololing. A solution of Compound IIb-a (86.0 g) obtained in Step A in THF (500 ml) was dropwise added to the suspension under ice-cooling, followed by stirring for 2 hours. Water was added to the mixture and the resultant was extracted with ethyl acetate. The organic layer was washed with a saturated saline and and dried over anhydrous magnesium sulfate. The residue was purified by silica gel column chromatography (hexane:chloroform = 1:1) to give Compound IIb-b (74.8 g, 87.9%) as a pale-yellow oily substance.
NMR(DMSO-d₆, δ, ppm): 1.00(t, J=7.4Hz, 3H), 1.37(d, J=6.4Hz, 3H), 2.04(m, 2H), 3.32(s, 3H), 4.84-4.91(m, 1H), 4.86(s, 1H), 5.05(s, 1H), 6.90(d, J=7.4Hz, 1H), 7.02-7.05(m, 2H)
MASS(m/e): 286, 284(M⁺)

### (Step C) 3-Bromo-2-(2-ethyl-2-buten-1-yl)-6-methoxyphenol (Compound IIb-c)

Compound IIb-b (62.0 g) obtained in Step B was dissolved in 1-methylpyrrolidinone (68 ml) followed by stirring at 170°C for 2 hours. After being allowed to stand for cooling, a saturated saline was added to the mixture followed by extraction with ethyl acetate. The organic layer was dried over anhydrous magnesium sulfate and the solvent was distilled off to give Compound IIb-c (73.9 g) as a crude pale-yellow oily substance.
NMR(DMSO-d₆, δ, ppm): 0.99(t, J=7.4Hz, 3H), 1.48(d, J=6.9Hz, 3H), 2.04(q, J=7.4Hz, 2H), 3.37(s, 2H), 4.71(q, J=6.9Hz, 1H), 6.79(d, J=8.9Hz, 1H), 6.98 (d, J=8.91Hz, 1H), 8.86(brs, 1H)
MASS(m/e): 286, 284(M⁺)

### (Step D) 4-Bromo-2,2-diethyl-7-methoxy-2,3-dihydrobenzofuran (Compound IIb-d)

Compound IIb-c (73.9 g) obtained in Step C was dissolved in methanol (740 ml), and sulfuric acid (74 ml) was dropwise added thereto under ice-cooling, followed by heating under reflux for 3 hours. After being allowed to stand for cooling, the mixture was concentrated and water was added thereto, followed by extraction with ethyl acetate. The organic layer was washed with a saturated saline and dried over anhydrous magnesium sulfate, and the solvent was distilled off. The residue was purified by silica gel column chromatography (hexane:ethyl acetate = 8:1) to give Compound IIb-d (70.9 g, 83.0% from Compound IIb-b) as a pale-yellow oily substance.
NMR(DMSO-d₆, δ, ppm): 0.86(t, J=7.4Hz, 6H), 1.69(q, J=7.4Hz, 4H), 2.95(s, 2H), 3.73(s, 3H), 6.77(d, J=8.9Hz, 1H), 6.90(d, J=8.4Hz, 1H)

### (Step E) 2,2-Diethyl-7-methoxy-2,3-dihydrobenzofuran-4-carbaldehyde (Compound IIb)

Under an argon atmosphere, a solution (600 ml) of Compound IIb-d (61.6 g) obtained in Step D in THF was cooled to -78°C, and then a 1.69M solution (197 ml) of n-butyl lithium in hexane was dropwise added, followed by stirring at the same temperature for 2 hours. DMF (37 ml) was added to the reaction solution and the mixture was stirred at room temperature for 2 hours. A small amount of water was added to the reaction solution and the mixture was extracted with ethyl acetate. The organic layer was washed with a saturated saline and dried over anhydrous magnesium sulfate, and the solvent was distilled off. The residue was purified by silica gel column chromatography (ethyl acetate/hexane = 1/5) to give Compound IIb (43.6 g, 86.0%) as colorless crystals.
NMR(DMSO-d₆, δ, ppm) : 0.85 (t, J=7.4Hz, 6H), 1.70(q, J=7.4Hz, 4H), 3.26(s, 2H), 3.87 (s, 3H), 7.03(d, J=8.4Hz, 1h), 7.38(d, J=8.4Hz, 1H), 9.88 (s, 1H)
MASS (m/e) : 234 (M⁺), 205

### Reference Example 3

### 7-Methoxy-spiro[2,3-dihydrobenzofuran-2,1'-cyclopentane]-4-carbaldehyde (Compound IIc)

### (Step A) 4-Bromo-2-(2-oxocyclopentyloxy)anisole (Compound IIc-a)

A mixture of 5-bromo-2-methoxyphenol (120.0 g), 2-chloro-1-cyclopentanone (100.0 g), potassium carbonate (163.3 g), and DMF (1.2 ℓ) was stirred at 70°C for 3 hours. After being allowed to stand for cooling, water was added to the mixture followed by extraction with ethyl acetate. The organic layer was washed with a saturated saline and dried over anhydrous magnesium sulfate, and the solvent was distilled off. The residue was purified by silica gel column chromatography (hexane:ethyl acetate = 9:1) to give Compound IIc-a (141.43 g, 83.9%) as a pale-yellow oily substance.
NMR(DMSO-d₆, δ, ppm) : 1.78-1.99(m, 3H), 2.21-2.40(m, 3H), 3.74(s, 3H), 4.95(t, J=7.9Hz, 1H), 6.92(d, J=9.4Hz, 1H), 7.09(dd, J=2.0Hx, 9.4Hz, 1H), 7.22 (d, J=2.0Hz, 1H)
MASS(m/z) : 286, 284(M⁺)

### (Step B) 4-Bromo-2-(2-methylenecyclopentyloxy)anisole (Compound IIc-b)

Methyltriphenylphosphonium bromide (510.3 g) was suspended in THF (2.5 ℓ), and potassium t-butoxide (153.1 g) was added thereto under ice-cooling, followed by stirring for 3 hours under ice-cooling. A solution of Compound IId-a (141.43 g) obtained in Step A in THF (1.0 ℓ) was dropwise added to the suspension under ice-cooling, followed by stirring for one hour. Water was added to the mixture followed by extraction with ether. The organic layer was washed with a saturated saline and and dried over anhydrous magnesium sulfate. The residue was purified by silica gel column chromatography (hexane:chloroform = 1:1) to give Compound IIc-b (108.4 g, 70.7%) as a pale-yellow oily substance.
NMR(DMSO-d₆, δ, ppm): 1.66-1.98(m, 4H), 2.21-2.42(m, 2H), 3.74(s, 3H), 5.01-5.05(m, 3H), 6.92(d, J=8.6Hz, 1H), 7.08(dd, J=1.0Hz, 8.6Hz, 1H), 7.22 (d, J=1.0Hz, 1H)
MASS(m/e): 284, 282(M⁺)

### (Step C) 3-Bromo-2-[(2-cyclopenten-1-yl)methyl]-6-methoxyphenol (Compound IIc-c)

Compound IIc-b (108.4 g) obtained in Step B was dissolved in 1-methylpyrrolidinone (110 ml) followed by stirring at 170°C for 3 hours. After being allowed to stand for cooling, a saturated saline was added to the mixture followed by extraction with ethyl acetate. The organic layer was dried over anhydrous magnesium sulfate and the solvent was distilled off to give Compound IIc-c (129.7 g) as a crude pale-yellow oily substance.
NMR(DMSO-d₆, δ, ppm): 1.78(m, 2H), 2.19-2 .25(m, 4H), 3.43(s, 2H), 3.78(s, 3H), 5.06(t, J=2.0Hz, 1H), 6.79(d, J=8.9, 1H), 6.99(d, J=8.9Hz, 1H), 8.92(s, 1H)
MASS (m/e) : 285, 283(M⁺)

### (Step D) 4-Bromo-7-methoxy-spiro[2,3-dihydrobenzofuran-2,1'-cyclopentane] (Compound IIc-d)

Compound IIc-c (129.7 g) obtained in Step C was dissolved in methanol (1.3 ℓ), and sulfuric acid (130 ml) was dropwise added thereto under ice-cooling, followed by heating under reflux for 3 hours. After being allowed to stand for cooling, water was added followed by extraction with ethyl acetate. The organic layer was successively washed with a saturated aqueous solution of sodium bicarbonate and a saturated saline and dried over anhydrous magnesium sulfate, and the solvent was distilled off. The residue was purified by silica gel column chromatography (hexane:ethyl acetate = 9:1) to give Compound IIc-d (102.7 g, 94.7% yield from Compound IId-b) as pale-yellow crystals.
Melting point: 45-47 °C
NMR(DMSO-d₆, δ, ppm): 1.71-1.80(m, 6H), 1.96-2.01(m, 2H), 3.16(s, 2H), 3.74(s, 3H), 6.78(d, J=8.4Hz, 1H), 6.92(d, J=8.9Hz, 1H)
MASS (m/e) : 285, 283(M⁺)

### (Step E) (Compound IIc)

Under an argon atmosphere, a solution (700 ml) of Compound IIc-d (102.7 g) obtained in Step D in THF was cooled to -78°C, and then a 1.56M solution (360 ml) of n-butyl lithium in hexane was dropwise added thereto, followed by stirring at the same temperature for one hour. DMF (62 ml) was added to the reaction solution and the mixture was stirred at the same temperature for 2 hours. A small amount of water was added to the reaction solution and the mixture was extracted with ethyl acetate. The organic layer was washed with a saturated saline and dried over anhydrous magnesium sulfate, and the solvent was distilled off. The residue was purified by silica gel column chromatography (chloroform) to give Compound IIc (77.4 g, 91.9%) as colorless crystals.
Melting point: 50-52 °C
NMR(DMSO-d₆, δ, ppm) : 1.75-1.86(m, 6H), 1.92-2.02(m, 2H), 3.46(s, 2H), 3.86(s, 3H), 7.04(d, J=8.4Hz, 1H), 7.40(d, J=8.4Hz, 1H), 9.88(s, 1H)
MASS (m/e) : 232 (M⁺)

### Reference Example 4

### 7-Methoxy-spiro[2,3-dihydrobenzofuran-2,1'-cyclohexane]-4-carbaldehyde (Compound IId)

### (Step A) 4-Bromo-2-(2-oxocyclohexyloxy)anisole (Compound IId-a)

A mixture of 5-bromo-2-methoxyphenol (120.0 g), 2-chloro-1-cyclohexanone (108.0 g), potassium carbonate (163.3 g), and DMF (1.2 ℓ) was stirred at 70°C for 3 hours. After being allowed to stand for cooling, water was added to the mixture followed by extraction with ethyl acetate. The organic layer was washed with a saturated saline and dried over anhydrous magnesium sulfate, and the solvent was distilled off. The residue was purified by silica gel column chromatography (hexane:ethyl acetate = 9:1) to give Compound IId-a (138.5 g, 78.3%) as pale-yellow crystals.
Melting point: 71-73 °C
NMR(DMSO-d₆, δ, ppm): 1.54-2.02(m, 5H), 2.28-2.33(m, 2H), 2.50-2.73(m, 1H), 3.75(s, 3H), 5.03(m, 1H), 6.91(d, J=8.4Hz, 1H), 6.99(d, J=2.5Hz, 1H), 7.04 (dd, J=8.4, 2.0Hz, 1H)
MASS(m/e): 300, 298(M⁺), 204, 202

### (Step B) 4-Bromo-2-(2-methylenecyclohexyloxy)anisole (Compound IId-b)

Methyltriphenylphosphonium bromide (476.0 g) was suspended in THF (1.3 ℓ), and potassium t-butoxide (143.0 g) was added thereto under ice-cooling, followed by stirring for 3 hours under ice-cooling. A solution of Compound IId-a (138.5 g) obtained in Step A in THF (1.0 ℓ) was dropwise added to the suspension under ice-cooling, followed by stirring at room temperature for one hour. Water was added to the mixture followed by extraction with ether. The organic layer was washed with a saturated saline and dried over anhydrous magnesium sulfate. The residue was purified by silica gel column chromatography (hexane:chloroform = 1:1) to give Compound IId-b (133.7 g, 97.3%) as a pale-yellow oily substance.
NMR (DMSO-d₆, δ, ppm) : 1.44-1.89 (m, 6H), 2.06-2.11 (m, 1H), 2.26-2.30 (m, 1H), 3.76 (s, 3H), 4.76 (t, J=4.0Hz, 1H), 4.79(s, 2H), 6.90(d, J=8.4Hz, 1H), 7.05 (dd, J=2.5, 8.4Hz, 1H), 7.08(d, J=2.5Hz, 1H)
MASS(m/e): 298, 296(M⁺), 204, 202

### (Step C) 3-Bromo-2-[(2-cyclohexen-1-yl)methyl]-6-methoxyphenol (Compound IId-c)

Compound IId-b (133.7 g) obtained in Step B was dissolved in 1-methylpyrrolidinone (160 ml) followed by stirring at 170°C for 2 hours. After being allowed to stand for cooling, a saturated saline was added to the mixture followed by extraction with ethyl acetate. The organic layer was dried over anhydrous magnesium sulfate and the solvent was distilled off to give Compound IId-c (169.5 g) as a crude pale-yellow oily substance.
NMR(DMSO-d₆, δ·ppm): 1.44-1.59(m, 4H), 1.87-1.99(m, 4H), 3.31(s, 2H), 3.79(s, 3H), 5.05(t, J=1.5Hz, 1H), 6.78(d, J=8.9Hz, 1H), 6.98(d, J=8.9Hz, 1H), 8.85(s, 1H)
MASS(m/e): 298, 296(M⁺), 217, 215

### (Step D) 4-Bromo-7-methoxy-spiro[2,3-dihydrobenzofuran-2,1'-cyclohexane] (Compound IId-d)

Compound IId-c (169.5 g) obtained in Step C was dissolved in methanol (1.4 ℓ), and sulfuric acid (170 ml) was dropwise added thereto under ice-cooling, followed by heating under reflux for 2 hours. After being allowed to stand for cooling, the mixture was concentrated, and water was added thereto, followed by extraction with ethyl acetate. The organic layer was successively washed with a saturated aqueous solution of sodium bicarbonate and a saturated saline and dried over anhydrous magnesium sulfate, and the solvent was distilled off. The residue was purified by silica gel column chromatography (hexane:ethyl acetate = 9:1) to give Compound IId-d (127.8 g, 95.6% from Compound IId-b) as orange-yellow crystals.
NMR(DMSO-d₆, δ, ppm): 1.43-1.50(m, 4H), 1.65-1.77(m, 6H), 2.94(s, 2H), 3.74(s, 3H), 6.78(d, J=8.4Hz, 1H), 6.92(d, J=8.4Hz, 1H)
MASS(m/e): 298, 296(M⁺), 217, 215

### (Step E) (Compound IId)

Under an argon atmosphere, a solution (1.0 ℓ) of Compound IId-d (100.0 g) obtained in Step D in THF was cooled to -78°C, and a 1.70M solution (307 ml) of n-butyl lithium in hexane was dropwise added thereto, followed by stirring at the same temperature for one hour. DMF (60 ml) was added to the reaction solution and the mixture was stirred at the same temperature for 2 hours. A small amount of water was added to the reaction solution followed by extraction with ethyl acetate. The organic layer was washed with a saturated saline and dried over anhydrous magnesium sulfate, and the solvent was distilled off. The residue was purified by silica gel column chromatography (hexane/ethyl acetate = 6/1) to give Compound IId (78.9 g, 95.1%) as colorless crystals.
NMR(DMSO-d₆, δ, ppm): 1.30-1.61(m, 4H), 1.66-1.76(m, 6H), 3.25 (s, 2H), 3.87 (s, 3H), 7.04 (d, J=8.4Hz, 1H), 7.39(d, J=8.4Hz, 1H), 9.87(s, 1H)
MASS(m/e) : 246(M⁺)

### Reference Example 5

### (±)-7-Methoxy-3-methyl-2,3-dihydrobenzofuran-4-carbaldehyde (Compound IIe)

### (Step A) 3-Allyloxy-2-bromo-4-methoxybenzaldehyde (Compound IIe-a)

2-Bromo-3-hydroxy-4-methoxybenzaldehyde (1.68 g) was dissolved in DMF (17 ml), and sodium hydride (0.209 g) was added thereto under ice-cooling, followed by stirring for 30 minutes. Allyl bromide (0.944 ml) was added to the mixture, followed by stirring at 60°C for one hour. After being allowed to stand for cooling, water was added to the mixture and the precipitated solid was collected by filtration. The obtained crude crystals were recrystallized from isopropanol to give Compound IIe-a (1.30 g, 66%).
Melting point: 75-78 °C
NMR(CDCl₃, δ, ppm): 3.96(s, 3H), 4.57(d, J=8.3Hz, 2H), 5.19-5.50(m, 2H), 6.02-6.27(m, 1H), 6.95(d, J=9.3Hz, 1H), 7.75(d, J=9.3Hz, 1H), 10.27(s, 1H)

### (Step B) (Compound IIe)

A mixture of Compound IIe-a (0.436 g) obtained in Step A, tributyltin hydride (0.519 ml), and azobisisobutyronitrile (AIBN) (26.4 mg) was heated under reflux for 5 hours. Further, tributyltin hydride (1.3 ml) and AIBN (52 mg) were added to the mixture followed by heating under reflux one night. After being allowed to stand for cooling, ether and a 50% aqueous solution of KF were added to the mixture, followed by stirring at room temperature for 5 hours. The insoluble matters were filtered off and the filtrate was extracted with ether. The organic layer was dried over anhydrous magnesium sulfate and concentrated. The residue was purified by silica gel column chromatography (toluene/ethyl acetate = 20/1) to give Compound IIe (0.184 g, 60%) as an oily substance.
NMR(CDCl₃, δ, ppm): 1.31(d, J=7.2Hz, 3H), 3.86-4.07(m, 1H), 3.97(s, 3H), 4.40(dd, J=8.8, 4.5Hz, 1H), 4.60-4.72(m, 1H), 6.89(d, J=9.0Hz, 1H), 7.36(d, J=9.0Hz, 1H), 9.91(s, 1H)

### Reference Example 6

### 7-Methoxy-2-(4-pyridyl)benzofuran-4-carbaldehyde (Compound IIf)

### (Step A) 7-Methoxy-2-(4-pyridyl)benzofuran (Compound IIf-a)

Ortho-vanillin (93.0 g) and 4-picolyl chloride hydrochloride (100 g) as starting materials were dissolved in DMF (1200 ml), and potassium carbonate (337 g) and potassium iodide (30 g) were added thereto, followed by heating under reflux for 24 hours while stirring. The reaction solution was filtered using celite, the solvent was distilled off under reduced pressure, and the residue was extracted with ethyl acetate. The organic layer was washed with a saturated saline and dried over.anhydrous magnesium sulfate, and the solvent was distilled off under reduced pressure. The residue was purified by silica gel column chromatography (hexane:ethyl acetate = 1:2) and further washed with diethyl ether to give Compound IIf-a (26.7 g, 19.4%) as pale-yellow needles.
NMR(CDCl₃, δ, ppm): 4.09(s, 3H), 6.84(dd, J=2Hz, 7Hz, 1H), 7.1-7.2(m, 3H), 7.70(d, J=6Hz, 2H), 8.65(d, J=6Hz, 2H)
MASS(m/e): 225(M⁺)

### (Step B) (Compound IIf)

Under a nitrogen stream, Compound IIf-a (3.70 g) obtained in Step A was dissolved in dichloromethane (60 ml) followed by stirring at -10°C, and titanium tetrachloride (4.00 ml) dissolved in dichloromethane (10 ml) was dropwise added thereto over 5 minutes at the same temperature. Then, dichloromethyl methyl ether (1.60 ml) was added to the mixture at the same temperature, and the mixture was warmed to room temperature followed by stirring for 20 minutes. The reaction solution was poured into ice-water containing potassium hydroxide (about 10 g) followed by stirring for some time, and the mixture was filtered using celite. The filtrate was extracted with ethyl acetate, the organic layer was washed with a saturated saline and dried over anhydrous magnesium sulfate, and the solvent was distilled off under reduced pressure. The residue was purified by silica gel column chromatography (hexane:ethyl acetate = 1:2) to give Compound IIf (2.60 g, 62.9%) as a white solid.
Melting point: 178-179 °C
NMR(CDCl₃, δ, ppm) : 4.15(s, 3H), 6. 96 (d, J=8Hz, 1H), 7.72(d, J=8Hz, 1H), 7.78(d, J=6Hz, 2H), 8.01(s, 1H), 8.72(d, J=6Hz, 2H), 10.06(s, 1H)
MASS(m/e): 252(M⁺-1), 224
IR(KBr, cm⁻¹): 1670, 1606, 1573

### Reference Example 7

### 7-Methoxy-2-(2-pyridyl)benzofuran-4-carbaldehyde (Compound IIg)

### (Step A) 7-Methoxy-2-(2-pyridyl)benzofuran (Compound IIg-a)

Substantially the same procedure as in Step A of Reference Example 6 was repeated using ortho-vanillin (10.0 g) and using 2-picolyl chloride hydrochloride (11.0 g) instead of 4-picolyl chloride hydrochloride to give Compound IIg-a (4.23 g, 26.5%) as colorless needles.
NMR(CDCl₃, δ, ppm): 4.03(s, 3H), 6.84(dd, J=1Hz, 8Hz, 1H), 7.18(dd, J=8Hz, 8Hz, 1H), 7.23(ddd, J=1Hz, 5Hz, 8Hz, 1H), 7.25(dd, J=1Hz, 8Hz, 1H), 7.45(s, 1H), 7.76(ddd, J=2H z, 8Hz, 8Hz, 1H), 7.98(ddd, J=1Hz, 1Hz, 8Hz, 1H), 8.65(ddd, J=1Hz, 2Hz, 5Hz, 1H)
MASS(m/e): 225(M⁺)

### (Step B) (Compound IIg)

Substantially the same procedure as in Step B of Reference Example 6 was repeated using Compound IIg-a (5.00 g) obtained in Step A to give Compound IIg (3.81 g, 67.8%) as a white solid.
Melting point: 143-144 °C
NMR(CDCl₃, δ, ppm): 4.11(s, 3H), 6.92(d, J=9Hz, 1H), 7.27(dd, J=6Hz, 8Hz, 1H), 7.72(d, J=9Hz, 1H), 7.79(ddd, J=2Hz, 8Hz, 8Hz, 1H), 7.95(d, J=8Hz, 1H), 8.12(s, 1H), 8.72(dd, J=2Hz, 6Hz, 1H), 10.09(s, 1H)
MASS(m/e) : 253(M⁺), 252
IR(KBr·cm⁻¹): 1670, 1575, 1475, 1309

### Reference Example 8

### 7-Methoxy-2-phenylbenzofuran-4-carbaldehyde (Compound IIh)

### (Step A) 7-Methoxy-2-(4-nitrophenyl)benzofuran (Compound I Ih-a)

Substantially the same procedure as in Step A of Reference Example 6 was repeated using ortho-vanillin (50.0 g) and using 4-nitrobenzyl chloride (59.0 g) instead of 4-picolyl chloride hydrochloride to give Compound IIh-a (53.0 g, 59.8%) as a yellow solid.
NMR(CDCl₃, δ, ppm): 4.03(s, 3H), 6.89(dd, J=2Hz, 8Hz, 1H), 7.1-7.3(m, 3H), 8.00(d, J=9Hz, 2H), 8.29(d, J=9Hz, 2H)
MASS(m/e): 269(M⁺), 239, 223

### (Step B) 7-Methoxy-2-phenylbenzofuran (Compound IIh-b)

Compound IIh-a (26.0 g) obtained in Step A was dissolved in ethanol (400 ml)/distilled water (40 ml), and reduced iron (26.0 g) and iron (III) chloride (1.56 g) were added thereto, followed by heating under reflux for 2 hours. The reaction solution was filtered using celite, the filtrate was concentrated under reduced pressure, and the residue was extracted with ethyl acetate. The organic layer was dried over magnesium sulfate and the solvent was distilled off under reduced pressure. The residue was dissolved in tetrahydrofuran (400 ml), and sodium nitrite (10 g) and phosphinic acid (a 32-36% aqueous solution, 400 ml) were added thereto with stirring at 0°C, followed by stirring for 7 hours. The reaction solution was adjusted to alkaline by slowly adding a 1N aqueous solution of potassium hydroxide, and then the organic layer was extracted with dichloromethane. The organic layer was dried over magnesium sulfate and the solvent was distilled off under reduced pressure. The residue was purified by silica gel column chromatography (hexane:ethyl acetate = 8:1) to give Compound IIh-b (16.6 g, 77.1%) as a white solid.
NMR(CDCl₃, δ, ppm) : 4.06(s, 3H), 6.81(dd, J=2Hz, 7Hz, 1H), 7.02(s, 1H), 7.15(dd, J=7Hz, 7Hz, 1H), 7.17 (t, J=7Hz, 1H), 7.36(dd, J=2Hz, 7Hz, 1H), 7.44 (dd, J=7Hz, 8Hz, 2H), 7.89(d, J=8Hz, 2H)
MASS(m/e): 224(M⁺)

### (Step C) (Compound IIh)

Substantially the same procedure as in Step B of Reference Example 6 was repeated using Compound IIh-b (16.0 g) obtained in Step B to give Compound IIh (6.86 g, 38.0%) as a white solid.
Melting point: 110-111 °C
NMR(CDCl₃, δ, ppm): 4.12(s, 3H), 6.87(d, J=9Hz, 1H), 7.3-7.5(m, 3H), 7.62(d, J=9Hz, 1H), 7.78(s, 1H), 7.91(d, J=8Hz, 2H), 10.05(s, 1H)
MASS(m/e): 252(M⁺), 251
IR(KBr, cm⁻¹): 1683, 1621, 1581, 1396, 1265, 1174

### Reference Example 9

### 2-Cyano-7-methoxybenzofuran-4-carbaldehyde (Compound IIi)

A mixture of 2-cyano-7-methoxybenzofuran (4.17 g), hexamethylenetetramine (3.38 g), and trifluoroacetic acid (62 ml) was stirred at 60 to 70°C for one hour. The mixture was concentrated and the residue was purified by silica gel column chromatography (toluene/ethyl acetate = 20/1) to give Compound IIi (1.02 g, 21%) as colorless crystals.
Melting point: 170-178 °C
NMR(CDCl₃, δ, ppm): 4.14(s, 3H), 7.10(d, J=8.1Hz, 1H), 7.82(d, J=8.1Hz, 1H), 8.21(s, 1H), 10.05(s, 1H)

### Reference Example 10

### 3-Ethoxycarbonylmethyl-7-methoxybenzofuran-4-carbaldehyde (Compound IIj)

### (Step A) 3-[(E)-3-Ethoxycarbonyl-2-propen-1-oxy]-2-iodo-4-methoxybenzaldehyde (Compound IIj-a)

Substantially the same procedure as in Step A of Reference Example 1 was repeated using 3-hydroxy-2-iodo-4-methoxybenzaldehyde (13 g) to give Compound IIj-a (18 g, 100%) as a dark brown oily substance.
NMR(CDCl₃, δ, ppm): 1.32(t, J=7Hz, 3H), 3.95(s, 3H), 4.24(q, J=7Hz, 2H), 4.68(dd, J=2, 4Hz, 2H), 6.35 (dt, J=2, 16Hz, 1H), 7.00(d, 9Hz, 1H), 7.13(dt, J=4, 16Hz, 1H), 7.75(d, J=9Hz, 1H), 10.0(s, 1H)
MASS(m/e): 390(M⁺)

### (Step B) (Compound IIj)

A mixture of Compound IIj-a (18 g) obtained in Step A, THF-acetonitrile (1:1) (18 ml), triethylamine (7.8 ml), and palladium acetate (0.73 g) was heated under reflux for 3 hours. The catalyst was removed and the filtrate was concentrated. Ethyl acetate was added to the residue, and the mixture was washed with dilute hydrochloric acid and a saturated saline and dried over sodium sulfate. The solvent was distilled off and the residue was purified by silica gel column chromatography (hexane:ethyl acetate = 2:1) to give Compound IIj (11 g, 87%) as pale-yellow crystals.
Melting point: 45-50 °C
NMR(CDCl₃, δ, ppm) : 1.27(t, J=7Hz, 3H), 4.05(s, 2H), 4.09(s, 3H), 4.18(q, J=7Hz, 2H), 6.91(d, 9Hz, 1H), 7.70(d, J=9Hz, 1H), 9.93(s, 1H)

### Reference Example 11

### Methyl 2,2-diethyl-7-methoxy-2,3-dihydrobenzofuran-4-carboxylate (Compound IIk)

### (Step A) Methyl 4-methoxy-3-(1-methyl-2-oxobutan-1-yloxy) benzoate (Compound IIk-a)

A mixture of methyl 3-hydroxy-4-methoxy benzoate (19.3 g), 2-bromo-3-pentanone (19.2 ml), potassium carbonate (29.3 g), and DMF (193 ml) was stirred at 90°C for 2 hours. After being allowed to stand for cooling, water was added to the mixture followed by extraction with toluene. The organic layer was washed with a saturated saline and dried over sodium sulfate, and the solvent was distilled off. The residue was purified by column chromatography (silica gel, hexane:ethyl acetate = 3:1) to give Compound IIk-a (25.8 g, 91.5%) as a colorless oily substance.
NMR(CDCl₃, δ, ppm) : 1.08(t, J=5.8Hz, 3H), 1.52(d, J=7.0Hz, 3H), 2.47-2.90(m, 2H), 3.88(s, 3H), 3.93(s, 3H), 4.71(q, J=7.0Hz, 1H), 6.92(d, J=8.6Hz, 1H), 7.47(d, J=1.0Hz, 1H), 7.74(dd, J=1.0, 8.6Hz, 1H)
MASS (m/e) : 266(M⁺)

### (Step B) Methyl 4-methoxy-3-(1-methyl-2-methylenebutan-1-yloxy)benzoate (Compound IIk-b)

Methyltriphenylphosphonium bromide (48.5 g) was suspended in ether (485 ml), and a 1.7N solution (78.8 ml) of n-butyl lithium in hexane was dropwise added thereto under ice-cooling. The mixture was stirred at room temperature for 30 minutes and then cooled with ice again. Compound IIb-a (25.8 g) obtained in Step A was dissolved in ether (120 ml). The solution was dropwise added to the mixture, followed by stirring for 30 minutes under ice-cooling. Water was added to the mixture followed by extraction with ethyl acetate. The organic layer was washed with a saturated saline and dried over sodium sulfate, and the solvent was distilled off. The residue was purified by column chromatography (silica gel, hexane:ethyl acetate = 10:1) to give Compound IIk-b (20.5 g, 80%) as a colorless oily substance.
NMR(CDCl₃, δ, ppm): 1.10(t, J=7.6Hz, 3H), 1.51(d, J=7.0Hz, 3H), 2.02-2.20(m, 2H), 3.88(s, 3H), 3.91(s, 3H), 4.81(q, J=7.0Hz, 1H), 4.90(s, 1H), 5.10(s, 1H), 6.88(d, J=8.4Hz, 1H), 7.53(d, J=1.1Hz, 1H), 7.65(dd, J=1.1, 8.4Hz, 1H)
MASS(m/e): 264(M⁺)

### (Step C) Methyl 3-hydroxy-4-methoxy-2-(2-ethyl-2-buten-1-yl)benzoate (Compound IIk-c)

Compound IIk-b (20.3 g) obtained in Step B was dissolved in 1-methylpiperidone (22 ml) followed by stirring at 120°C one night and then at 180°C for 2 hours. After being allowed to stand for cooling, a saturated saline was added to the mixture followed by extraction with ethyl acetate. The organic layer was dried over sodium sulfate and the solvent was distilled off. The residue was purified by column chromatography (silica gel, hexane:ethyl acetate = 10:1) to give an oily Compound IIk-c (17.0 g, 84%) as a mixture of isomers (5:1).
NNR(CDCl₃, δ, ppm): 0.89(t, J=7.6Hz, 0.2H), 1.06(t, J=7.6Hz, 0.8H), 1.52(d, J=7.7Hz, 0.8H), 1.75(d, J=7.7Hz, 0.2H), 2.10 and 2.12(each q, J=7.6Hz, total 2H), 3.77 and 3.78(each s, total 2H), 3.81 and 3.82(each s, total 3H), 3.91 and 3.92 (each s, total 3H), 4.80(q, J=7.7Hz, 0.8Hz), 5.31(q, J=7.7Hz, 0.2H), 5.79(s, 0.8H), 5.87(s, 0.2H), 6.74 and 6.76(each d, J=8.4Hz, total 1H), 7.40 (d, J=8.4Hz, 0.2H), 7.49(d, J=8.4Hz, 0.8H)
MASS (m/e) : 264 (M⁺)

### (Step D) Methyl 2,2-diethyl-7-methoxy-2,3-dihydrobenzofuran-4-carboxylate (Compound IIk)

Compound IIk-c (16.8 g) obtained in Step C was dissolved in methanol (170 ml), and sulfuric acid (20 ml) was dropwise added thereto under ice-cooling, followed by heating under reflux one night. After being allowed to stand for cooling, the mixture was concentrated and poured into a 1N aqueous solution of sodium hydroxide under ice-cooling. The mixture was extracted with ethyl acetate, and the organic layer was washed with a saturated saline and dried over sodium sulfate. The solvent was distilled off, and the residue was purified by column chromatography (silica gel, hexane:ethyl acetate = 10:1 and 3:1) to give Compound IIk (12.0 g, 73%) as a pale-yellow oily substance.
NMR(CDCl₃, δ, ppm) : 0.95(t, J=8.0Hz, 6H), 1.80(q, J=8.0Hz, 4H), 3.34(s, 2H), 3.88(s, 3H), 3.92(s, 3H), 6.77(d, J=8.4Hz, 1H), 7.52(d, J=8.4Hz, 1H)
MASS (m/e) : 264 (M⁺).

### Reference Example 12

### Methyl 7-methoxy-spiro(2,3-dihydrobenzofuran-2,1'-cyclopentane)-4-carboxylate (Compound III)

### (Step A) 4-Bromo-2-(2-oxocyclopentyloxy)anisole (Compound IIl-a)

A mixture of 5-bromo-2-methoxyphenol (6.31 g), α-chlorocyclopentanone (6.9 ml), potassium carbonate (9.57 g), and DMF (63 ml) was Stirred at 90°C for 2 hours, α-Chlorocyclopentanone (14 ml) was further added to the mixture, followed by stirring at 90°C for one hour. After being allowed to stand for cooling, water was added to the mixture followed by extraction with ether. The organic layer was washed with a 1N aqueous solution of sodium hydroxide and then with a saturated saline, and dried over sodium sulfate. The solvent was distilled off, and the residue was purified by column chromatography (silica gel, hexane:ethyl acetate = 2:1) to give Compound IIl-a (11.8 g, 99%) as an oily substance.
NMR(CDCl₃, δ, ppm): 1.80-2.60(m, 6H), 3.89(s, 3H), 3.90(s, 3H), 4.65-4.77(m, 1H), 6.90(d, J=8.4Hz, 1H), 7.62(d, J=2.0Hz, 1H), 7.72(dd, J=8.4, 2.0Hz, 1H)
MASS(m/z) : 264(M⁺)

### (Step B) 4-Bromo-2-(2-methylenecyclopentyloxy)anisole (Compound II1-b)

Methyltriphenylphosphonium bromide (66.2 g) was suspended in THF (600 ml), and a 1M solution (185 ml) of potassium t-butoxide in THF was dropwise added thereto under ice-cooling, followed by stirring for 30 minutes under ice-cooling. Compound IIl-a (35.0 g) obtained in Step A was dissolved in THF (150 ml). The solution was dropwise added to the mixture under ice-cooling followed by stirring for 15 minutes. Water was added to the mixture followed by extraction with ethyl acetate. The organic layer was washed with a saturated saline and dried over sodium sulfate. The residue was purified by column chromatography (silica gel, hexane:ethyl acetate = 10:1) to give Compound IIl-b (24.5 g, 71%) as an oily substance.
NMR(CDCl₃, δ, ppm): 1.60-2.65(m, 6H), 3.90(s, 3H), 3.91(s, 3H), 4.95-5.05(m, 1H), 5.09-5.20(m, 2H), 6.90(d, J=8.4Hz, 1H), 7.62(d, J=2.2Hz, 1H), 7.70 (dd, J=8.4, 2.2Hz, 1H)
MASS(m/e): 262(M⁺)

### (Step C) 3-Bromo-2-[(2-cyclopenten-1-yl)methyl]-4-methoxyphenol (Compound IIl-c)

Compound IIl-b (29.4 g) obtained in Step B was dissolved in 1-methylpiperidinone (32 ml) followed by stirring at 140°C for 3 hours. After being allowed to stand for cooling, a saturated saline was added to the mixture followed by extraction with ethyl acetate. The organic layer was dried over sodium sulfate and the solvent was distilled off. The residue was purified by column chromatography (silica gel, hexane:ethyl acetate = 7:1) to give Compound IIl-c (26.4 g, 90%) as an oily substance.
NMR(CDCl₃, δ, ppm): 1.76-1.93(m, 2H), 2.16-2.38(m, 4H), 3.82(s, 2H), 3.82(s, 3H), 3.94(s, 3H), 5.01-5.11(m, 1H), 5.78(s, 1H), 6.75(d, J=8.5Hz, 1H), 7.50(d, J=8.5Hz, 1H)
MASS(m/e): 262(M⁺)

### (Step D) (Compound IIl)

Compound IIl-c (0.274 g) obtained in Step C was dissolved in methanol (10 ml), and sulfuric acid (1 ml) was dropwise added thereto under ice-cooling, follwed by heating under reflux one night. After being allowed to stand for cooling, the mixture was concentrated and poured into a 1N aqueous solution of sodium hydroxide under ice-cooling. The mixture was extracted with ethyl acetate, and the organic layer was washed with a saturated saline and dried over sodium sulfate.

The solvent was distilled off, and the residue was purified by column chromatography (silica gel, hexane:ethyl acetate = 10:1) to give Compound III (0.223 g, 82%) as an oily substance.
NMR(CDCl₃, δ, ppm): 1.66-2.25(m, 8H), 3.51(s, 2H), 3.89(s, 3H), 3.92(s, 3H), 6.78(d, J=8.7Hz, 1H), 7.53(d, J=8.7Hz, 1H)

### Reference Example 13

### Methyl 7-methoxy-spiro[2,3-dihydrobenzofuran-2,1'-cyclohexane]-4-carboxylate (Compound IIm)

### (Step A) Methyl 4-methoxy-3-(2-oxocyclohexyloxy)benzoate (Compound IIm-a)

A mixture of methyl 3-hydroxy-4-methoxybenzoate (2.47 g), α-chlorocyclohexanone (2.33 ml), potassium carbonate (3.76 g), and DMF (25 ml) was stirred at 90°C for 2 hours. α-Chlorocyclohexanone (2.0 ml) was further added to the mixture, followed by stirring at 90°C for one hour. After being allowed to stand for cooling, water was added to the mixture followed by extraction with ether. The organic layer was washed with a 1N aqueous solution of sodium hydroxide and then with a saturated saline, and dried over sodium sulfate. The solvent was distilled off, and the residue was purified by column chromatography (silica gel, hexane:ethyl acetate = 2:1) to give Compound IIm-a (3.16 g, 83%) as an oily substance.
Melting point: 66-69 °C
NMR(CDCl₃, δ, ppm): 1.65-1.90(m, 2H), 1.96-2.14(m, 3H), 2.32-2.72(m, 3H), 3.87(s, 3H), 3.92(s, 3H), 4.69-4.82(m, 1H), 6.90(d, J=8.0Hz, 1H), 7.43(d, J=1.5Hz, 1H), 7.70(dd, J=8.0, 1.5Hz, 1H)
MASS(m/e) : 278(M⁺)

### (Step B) Methyl 3-(2-methylenecyclohexyloxy)-4-methoxy-benzoate (Compound IIm-b)

Methyltriphenylphosphonium bromide (40.4 g) was suspended in ether (400 ml), and a 1.7N solution (64.8 ml) of n-butyl lithium in hexane was dropwise added thereto under ice-cooling, followed by stirring at room temperature for 10 minutes and then cooling with ice again. Compound IIm-a (15.7 g) obtained in Step A was dissolved in ether (16 ml). The solution was dropwise added to the mixture followed by stirring at room temperature for one hour. Water was added to the mixture under ice-cooling followed by extraction with ethyl acetate. The organic layer was washed with a saturated saline and dried over sodium sulfate. The solvent was distilled off, and the residue was purified by column chromatography (silica gel, hexane:ethyl acetate = 10:1) to give Compound IIm-b (9.15 g, 59%) as an oily substance.
NMR(CDCl₃, δ, ppm): 1.45-2.18(m, 7H), 2.37-2.52(m, 1H), 3.88(s, 3H), 3.91(s, 3H), 4.62-4.75(m, 1H), 4.82(s, 1H), 4.90(s, 1H), 6.90(d, J=8.2Hz, 1H), 7.55(d, J=1.3H=, 1H), 7.67(dd, J.=8.2, 1.3Hz, 1H)
MASS(m/e): 276(M⁺)

### (Step C) Methyl 2-[(2-cyclohexen-1-yl)methyl]-3-hydroxy-4-methoxybenzoate (Compound IIm-c)

Compound IIm-b (9.0 g) obtained in Step B was dissolved in 1-methylpiperidinone (10 ml) followed by stirring at 140°C for 3 hours and then at 150°C for 2 hours. After being allowed to stand for cooling, a saturated saline was added to the mixture followed by extraction with ethyl acetate, and the organic layer was dried over sodium sulfate. The solvent was distilled off, and the residue was purified by column chromatography (silica gel, hexane:ethyl acetate = 10:1) to give Compound IIm-c (7.63 g, 85%) as an oily substance.
NMR(CDCl₃, δ, ppm): 1.44-1.70(m, 4H), 1.85-2.07(m, 4H), 3.70(s, 2H), 3.82 (s, 3H), 3.95(s, 3H), 5.07-5.18(m, 1H), 5.79(s, 1H), 6.77(d, J=8.0Hz, 1H), 7.48(d, J=8.0Hz, 1H)
MASS(m/e): 276(M⁺)

### (Step D) (Compound IIm)

Compound IIm-c (7.6 g) obtained in Step C was dissolved in methanol (100 ml), and sulfuric acid (10 ml) was dropwise added thereto under ice-cooling, followed by heating under reflux one night. After being allowed to stand for cooling, the mixture was concentrated, and the residue was poured into a saturated aqueous solution of sodium bicarbonate under ice-cooling. The mixture was extracted with ethyl acetate, the organic layer was washed with a saturated saline, and the solvent was distilled off. The residue was purified by column chromatography (silica gel, hexane:ethyl acetate = 10:1) to give Compound IIm (3.42 g, 45%) as an oily substance.
Melting point: 81-83 °C
NMR(CDCl₃, δ, ppm): 1.25-1.95(m, 10H), 3.32(s, 2H), 3.87(s, 3H), 3.92(s, 3H), 6.77(d, J=8.2Hz, 1H), 7.51(d, J=8.2Hz, 1H).
MASS(m/e): 276(M⁺)

### Reference Example 14

### Methyl (±)-cis-6-methoxy-1,2,3,4,4a,9b-hexahydrodibenzofuran-9-carboxylate (Compound IIn)

### (Step A) 2-Bromo-3-(cyclohex-2-en-1-oxy)-4-methoxy-benzaldehyde (Compound IIn-a)

Diethyl azodicarboxylate (2.7 ml) was dropwise added to a mixture of 2-bromo-3-hydroxy-4-methoxy-benzaldehyde (4.0 g), THF (80 ml), 2-cyclohexen-1-ol (1.2 ml), and triphenylphosphine (4.5 g) under ice-cooling, followed by stirring at room temperature for 2 hours. The mixture was poured into water followed by extraction with ether. The organic layer was washed with a 1N aqueous solution of sodium hydroxide and with a saturated saline, and dried over sodium sulfate. The solvent was distilled off, and the residue was purified by column chromatography (hexane:ethyl acetate = 10:1 and 5:1) to give Compound IIn-a (1.8 g, 47%) as a pale-yellow oily substance.
NMR(CDCl₃, δ, ppm): 1.50-2.25(m, 6H), 3.94(s, 3H), 4.70-4.85(m, 1H), 5.20-6.02(m, 2H), 6.96(d, J=8Hz, 1H), 7.72(d, J=8Hz, 1H), 10.3(s, 1H)
MASS(m/e): 311(M⁺)

### (Step B) (±)-cis-6-Methoxy-1,2,3,4,9a,9b-hexahydrodibenzofuran-9-carbaldehyde (Compound IIn-b)

Substantially the same procedure as in Step B of Reference Example 5 was repeated using Compound IIn-a (1.1 g) obtained in Step A to give Compound IIn-b (0.45 g, 56%) as colorless crystals.
NMR(CDCl₃, δ, ppm): 0.90-1.10(m, 1H), 1.15-1.42(m, 1H), 1.46-1.84(m, 4H), 2.00-2.20(m, 1H), 2.35-2.55(m, 1H), 3.54-3.70(m, 1H), 3.97(s, 3H), 4.60-4.71(m, 1H), 6.88(d, J=8Hz, 1H), 7.35(d, J=8Hz, 1H), 9.90(s, 1H)
MASS (m/e) : 232(M⁺)

### (Step C) (Compound IIn)

Compound IIn-b (0.42 g) obtained in Step B was dissolved in a mixed solvent of dichloromethane (5 ml) and methanol (5 ml) followed by stirring at 0°C, and potassium hydroxide (1.6 g) was added thereto. The mixture was warmed to the room temperature and stirred for 8 hours, while iodine (0.93 g) dissolved in methanol (3 ml) was slowly and dropwise added thereto. Water was added to the reaction solution followed by extraction with dichloromethane. The organic layer was dried over magnesium sulfate and the solvent was distilled off under reduced pressure. The residue was purified by silica gel column chromatography (ethyl acetate/n-hexane = 1/5) to give Compound IIn (0.41 g, 88%) as pale-yellow crystals.
NMR (CDCl₃, δ, ppm) : 0.90-1.10(m, 1H), 1.15 -1.35(m, 1H), 1.45-1.85(m, 4H), 2.05-2.22(m, 1H), 2.35-2.45(m, 1H), 3.50-3.65(m, 1H), 3.87(s, 3H), 3.94 (s, 3H), 4.58-4.66(m, 1H), 6.77(d, J=9Hz, 1H), 7.56(d, J=9Hz, 1H)
MASS (m/e) : 262 (M⁺)

### Reference Example 15

### Methyl 2-butyl-7-methoxybenzofuran-4-carboxylate (Compound IIo)

Compound IIad (1.3 g) obtained in Reference Example 30 was dissolved in methanol (16 ml), and concentrated sulfuric acid (5 ml) was dropwise added thereto under ice-cooling, followed by heating under reflux for one hour. After being allowed to stand for cooling, the solvent was distilled off, and the residue was poured into a 1N aqueous solution of sodium hydroxide. The precipitate was collected by filtration and dried to give Compound IIo (0.82 g, 56%) as a pale-yellow oily substance.
NMR(CDCl₃, δ, ppm) : 0.954(t, J=8Hz, 3H), 1.30-1.56(m, 2H), 1.64-1.89(m, 2H), 2.82(t, J=8Hz, 2H), 3.94 (s, 3H), 4.06(s, 3H), 6.76(d, J=9Hz, 1H), 6.98(s, 1H), 7.91(d, J=9H z, 1H)
MASS(m/e): 262(M⁺)

### Reference Example 16

### Methyl 7-methoxy-2-(2-methylpropyl)benzofuran-4-carboxylate (Compound IIp)

### (Step A) 7-Methoxy-2-(2-methyl-1-propen-1-yl)benzofuran (Compound IIp-a)

Substantially the same procedure as in Step B of Reference Example 2 was repeated using Compound IIad-a (6.2 g) obtained in Step A of Reference Example 30, 2-propyltriphenylphosphonium iodide (20 g), and potassium tert-butoxide (5.1 g) to give Compound IIp-a (5.7 g, 81%) as a pale-yellow oily substance.
NMR(CDCl₃, δ, ppm): 1.96(s, 3H), 2.09(s, 3H), 4.01(s, 3H), 6.20-6.23(brs, 1H), 6.51(s, 1H), 6.75(dd, J=4, 6Hz, 1H), 7.05-7.15(m, 2H)

### (Step B) 7-Methoxy-2-(2-methylpropyl)benzofuran (Compound IIp-b)

Substantially the same procedure as in Step C of Reference Example 30 was repeated using Compound IIp-a (0.4 g) obtained in Step A to give Compound IIp-b (0.8 g, 93%) as a pale-yellow oily substance.
NMR(CDCl₃, δ, ppm): 0.980(d, J=7Hz, 6H), 2.05-2.22(m, 1H), 2.65(d, J=7Hz, 2H), 4.00(s, 3H), 6.37(s, 1H), 6.68-6.80(m, 1H), 7.05-7.15(m, 2H)

### (Step C) 7-Methoxy-2-(2-methylpropyl)benzofuran-4-carbaldehyde (Compound IIp-c)

Substantially the same procedure as in Step D of Reference Example 30 was repeated using Compound IIp-b (0.38 g) obtained in Step B to give Compound IIp-c (0.29 g, 66%) as a pale-yellow oily substance.
NMR(CDCl₃, δ, ppm): 0.999(d, J=8Hz, 6H), 2.05-2.23(m, 1H), 2.70(d, J=8Hz, 2H), 4.10(s, 3H), 6.84(d, J=8Hz, 1H), 7.17(s, 1H), 7.63(d, J=8Hz, 1H), 10.0(s, 1H)

### (Step D) (Compound IIp)

Substantially the same procedure as in Step C of Reference Example 14 was repeated using Compound IIp-c (2.7 g) obtained in Step C to give Compound IIp (3.0 g, 100%) as pale-yellow crystals.
NMR(CDCl₃, δ, ppm): 1.00(d, J=7Hz, 6H), 2.05-2.25(m, 1H), 2.69(d, J=7Hz, 2H), 3.94(s, 3H), 4.06(s, 3H), 6.76(d, J=8Hz, 1H), 6.99(s, 1H), 7.91(d, J=8Hz, 1H)

### Reference Example 17

### Methyl 7-methoxy-2-(4-pyridyl)benzofuran-4-carboxylate (Compound IIq)

Compound IIf (1.80 g) obtained in Reference Example 6 was dissolved in a mixed solvent of dichloromethane (40 ml) and methanol (80 ml) followed by stirring at 0°C, and potassium hydroxide (8.0 g) was added thereto. The mixture was warmed to the room temperature and stirred for 12 hours, while iodine (13.5 g) dissolved in methanol (30 ml) was slowly and dropwise added thereto. The reaction solution was extracted with dichloromethane, the organic layer was dried over magnesium sulfate, and the solvent was distilled off under reduced pressure. The residue was purified by silica gel column chromatography (hexane:ethyl acetate = 1:3) to give Compound IIq (1.50 g, 74.5%) as a white solid.
NMR(CDCl₃, δ, ppm): 4.00(s, 3H), 4.10(s, 3H), 6.87(d, J=9Hz, 1H), 7.78(d, J=7Hz, 2H), 7.85(s, 1H), 7.99(d, J=9Hz, 1H), 8.70(d, J=7Hz, 2H)

### Reference Example 18

### Methyl 7-methoxy-2-(2-pyridyl)benzofuran-4-carboxylate (Compound IIr)

Substantially the same procedure as in Reference Example 17 was repeated using Compound IIg (5.50 g) obtained in Reference Example 7 to give Compound IIr (4.05 g, 65.9%) as a white solid.
Melting point: 148-149 °C
NMR(CDCl₃, δ, ppm): 3.99(s, 3H), 4.10(s, 3H), 6.87(d, J=8Hz, 1H), 7.27(dd, J=6Hz, 8Hz, 1H), 7.78(ddd, J=2Hz, 8Hz, 8Hz, 1H), 7.95(s, 1H), 7.97(d, J=8Hz, 1H), 7.97(d, J=8Hz, 1H), 8.71(dd, J=2Hz, 6Hz, 1H)
MASS(m/e): 283(M⁺), 252
IR(KBr, cm⁻¹): 1712, 1585, 1274, 1265, 1193, 1147

### Reference Example 19

### Methyl 7-methoxy-2-phenylbenzofuran-4-carboxylate (Compound IIs)

Substantially the same procedure as in Reference Example 17 was repeated using Compound IIh (3.00 g) obtained in Reference Example 8 to give Compound IIs (2.72 g, 85.8%) as a white solid.
Melting point: 117-118 °C
NMR(CDCl₃, δ, ppm): 3.97(s, 3H), 4.09(s, 3H), 6.81(d, J=9Hz, 1H), 7.3-7.5(m, 3H), 7.62(s, 1H), 7.93(d, J=9Hz, 1H), 7.94(d, J=9Hz.2H)
MASS (m/e) : 282(M⁺), 251
IR(KBr, cm⁻¹): 1701, 1620, 1292, 1220, 1095

### Reference Example 20

### Methyl 2-(2-ethylphenyl)-7-methoxybenzofuran-4-carboxylate (Compound IIt)

### (Step A) 2-(2-Cyanophenyl)-7-methoxybenzofuran (Compound IIt-a)

Substantially the same procedure as in Step A of Reference Example 17 was repeated using ortho-vanillin (38.8 g) and using α-bromoorthotolunitrile (50.0 g) instead of 4-picolyl chloride hydrochloride to give Compound IIt-a (39.6 g, 62.3%) as colorless needles.
NMR(CDCl₃, δ, ppm): 4.05(s, 3H), 6.87(d, J=8Hz, 1H), 7.1-7.3(m, 2H), 7.41(dd, J=7Hz, 7Hz, 1H), 7.70 (dd, J=8Hz, 8Hz, 1H), 7.74(s, 1H), 7.77(d, J=8Hz, 1H), 8.17(d, J=7Hz, 1H)

### (Step B) 2-(2-formylphenyl)-7-methoxybenzofuran (Compound IIt-b)

Compound IIt-a (26.0 g) obtained in Step A was dissolved in dry dichloromethane (500 ml), and the solution was cooled to -78°C followed by stirring. A 1.0M solution (156 ml) of diisobutylaluminium hydride in toluene was dropwise added to the mixture followed by stirring for one hour while warming the solution to the room temperature. A saturated aqueous solution of ammonium chloride was added to the reaction solution, and ethyl acetate and a 5% aqueous solution of sulfuric acid were added thereto, followed by stirring at room temperature for 30 minutes. The mixture was extracted with ethyl acetate, the organic layer was washed with a saturated saline and dried over anhydrous magnesium sulfate, and the solvent was distilled off under reduced pressure. The residue was washed with diethyl ether to give Compound IIt-b (20.0 g, 76.0%) as a pale-yellow solid.
NMR (CDCl₃, δ, ppm) : 4.03 (s, 3H), 6.86(dd, J=2Hz, 7Hz, 1H), 6.95(s, 1H), 7.2-7.3(m, 2H), 7.53(dd, J=7.5Hz, 7.5Hz, 1H), 7.67(dd, J=2Hz, 6Hz, 1H), 7.87(d, J=8Hz, 1H), 8.04(d, J=7.5Hz, 1H), 10.47 (s, 1H)

### (Step C) 2-(2-Ethenylphenyl)-7-methoxybenzofuran (Compound IIt-c)

Methyltriphenylphosphonium bromide (33.1 g) was dissolved in dry tetrahydrofuran (300 ml) followed by stirring at 0°C, and potassium tert-butoxide (10.0 g) was added thereto, followed by stirring at the same temperature for 30 minutes. Compound IIt-b (9.0 g) obtained in Step B was added to the reaction solution followed by stirring at room temperature for 10 minutes. Then, distilled water was added to the mixture followed by extraction with diethyl ether. The organic layer was washed with a saturated saline and dried over anhydrous magnesium sulfate, and the solvent was distilled off under reduced pressure. The residue was purified by silica gel column chromatography (hexane:ethyl acetate = 3:1) to give Compound IIt-c (7.71 g, 86.3%) as a pale-yellow oily substance.
NMR(CDCl₃, δ, ppm): 4.04(s, 3H), 5.36(d, J=11Hz, 1H), 5.73(d, J=17Hz, 1H), 6.83(dd, J=1Hz, 8Hz, 1H), 6.86(s, 1H), 7.1-7.25(m, 3H), 7.3-7.4(m, 2H), 7.58(m, 1H), 7.85(m, 1H)
MASS(m/e): 250 (M⁺), 207, 165

### (Step D) 2-(2-Ethylphenyl)-7-methoxybenzofuran (Compound IIt-d)

Compound IIt-c (7.7 g) obtained in Step C and palladium-carbon (1.9 g) were added to diethyl ether (200 ml) and the mixture was subjected to hydrogenation while stirring at room temperature. After one hour, the reaction solution was filtered with celite; and the solvent was distilled off under reduced pressure from the filtrate to give Compound IIt-d as a pale-yellow oily substance.
NMR(CDCl₃, δ, ppm): 1.30(t, J=7.5Hz, 3H), 2.93(q, J=7.5Hz, 2H), 4.03(s, 3H), 6.80(dd, J=1.5Hz, 7Hz, 1H), 6.84 (s, 1H), 7.1-7.4(m, 5H), 7.75(d, J=7Hz, 1H)
MASS(m/e): 252 (M⁺), 237, 194

### (Step E) 2-(2-Ethylphenyl)-7-methoxybenzofuran-4-carbaldehyde (Compound IIt-e)

Substantially the same procedure as in Step B of Reference Example 6 was repeated using Compound IIt-d (7.50 g) obtained in Step D to give Compound IIt-e (5.17 g, 62.1%) as a white solid.
NMR(CDCl₃, δ, ppm): 1.29(t, J=7.5Hz, 3H), 2.96(q, J=7.5Hz, 2H), 4.13(s, 3H), 6.91(d, J=8Hz, 1H), 7.2-7.4(m, 3H), 7.64(s, 1H), 7.69(d, J=8Hz, 1H), 7.80(d, J=7Hz, 1H), 10.07 (s, 1H)
MASS(m/e): 280 (M⁺), 265, 247

### (Step F) (Compound IIt)

Substantially the same procedure as in Step C of Reference Example 14 was repeated using Compound IIt-d (5.00 g) obtained in Step D to give Compound IIt (4.43 g, 80.0%) as a white solid.
NMR(CDCl₃, δ, ppm): 1.29(t, J=6.5Hz, 3H), 2.94(q, J=7.5Hz, 2H), 3.96(s, 3H), 4.08(s, 3H), 6.82(d, J=8.5Hz, 1H), 7.2-7.4(m, 3H), 7.47(s, 1H), 7.77 (d, J=7Hz, 1H), 7.96(d, J=8.5Hz, 1H)
MASS(m/e): 311 (M⁺), 279

### Reference Example 21

### Methyl 2-[2-(2-propyl)phenyl]-7-methoxybenzofuran-4-carboxylate (Compound IIu)

### (Step A) 2-(2-Acetylphenyl)-7-methoxybenzofuran (Compound IIu-a)

Compound IIt-b (18.4 g) obtained in Step B of Reference Example 20 was dissolved in dry tetrahydrofuran (500 ml), and the solution was cooled to -78°C followed by stirring. A 3.0M solution (36.4 ml) of methylmagnesium bromide in diethyl ether was dropwise added to the mixture and the reaction solution was slowly warmed to the room temperature. Distilled water was added to the mixture to cease the reaction and the solution was extracted with ethyl acetate. The organic layer was washed with a saturated saline and dried over anhydrous magnesium sulfate, and the solvent was distilled off under reduced pressure. The residue was purified by silica gel column chromatography (hexane:ethyl acetate = 3:1) to give 2-(2-(1-hydroxyethyl)phenyl)-7-methoxybenzofuran (17.8 g, 91.0%) as a colorless solid. Then, the solid was dissolved in dry dichloromethane (400 ml), and pyridinium chlorochromate (PCC, 27.0 g) and molecular sieve (3A, 30.0 g) were added thereto, followed by stirring at room temperature for one hour. Then, dichloromethane and 5% sulfuric acid were added to the reaction solution, the mixture was filtered with celite, and the filtrate was extracted with dichloromethane. The organic layer was washed with a saturated saline and dried over anhydrous magnesium sulfate, and the solvent was distilled off under reduced pressure. The residue was purified by silica gel column chromatography (hexane:ethyl acetate = 3:1) to give Compound IIu-a (16.6 g, 98.4%) as a pale-yellow oily substance.
NMR(CDCl₃, δ, ppm): 2.37(s, 3H), 4.01(s, 3H), 6.82(dd, J=2Hz, 6.5Hz, 1H), 6.89(s, 1H), 7.1-7.2(m, 2H), 7.4-7.6(m, 3H), 7.78(d, J=6Hz, 1H)
MASS(m/e): 266 (M⁺), 207

### (Step B) 2-[2-(1-Methylethenyl)-7-methoxybenzofuran (Compound IIu-b)

Substantially the same procedure as in Step C of Reference Example 20 was repeated using Compound IIu-a (16.0 g) obtained in Step A to give Compound 21 (15.6 g, 98.0%) as a pale-yellow oily substance.
NMR(CDCl₃, δ, ppm): 1.98(bs, 3H), 4.02(s, 3H), 5.07 (bs, 1H), 5.22(bs, 1H), 6.78(dd, J=1.5Hz, 7Hz, 1H), 7.05(s, 1H), 7.1-7.4(m, 5H), 7.91(dd, J=1.5Hz, 5Hz, 1H)
MASS(m/e): 264(M⁺)

### (Step C) 2-(2-Isopropylphenyl)-7-methoxybenzofuran (Compound IIu-c)

Substantially the same procedure as in Step D of Reference Example 20 was repeated using Compound IIu-b (15.3 g) obtained in Step B to give Compound IIu-c (14.0 g, 91.1%) as a colorless oily substance.
NMR(CDCl₃, δ, ppm): 1.26(d, J=7Hz, 6H), 3.45(sep, J=7Hz, 1H), 4.00(s, 3H), 6.77(s, 1H), 6.78(dd, J=1.5, 7.5Hz, 1H), 7.1-7.3(m, 3H), 7.3-7.5(m, 2H), 7.61(d, J=7.5Hz, 1H)
MASS(m/e): 266(M⁺), 219

### (Step D) 2-(2-Isopropylphenyl)-7-methoxybenzofuran-4-carbaldehyde (Compound IIu-d)

Substantially the same procedure as in Step B of Reference Example 6 was repeated using Compound IIu-c (1.00 g) obtained in Step C to give Compound IIu-d (0.67 g, 60.7%) as a pale-yellow oily substance.
NMR(CDCl₃, δ, ppm): 1.29(d, J=7Hz, 6H), 3.45(sep, J=7Hz, 1H), 4.12(s, 3H), 6.91(d, J=8Hz, 1H), 7.25(m, 1H), 7.35-7.5(m, 2H), 7.57(s, 1H), 7.63 (d, J=7.5Hz, 1H), 7.68(d, J=8Hz, 1H), 10.08(s, 1H)
MASS(m/e): 294(M⁺), 280, 261

### (Step E) (Compound IIu)

Substantially the same procedure as in Step C of Reference Example 14 was repeated using Compound IIu-d (5.40 g) obtained in Step D to give Compound IIu (5.00 g, 84.0%) as a white solid.
NMR(CDCl₃, δ, ppm): 1.29(d, J=7Hz, 6H), 3.47(sep, J=7Hz, 1H), 3.97(s, 3H), 4.09(s, 3H), 6.83(d, J=8Hz, 1H), 7.25(m, 1H), 7.41(s, 1H), 7.4-7.5(m, 2H), 7.63(dd, J=1Hz, 8.5Hz, 1H), 7.97(d, J=8Hz, 1H)
MASS (m/e) : 324 (M⁺), 277

### Reference Example 22

### Methyl 7-methoxy-3-phenylbenzofuran-4-carboxylate (Compound IIv)

Substantially the same procedure as in Reference Example 15 was repeated using Compound IIah (1.32 g) obtained in Reference Example 34 to give Compound IIv (1.26 g, 91%) as a colorless oily substance.
NMR(CDCl₃, δ, ppm) : 3.16(s, 3H), 4.07(s, 3H), 6.87 (d, J=9Hz, 1H), 7.31-7.44 (m, 5H), 7.68(s, 1H), 7.81 (d, J=9Hz, 1H)
MASS(m/e): 282(M⁺)

### Reference Example 23

### 7-Methoxy-2,3-dihydrobenzofuran-4-carboxylic acid (Compound IIw)

### (Step A) Methyl 7-methoxybenzofuran-4-carboxylate (Compound IIw-a)

7-Methoxybenzofuran-4-carboxylic acid (0.50 g) was dissolved in methanol (10 ml), and sulfuric acid (0.6 ml) was dropwise added thereto under ice-cooling, followed by heating under reflux for one hour. Sulfuric acid (0.2 ml) was further added to the mixture followed by heating under reflux for 30 minutes. After being allowed to stand for cooling, the solvent was distilled off, and the residue was poured into a 1N aqueous solution of sodium hydroxide. The precipitate was collected by filtration and dried to give Compound IIw-a (0.53 g, 99%) as a white solid.
Melting point: 87-89 °C
NMR(CDCl₃, δ, ppm) : 3.96(s, 3H), 4.09(s, 3H), 6.83 (d, J=9Hz, 1H), 7.36(d, J=1Hz, 1H), 7.70(d, J=1Hz, 1H), 7.98(d, J=9Hz, 1H)
MASS(m/e) : 206 (M⁺)

### (Step B) Methyl 7-methoxy-2,3-dihydrobenzofuran-4-carboxylate (Compound IIw-b)

Compound IIw-a (0.84 g) obtained in Step A was dissolved in ethanol (16 ml), and 5% rhodium carbon (0.17 g) was added thereto, followed by hydrogenation at normal temperature and normal pressure for 10 hours. The catalyst was removed, and then the filtrate was concentrated to give Compound IIw-b (0.80 g, 95%) a white solid.
Melting point: 68-78 °C
NMR(CDCl₃, δ, ppm): 3.56(t, J=9Hz, 2H), 3.89(s, 3H), 3.93(s, 3H), 4.67(t, J=9Hz, 2H), 6.77(d, J=8Hz, 1H), 7.59(d, J=8Hz, 1H)

### (Step C) (Compound IIw)

A mixture of Compound IIw-b (0.76 g) obtained in Step B, ethanol (3 ml), and a 2N solution of sodium hydroxide (3 ml) was heated under reflux for 3 hours. The mixture was adjusted to pH 1 by adding dilute hydrochloric acid under ice-cooling. The precipitate was collected by filtration and dried to give Compound IIw (0.64 g, 90%) as a white solid.
Melting point: 202-207 °C
NMR(CDCl₃, δ, ppm) : 3.61(t, J=9Hz, 2H), 3.95(s, 3H), 4.70 (t, J=9Hz, 2H), 6.80 (d, J=8Hz, 1H), 7.65 (d, J=8Hz, 1H)
MASS(m/e): 194(M⁺)

### Reference Example 24

### (±)-7-Methoxy-3-methyl-2,3-dihydrobenzofuran-4-carboxylic acid (Compound IIx)

Compound IIe (0.184 g) obtained in Reference Example 5 was dissolved in acetone (2 ml), and an aqueous solution of potassium permanganate (0.182 g) was slowly added thereto with stirring at room temperature. The insoluble matters were filtered off, and concentrated hydrochloric acid was added to the filtrate. The precipitated solid was collected by filtration and dried to give Compound IIx (0.116 g, 58.3%) as colorless crystals.
Melting point: 194-197 °C
NMR(CDCl₃, δ, ppm): 1.36(d, J=8.0Hz, 3H), 3.89-4.09(m, 1H), 3.96(s, 3H), 4.40(dd, J=9.3, 3.0Hz, 1H), 4.56-4.70(m, 1H), 6.82(d, J=8.9Hz, 1H), 7.69(d, J=8.9Hz, 1H)

### Reference Example 25

### (±)-3-Ethyl-7-methoxy-2,3-dihydrobenzofuran-4-carboxylic acid (Compound IIy)

Substantially the same procedures as in Reference Example 5 and then as in Reference Example 24 were repeated using 2-bromo-3-hydroxy-4-methoxybenzaldehyde (0.64 g) and 1-bromo-2-butene to give Compound IIy (0.37 g) as colorless crystals.
Melting point: 174-177 °C
NMR (CDCl₃, δ, ppm): 0.92(t, J=8.1Hz, 3H), 1.51-1.89(m, 2H), 3.78-4.02(m, 1H), 3.95(s, 3H), 4.50-4.66(m, 2H), 6.82(d, J=9.0Hz, 1H), 7.70(d, J=9.0Hz, 1H)

### Reference Example 26

### (±)-7-Methoxy-3-(2-propyl)-2,3-dihydrobenzofuran-4-carboxylic acid (Compound IIz)

Substantially the same procedures as in Reference Example 5 and then as in Reference Example 24 were repeated using 2-bromo-3-hydroxy-4-methoxybenzaldehyde (0.21 g) and 1-bromo-3-methyl-2-butene to give Compound IIz (0.163 g) as colorless crystals.
Melting point: 179-183 °C
NMR(CDCl₃, δ, ppm) : 0.67(d, J=8.7Hz, 3H), 1.01(d, J=8.7Hz, 3H), 2.14-2.32(m, 1H), 3.82-4.01(m, 1H), 3.95(s, 3H), 4.41-4.51(m, 1H), 4.68(dd, J=9.2, 3.0Hz, 1H), 6.82(d, J=9.0Hz, 1H), 7.69(d, J=9.0Hz, 1H)

### Reference Example 27

### (±)-3-Ethoxycarbonylmethyl-7-methoxy-2,3-dihydrobenzofuran-4-carboxylic acid (Compound IIaa)

Substantially the same procedures as in Reference Example 5 and then as in Reference Example 24 were repeated using 2-bromo-3-hydroxy-4-methoxybenzaldehyde (2.14 g) and ethyl bromocrotonate to give Compound IIaa (2.45 g) as white crystals.
NMR(CDCl₃, δ, ppm): 1.27(t, J=5.7Hz, 3H), 2.52(dd, J=17.2, 12.3Hz, 1H), 2.98(dd, J=17.2, 4.1Hz, 1H), 3.95(s, 3H), 4.17(q, J=5.7Hz, 2H), 4.23-4.37 (m, 1H), 4.50-4.77(m, 2H), 6.85(d, J=8.2Hz, 1H), 7.70(d, J=8.2Hz, 1H)

### Reference Example 28

### 2-Cyano-7-methoxybenzofuran-4-carboxylic acid (Compound IIab)

A mixture of Compound IIi (0.2 g) obtained in Reference Example 9, a 80% aqueous solution (2 ml) of acetic acid, sulfamic acid (0.145 g), and a 80% aqueous solution (0.084 g) of sodium chlorite was stirred at room temperature one night. The mixture was diluted with water, and then the precipitated solid was collected by filtration and dried to give Compound IIab (0.259 g, 83%) as white crystals.
NMR(DMSO-d₆, δ, ppm): 4.05(s, 3H), 7.30 (d, J=9.1Hz, 1H), 8.00(d, J=9.1Hz, 1H), 8.30(s, 1H), 12.98-13.22(br, 1H)

### Reference Example 29

### 7-Methoxybenzofuran-4-carboxylic acid (Compound IIac)

Compound IIac was synthesized according to the method described in Org. Prep. Proced. Int., 763 (1989).
Melting point: 224-226 °C
NMR(DMSO-d₆, δ, ppm): 4.00(s, 3H), 7.02(d, J=9Hz, 1H), 7.30(d, J=3Hz, 1H), 7.88(d, J=9Hz, 1H), 8.10(d, J=3Hz, 1H), 12.7-12.8(brs, 1H)
MASS (m/e) : 192(M⁺)

### Reference Example 30

### 2-Butyl-7-methoxybenzofuran-4-carboxylic acid (Compound IIad)

### (Step A) 7-Methoxybenzofuran-2-carbaldehyde (Compound IIad-a)

2-Cyano-7-methoxybenzofuran (0.736 g) was dissolved in dichloromethane (10 ml), and a 1.02N DIBAL solution (5.4 ml) in toluene was added thereto at -4 to -30°C, followed by stirring for one hour. Methanol and dilute hydrochloric acid were added to the mixture, and the solvent was distilled off. The obtained residue was purified by column chromatography (hexane/ethyl acetate = 10/1) to give Compound IIad-a (0.371 g, 50%) as an oily substance.
NMR(CDCl₃, δ, ppm): 4.04(s, 3H), 6.92-7.03(m, 1H), 7.17-7.40(m, 2H), 7.54(s, 1H), 9.90(s, 1H)

### (Step B) (E/Z)-2-(1-Buten-1-yl)-7-methoxybenzofuran (Compound IIad-b)

1-Propyltriphenylphosphonium bromide (0.907 g) was suspended in ether (10 ml), and a 1.7N solution (1.42 ml) of butyl lithium in hexane was added thereto under ice-cooling, followed by stirring for one hour. A solution of Compound IIad-a (0.319 g) dissolved in ether (3.2 ml) was dropwise added to the mixture, followed by stirring for 10 minutes. Water was added to the mixture followed by extraction with ethyl acetate. The organic layer was washed with a saturated saline and dried over sodium sulfate, and the solvent was distilled off. The residue was purified by silica gel column chromatography (hexane/ethyl acetate = 30/1) to give Compound IIad-b (0.28 g, 78%) as a colorless oily mixture of isomers (2:5).
NMR (CDCl₃, δ, ppm): 1.11 and 1.14 (each t, J=7Hz, total 3H), 2.16-2.33(m, 0.3H), 2.48-2.67(m, 0.7H), 4.01 and 4.02(each s, total 3H), 5.80(dt, J=8, 10Hz, 0.7H), 6.23-6.39(m, 1H), 6.48(s, 0.3H), 6.60(dt, J=8, 14Hz, 0.3H), 6.61(s, 0.7H), 6.70-6.83(m, 1H), 7.04-7.20(m, 2H)

### (Step C) 2-Butyl-7-methoxybenzofuran (Compound IIad-c)

Compound IIad-b (0.27 g) was dissolved in methanol (5.4 ml), and 10% palladium carbon (27 mg) was added thereto, followed by hydrogenation at normal temperature and normal pressure for 3 hours. The catalyst was removed, and then the filtrate was concentrated to give Compound IIad-c (0.248 g, 91%) as an oily substance.
NMR(CDCl₃, δ, ppm): 0.94(t, J=8Hz, 3H), 1.30-1.51(m, 2H), 1.64-1.82(m, 2H), 2.79(t, J=7Hz, 2H), 4.00 (s, 3H), 6.38(s, 1H), 6.68-6.80(m, 1H), 7.02-7.17(m, 2H)

### (Step D) 2-Butyl-4-formyl-7-methoxybenzofuran (Compound IIad-d)

Compound IIad-c (1.70 g) was dissolved in DMF (17 ml), and phosphorus oxychloride (2.3 ml) was added thereto under ice-cooling, followed by stirring at 80°C for one hour. Phosphorus oxychloride (2.3 ml) was further added to the mixture under ice-cooling, followed by stirring at 80°C for 2 hours. After being allowed to stand for cooling, the mixture was poured into ice-water followed by extraction with ether. The organic layer was washed with a saturated saline and dried over sodium sulfate, and the solvent was distilled off. The residue was purified by silica gel column chromatography (hexane/ethyl acetate = 10/1) to give Compound IIad-d (1.19 g, 62%) as an oily substance.
NMR(CDCl₃, δ, ppm) : 0.97(t, J=7Hz, 3H), 1.31-1.52(m, 2H), 1.67-1.88(m, 2H), 2.83(t, J=8Hz, 2H), 4.09 (s, 3H), 6.83(d, J=9Hz, 1H), 7.14(s, 1H), 7.61(d, J=9Hz, 1H), 10.0(s, 1H)
MASS (m/e) : 232(M⁺)

### (Step E) (Compound IIad)

Substantially the same procedure as in Reference Example 24 was repeated using Compound IIad-d (0.500 g) to give Compound IIad (0.467 g, 88%) as a white solid.
Melting point: 114-120 °C
NMR(CDCl₃, δ, ppm): 0.97(t, J=8Hz, 3H), 1.31-1.54(m, 2H), 1.68-1.87(m, 2H), 2.85 (t, J=8Hz, 2H), 4.09 (s, 3H), 6.80(d, J=9Hz, 1H), 7.07(s, 1H), 8.00(d, J=9Hz, 1H)
MASS(m/e): 248(M⁺)

### Reference Example 31

### 7-Methoxy-2-(4-pyridyl)benzofuran-4-carboxylic acid·hydrochloride (Compound IIae)

Distilled water (350 ml) and sodium hydroxide (544 mg) were added to Compound IIq (3.50 g) obtained in Reference Example 17, followed by heating under reflux for 2 hours. The solvent was distilled off from the reaction solution under reduced pressure, and the residue was dissolved in hot ethanol (500 ml). The mixture was cooled to 0°C followed by stirring. A hydrochloric acid-ethanol solution was dropwise added to the mixture followed by stirring for 20 minutes. The precipitated crystals were collected by filtration to give Compound IIae (1.83 g, 48.2%) as a white solid.
NMR(D₂O, δ, ppm): 3.61(s, 3H), 6.44(d, J=9Hz, 1H), 7.00(s, 1H), 7.12(d, J=9Hz, 1H), 7.58(d, J=7Hz, 2H), 8.30(d, J=7Hz, 2H)

### Reference Example 32

### 7-Methoxy-2-(2-pyridyl)benzofuran-4-carboxylic acid-hydrochloride (Compound IIaf)

Substantially the same procedure as in Reference Example 31 was repeated using Compound IIr (5.00 g) obtained in Reference Example 18 to give Compound IIaf (5.04 g, 93.3%) as a white solid.
NMR(DMSC-d₆, δ, ppm): 4.07(s, 3H), 7.14(d, J=8Hz, 1H), 7.53(dd, J=6Hz, 8Hz, 1H), 7.91(d, J=8Hz, 1H), 8.02(s, 1H), 8.05-8.15(m, 2H), 8.73(d, J=6Hz, 1H)

### Reference Example 33

### 2-Benzyl-7-methoxybenzofuran-4-carboxylic acid (Compound IIag)

### (Step A) 2-Benzoyl-7-methoxybenzofuran (Compound IIag-a)

Substantially the same procedure as in Step A of Reference Example 6 was repeated using ortho-vanillin (7.8 g) and using phenacyl chloride (9.5 g) instead of 4-picolyl chloride hydrochloride to give Compound IIag-a (13.9 g, quant.) as a pale-yellow solid.
NMR(CDCl₃, δ, ppm): 4.01(s, 3H), 6.94(dd, J=1Hz, 8Hz, 1H), 7.29-7.21(m, 2H), 7.63-7.48(m, 4H), 8.06(dd, J=1Hz 8Hz, 2H)
MASS (m/e): 252(M⁺)

### (Step B) 2-Benzyl-7-methoxybenzofuran (Compound IIag-b)

Compound IIag-a (10.00 g) obtained in Step A was suspended in diethylene glycol (100 ml), and potassium hydroxide (7.57 g) and hydrazine-monohydrate (5.77 ml) were added thereto with stirring at room temperature, followed by heating under reflux for 2 hours with stirring. The reaction solution was poured into ice-water, and the mixture was adjusted to weak acidic with dilute hydrochloric acid, followed by extraction with ether. The organic layer was washed with a saturated saline and dried over anhydrous magnesium sulfate, and the solvent was distilled off under reduced pressure. The residue was purified by silica gel column chromatography (hexane:ethyl acetate = 30:1) to give Compound IIag-b (7.35 g, 77.8%) as a yellow oily substance.
NMR(CDCl₃, δ, ppm) 3.98(s, 3H), 4.12(s, 2H), 6.31(s, 1H), 6.73(dd, J=1Hz, 7Hz, 2H), 7.12-7.03(m, 2H), 7.35-7.22(m, 5H)
MASS(m/e): 238(M⁺)

### (Step C) 2-Benzyl-7-methoxybenzofuran-4-carbaldehyde (Compound IIag-c)

Substantially the same procedure as in Step B of Reference Example 6 was repeated using Compound IIag-b (7.35 g) obtained in Step B to give Compound IIag-c (2.70 g, 32.9%) as a white solid.

### (Step D) Methyl 2-benzyl-7-methoxybenzofuran-4-carboxylate (Compound IIag-d)

Substantially the same procedure as in Reference Example 17 was repeated using Compound IIag-c (2.70 g) obtained in Step C to give Compound IIag-d (1.20 g, 39.9%) as a white solid.

### (Step E) 2-Benzyl-7-methoxybenzofuran-4-carboxylic acid (Compound IIag)

Substantially the same procedure as in Reference Example 31 was repeated using Compound IIag-d (1.20 g) obtained in Step D to give Compound IIag (0.39 g, 34.1%) as a white solid.
NMR(DMSO-d₆, δ, ppm): 4.01(s, 3H), 4.20(s, 2H), 6.65 (s, 1H), 7.26(d, 1H, J=8Hz), 7.39-7.28(m, 5H), 7.53(d, 1H, J=8Hz)
MASS(m/e): 282(M⁺)

### Reference Example 34

### 7-Methoxy-3-phenylbenzofuran-4-carboxylic acid (Compound IIah)

### (Step A) 4-Bromo-2-phenacyloxyanisole (Compound IIah-a)

Substantially the same procedure as in Step A of Reference Example 6 was repeated using 4-bromo-2-methoxyphenol (7.0 g) and phenacyl bromide (10.6 g) to give Compound IIah-a (9.8 g, 74%) as a pale-yellow oily substance.
NMR(CDCl₃, δ, ppm): 3.83(s, 3H), 5.33(s, 2H), 6.76(d, J=8Hz, 1H), 6.95(d, J= 2Hz, 1H), 6.76(d, J=8Hz, 1H), 7.06(dd, J=2, 8Hz, 1H), 7.45-7.63(m, 3H), 7.96-7.99(m, 2H)
MASS(m/e): 320(M⁺)

### (Step B) 4-Bromo-7-methoxy-3-phenylbenzofuran (Compound IIah-b)

Polyphosphoric acid (50 ml) was added to Compound IIah-a (10.8 g) obtained in Step A followed by heating at 60°C for 4 hours. After being allowed to stand for cooling, the reaction solution was poured into ice followed by extraction with ether. The organic layer was washed with a saturated saline and dried over magnesium sulfate. The solvent was distilled off and the residue was purified by silica gel column chromatography (hexane:ethyl acetate = 30:1) to give Compound IIah-b (5.9 g, 58%) as a pale-yellow oily substance.
NMR(CDCl₃, δ, ppm): 4.02(s, 3H), 6.72(d, J=9Hz, 1H), 7.32(d, J=9Hz, 1H), 7.40-7.51(m, 5H), 7.62(s, 1H)
MASS(m/e): 302(M⁺)

### (Step C) (Compound IIah)

Substantially the same procedure as in Step D of Reference Example 1 was repeated using Compound IIah-b (4.0 g) obtained in Step B and using dry ice instead of DMF to give Compound IIah (1.5 g, 42%) as white crystals.
NMR(CDCl₃, δ, ppm): 4.10(s, 3H), 6.88(d, J=9Hz, 1H), 7.31-7.35(m, 5H), 7.71(s, 1H), 7.88(d, J=9Hz, 1H)
MASS (m/e) : 268(M⁺)

### Reference Example 35

### 3-Ethoxycarbonylmethyl-7-methoxybenzofuran-4-carboxylic acid (Compound IIai)

Substantially the same procedure as in Reference Example 24 was repeated using Compound IIj (4.9 g) obtained in Reference Example 10 to give Compound IIai (4.4 g, 85%) as white crystals.
Melting point: 170-177 °C
NMR(CDCl₃, δ, ppm): 1.26(t, J=7Hz. 3H), 3.98(s, 2H), 4.08(s, 3H), 4.17(q, J=7Hz, 2H), 6.85(d, J=9Hz, 1H), 7.65(s, 1H), 8.06(d, J=9Hz, 1H)

### Reference Example 36

### 4-Benzoyl-7-methoxy-2,2-dimethyl-2,3-dihydrobenzofuran (Compound IIaj)

### (Step A) 4-(1-Hydroxy-1-phenylmethyl)-7-methoxy-2,2-dimethyl-2,3-dihydrobenzofuran (Compound IIaj-a)

Under an argon atmosphere, a solution of Compound IIa (4.6 g) obtained in Reference Example 1 in THF (25 ml) was cooled to -78°C, and a 1.0M solution (26 ml) of phenylmagnesium bromide in THF was slowly and dropwise added thereto, followed by stirring at 0°C for one hour. A saturated aqueous solution of ammonium chloride was added to the reaction solution followed by extraction with methylene chloride. The organic layer was washed with a saturated saline and dried over anhydrous magnesium sulfate, and the solvent was distilled off under reduced pressure. The residue was purified by silica gel column chromatography (chloroform/methanol = 50/1) to give Compound IIaj-a (4.6 g, 72.2%) as a pale-yellow oily substance.
NMR(DMSO-d₆, δ, ppm): 1.32(s, 3H), 1.34(s, 3H), 2.84 (s, 2H), 3.71(s, 3H), 6.74-6.8.1(m, 2H), 7.28-7.30(m, 5H)

### (Step B) (Compound IIaj)

Compound Ilaj-a (4.0 g) obtained in Step A was dissolved in methylene chloride (140 ml), and manganese dioxide (4.0 g) was added thereto, followed by stirring at room temperature for 5 hours. The reaction solution was filtered and the obtained filtrate was concentrated under reduced pressure. The residue was purified by silica gel column chromatography (chloroform/hexane = 1/2) to give Compound IIaj (2.0 g, 67.4%) as colorless crystals.
Melting point: 65-69 °C
NMR(DMSO-d₆, δ, ppm): 1.43(s, 6H), 3.34(s, 2H), 3.85 (s, 3H), 6.94(d, J= 8.25Hz, 1H), 7.04(d, J=8.25Hz, 1H), 7.39-7.69(m, 5H)
IR(KBr, cm⁻¹): 1637, 1608, 1576, 1506, 1446
MASS (m/z): 282(M⁺)

### Reference Example 37

### (±)-4-Benzoyl-7-methoxy-3-methyl-2,3-dihydrobenzofuran (Compound IIak)

### (Step A) 4-(1-Hydroxy-1-phenylmethyl)-7-methoxy-3-methyl-2,3-dihydrobenzofuran (Compound IIak-a)

Under an argon atmosphere, a solution of Compound IIe (7.0 g) obtained in Reference Example 5 in THF (70 ml) was cooled to -78°C, and a 1.0M solution (41 ml) of phenylmagnesium bromide in THF was slowly and dropwise added thereto, followed by stirring at 0°C for one hour. A saturated aqueous solution of ammonium chloride was added to the reaction solution followed by extraction with methylene chloride. The organic layer was washed with a saturated saline and dried over anhydrous magnesium sulfate, and the solvent was distilled off under reduced pressure. The residue was purified by silica gel column chromatography (chloroform/methanol = 50/1) to give Compound IIaj-a (7.8 g, 79.4%) as a pale-yellow oily substance.
NMR(DMSO-d₆, δ, ppm): 1.18(d, J=6.93Hz, 3H), 3.25-3.40(m, 1H), 3.72(s, 3H), 4.13(dd, J=8.75Hz, 3.30Hz, 1H), 4.39(t, J=8.58Hz, 1H), 6.80(d, J=8.58Hz, 1H), 6.87(d, J=8.58Hz, 1H), 7.20-7.31 (m, 5H)

### (Step B) Compound IIaj

Compound IIaj-a (5.0 g) obtained in Step A was dissolved in methylene chloride (240 ml), and manganese dioxide (5.0 g) was added thereto, followed by stirring at room temperature for 5 hours. The reaction solution was filtered and the obtained filtrate was concentrated under reduced pressure. The residue was purified by silica gel column chromatography (chloroform/hexane = 1/2) to give Compound IIaj (4.62 g, 93.1%) as a yellowish brown oily substance.
NMR(DMSO-d₆, δ, ppm): 1.10(d, J=6.93Hz, 3H), 3.79-3.86(m, 1H), 3.86(s, 3H), 4.24(dd, J=4.29Hz, 8.91Hz, 1H), 4.62(t, J=8.91Hz, 1H), 6.96(d, J=8.25Hz, 1H), 7.02(d, J=8.25Hz, 1H), 7.52-7.57 (m, 2H), 7.64-7.71(m, 3H)

### Reference Example 38

### 4-Benzoyl-7-methoxy-2-(4-pyridyl)benzofuran (Compound IIal)

Substantially the same procedure as in Reference Example 36 was repeated using Compound IIaf (6.0 g) obtained in Reference Example 6 to give Compound IIal (5.6 g, 75%) as a pale-yellow oily substance.
NMR(CDCl₃, δ, ppm): 4.12(s, 3H), 6.83(d, J=8Hz, 1H), 7.4-7.6(m, 4H), 7.7-7.9(m, 5H), 8.69(d, J=5.5Hz, 2H)

### Reference Example 39

### 4-Acetyl-7-methoxy-2,2-dimethyl-2,3-dihydrobenzofuran (Compound IIam)

### (Step A) 4-(1-Hydroxyethyl)-7-methoxy-2,2-dimethyl-2,3-dihydrobenzofuran (Compound IIam-a)

Under an argon atmosphere, a solution of Compound IIa (21 g) obtained in Reference Example 1 in THF (100 ml) was cooled to -78°C, and a 1.0M solution (122 ml) of methylmagnesium bromide in THF was slowly and dropwise added thereto, followed by stirring at 0°C for one hour. A saturated aqueous solution of ammonium chloride was added to the reaction solution followed by extraction with methylene chloride. The organic layer was washed with a saturated saline and dried over anhydrous magnesium sulfate, and the solvent was distilled off under reduced pressure. The residue was purified by silica gel column chromatography (chloroform/methanol = 50/1) to give Compound IIam-a (24.4 g, quant.) as a pale-yellow oily substance.
NMR(DMSO-d₆, δ, ppm): 1.26(d, J=6.3Hz, 3H), 1.39(s, 3H), 1.41(s, 3H), 3.00(s, 2H), 3.71(s, 3H), 4.60-4.64(m, 1H), 4.94(d, J=4.0Hz, 1H), 6.75(s, 2H)
MASS (m/z) : 282(M⁺)

### (Step B) (Compound IIam)

Compound IIam-a (20.9 g) obtained in Step A was dissolved in methylene chloride (200 ml), and manganese dioxide (31 g) was added thereto, followed by stirring at room temperature for 5 hours. The reaction solution was filtered off and the obtained filtrate was concentrated under reduced pressure. The residue was purified by silica gel column chromatography (chloroform/hexane = 1/2) to give Compound IIam (12.2 g, 59.0%) as colorless crystals.
NMR(DNSO-d₆, δ, ppm): 1.40(s, 6H), 2.49(s, 3H), 3.27 (s, 2H), 3.83(s, 3H), 6.94(d, J=8.6Hz, 1H), 7.49 (d, J=8.6Hz, 1H)
MASS(m/e): 220(M⁺)

### Reference Example 40

### 4-Acetyl-7-methoxy-spiro[2,3-dihydrobenzofuran-2,1'-cyclopentane] (Compound IIan)

### (Step A) 4-(1-Hydroxyethyl)-7-methoxy-spiro[2,3-dihydrobenzofuran-2,1'-cyclopentane] (Compound IIan-a)

Under an argon atmosphere, a solution of Compound IIc (5.5 g) obtained in Reference Example 3 in THF (20 ml) was cooled to -78°C, and a 0.95M solution (30 ml) of methylmagnesium bromide in THF was slowly and dropwise added thereto, followed by stirring at 0°C for one hour. A saturated aqueous solution of ammonium chloride was added to the reaction solution followed by extraction with methylene chloride. The organic layer was washed with a saturated saline and dried over anhydrous magnesium sulfate, and the solvent was distilled off under reduced pressure. The residue was purified by silica gel column chromatography (chloroform/methanol = 50/1) to give Compound IIan-a (6.7 g, quant.) as a pale-yellow oily substance.
NMR(DMSO-d₆, δ, ppm): 1.25(d, J=6.6Hz, 3H), 1.71-1.86 (m, 8H), 3.17(s, 2H), 3.71(s, 3H), 4.60-4.65(m, 1H), 4.96(d, J=4.0Hz, 1H), 6.74(s, 2H)

### (Step B) (Compound IIan)

Compound IIan-a (6.5 g) obtained in Step A was dissolved in methylene chloride (260 ml), and pyridinium chlorochromate (6.8 g) was added thereto, followed by stirring at room temperature for 2 hours. The reaction solution was filtered and the obtained filtrate was concentrated under reduced pressure. The residue was purified by silica gel column chromatography (ethyl acetate/hexane = 1/9) to give Compound IIan (2.98 g, 52.8%) as colorless crystals.
NMR(DMSO-d₆, δ, ppm): 1.71-1.99(m, 8H), 2.49(s, 3H), 3.44(s, 2H), 3.83(s, 3H), 6.93(d, J=8.6Hz, 1H), 7.48(d, J=8.6Hz, 1H)

### Reference Example 41

### 8-Methoxy-2,2-dimethylbenzopyran-5-carboxylic acid (Compound IIao)

### (Step A) Methyl 3-(1,1-dimethyl-2-propyn-1-yloxy)-4-methoxybenzoate (Compound IIao-a)

A mixture of methyl 3-hydroxy-4-methoxybenzoate (5.41 g), 3-chloro-3-methyl-1-butyne (10 ml), cesium carbonate (19.4 g), and DMF (54 ml) was stirred at 80°C for one hour. 3-Chloro-3-methyl-1-butyne (5 ml) was further added to the mixture followed by stirring at 90°C for 3 hours. After being allowed to stand for cooling, water was added to the mixture followed by extraction with ether. The organic layer was washed with a 1N aqueous solution of sodium hydroxide and with a saturated saline and dried over sodium sulfate, and the solvent was distilled off. The residue was purified by silica gel column chromatography (hexane/ethyl acetate = 10/1 and 7/1) to give Compound IIao-a (2.31 g, 31%) as a brown oily substance.
NMR(CDCl₃, δ, ppm) : 1.68(s, 6H), 2.54(s, 1H), 3.87(s, 3H), 3.88(s, 3H), 6.90(d, J=8Hz, 1H), 7.79(dd, J=1, 8Hz, 1H), 8.09(d, J=1Hz, 1H)

### (Step B) Methyl 8-methoxy-2,2-dimethylbenzopyran-5-carboxylate (Compound IIao-b)

Compound IIao-a (2.30 g) obtained in Step A was dissolved in diethylaniline (14 ml) followed by stirring at 160°C for 5 hours. After being allowed to stand for cooling, dilute hydrochloric acid was added to the mixture followed by extraction with ether. The organic layer was washed with a saturated saline end dried over sodium sulfate, and the solvent was distilled off. The residue was purified by silica gel column chromatography (hexane/ethyl acetate = 10/1 and 7/1) to give Compound IIao-b (2.12 g, 92%) as a pale-yellow oily substance.
NMR (CDCl₃, δ, ppm) : 1.48(s, 6H), 3.86(s, 3H), 3.90(s, 3H), 5.78(d, J=9Hz, 1H), 6.78(d, J=8Hz, 1H), 7.33(d, J=9Hz, 1H), 7.56(d, J=8Hz, 1H)

### (Step C) (Compound IIao)

Substantially the same procedure as in Reference Example 31 was repeated using Compound IIao-b (0.38 g) obtained in Step B to give Compound IIao (0.34 g, 96%) as a white solid.
Melting point: 159-166 °C
NMR(CDCl₃, δ, ppm): 1.50(s, 6H), 3.92(s, 3H), 5.80(d, J=9Hz, 1H), 6.80(d, J=9Hz, 1H), 7.41(d, J=9Hz, 1H), 7.69(d, J=9Hz, 1H)
MASS(m/e): 234(M⁺)

### Reference Example 42

### 8-Methoxy-2,2-dimethyl-3,4-dihydrobenzopyran-5-carboxylic acid (Compound IIap)

### (Step A) Methyl 8-methoxy-2,2-dimethyl-3,4-dihydrobenzopyran-5-carboxylate (Compound IIap-a)

Substantially the same procedure as in Step C of Reference Example 30 was repeated using Compound IIao-b (1.78 g) obtained in Step B of Reference Example 41 and 10% palladium carbon (0.36 g) to give Compound IIap-a (1.31g, 73%) as a white solid.
NMR(CDCl₃, δ, ppm): 1.40(s, 6H), 1.70-1.87(m, 2H), 3.03-3.20(m, 2H), 3.85(s, 3H), 3.90(s, 3H), 6.73 (d, J=8Hz, 1H), 7.57(d, J=8Hz, 1H)
MASS(m/e): 250 (M⁺)

### (Step B) (Compound IIap)

Substantially the same procedure as in Reference Example 31 was repeated using Compound IIap-a (1.27 g) obtained in Step A to give Compound IIap (1.3 g, 96%) as a white solid.
NMR(CDCl₃, δ, ppm): 1.40(s, 6H), 1.75-1.90(m, 2H), 3.11-3.26(m, 2H), 3.91(s, 3H), 6.78(d, J=9Hz, 1H), 7.73(d, J=9Hz, 1H)
MASS(m/e): 236(M⁺)

### Reference Example 43

### 5-Carboxy-8-methoxy-spiro[benzopyran-2,1'-cyclopentane] (Compound IIaq)

### (Step A) 8-Methoxy-4-oxo-spiro[3,4-dihydrobenzopyran-2,1'-cyclopentane] (Compound IIaq-a)

A mixture of methyl 2-hydroxy-3-methoxyacetophenone (16 g), cyclopentanone (33 ml), pyrrolidine (15 ml), and toluene (200 ml) was heated under reflux for 3 hours. Cyclopentanone (6 ml) was further added to the mixture followed by heating under reflux for 2 hours. After being allowed to stand for cooling, ether was added to the mixture followed by washing with dilute hydrochloric acid and with a saturated saline. The mixture was dried over sodium sulfate and the solvent was distilled off to give Compound IIaq-a (20 g, 90%) as a brown oily substance.
NMR(CDCl₃, δ, ppm): 1.54-2.00(m, 6H), 2.02-2.26(m, 2H), 2.85(s, 2H), 3.89(s, 3H), 6.90(dd, J=9, 9Hz, 1H), 7.02(d, J=9Hz, 1H), 7.48(d, J=9Hz, 1H)
MASS(m/e): 232(M⁺)

### (Step B) 4-Hydroxy-8-methoxy-spiro[3,4-dihydrobenzopyran-2,1'-cyclopentane] (Compound IIaq-b)

Compound IIaq-a (39 g) obtained in Step A was dissolved in methanol (300 ml), and sodium borohydride (7.5 g) was added thereto under ice-cooling, followed by stirring at room temperature for one hour. The mixture was cooled with ice again, dilute hydrochloric acid was added thereto, and the solvent was distilled off. Water was added to the residue followed by extraction with ethyl acetate. The organic layer was washed with a saturated saline and dried over sodium sulfate, and the solvent was distilled off. The residue was purified by silica gel column chromatography (hexane/ethyl acetate = 6/1 and 2/1) to give Compound IIaq-b (29 g, 74%) as a pale-yellow oily substance.
NMR(CDCl₃, δ, ppm) : 1.46-2.18(m, 9H), 2.25(dd, J= 8, 12Hz, 1H), 3.82(s, 3H), 4.78-4.92(m, 1H), 6.80 (dd, J=2, 8Hz, 1H), 6.88(dd, J=8, 9Hz, 1H), 7.07 (dd, J=2, 8Hz, 1H)
MASS(m/e): 234(M⁺)

### (Step C) 8-Methoxy-spiro[benzopyran-2,1'-cyclopentane] (Compound IIaq-c)

Methanesulfonyl chloride (4.9 ml) was dropwise added to a mixture of Compound IIaq-b (11 g) obtained in Step B, triethylamine (8.8 ml), and dichloromethane (114 ml) under ice-cooling, followed by stirring at room temperature for 30 minutes. DBU (9.5 ml) was added to the mixture followed by heating under reflux for 7 hours. After being allowed to stand for cooling, water was added to the mixture followed by extraction with hexane. The organic layer was washed with a saturated saline and dried over sodium sulfate, and the solvent was distilled off to give Compound IIaq-c (11 g, 99%) as a brown oily substance.
NMR(CDCl₃, δ, ppm): 1.50-1.79(m, 4H), 1.82-2.08(m, 2H), 2.11-2.32(m, 2H), 3.84(s, 3H), 5.79(d, J=10Hz, 1H), 6.35(d, J=10Hz, 1H), 6.61(dd, J=4, 6Hz, 1H), 6.71-6.87(m, 2H)

### (Step D) 8-Methoxy-spiro[benzopyran-2,1'-cyclopentane]-5-carbaldehyde (Compound IIaq-d)

Phosphorus oxychloride (18 ml) was dropwise added to a mixture of Compound IIaq-c (11 g) obtained in Step C, N-methylformanilide (24 ml), and dichloroethane (53 ml) under ice-cooling, followed by stirring at 90°C for 2 hours. After being allowed to stand for cooling, the reaction solution was poured into ice-water followed by extraction with ethyl acetate. The organic layer was washed with a saturated saline and dried over sodium sulfate. The solvent was distilled off and the residue was purified by silica gel column chromatography (hexane/ethyl acetate = 8/1) to give Compound IIaq-d (7.8 g, 65%) as an oily mixture of isomers (1:3).
NMR(CDCl₃, δ, ppm): 1.50-1.80(m, total 4H), 1.81-2.08 (m, total 2H), 2.10-2.32(m, total 2H), 3.90 and 3.91(each s, total 3H), 5.71(d, J=9Hz, 0.75H), 5.90(d, J=9Hz, 0.25H), 6.39(d, J=9Hz, 0.75H), 6.85(d, J=8Hz, 0.25H), 7.15(d, J=1Hz, 0.75H), 7.29(d, J=1Hz, 0.75H), 7.30(d, J=8Hz, 0.25H), 7.48(d, J=9Hz, 0.25H), 9.80(s, 0.75H), 10.0(s, 0.25H)

### (Step E) 8-Methoxy-5-methoxycarbonyl-spiro[benzopyran-2,1'-cyclopentane] (Compound IIaq-e)

Compound IIaq-d (21 g) obtained in Step D was dissolved in a 5% solution (400 ml) of potassium hydroxide in methanol, and iodine (45 g) was portionwise added thereto under ice-cooling, followed by stirring at room temperature for 6 hours. The mixture was cooled again with ice, the mixture was adjusted to pH 3 by adding dilute hydrochloric acid, and the solvent was distilled off. Water was added to the mixture followed by extraction with ethyl acetate. The organic layer was washed with a saturated saline and dried over sodium sulfate, and the solvent was distilled off. The residue was purified twice by silica gel column chromatography (hexane/ethyl acetate = 10/1 and toluene/ether = 80/1) to give Compound IIaq-e (5.5 g, 23%) as a pale-yellow solid.
Melting point: 48-50 °C
NMR(CDCl₃, δ, ppm): 1.45-2.30(m, 8H), 3.85(s, 3H), 3.86(s, 3H), 5.82(d, J=9Hz, 1H), 6.76(d, J=8Hz, 1H), 7.37(d, J=9Hz, 1H), 7.53(d, J=8Hz, 1H)
MASS(m/e): 274(M⁺)

### (Step F) 5-Carboxy-8-methoxy-spiro[benzopyran-2,1'-cyclopentane] (Compound IIaq)

Substantially the same procedure as in Reference Example 31 was repeated using Compound IIaq-e (1.9 g) obtained in Step E to give Compound IIaq (1.7 g, 95%) as a white solid.
Melting point: 177-189 °C
NMR(CDCl₃, δ, ppm): 1.52-2.32(m, 8H), 3.90(s, 3H), 5.88(d, J=9Hz, 1H), 6.80(d, J=9Hz, 1H), 7.45(d, J=9Hz, 1H), 7.70(d, J=9Hz, 1H)
MASS(m/e): 260 (M⁺)

### Reference Example 44

### Methyl 8-methoxy-spiro[3,4-dihydrobenzopyran-2,1'-cyclopentane]-5-carboxylate (Compound IIar)

Substantially the same procedure as in Step A of Reference Example 42 was repeated using Compound (2.0 g) obtained in Step E of Reference Example 43 to give Compound IIar (2.0 g, 100%) as an oily substance.
NMR(CDCl₃, δ, ppm): 1.47-2.08(m, 10H), 3.17(t, J=7Hz, 2H), 3.83(s, 3H), 3.88(s, 3H), 6.70(d, J=9Hz, 1H), 7.56(d, J=9Hz, 1H)

### Reference Example 45

### 8-Methoxy-spiro[3,4-dihydrobenzopyran-2,1'-cyclopentane]-5-carboxylic acid (Compound IIas)

Substantially the same procedure as in Reference Example 31 was repeated using Compound IIar (2.0 g) obtained in Reference Example 44 to give Compound IIas (1.8 g, 96%) as white crystals.
Melting point: 182-189 °C
NMR(CDCl₃, δ, ppm): 1.50-2.10(m, 10H), 3.22(t, J=6Hz, 2H), 3.90(s, 3H), 6.75(d, J=8Hz, 1H), 7.70(d, J=8Hz, 1H)
MASS (m/e) : 262(M⁺)

### Reference Example 46

### Methyl 8-methoxy-spiro[3,4-dihydrobenzopyran-2,1'-cyclohexane]-5-carboxylate (Compound IIat)

### (Step A) 8-Methoxy-4-oxo-spiro[3,4-dihydrobenzopyran-2,1'-cyclohexane] (Compound IIat-a)

Substantially the same procedure as in Step A of Reference Example 43 was repeated using 2-hydroxy-3-methoxyacetophenone (40 g), cyclohexanone (100 ml), and pyrrolidine (40 ml) to give Compound at-a (59 g, 100%) as a brown oily substance.
NMR(CDCl₃, δ, ppm): 1.20-2.10(m, 10H), 2.74(s, 2H), 3.90(s, 3H), 6.90(dd, J=8, 8Hz, 1H), 7.05(dd, J=1, 8Hz, 1H), 7.46(d, J=1, 8Hz, 1H)
MASS(m/e): 246(M⁺)

### (Step B) 4-Hydroxy-8-methoxy-spiro[3,4-dihydrobenzopyran-2,1'-cyclohexane] (Compound IIat-b)

Substantially the same procedure as in Step B of Reference Example 43 was repeated using Compound at-a (59 g) obtained in Step A and sodium borohydride (18 g) to give Compound (51 g, 80%) as a pale-yellow oily substance.
NMR(CDCl₃, δ, ppm): 1.20-2.05(m, 11H), 2.26(dd, J=6, 13Hz, 1H), 3.95(s, 3H), 4.75-4.90(m, 1H), 6.80 (dd, J=1, 8Hz, 1H), 6.88(dd, J=8, 8Hz, 1H), 7.03 (dd, J=1, 8Hz, 1H)
MASS (m/e): 248(M⁺)

### (Step C) 8-Methoxy-spiro[benzopyran-2,1'-cyclohexane] (Compound IIat-c)

Substantially the same procedure as in Step C of Reference Example 43 was repeated using Compound at-b (50 g) obtained in Step B, triethylamine (54 ml), methanesulfonyl chloride (33 ml), and DBU (58 ml) to give Compound at-c (46 g, 100%) as a brown oily substance.
NMR(CDCl₃, δ, ppm): 1.20-2.08(m, 10H), 3.85(s, 3H), 5.70(d, J=9Hz, 1H), 6.33(d, J=9Hz, 1H), 6.57-6.85(m, 3H)
MASS (m/e): 230(M⁺)

### (Step D) 8-Methoxy-spiro[benzopyran-2,1'-cyclohexane]-5-carbaldehyde (Compound IIat-d)

Substantially the same procedure as in Step D of Reference Example 43 was repeated using Compound at-c (46 g) obtained in Step C, N-methylformanilide (100 ml), and phosphorus oxychloride (76 ml) to give Compound at-d (36 g, 69%) as an oily mixture of isomers (1:3).
NMR(CDCl₃, δ, ppm): 1.25-2.10(m, total 10H), 3.91 and 3.94(each s, total 3H); 5.80(d, J=9Hz, 0.75H), 5.90(d, J=9Hz, 0.25H), 6.39(d, J=9Hz, 0.75H), 6.90(d, J=8Hz, 0.25H), 7.16(d, J=1Hz, 0.75H), 7.28(d, J=1Hz, 0.75H), 7.32(d, J=8Hz, 0.25H), 7.45(d, J=9Hz, 0.25H), 9.80(s, 0.75H), 10.0 (s, 0.25H)

### (Step E) Methyl 8-methoxy-spiro[benzopyran-2,1'-cyclohexane]-5-carboxylate (Compound IIat-e)

Substantially the same procedure as in Step E of Reference Example 43 was repeated using Compound at-d (36 g) obtained in Step D and iodide (71 g) to give Compound at-e (4.8 g, 12%) as a pale-yellow solid.
Melting point: 70-75 °C
NMR(CDCl₃, δ, ppm): 1.20-2.03(m, 10H), 3.85(s, 3H), 3.90(s, 3H), 5.83(d, J=9Hz, 1H), 6.77(d, J=8Hz, 1H), 7.32(d, J=9Hz, 1H), 7.55(d, J=8Hz, 1H)
MASS (m/e): 288(M⁺)

### (Step F) Methyl 8-methoxy-spiro[3,4-dihydrobenzopyran-2,1'-cyclohexane]-5-carboxylate (Compound IIat)

Substantially the same procedure as in Step A of Reference Example 42 was repeated using Compound at-e (2.1 g) obtained in Step E to give Compound IIat (2.1 g, 100%) as a pale-yellow oily substance.
NMR(CDCl₃, δ, ppm): 1.25-1.94(m, 12H), 3.10(t, 7Hz, 2H), 3.84(s, 3H), 3.89(s, 3H), 6.73(d, J=9Hz, 1H), 7.55(d, J=9Hz, 1H)

### Reference Example 47

### 4-Methoxy-spiro[2,3-dihydrobenzofuran-2,1'-cyclopentane]-7-carbaldehyde (Compound IIau)

### (Step A) 4-Bromo-3-(2-oxocyclopentyloxy)anisole (Compound IIau-a)

Substantially the same procedure as in Step A of Reference Example 3 was repeated using 2-bromo-5-methoxy-phenol [Journal of Medicinal Chemistry, 1263, (1985)] (13.0 g) to give Compound IIau-a (15.1 g, 83%) as a pale-yellow oily substance.
NMR(CDCl₃, δ, ppm): 1.85-2.50(m, 6H), 3.78(s, 3H), 4.53-4.59(m, 1H), 6.45(dd, J=9, 3Hz, 1H), 6.67(d, J=3Hz, 1H), 7.39(d, J=9H=, 1H)
MASS (m/z) : 284 (M⁺)

### (Step B) 2-Bromo-4-(2-methylenecyclopentyloxy)anisole (Compound IIau-b)

Substantially the same procedure as in Step B of Reference Example 3 was repeated using Compound IIau-a (10.5 g) obtained in Step A to give Compound IIau-b (8.2 g, 79%) as a pale-yellow oily substance.
NMR(CDCl₃, δ, ppm) : 1.66-2.62(m, 6H), 3.77(s, 3H), 4.89-5.92(m, 1H), 5.11-5.12(m, 1H), 5.22-5.23(m, 1H), 6.40(dd, J=9, 3Hz, 1H), 6.57(d, J=3Hz, 1H), 7.40(d, J=9Hz, 1H)
MASS(m/e): 282(M⁺)

### (Step C) 6-Bromo-2-[(2-cyclopenten-1-yl)methyl]-3-methoxyphenol (Compound IIau-c)

Substantially the same procedure as in Step C of Reference Example 3 was repeated using Compound IIau-b (8.2 g) obtained in Step B to give Compound IIau-c (7.6 g, 93%) as a brown oily substance.
NMR(CDCl₃, δ, ppm): 1.80-1.91(m, 2H), 2.24-2.30(m, 4H), 3.47(s, 2H), 3.78(s, 3H), 5.25(s, 1H), 5.62 (s, 1H), 6.41(d, J=9Hz, 1H), 7.27(d, J=9Hz, 1H)
MASS(m/e): 282(M⁺)

### (Step D) 7-Bromo-4-methoxy-spiro[2,3-dihydrobenzofuran-2,1'-cyclopentane] (Compound IIau-d)

Substantially the same procedure as in Step D of Reference Example 3 was repeated using Compound IIau-c (5.7 g) obtained in Step C to give Compound IIau-d (5.5 g, 96%) as a brown oily substance.
NMR(CDCl₃, δ, ppm): 1.65-2.20(m, 8H), 3.17(s, 2H), 3.79(s, 3H), 6.28(d, J=9Hz, 1H), 7.18(d, J=9Hz, 1H)
MASS(m/e): 282(M⁺)

### (Step E) 4-Methoxy-spiro[2,3-dihydrobenzofuran-2,1'-cyclopentane]-7-carbaldehyde (Compound IIau)

Substantially the same procedure as in Step E of Reference Example 3 was repeated using Compound IIau-d (5.5 g) obtained in Step D to give Compound IIau (4.3 g, 95%) as colorless crystals.
NMR(CDCl₃, δ, ppm): 1.70-2.19(m, 8H), 3.09(s, 2H), 3.88(s, 3H), 6.47(d, J=9Hz, 1H), 7.63(d, J=9Hz, 1H), 10.08(s, 1H)
MASS(m/e): 232(M⁺)

### Reference Example 48

### 4-Methoxy-spiro[2,3-dihydrobenzofuran-2,1'-cyclopentane]-7-carboxylic acid (Compound IIav)

Substantially the same procedure as in Step E of Reference Example 47 was repeated using Compound (6.9 g) obtained in Step D of Reference Example 47 and using dry ice instead of DMF to give Compound IIav (3.5 g, 58%) as white crystals.
NMR (CDCl₃, δ, ppm): 1.68-2.23(m, 8H), 3.17(s, 2H), 3.90 (s, 3H),6.55(d, J=9Hz, 1H), 7.83(d, J=9Hz, 1H), 9.63 (brs, 1H)
MASS (m/e) : 248(M⁺)

### Reference Example 49

### Methyl 4-methoxy-spiro[2,3-dihydrobenzofuran-2,1'-cyclopentane]-7-carboxylate (Compound IIaw)

Substantially the same procedure as in Reference Example 15 was repeated using Compound IIav (1.0 g) obtained in Reference Example 48 to give Compound IIaw (0.86 g, 81%) as colorless crystals.
NMR (CDCl₃, δ, ppm): 1.70-2.22(m, 8H), 3.06(s, 2H), 3.85 (s, 3H), 3.87(s, 3H), 6.42(d, J=9Hz, 1H), 7.75(d, J=9Hz, 1H)
MASS (m/e) : 262 (M⁺)

### Reference Example 50

### 7-Methoxy-spiro[1,3-benzodioxole-2,1'-cyclopentane]-4-carbaldehyde (Compound IIax)

### (Step A) 7-Methoxy-spiro[1,3-benzodioxole-2,1'-cyclopentane] (Compound IIax-a)

A mixture of 3-methoxycatechol (22.6 g), cyclopentanone (27.1 g), methyl orthoformate (34.2 g), p-toluenesulfonic acid·monohydrate (0.2 g), and benzene (300 ml) was heated under reflux for 24 hours. After being allowed to stand for cooling, a dilute solution of sodium hydroxide was added to the mixture followed by extraction with ether. The organic layer was washed with a saturated saline and dried over anhydrous potassium carbonate. The solvent was distilled off under reduced pressure to give Compound IIax-a (30 g, 90%) as a colorless oily substance.
NMR(CDCl₃, δ, ppm): 1.79-1.89(m, 4H), 2.06-2.21(m, 4H), 3.89(s, 3H), 6.94-6.50(m, 2H), 6.74(t, J=8Hz, 1H)
MASS(m/e): 206(M⁺)

### (Step B) (Compound IIax)

Compound IIax-a (17.0 g) obtained in Step A was dissolved in dimethylformamide (100 ml), and phosphorus oxychloride (23.1 ml) was added thereto, followed by heating at 60°C for 6 hours. After being allowed to stand for cooling, the reaction solution was poured into ice followed by extraction with ether. The organic layer was washed with a saturated saline and dried over anhydrous potassium carbonate. The solvent was distilled off and the residue was purified by silica gel column chromatography (hexane:ethyl acetate = 20:1) to give Compound IIax (2.1 g, 11%) as colorless crystals.
NMR(CDCl₃, δ, ppm): 1.83-1.91(m, 4H), 2.14-2.24(m, 4H), 3.97(s, 3H), 6.58 (d, J=9Hz, 1H), 7.27(d, J=9Hz, 1H), 9.99(s, 1H)
MASS(m/e): 234(M⁺)

### Reference Example 51

### Methyl 7-methoxy-spiro[1,3-benzodioxole-2,1'-cyclopentane]-4-carboxylate (Compound IIay)

Substantially the same procedure as in Step C of Reference Example 14 was repeated using Compound IIay (3.7 g) obtained in Reference Example 50 to give Compound IIay (2.7 g, 64%) as a colorless oily substance.
NMR(CDCl₃, δ, ppm) : 1.84-1.90(m, 4H), 2.11-2.25(m, 4H), 3.88(s, 3H), 3.94(s, 3H), 6.52(d, J=9Hz, 1H), 7.40(d, J-9H=, 1H)
MASS (m/e) : 264(M⁺)

### Reference Example 52

### 7-Methoxy-spiro[1,3-benzodioxole-2,1'-cyclopentane]-4-carboxylic acid (Compound IIaz)

Substantially the same procedure as in Reference Example 31 was repeated using Compound IIay (1.70 g) obtained in Reference Example 51 to give Compound IIaz (1.54 g, 96%) as colorless crystals.
NMR(COCl₃, δ, ppm): 1.83-1.91(m, 4H), 2.14-2.24(m, 4H), 3.97(s, 3H), 6.58(d, J=9Hz, 1H), 7.27(d, J=9Hz, 1H), 9.63(brs, 1H)
MASS(m/e): 250(M⁺)

### Reference Example 53

### 7-Benzoyl-4-methoxy-spiro[1,3-benzodioxole-2,1'-cyclopentane] (Compound IIba)

### (Step A) 7-(1-Hydroxy-1-phenyl)methyl-4-methoxy-spiro[1,3-benzodioxole-2,1'-cyclopentane] (Compound IIba-a)

Substantially the same procedure as in Step A of Reference Example 36 was repeated using Compound IIax (4.4 g) obtained in Reference Example 50 to give Compound IIba-a (5.6 g, 95%) as a pale-yellow oily substance.
NMR(CDCl₃, δ, ppm): 1.77-1.87(m, 4H), 2.03-2.18(m, 4H), 2.48(d, J=4Hz, 1H), 3.85(s, 3H), 5.92(d, J=4Hz, LH), 6.43(d, J=9Hz, 1H), 7.15(d, J=9Hz, 1H), 7.22-7.43(m, 5H)
MASS(m/e): 312(M⁺)

### (Step B) (Compound IIba)

Substantially the same procedure as in Step B of Reference Example 36 was repeated using Compound IIba-a (5.6 g) obtained in Step A to give Compound IIba (4.9 g, 88%) as a colorless oily substance.
NMR(CDCl₃, δ, ppm): 1.72-1.83(m, 4H), 2.04-2.18(m, 4H), 3.94(s, 3H), 6.56(d, J=9Hz, 1H), 6.68(d, J=9Hz, 1H), 7.40-7.57(m, 3H), 7.77-7.81(m, 2H)
MASS(m/e): 310(M⁺)

### Preparation Example 1 Tablet

Tablets having the following composition are prepared according to a conventional method.

| | |
|---|---|
| Compound 68 | 50 mg |
| Lactose | 60 mg |
| Potato starch | 50 mg |
| Polyvinyl alcohol | 2 mg |
| Magnesium stearate | 1 mg |
| Tar dye | a trace amount |

### Preparation Example 2 Powder

Powder having the following composition is prepared according to a conventional method.

| | |
|---|---|
| Compound 68 | 50 mg |
| Lactose | 250 mg |

### preparation Example 3 Nasal inhalation

A nasal inhalation having the following composition is prepared according to a conventional method.

| | |
|---|---|
| Compound 68 | 1 mg |
| Lactose | 20 mg |

### Preparation Example 4 Ophthalmic preparation

An ophthalmic preparation having the following composition is prepared according to a conventional method.

| | |
|---|---|
| Compound 68 | 10 mg |
| Sodium chloride | 20 mg |
| Methylparaben | 0.1 mg |
| Propylparaben | 0.1 mg |
| Injectable water | q.s. 1.0 ml |

### Preparation Example 5 Transdermal therapeutic system

A transdermal therapeutic system having the following composition is prepared according to a conventional method.

| | |
|---|---|
| Compound 68 | 10 g |
| White beeswax | 80 g |
| Stearyl alcohol | 30 g |
| Cholesterol | 30 g |
| White vaseline | q.s. 1,000 g |

### Preparation Example 6 Suppository

A suppository having the following composition is prepared according to a conventional method.

| | |
|---|---|
| Compound 68 | 10 mg |
| Witepsol W-15 | 1.79 g |

### Preparation Example 7 Injectable Preparation

An injectable preparation having the following composition is prepared according to a conventional method.

| | |
|---|---|
| Compound 68 | 10 mg |
| Injectable water | q.s. 1.0 ml |

### Preparation Example 8 Syrup

A syrup having the following composition is prepared according to a conventional method.

| | |
|---|---|
| Compound 68 | 10 mg |
| Sucrose | 300 mg |
| Methylparaben | 0.5 mg |
| Sodium benzoate | 0.5 mg |
| Lemon flavor | as necessary |
| Dye | as necessary |
| Purified water | q.s. 1.0 ml |

### Preparation Example 9 Nasal spray

A nasal spray having the following composition is prepared according to a conventional method.

| | |
|---|---|
| Compound 68 | 10 mg |
| Sodium chloride | 8 mg |
| Benzalkonium chloride | 0.1 mg |
| Carbopol | 10 mg |
| Purified water | q.s. 1.0 ml |

### Preparation Example 10 Tablet

Tablets having the following composition are prepared according to a conventional method.

| | |
|---|---|
| Compound 68 | 10 mg |
| Lactose | 140 mg |
| Corn starch | 45 mg |
| Sodium croscarmellose | 10 mg |
| Hydroxypropyl cellulose L | 4 mg |
| Magnesium stearate | 1 mg |

### Preparation Example 11 Capsule

Capsules having the following composition are prepared according to a conventional method.

| | |
|---|---|
| Compound 68 | 10 mg |
| Lactose | 185 mg |
| Sodium croscarmellose | 10 mg |
| Hydroxypropyl cellulose L | 4 mg |
| Magnesium stearate | 1 mg |

### Preparation Example 12 Dry syrup

A dry syrup having the following composition is prepared according to a conventional method.

| | |
|---|---|
| Compound 68 | 10 mg |
| Sucrose | 0.7 g |
| D-mannitol | 0.28 g |
| Pullulan | 20 mg |

### Preparation Example 13 Granules

Granules having the following composition are prepared according to a conventional method.

| | |
|---|---|
| Compound 68 | 10 mg |
| Lactose | 0.8 g |
| Corn starch | 0.17 g |
| Hydroxypropyl cellulose L | 30 mg |

### Industrial Applicability

The present invention can provide oxygen-containing heterocyclic compounds which exhibit PDE IV inhibitory activity and which are useful as therapeutic agents for asthma, allergy, rheumatoid arthritis, psoriasis, myocardial infarction, depression, amnesia, multiple sclerosis, Crohn's disease, systemic lupus erythematosus, diabetes, wounds, AIDS, and the like.

## Claims

1. An oxygen-containing heterocyclic compound represented by following Formula (I): 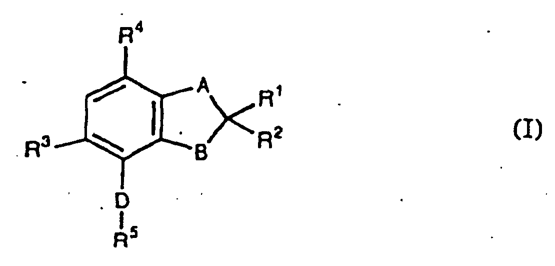 wherein R¹ and R² independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, aralkyl, cyano, or -(CH₂)ₙ-E¹-CO-G¹ (wherein E¹ represents a bond, O, or NH; and G¹ represents hydrogen, substituted or unsubstituted C₁₋₈ alkyl, cycloalkyl, polycycloalkyl, substituted or unsubstituted aryl, a substituted or unsubstituted aromatic heterocyclic group, aralkyl, OR⁶ (wherein R⁶ represents hydrogen, C₁₋₈ alkyl, cycloalkyl, polycycloalkyl, substituted or unsubstituted aryl, a substituted or unsubstituted aromatic heterocyclic group, or aralkyl), or NR⁷R⁸ (wherein R⁷ and R⁸ independently represent hydrogen, C₁₋₈ alkyl, cycloalkyl, polycycloalkyl, substituted or unsubstituted aryl, a substituted or unsubstituted aromatic heterocyclic group, substituted or unsubstituted aralkyl, or heteroarylalkyl; or R⁷ and R⁸ are combined to represent a substituted or unsubstituted heterocyclic group containing a nitrogen atom adjacent thereto); and n represents an integer of 0 to 4}; R¹ and R² are combined to represent a saturated carbon ring together with a carbon atom adjacent thereto; or R², and R¹³ described below are combined to form a single bond;
R³ represents hydrogen, phenyl, or halogen;
R⁴ represents hydroxy or substituted or unsubstituted C₁₋₈ alkoxy;
R⁵ represents substituted or unsubstituted aryl, a substituted or unsubstituted aromatic heterocyclic group (said substituted aromatic heterocyclic group has 1 to 3 substituents), cycloalkyl, pyridine-N-oxide, cyano, or C₁₋₈ alkoxycarbonyl;
A represents -C(R⁹)(R¹⁰)- (wherein R⁹ and R¹⁰ independently represent hydrogen, substituted or unsubstituted C₁₋₈ alkyl, cycloalkyl, or polycycloalkyl) or O;
B represents O,
-C(R¹²)(R¹³)- {wherein R¹² and R¹³ independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, aralkyl, cyano, or -(CH₂)ₚ-E³-CO-G³ (wherein E³, G³, and p have the same meanings as the above-described E¹, G¹, and n, respectively) ; R¹³ and R² are combined to form a single bond; or R¹³ and R² are combined to form a saturated carbon ring together with two carbon atoms adjacent thereto}; or
-C(R¹⁴)(R¹⁵)-C(R¹⁶)(R¹⁷) - {wherein R¹⁴ and R¹⁵ independently represent hydrogen, substituted or unsubstituted C₁₋₈ alkyl, cycloalkyl, aralkyl, substituted or unsubstituted aryl, or a substituted or unsubstituted aromatic heterocyclic group; or
R¹⁴ and R¹⁵ are combined to form O; and R¹⁶ and R¹⁷ independently represent hydrogen, substituted or unsubstituted C₁₋₈ alkyl, cycloalkyl, aralkyl, substituted or unsubstituted aryl, or a substituted or unsubstituted aromatic heterocyclic group; or
R¹⁷ and R¹⁵ are combined to form a single bond; or R¹⁷ and R¹⁵ are combined to form a saturated carbon ring together with two carbon atoms adjacent thereto},
with the proviso that when A represents -C(R⁹)(R¹⁰)-, B does not represent O; and
D represents (i) -C(R¹⁸)(R¹⁹)-X- {wherein R¹⁸ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy; and R¹⁹ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano; or R¹⁸ and R¹⁹ are combined to form O, S, or NR²⁰ (wherein R²⁰ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy); X represents
(a) -C(R²¹)(R²²)- (wherein R²¹ and R²² independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, C₂₋₉ alkanoyl, cycloalkanoyl, C₁₋₈ alkoxycarbonyl, or cyano) or (b) S; or X represents (c) NR²³ (wherein R²³ represents hydrogen, C₁₋₈ alkyl, cycloalkyl, substituted or unsubstituted aryl, a substituted or unsubstituted aromatic heterocyclic group, or aralkyl) unless R¹ and R² simultaneously represent substituted or unsubstituted C₁₋₈ alkyl, cycloalkyl, polycycloalkyl, C₂₋₈ alkenyl, or cycloalkenyl in the above definition or unless R¹ and R² are combined to represent a saturated carbon ring together with a carbon atom adjacent thereto and R⁵ represents substituted or unsubstituted phenyl or substituted or unsubstituted pyridyl}, or
(ii) -C(R^{19a})=Y- {wherein R^{19a} represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano; and Y represents -C(R²⁴)-Z-(wherein R²⁴ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, C₂₋₉ alkanoyl, cycloalkanoyl, C₁₋₈ alkoxycarbonyl, or cyano; or R²⁴ and R^{19a} are combined to form a single bond; and Z represents CONH, CONKCH₂, or a bond) or N), or D represents (iii) a bond Unless R⁵ represents substituted or unsubstituted phenyl, cyano, or C₁₋₈ alkoxycarbonyl,
with the proviso that when R⁵ represents substituted or unsubstituted phenyl or C₁₋₈ alkoxycarbonyl, D does not represent -CH₂CH₂- or -CH=CH-;
or a pharmaceutically acceptable salt thereof and optionally on a form of adducts with water or solvents.

2. The oxygen-containing heterocyclic compound according to claim 1 wherein A is O and B is O; or a pharmaceutically acceptable salt thereof and optionally on a form of adducts with water or solvents.

3. The oxygen-containing heterocyclic compound according to claim 1 or 2 wherein D is -C(R¹⁸)(R¹⁹)-X-; or a pharmaceutically acceptable salt thereof and optionally on a form of adducts with water or solvents.

4. The oxygen-containing heterocyclic compound according to claim 3 wherein R¹⁸ and R¹⁹ are combined to form O; or a pharmaceutically acceptable salt thereof and optionally on a form of adducts with water or solvents.

5. The oxygen-containing heterocyclic compound according to claim 3 or 4 wherein X is -C(R²¹)(R²²)-; or a pharmaceutically acceptable salt thereof and optionally on a form of adducts with water or solvents.

6. The oxygen-containing heterocyclic compound according to any one of claims 1 to 5 wherein R¹ and R² are combined to represent a saturated carbon ring together with a carbon atom adjacent thereto; or a pharmaceutically acceptable salt thereof and optionally on a form of adducts with water or solvents.

7. The oxygen-containing heterocyclic compound according to any one of claims 1 to 6 wherein R⁵ is a substituted or unsubstituted aromatic heterocyclic group: or a pharmaceutically acceptable salt thereof and optionally on a form of adduct with water or solvents.

8. A use of an oxygen-containing heterocyclic compound represented by following Formula (Z): 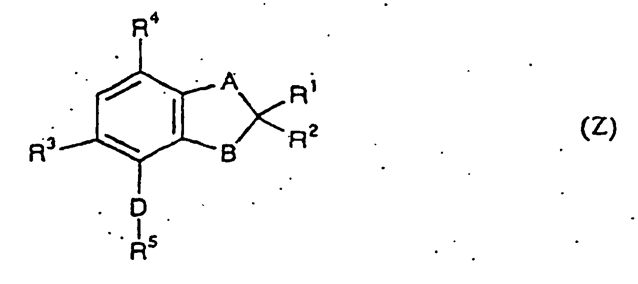 whereinR¹and R² independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, aralkyl, cyano, or -(CH₂)ₙ-E¹-CO-G¹ {wherein E¹ represents a, bond, O, or NH: and G¹ represents hydrogen, substituted or unsubstituted C₁₋₈ alkyl, cycloalkyl, polycycloalkyl, substituted or unsubstixuxed aryl, a substituted or unsubstituted aromatic heterocyclic group, aralkyl, OR⁶ (wherein R⁶ represents hydrogen, C₁₋₈ alkyl, cycloalkyl,-polycycloalkyl, substituted or unsubstituted aryl, a substituted or unsubstituted aromatic heterocyclic group, .or aralkyl), or NR⁷R⁸ (wherein R⁷ and R⁸ independently represent hydrogen, C₁₋₈ alkyl, cycloalkyl, polycycloalkyl, substituted or unsubstituted aryl, a substituted or unsubstituted aromatic heterocyclic group, substituted or unsubstituted aralkyl, or hoteroarylalkyl; or R⁷ and R⁸ are combined to represent a substituted or unsubstituted heterocyclic group containing a nitrogen atom adjacent thereto); and n represents an integer of 0 to 4}: R¹ and R² are combined to represent a saturated carbon ring together with a carbon atom adjacent thereto; or R², and R¹³ described below are combined to form a single bond;
R³ represents hydrogen, phenyl, or halogen;
R⁴ represents hydroxy or substituted or unsubstituted C₁₋₈ alkoxy;
R⁵ represents substituted or unsubstituted aryl, a substituted or unsubstituted aromatic heterocyclic group (said substituted aromatic heterocyclic group has 1 to 3 substituents), cycloalkyl, pyridine-N-oxide, cyano, or C₁₋₈ alkoxycarbonyl;
A represents -C(R⁹)(R¹⁰)- (wherein R⁹ and R¹⁰ independently represent hydrogen, substituted or unsubstituted C₁₋₈ alkyl, cycloalkyl, or polycycloalkyl) or O;
B represents O,
-C(R¹²)(R¹³)- {wherein R¹² and R¹³ independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, aralkyl, cyano, or -(CH₂)ₚ-E³-CO-G³ (wherein E³, G³, and p have the same meanings as the above-described E¹, G¹, and n, respectively); R¹³ and R² are combined to form a single bond; or R¹³ and R² are combined to form a saturated carbon ring together with two carbon atoms adjacent thereto}; or
-C(R¹⁴)(R¹⁵)-C(R¹⁶)(R¹⁷)- {wherein R¹⁴ and R¹⁵ independently represent hydrogen, substituted or unsubstituted C₁₋₈ alkyl, cycloalkyl, aralkyl, substituted or unsubstituted aryl, or a substituted or unsubstituted aromatic heterocyclic group; or R¹⁴ and R¹⁵ are combined to form O; and R¹⁶ and R¹⁷ independently represent hydrogen, substituted or unsubstituted C₁₋₈ alkyl, cycloalkyl, aralkyl, substituted or unsubstituted aryl, or a substituted or unsubstituted aromatic heterocyclic group; or R¹⁷ and R¹⁵ are combined to form a single bond; or R¹⁷ and R¹⁵ are combined to form a saturated carbon ring together with two carbon atoms adjacent thereto}; and
D represents (i) -C(R¹⁸)(R¹⁹)-x- {wherein R¹⁸ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy; and R¹⁹ represents hydrogen, substituted or unsubstituted C₁₋₈ alkyl, cycloalkyl, polycycloalkyl. C₂₋₈ alkenyl, cycloalkenyl, substituted or unsubstituted aryl, a substituted or unsubstituted aromatic heterocyclicgroup, hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano: or R¹⁸ and R¹⁹ are combined to form O, S, or NR²⁰ (wherein R²⁰ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy); X represents (a) -C(R²¹)(R²²)- (wherein R²¹ and R²² independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, C₂₋₉ alkanoyl, cycloalkanoyl, C₁₋₈ alkoxycarbonyl, or cyano) or (b) S; or X represents (c) NR²³ (wherein R²³ represents hydrogen, C₁₋₈ alkyl, cycloalkyl, substituted or unsubstituted aryl, a substituted or unsubstituted aromatic heterocyclic group, or aralkyl) unless R¹ and R² simultaneously represent substituted or unsubstituted C₁₋₈ alkyl, cycloalkyl, polycycloalkyl, C₂₋₈ alkenyl, or cycloalkenyl in the above definition or unless R¹ and R² are combined to represent a saturated carbon ring together with a carbon atom adjacent thereto and R⁵ represents substituted or unsubstituted phenyl or substituted or substituted pyridyl},
(ii) -C(R^{19a})=Y- {wherein R^{19a} represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano: and Y represents -C(R²⁴)-Z- (wherein R²⁴ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, C₂₋₉ alkanoyl, cycloalkanoyl, C₁₋₈ alkoxycarbonyl, or cyano; or R²⁴ and R^{19a} are combined to form a single bond; and Z represents CONH, CONHCH₂,
or a bond) or N}, or (iii) a bond unless R₅ represents substituted or unsubstituted phenyl, cyano, or C₁₋₈ alkoxycarbonyl
or a pharmaceutically acceptable salt thereof and optionally on a form of adducts with water or solvents for the preparation of a phosphodiesterase IV inhibitor.

9. Use according to claim 8, of the oxygen-containing heterocyclic compound according to any one of claims 1 to 7 or a pharmaceutically acceptable salt thereof and optionally on a form of addsuts with water or solvents

10. A use of an oxygen-containing heterocyclic compound represented by following Formula (ZA): 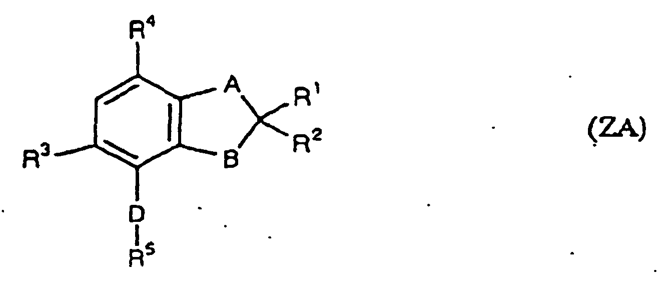 wherein R¹ and R² independently represent hydrogen, substituted or unsubstituted C₁₋₈ alkyl, cycloalkyl, polycycloalkyl, C₂₋₈ alkenyl, cycloalkenyl, substituted or unsubsritured aryl, a substituted or unsubstituted aromatic heterocyclic group, aralkyl, cyano, or -(CH₂)ₙ-E¹-CO-G¹ {wherein E¹ represents a bond, O, or NH: and G¹ represents hydrogen, substituted or unsubstituted C₁₋₈ alkyl, cycloalkyl, polycycloalkyl, substituted or unsubstituted aryl, a substituted or unsubstituted aromatic heterocyclic group, aralkyl, OR⁶ (wherein R⁶ represents hydrogen, C₁₋₈ alkyl, cycloalkyl, polycycloalkyl, substituted or unsubstituted aryl, a substituted or unsubstituted aromatic heterocyclic group, or aralkyl), or NR⁷R⁸ (wherein R⁷ and R⁸ independently represent hydrogen, C₁₋₈ alkyl, cycloalkyl, polycycloalkyl, substituted or unsubstituted aryl, a substituted or unsubstituted aromatic heterocyclic group, substituted or unsubstituted aralkyl, or heteroarylalkyl; or R⁷ and R⁸ are combined to represent a substituted or unsubstituted heterocyclic group containing a nitrogen atom adjacent thereto): and n represents an integer of 0 to 4}; R¹ and R² are combined to represent a saturated carbon ring together with a carbon atom adjacent thereto; or R², and R¹³ described below are combined to form a single bond;
R³ represents hydrogen, phenyl, or halogen:
R⁴ represents hydroxy or substituted or unsubstituted C₁₋₈ alkoxy;
R⁵ represents substituted or unsubstituted aryl, a substituted or unsubstituted aromaticheterocyclic group (said substituted aromatic heterocyclic group has 1 to 3 substituents), cycloalkyl, pyridine-N-oxide, cyano, or C₁₋₈ alkoxycarbonyl;
A represents -C(R⁹)(R¹⁰)- (wherein R⁹ and R¹⁰ independently represent hydrogen, substituted or unsubstituted C₁₋₈ alkyl, cycloalkyl, or polycycloalkyl) or O;
B represents O,
-C(R¹²)(R¹³)- {wherein R¹² and R¹³ independently represent hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, aralkyl, cyano, or -(CH₂)ₚ-E³-CO-G³ (wherein E³, G³, and p have the same meanings as the above-described E¹. G¹, and n, respectively); R¹³ and R² are combined to form a single bond; or R¹³ and R² are combined to form a saturated carbon ring together with two carbon atoms adjacent thereto}; or
-C(R¹⁴)(R¹⁵)-C(R¹⁶)(R¹⁷)- {wherein R¹⁴ and R¹⁵ independently represent hydrogen, substituted or unsubstituted C₁₋₈ alkyl, cycloalkyl, aralkyl, substituted or unsubstituted aryl, or a substituted or unsubstituted aromatic heterocyclic group; or R¹⁴ and R¹⁵ are combined to form O; and R¹⁶ and R¹⁷ independently represent hydrogen, substituted or unsubstituted C₁₋₈ alkyl, cycloalkyl, aralkyl, substituted or unsubstituted aryl, or a substituted or unsubstituted aromatic heterocyclic group; or R¹⁷ and R¹⁵ are combined to form a single bond; or R¹⁷ and R¹⁵ are combined to form a saturated carbon ring together with two carbon atoms adjacent thereto}; and
D represents (i) -C(R¹⁸)(R¹⁹)-X- {wherein R¹⁸ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy; and R¹⁹ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, C₂₋₉ alkanoyloxy, C₂₋₉ alkanoyl, cycloalkanoyl, C₁₋₈ alkoxycarbonyl, or cyano; or R¹⁸ and R¹⁹ are combined to form O, S, or NR²⁰ (wherein R²⁰ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, or C₂₋₉ alkanoyloxy); X represents (a) -C(R²¹)(R²²)- (wherein R²¹ and R²² independently represent hydrogen, substituted or unsubstituted C₁₋₈ alkyl, cycloalkyl, polycycloalkyl. C₂₋₈ alkenyl, cycloalkenyl, subscttuted or unsubstituted aryl, a substituted or unsubstituted aromaticheterocyclic group, C₂₋₉ alkanoyl, cycloalkanoyl, C₁₋₈ alkoxycarbonyl, or cyano) or (b) S; or X represents (c) NR²³ (wherein R²³ represents hydrogen. C₁₋₈ alkyl, cycloalkyl, substituted or unsubstituted aryl, a substituted or unsubstituted aromatic heterocyclic group, or aralkyl) unless R¹ and R² simultaneously represent substituted or unsubstituted C₁₋₈ alkyl, cycloalkyl, polycycloalkyl, C₂₋₈ alkenyl, or cycloalkenyl in the above definition or unless R¹ andR² are combined to represent a saturated carbon ring together with a carbon atom adjacent thereto and R⁵ represents substituted or unsubstituted phenyl or substituted or substituted pyridyl}, or
(ii) -C(R^{19a})=Y- {wherein R^{19a} represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, hydroxy, substituted or unsubstituted C₁₋₈ alkoxy, C₂₋₉ alkanoyloxy. C₂₋₉ alkanoyl, cycloalkanoyl, C₁₋₈ alkoxycarbonyl, or cyano; and Y represents -C(R²⁴)-Z- (wherein R²⁴ represents hydrogen, substituted or unsubstituted C₁₋₈ alkyl, cycloalkyl, polycycloalkyl, C₂₋₈ alkenyl, cycloalkenyl, substituted or unsubstituted aryl, a substituted or unsubstituted aromatic heterocyclic group, C₂₋₉ alkanoyl, cycloalkanoyl, C₁₋₈ alkoxycarbonyl, or cyano; or R²⁴ and R^{19a} are combined to form a single bond; and Z represents CONH, CONHCH₂, or a bond) or N}, orD represents (iii) a bond unless R⁵ represents substituted or unsubstituted phenyl, cyano, or C₁₋₈ alkoxycarbonyl,
or a pharmaceutically acceptable salt thereof and optionally on a form of adducts with water or solvents for the preparation of a pharmaceutical composition which is useful for the treatment of inflammatory allergic diseases.

11. A use according to claim 10, of the oxygen-containing heterocyclic compound according to any one of claims 1 to 7 or a pharmaceutically acceptable salt thereof and optionally on a form of adducts with water or solvents.

## Revendications

1. Composé hétérocyclique oxygéné, représenté par la formule (I) suivante : 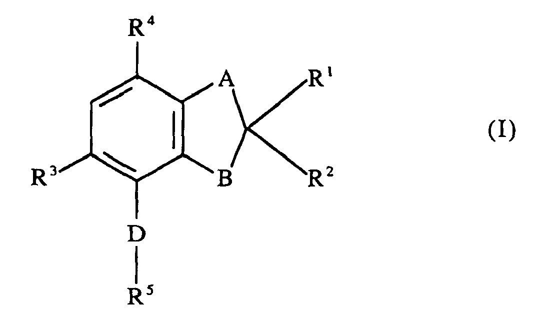 dans laquelle
- R¹ et R² représentent chacun, indépendamment, un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, aralkyle ou cyano, ou encore un fragment de formule -(CH₂)ₙ-E¹-CO-G¹ {où E¹ représente une liaison
ou un chaînon -O- ou -NH-, G¹ représente un atome d'hydrogène ou un groupe alkyle en C₁₋₈ avec ou sans substituant, cycloalkyle, polycycloalkyle, aryle avec ou sans substituant, hétéroaryle avec ou sans substituant ou aralkyle, ou encore un groupe de formule OR⁶ (où R⁶ représente un atome d'hydrogène ou un groupe alkyle en C₁₋₈, cycloalkyle, polycycloalkyle, aryle avec ou sans substituant, hétéroaryle avec ou sans substituant ou aralkyle) ou NR⁷R⁸ (où R⁷ et R⁸ représentent chacun, indépendamment, un atome d'hydrogène ou un groupe alkyle en C₁₋₈, cycloalkyle, polycycloalkyle, aryle avec ou sans substituant, hétéroaryle avec ou sans substituant, aralkyle avec ou sans substituant ou hétéroaryl-alkyle, ou bien R⁷ et R⁸ représentent conjointement un groupe hétérocyclique avec ou sans substituant, comportant l'atome d'azote adjacent), et n représente un nombre entier valant de 0 à 4}, ou bien R¹ et R² représentent conjointement un carbocycle saturé formé avec l'atome de carbone adjacent, ou encore R² représente une liaison, conjointement avec le symbole R¹³ traité plus loin ;
- R³ représente un atome d'hydrogène ou d'halogène ou un groupe phényle ;
- R⁴ représente un groupe hydroxyle ou alcoxy en C₁₋₈ avec ou sans substituant ;
- R⁵ représente un groupe aryle avec ou sans substituant, hétéroaryle sans substituant ou portant 1 à 3 substituants, cycloalkyle, N-oxydo-pyridyle, cyano ou (alcoxy en C₁₋₈)carbonyle,
- A représente un chaînon -O- ou -C(R⁹)(R¹⁰)- où R⁹ et R¹⁰ représentent chacun, indépendamment, un atome d'hydrogène ou un groupe alkyle en C₁₋₈ avec ou sans substituant, cycloalkyle ou polycycloalkyle,
- B représente un chaînon -O- ou -C(R¹²)(R¹³)- {où R¹² et R¹³ représentent chacun, indépendamment, un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, aralkyle ou cyano, ou encore un fragment de formule -(CH₂)ₚ-E³-CO-G³ (où E³, G³ et p ont respectivement les mêmes significations que les symboles E¹, G¹ et n traités plus haut), ou bien R¹³ et R² représentent conjointement une liaison, ou encore R¹³ et R² représentent conjointement un carbocycle saturé formé avec les deux atomes de carbone adjacents}, ou un fragment de formule -C(R¹⁴)(R¹⁵)-C(R¹⁶)(R¹⁷)-{où R¹⁴ et R¹⁵ représentent chacun, indépendamment, un atome d'hydrogène ou un groupe alkyle en C₁₋₈ avec ou sans substituant, cycloalkyle, aralkyle, aryle avec ou sans substituant ou hétéroaryle avec ou sans substituant, ou bien R¹⁴ et R¹⁵ représentent conjointement un atome d'oxygène, et R¹⁶ et R¹⁷ représentent chacun, indépendamment, un atome d'hydrogène ou un groupe alkyle en C₁₋₈ avec ou sans substituant, cycloalkyle, aralkyle, aryle avec ou sans substituant ou hétéroaryle avec ou sans substituant, ou bien R¹⁷ et R¹⁵ représentent conjointement une liaison,
ou R¹⁷ et R¹⁵ représentent conjointement un carbocycle saturé formé avec les deux atomes de carbone adjacents} ;
sous réserve que, si A représente -C(R⁹)(R¹⁰)-, B ne représente pas un chaînon ―O― ;
- et D représente
i) un fragment de formule -C(R¹⁸)(R¹⁹)-X- où R¹⁸ représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, hydroxyle, alcoxy en C₁₋₈ avec ou sans substituant ou alcanoyloxy en C₂₋₉, R¹⁹ représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, hydroxyle, alcoxy en C₁₋₈ avec ou sans substituant, alcanoyloxy en C₂₋₉, alcanoyle en C₂₋₉, cycloalcanoyle, (alcoxy en C₁₋₈)carbonyle ou cyano, ou bien R¹⁸ et R¹⁹ représentent conjointement un atome d'oxygène ou de soufre ou un groupe de formule NR²⁰ (où R²⁰ représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, hydroxyle, alcoxy en C₁₋₈ avec ou sans substituant ou alcanoyloxy en C₂₋₉), et X représente
a) un chaînon -C(R²¹)(R²²)- où R²¹ et R²² représentent chacun, indépendamment, un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, alcanoyle en C₂₋₉, cycloalcanoyle, (alcoxy en C₁₋₈)carbonyle ou cyano,
b) un chaînon -S-,
c) ou un chaînon -N(R²³)- où R²³ représente un atome d'hydrogène ou un groupe alkyle en C₁₋₈, cycloalkyle, aryle avec ou sans substituant, hétéroaryle avec ou sans substituant ou aralkyle,
sauf si R¹ et R² représentent simultanément, parmi leurs significations indiquées plus haut, des groupes alkyle en C₁₋₈ avec ou sans substituant, cycloalkyle, polycycloalkyle, alcényle en C₂₋₈ ou cycloalcényle, ou sauf si R¹ et R² représentent conjointement un carbocycle saturé formé avec l'atome de carbone adjacent et R⁵ représente un groupe phényle avec ou sans substituant ou pyridyle avec ou sans substituant,
ii) un fragment de formule -C(R^{19A})=Y- où R^{19A} représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, hydroxyle, alcoxy en C₁₋₈ avec ou sans substituant, alcanoyloxy en C₂₋₉, alcanoyle en C₂₋₉, cycloalcanoyle, (alcoxy en C₁₋₈)carbonyle ou cyano, et Y représente un atome d'azote ou un fragment de formule -C(R²⁴)-Z- où R²⁴ représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, alcanoyle en C₂₋₉, cycloalcanoyle, (alcoxy en C₁₋₈)carbonyle ou cyano, ou bien R²⁴ et R^{19A} représentent conjointement une liaison, et Z représente une liaison ou un groupe de formule -CONH- ou -CONH-CH₂- ;
iii) ou une liaison, sauf si R⁵ représente un groupe phényle avec ou sans substituant, cyano ou (alcoxy en C₁₋₈)carbonyle,
sous réserve que, si R⁵ représente un groupe phényle avec ou sans substituant ou (alcoxy en C₁₋₈)carbonyle, D ne représente pas -CH₂CH₂- ou -CH=CH- ;
ou sel pharmacologiquement admissible d'un tel composé, éventuellement sous la forme d'hydrate ou de solvate.

2. Composé hétérocyclique oxygéné, conforme à la revendication 1, dans lequel A représente un chaînon -O- et B représente un chaînon -O-, ou sel pharmacologiquement admissible d'un tel composé, éventuellement sous la forme d'hydrate ou de solvate.

3. Composé hétérocyclique oxygéné, conforme à la revendication 1 ou 2, dans lequel D représente un fragment de formule -C(R¹⁸)(R¹⁹)-X-, ou sel pharmacologiquement admissible d'un tel composé, éventuellement sous la forme d'hydrate ou de solvate.

4. Composé hétérocyclique oxygéné, conforme à la revendication 3, dans lequel R¹⁸ et R¹⁹ représentent conjointement un atome d'oxygène, ou sel pharmacologiquement admissible d'un tel composé, éventuellement sous la forme d'hydrate ou de solvate.

5. Composé hétérocyclique oxygéné, conforme à la revendication 3 ou 4, dans lequel X représente un chaînon -C(R²¹)(R²²)-, ou sel pharmacologiquement admissible d'un tel composé, éventuellement sous la forme d'hydrate ou de solvate.

6. Composé hétérocyclique oxygéné, conforme à l'une des revendications 1 à 5, dans lequel R¹ et R² représentent conjointement un carbocycle saturé formé avec l'atome de carbone adjacent, ou sel pharmacologiquement admissible d'un tel composé, éventuellement sous la forme d'hydrate ou de solvate.

7. Composé hétérocyclique oxygéné, conforme à l'une des revendications 1 à 5, dans lequel R⁵ représente un groupe hétéroaryle avec ou sans substituant, ou sel pharmacologiquement admissible d'un tel composé, éventuellement sous la forme d'hydrate ou de solvate.

8. Emploi d'un composé hétérocyclique oxygéné, représenté par la formule (Z) suivante : 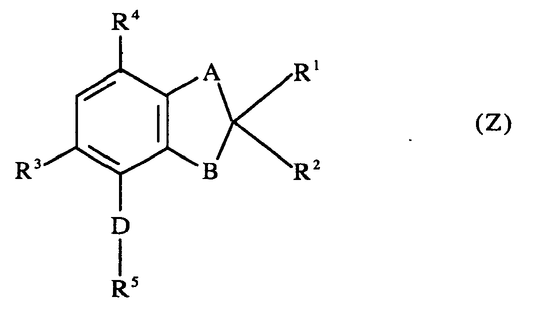 dans laquelle
- R¹ et R² représentent chacun, indépendamment, un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, aralkyle ou cyano, ou encore un fragment de formule -(CH₂)ₙ-E¹-CO-G¹ {où E¹ représente une liaison
ou un chaînon -O- ou -NH-, G¹ représente un atome d'hydrogène ou un groupe alkyle en C₁₋₈ avec ou sans substituant, cycloalkyle, polycycloalkyle, aryle avec ou sans substituant, hétéroaryle avec ou sans substituant ou aralkyle, ou encore un groupe de formule OR⁶ (où R⁶ représente un atome d'hydrogène ou un groupe alkyle en C₁₋₈, cycloalkyle, polycycloalkyle, aryle avec ou sans substituant, hétéroaryle avec ou sans substituant ou aralkyle) ou NR⁷R⁸ (où R⁷ et R⁸ représentent chacun, indépendamment, un atome d'hydrogène ou un groupe alkyle en C₁₋₈, cycloalkyle, polycycloalkyle, aryle avec ou sans substituant, hétéroaryle avec ou sans substituant, aralkyle avec ou sans substituant ou hétéroaryl-alkyle, ou bien R⁷ et R⁸ représentent conjointement un groupe hétérocyclique avec ou sans substituant, comportant l'atome d'azote adjacent), et n représente un nombre entier valant de 0 à 4}, ou bien R¹ et R² représentent conjointement un carbocycle saturé formé avec l'atome de carbone adjacent, ou encore R² représente une liaison, conjointement avec le symbole R¹³ traité plus loin ;
- R³ représente un atome d'hydrogène ou d'halogène ou un groupe phényle ;
- R⁴ représente un groupe hydroxyle ou alcoxy en C₁₋₈ avec ou sans substituant ;
- R⁵ représente un groupe aryle avec ou sans substituant, hétéroaryle sans substituant ou portant 1 à 3 substituants, cycloalkyle, N-oxydo-pyridyle, cyano ou (alcoxy en C₁₋₈)carbonyle,
- A représente un chaînon -O- ou -C(R⁹)(R¹⁰)- où R⁹ et R¹⁰ représentent chacun, indépendamment, un atome d'hydrogène ou un groupe alkyle en C₁₋₈ avec ou sans substituant, cycloalkyle ou polycycloalkyle,
- B représente un chaînon -O- ou -C(R¹²)(R¹³)- {où R¹² et R¹³ représentent chacun, indépendamment, un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, aralkyle ou cyano, ou encore un fragment de formule -(CH₂)p-E³-CO-G³ (où E³, G³ et p ont respectivement les mêmes significations que les symboles E¹, G¹ et n traités plus haut), ou bien R¹³ et R² représentent conjointement une liaison, ou encore R¹³ et R² représentent conjointement un carbocycle saturé formé avec les deux atomes de carbone adjacents}, ou un fragment de formule -C(R¹⁴)(R¹⁵)-C(R¹⁶)(R¹⁷)-{où R¹⁴ et R¹⁵ représentent chacun, indépendamment, un atome d'hydrogène ou un groupe alkyle en C₁₋₈ avec ou sans substituant, cycloalkyle, aralkyle, aryle avec ou sans substituant ou hétéroaryle avec ou sans substituant, ou bien R¹⁴ et R¹⁵ représentent conjointement un atome d'oxygène, et R¹⁶ et R¹⁷ représentent chacun, indépendamment, un atome d'hydrogène ou un groupe alkyle en C₁₋₈ avec ou sans substituant, cycloalkyle, aralkyle, aryle avec ou sans substituant ou hétéroaryle avec ou sans substituant, ou bien R¹⁷ et R¹⁵ représentent conjointement une liaison, ou R¹⁷ et R¹⁵ représentent conjointement un carbocycle saturé formé avec les deux atomes de carbone adjacents} ;
- et D représente
i) un fragment de formule -C(R¹⁸)(R¹⁹)-X- où R¹⁸ représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, hydroxyle, alcoxy en C₁₋₈ avec ou sans substituant ou alcanoyloxy en C₂₋₉, R¹⁹ représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, hydroxyle, alcoxy en C₁₋₈ avec ou sans substituant, alcanoyloxy en C₂₋₉, alcanoyle en C₂₋₉, cycloalcanoyle, (alcoxy en C₁₋₈)carbonyle ou cyano, ou bien R¹⁸ et R¹⁹ représentent conjointement un atome d'oxygène ou de soufre ou un groupe de formule NR²⁰ (où R²⁰ représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, hydroxyle, alcoxy en C₁₋₈ avec ou sans substituant ou alcanoyloxy en C₂₋₉), et X représente
a) un chaînon -C(R²¹)(R²²)- où R²¹ et R²² représentent chacun, indépendamment, un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, alcanoyle en C₂₋₉, cycloalcanoyle, (alcoxy en C₁₋₈)carbonyle ou cyano,
b) un chaînon -S-,
c) ou un chaînon -N(R²³)- où R²³ représente un atome d'hydrogène ou un groupe alkyle en C₁₋₈, cycloalkyle, aryle avec ou sans substituant, hétéroaryle avec ou sans substituant ou aralkyle,
sauf si R¹ et R² représentent simultanément, parmi leurs significations indiquées plus haut, des groupes alkyle en C₁₋₈ avec ou sans substituant, cycloalkyle, polycycloalkyle, alcényle en C₂₋₈ ou cycloalcényle, ou sauf si R¹ et R² représentent conjointement un carbocycle saturé formé avec l'atome de carbone adjacent et R⁵ représente un groupe phényle avec ou sans substituant ou pyridyle avec ou sans substituant,
ii) un fragment de formule -C(R^{19A})=Y- où R^{19A} représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, hydroxyle, alcoxy en C₁₋₈ avec ou sans substituant, alcanoyloxy en C₂₋₉, alcanoyle en C₂₋₉, cycloalcanoyle, (alcoxy en C₁₋₈)carbonyle ou cyano, et Y représente un atome d'azote ou un fragment de formule -C(R²⁴)-Z- où R²⁴ représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, alcanoyle en C₂₋₉, cycloalcanoyle, (alcoxy en C₁₋₈)carbonyle ou cyano, ou bien R²⁴ et R^{19A} représentent conjointement une liaison, et Z représente une liaison ou un groupe de formule -CONH- ou -CONH-CH₂- ;
iii) ou une liaison, sauf si R⁵ représente un groupe phényle avec ou sans substituant, cyano ou (alcoxy en C₁₋₈)carbonyle,
ou d'un sel pharmacologiquement admissible d'un tel composé, éventuellement sous la forme d'hydrate ou de solvate, en vue de la préparation d'un inhibiteur de phosphodiesterase IV.

9. Emploi, conforme à la revendication 8, d'un composé hétérocyclique oxygéné conforme à l'une des revendications 1 à 7 ou d'un sel pharmacologiquement admissible d'un tel composé, éventuellement sous la forme d'hydrate ou de solvate.

10. Emploi d'un composé hétérocyclique oxygéné, représenté par la formule (ZA) suivante : 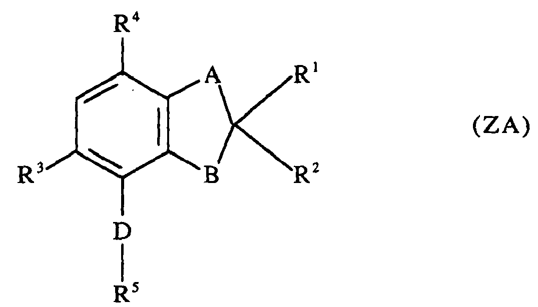 dans laquelle
- R¹ et R² représentent chacun, indépendamment, un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, aralkyle ou cyano, ou encore un fragment de formule -(CH₂)ₙ-E¹-CO-G¹ {où E¹ représente une liaison
ou un chaînon -O- ou -NH-, G¹ représente un atome d'hydrogène ou un groupe alkyle en C₁₋₈ avec ou sans substituant, cycloalkyle, polycycloalkyle, aryle avec ou sans substituant, hétéroaryle avec ou sans substituant ou aralkyle, ou encore un groupe de formule OR⁶ (où R⁶ représente un atome d'hydrogène ou un groupe alkyle en C₁₋₈, cycloalkyle, polycycloalkyle, aryle avec ou sans substituant, hétéroaryle avec ou sans substituant ou aralkyle) ou NR⁷R⁸ (où R⁷ et R⁸ représentent chacun, indépendamment, un atome d'hydrogène ou un groupe alkyle en C₁₋₈, cycloalkyle, polycycloalkyle, aryle avec ou sans substituant, hétéroaryle avec ou sans substituant, aralkyle avec ou sans substituant ou hétéroaryl-alkyle, ou bien R⁷ et R⁸ représentent conjointement un groupe hétérocyclique avec ou sans substituant, comportant l'atome d'azote adjacent), et n représente un nombre entier valant de 0 à 4}, ou bien R¹ et R² représentent conjointement un carbocycle saturé formé avec l'atome de carbone adjacent, ou encore R² représente une liaison, conjointement avec le symbole R¹³ traité plus loin ;
- R³ représente un atome d'hydrogène ou d'halogène ou un groupe phényle ;
- R⁴ représente un groupe hydroxyle ou alcoxy en C₁₋₈ avec ou sans substituant ;
- R⁵ représente un groupe aryle avec ou sans substituant, hétéroaryle sans substituant ou portant 1 à 3 substituants, cycloalkyle, N-oxydo-pyridyle, cyano ou (alcoxy en C₁₋₈)carbonyle,
- A représente un chaînon -O- ou -C(R⁹)(R¹⁰)- où R⁹ et R¹⁰ représentent chacun, indépendamment, un atome d'hydrogène ou un groupe alkyle en C₁₋₈ avec ou sans substituant, cycloalkyle ou polycycloalkyle,
- B représente un chaînon -O- ou -C(R¹²)(R¹³)- {où R¹² et R¹³ représentent chacun, indépendamment, un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, aralkyle ou cyano, ou encore un fragment de formule -(CH₂)ₚ-E³-CO-G³ (où E³, G³ et p ont respectivement les mêmes significations que les symboles E¹, G¹ et n traités plus haut), ou bien R¹³ et R² représentent conjointement une liaison, ou encore R¹³ et R² représentent conjointement un carbocycle saturé formé avec les deux atomes de carbone adjacents}, ou un fragment de formule -C(R¹⁴)(R¹⁵)-C(R¹⁶)(R¹⁷)-{où R¹⁴ et R¹⁵ représentent chacun, indépendamment, un atome d'hydrogène ou un groupe alkyle en C₁₋₈ avec ou sans substituant, cycloalkyle, aralkyle, aryle avec ou sans substituant ou hétéroaryle avec ou sans substituant, ou bien R¹⁴ et R¹⁵ représentent conjointement un atome d'oxygène, et R¹⁶ et R¹⁷ représentent chacun, indépendamment, un atome d'hydrogène ou un groupe alkyle en C₁₋₈ avec ou sans substituant, cycloalkyle, aralkyle, aryle avec ou sans substituant ou hétéroaryle avec ou sans substituant, ou bien R¹⁷ et R¹⁵ représentent conjointement une liaison,
ou R¹⁷ et R¹⁵ représentent conjointement un carbocycle saturé formé avec les deux atomes de carbone adjacents} ;
- et D représente
i) un fragment de formule -C(R¹⁸)(R¹⁹)-X- où R¹⁸ représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, hydroxyle, alcoxy en C₁₋₈ avec ou sans substituant ou alcanoyloxy en C₂₋₉, R¹⁹ représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, hydroxyle, alcoxy en C₁₋₈ avec ou sans substituant, alcanoyloxy en C₂₋₉, alcanoyle en C₂₋₉, cycloalcanoyle, (alcoxy en C₁₋₈)carbonyle ou cyano, ou bien R¹⁸ et R¹⁹ représentent conjointement un atome d'oxygène ou de soufre ou un groupe de formule NR²⁰ (où R²⁰ représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, hydroxyle, alcoxy en C₁₋₈ avec ou sans substituant ou alcanoyloxy en C₂₋₉), et X représente
a) un chaînon -C(R²¹)(R²²)- où R²¹ et R²² représentent chacun, indépendamment, un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, alcanoyle en C₂₋₉, cycloalcanoyle, (alcoxy en C₁₋₈)carbonyle ou cyano,
b) un chaînon -S-,
c) ou un chaînon -N(R²³)- où R²³ représente un atome d'hydrogène ou un groupe alkyle en C₁₋₈, cycloalkyle, aryle avec ou sans substituant, hétéroaryle avec ou sans substituant ou aralkyle,
sauf si R¹ et R² représentent simultanément, parmi leurs significations indiquées plus haut, des groupes alkyle en C₁₋₈ avec ou sans substituant, cycloalkyle, polycycloalkyle, alcényle en C₂₋₈ ou cycloalcényle, ou sauf si R¹ et R² représentent conjointement un carbocycle saturé formé avec l'atome de carbone adjacent et R⁵ représente un groupe phényle avec ou sans substituant ou pyridyle avec ou sans substituant,
ii) un fragment de formule -C(R^{19A})=Y- où R^{19A} représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, hydroxyle, alcoxy en C₁₋₈ avec ou sans substituant, alcanoyloxy en C₂₋₉, alcanoyle en C₂₋₉, cycloalcanoyle, (alcoxy en C₁₋₈)carbonyle ou cyano, et Y représente un atome d'azote ou un fragment de formule -C(R²⁴)-Z- où R²⁴ représente un atome d'hydrogène ou un groupe alkyle en C₁₋₈ avec ou sans substituant, cycloalkyle, polycycloalkyle, alcényle en C₂₋₈, cycloalcényle, aryle avec ou sans substituant, hétéroaryle avec ou sans substituant, alcanoyle en C₂₋₉, cycloalcanoyle, (alcoxy en C₁₋₈)carbonyle ou cyano, ou bien R²⁴ et R^{19A} représentent conjointement une liaison, et Z représente une liaison ou un groupe de formule -CONH- ou -CONH-CH₂- ;
iii) ou une liaison, sauf si R⁵ représente un groupe phényle avec ou sans substituant, cyano ou (alcoxy en C₁₋₈)carbonyle,
ou d'un sel pharmacologiquement admissible d'un tel composé, éventuellement sous la forme d'hydrate ou de solvate, en vue de la préparation d'une composition pharmaceutique utilisable dans le traitement de maladies allergiques inflammatoires.

11. Emploi, conforme à la revendication 10, d'un composé hétérocyclique oxygéné conforme à l'une des revendications 1 à 7 ou d'un sel pharmacologiquement admissible d'un tel composé, éventuellement sous la forme d'hydrate ou de solvate.

## Patentansprüche

1. Sauerstoffhaltige heterocyclische Verbindung, die durch die folgende Formel (I) repräsentiert wird: 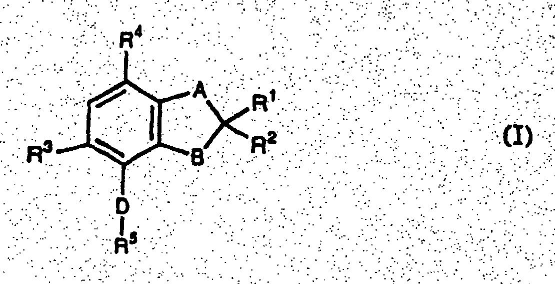 wobei R¹ und R² unabhängig voneinander Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Aralkyl, Cyan oder -(CH₂)ₙ-E¹-CO-G¹ repräsentieren {wobei E¹ eine Bindung, O oder NH repräsentiert; und G¹ Wasserstoff, substituiertes oder unsubstituiertes C₁₋₈-Alkyl, Cycloalkyl, Polycycloalkyl, substituiertes oder unsubstituiertes Aryl, eine substituierte oder unsubstituierte heterocyclische aromatische Gruppe, Aralkyl, OR⁶ (wobei R⁶ Wasserstoff, C₁₋₈-Alkyl, Cycloalkyl, Polycycloalkyl, substituiertes oder unsubstituiertes Aryl, eine substituierte oder unsubstituierte heterocyclische aromatische Gruppe oder Aralkyl repräsentiert), oder NR⁷R⁸ repräsentiert (wobei R⁷ und R⁸ unabhängig voneinander Wasserstoff, C₁₋₈-Alkyl, Cycloalkyl, Polycycloalkyl, substituiertes oder unsubstituiertes Aryl, eine substituierte oder unsubstituierte heterocyclische aromatische Gruppe, substituiertes oder unsubstituiertes Aralkyl oder Heteroarylalkyl repräsentieren; oder R⁷ und R⁸ kombiniert sind, um eine substituierte oder unsubstituierte heterocyclische Gruppe zu bilden, die ein daran angrenzendes Stickstoffatom enthält); und n eine ganze Zahl von 0 bis 4 repräsentiert}; R¹ und R² kombiniert sind, um zusammen mit einem daran angrenzenden Kohlenstoffatom einen gesättigten Kohlenstoffring zu repräsentieren; oder R² und R¹³, die nachstehend beschrieben werden, kombiniert sind, um eine Einfachbindung zu bilden;
R³ Wasserstoff, Phenyl oder Halogen repräsentiert;
R⁴ Hydroxyl oder substituiertes oder unsubstituiertes C₁₋₈-Alkoxyl repräsentiert;
R⁵ substituiertes oder unsubstituiertes Aryl, eine substituierte oder unsubstituierte heterocyclische aromatische Gruppe (die substituierte heterocyclische aromatische Gruppe hat 1 bis 3 Substituenten), Cycloalkyl, Pyridin-N-oxid, Cyan oder C₁₋₈-Alkoxycarbonyl repräsentiert;
A -C(R⁹)(R¹⁰)- (wobei R⁹ und R¹⁰ unabhängig voneinander Wasserstoff, substituiertes oder unsubstituiertes C₁₋₈-Alkyl, Cycloalkyl oder Polycycloalkyl repräsentieren) oder O repräsentiert;
B O, -C(R¹²)(R¹³)- {wobei R¹² und R¹³ unabhängig voneinander Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Aralkyl, Cyan oder -(CH₂)ₚ-E³⁻CO-G³ repräsentieren (wobei E³, G³ und p jeweils die gleichen Bedeutungen haben wie die vorstehend beschriebenen E¹, G¹ und n); R¹³ und R² kombiniert sind, um eine Einfachbindung zu bilden; oder R¹³ und R² kombiniert sind, um zusammen mit zwei daran angrenzenden Kohlenstoffatomen einen gesättigten Kohlenstoffring zu bilden}; oder -C(R¹⁴)(R¹⁵)-C(R¹⁶)(R¹⁷)-repräsentiert {wobei R¹⁴ und R¹⁵ unabhängig voneinander Wasserstoff, substituiertes oder unsubstituiertes C₁₋₈-Alkyl, Cycloalkyl, Aralkyl, substituiertes oder unsubstituiertes Aryl oder eine substituierte oder unsubstituierte heterocyclische aromatische Gruppe repräsentieren; oder R¹⁴ und R¹⁵ zu O kombiniert sind; und R¹⁶ und R¹⁷ unabhängig voneinander Wasserstoff, substituiertes oder unsubstituiertes C₁₋₈-Alkyl, Cycloalkyl, Aralkyl, substituiertes oder unsubstituiertes Aryl oder eine substituierte oder unsubstituierte heterocyclische aromatische Gruppe repräsentieren; oder R¹⁷ und R¹⁵ kombiniert sind, um eine Einfachbindung zu bilden; oder R¹⁷ und R¹⁵ kombiniert sind, um zusammen mit zwei daran angrenzenden Kohlenstoffatomen einen gesättigten Kohlenstoffring zu bilden},
mit der Maßgabe, dass, wenn A -C(R⁹)(R¹⁰)- repräsentiert, B nicht O repräsentiert; und
D (i) -C(R¹⁸)(R¹⁹)-X- repräsentiert {wobei R¹⁸ Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Hydroxyl, substituiertes oder unsubstituiertes C₁₋₈-Alkoxyl oder C₂₋₉-Alkanoyloxy repräsentiert; und R¹⁹ Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Hydroxyl, substituiertes oder unsubstituiertes C₁₋₈-Alkoxyl, C₂₋₉-Alkanoyloxy, C₂₋₉-Alkanoyl, Cycloalkanoyl, C₁₋₈₋Alkoxycarbonyl oder Cyan repräsentiert; oder R¹⁸ und R¹⁹ kombiniert sind, um O, S oder NR²⁰ zu bilden (wobei R²⁰ Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Hydroxyl, substituiertes oder unsubstituiertes C₁₋₈-Alkoxyl oder C₂₋₉-Alkanoyloxy repräsentiert); X (a) -C(R²¹)(R²²)- repräsentiert (wobei R²¹ und R²² unabhängig voneinander Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, C₂₋₉-Alkanoyl, Cycloalkanoyl, C₁₋₈-Alkoxycarbonyl oder Cyan repräsentieren), oder (b) S repräsentiert; oder X (c) NR²³ repräsentiert (wobei R²³ Wasserstoff, C₁₋₈-Alkyl, Cycloalkyl, substituiertes oder unsubstituiertes Aryl, eine substituierte oder unsubstituierte heterocyclische aromatische Gruppe oder Aralkyl repräsentiert), es sei denn, R¹ und R² repräsentieren gleichzeitig substituiertes oder unsubstituiertes C₁₋₈-Alkyl, Cycloalkyl, Polycycloalkyl, C₂₋₈-Alkenyl oder Cycloalkenyl mit der vorstehenden Definition, oder es sei denn, R¹ und R² sind kombiniert, um zusammen mit einem daran angrenzenden Kohlenstoffatom einen gesättigten Kohlenstoffring zu bilden, und R⁵ repräsentiert substituiertes oder unsubstituiertes Phenyl oder substituiertes oder unsubstituiertes Pyridyl}, oder (ii) -C(R^{19a})=Y- repräsentiert {wobei R^{19a} Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Hydroxyl, substituiertes oder unsubstituiertes C₁₋₈₋Alkoxyl, C₂₋₉-Alkanoyloxy, C₂₋₉-Alkanoyl, Cycloalkanoyl, C₁₋₈-Alkoxycarbonyl oder Cyan repräsentiert; und Y -C(R²⁴)-Z- (wobei R²⁴ Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, C₂₋₉-Alkanoyl, Cycloalkanoyl, C₁₋₈₋Alkoxycarbonyl oder Cyan repräsentiert; oder R²⁴ und R^{19a} kombiniert sind, um eine Einfachbindung zu bilden; und Z CONH, CONHCH₂ oder eine Bindung repräsentiert) oder N repräsentiert}, oder D (iii) eine Bindung repräsentiert, es sei denn, R⁵ repräsentiert substituiertes oder unsubstituiertes Phenyl, Cyan oder C₁₋₈₋Alkoxycarbonyl,
mit der Maßgabe, dass, wenn R⁵ substituiertes oder unsubstituiertes Phenyl oder C₁₋₈-Alkoxycarbonyl repräsentiert, D nicht -CH₂CH₂- oder -CH=CH- repräsentiert;
oder ein pharmazeutisch verwendbares Salz davon, fakultativ auf einer Form von Addukten mit Wasser oder Lösungsmitteln.

2. Sauerstoffhaltige heterocyclische Verbindung nach Anspruch 1, wobei A O ist und B O ist; oder ein pharmazeutisch verwendbares Salz davon, fakultativ auf einer Form von Addukten mit Wasser oder Lösungsmitteln.

3. Sauerstoffhaltige heterocyclische Verbindung nach Anspruch 1 oder 2, wobei D -C(R¹⁸)(R¹⁹)-X- ist; oder ein pharmazeutisch verwendbares Salz davon, fakultativ auf einer Form von Addukten mit Wasser oder Lösungsmitteln.

4. Sauerstoffhaltige heterocyclische Verbindung nach Anspruch 3, wobei R¹⁸ und R¹⁹ zu O kombiniert sind; oder ein pharmazeutisch verwendbares Salz davon, fakultativ auf einer Form von Addukten mit Wasser oder Lösungsmitteln.

5. Sauerstoffhaltige heterocyclische Verbindung nach Anspruch 3 oder 4, wobei X -C(R²¹)(R²²)- ist; oder ein pharmazeutisch verwendbares Salz davon, fakultativ auf einer Form von Addukten mit Wasser oder Lösungsmitteln.

6. Sauerstoffhaltige heterocyclische Verbindung nach einem der Ansprüche 1 bis 5, wobei R¹ und R² kombiniert sind, um zusammen mit einem daran angrenzenden Kohlenstoffatom einen gesättigten Kohlenstoffring zu bilden; oder ein pharmazeutisch verwendbares Salz davon, fakultativ auf einer Form von Addukten mit Wasser oder Lösungsmitteln.

7. Sauerstoffhaltige heterocyclische Verbindung nach einem der Ansprüche 1 bis 6, wobei R⁵ eine substituierte oder unsubstituierte heterocyclische aromatische Gruppe ist; oder ein pharmazeutisch verwendbares Salz davon, fakultativ auf einer Form von Addukten mit Wasser oder Lösungsmitteln.

8. Verwendung einer sauerstoffhaltigen heterocyclischen Verbindung, die durch die folgende Formel (Z) repräsentiert wird: 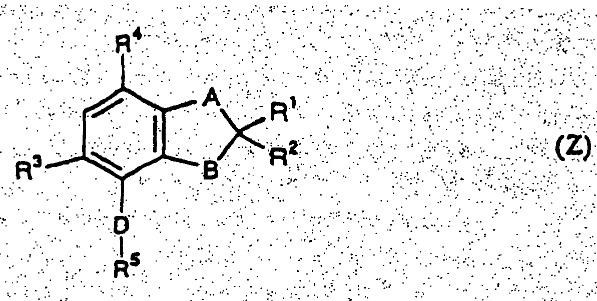 wobei R¹ und R² unabhängig voneinander Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Aralkyl, Cyan oder -(CH₂)ₙ-E¹-CO-G¹ repräsentieren {wobei E¹ eine Bindung, O oder NH repräsentiert; und G¹ Wasserstoff, substituiertes oder unsubstituiertes C₁₋₈-Alkyl, Cycloalkyl, Polycycloalkyl, substituiertes oder unsubstituiertes Aryl, eine substituierte oder unsubstituierte heterocyclische aromatische Gruppe, Aralkyl, OR⁶ (wobei R⁶ Wasserstoff, C₁₋₈-Alkyl, Cycloalkyl, Polycycloalkyl, substituiertes oder unsubstituiertes Aryl, eine substituierte oder unsubstituierte heterocyclische aromatische Gruppe oder Aralkyl repräsentiert), oder NR⁷R⁸ repräsentiert (wobei R⁷ und R⁸ unabhängig voneinander Wasserstoff, C₁₋₈-Alkyl, Cycloalkyl, Polycycloalkyl, substituiertes oder unsubstituiertes Aryl, eine substituierte oder unsubstituierte heterocyclische aromatische Gruppe, substituiertes oder unsubstituiertes Aralkyl oder Heteroarylalkyl repräsentieren; oder R⁷ und R⁸ kombiniert sind, um eine substituierte oder unsubstituierte heterocyclische Gruppe zu repräsentieren, die ein daran angrenzendes Stickstoffatom enthält); und n eine ganze Zahl von 0 bis 4 repräsentiert}; R¹ und R² kombiniert sind, um zusammen mit einem daran angrenzenden Kohlenstoffatom einen gesättigten Kohlenstoffring zu repräsentieren; oder R² und R¹³, die nachstehend beschrieben werden, kombiniert sind, um eine Einfachbindung zu bilden;
R³ Wasserstoff, Phenyl oder Halogen repräsentiert;
R⁴ Hydroxyl oder substituiertes oder unsubstituiertes C₁₋₈-Alkoxyl repräsentiert;
R⁵ substituiertes oder unsubstituiertes Aryl, eine substituierte oder unsubstituierte heterocyclische aromatische Gruppe (die substituierte heterocyclische aromatische Gruppe hat 1 bis 3 Substituenten), Cycloalkyl, Pyridin-N-oxid, Cyan oder C₁₋₈-Alkoxycarbonyl repräsentiert;
A -C(R⁹)(R¹⁰)- (wobei R⁹ und R¹⁰ unabhängig voneinander Wasserstoff, substituiertes oder unsubstituiertes C₁₋₈-Alkyl, Cycloalkyl oder Polycycloalkyl repräsentieren) oder O repräsentiert;
B O, -C(R¹²)(R¹³)- {wobei R¹² und R¹³ unabhängig voneinander Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Aralkyl, Cyan oder -(CH₂)ₚ-E³⁻CO-G³ repräsentieren (wobei E³, G³ und p jeweils die gleichen Bedeutungen haben wie die vorstehend beschriebenen E¹, G¹ und n); R¹³ und R² kombiniert sind, um eine Einfachbindung zu bilden; oder R¹³ und R² kombiniert sind, um zusammen mit zwei daran angrenzenden Kohlenstoffatomen einen gesättigten Kohlenstoffring zu bilden}; oder -C(R¹⁴)(R¹⁵)-C(R¹⁶)(R¹⁷)-repräsentiert {wobei R¹⁴ und R¹⁵ unabhängig voneinander Wasserstoff, substituiertes oder unsubstituiertes C₁₋₈-Alkyl, Cycloalkyl, Aralkyl, substituiertes oder unsubstituiertes Aryl oder eine substituierte oder unsubstituierte heterocyclische aromatische Gruppe repräsentieren; oder R¹⁴ und R¹⁵ zu O kombiniert sind; und R¹⁶ und R¹⁷ unabhängig voneinander Wasserstoff, substituiertes oder unsubstituiertes C₁₋₈-Alkyl, Cycloalkyl, Aralkyl, substituiertes oder unsubstituiertes Aryl oder eine substituierte oder unsubstituierte heterocyclische aromatische Gruppe repräsentieren; oder R¹⁷ und R¹⁵ kombiniert sind, um eine Einfachbindung zu bilden; oder R¹⁷ und R¹⁵ kombiniert sind, um zusammen mit zwei daran angrenzenden Kohlenstoffatomen einen gesättigten Kohlenstoffring zu bilden}, und
D (i) -C(R¹⁸)(R¹⁹)-X- repräsentiert {wobei R¹⁸ Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Hydroxyl, substituiertes oder unsubstituiertes C₁₋₈-Alkoxyl oder C₂₋₉-Alkanoyloxy repräsentiert; und R¹⁹ Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Hydroxyl, substituiertes oder unsubstituiertes C₁₋₈-Alkoxyl, C₂₋₉-Alkanoyloxy, C₂₋₉-Alkanoyl, Cycloalkanoyl, C₁₋₈₋Alkoxycarbonyl oder Cyan repräsentiert; oder R¹⁸ und R¹⁹ kombiniert sind, um O, S oder NR²⁰ zu bilden (wobei R²⁰ Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Hydroxyl, substituiertes oder unsubstituiertes C₁₋₈-Alkoxyl oder C₂₋₉-Alkanoyloxy repräsentiert); X (a) -C(R²¹)(R²²)- repräsentiert (wobei R²¹ und R²² unabhängig voneinander Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, C₂₋₉-Alkanoyl, Cycloalkanoyl, C₁₋₈-Alkoxycarbonyl oder Cyan repräsentieren), oder (b) S repräsentiert; oder X (c) NR²³ repräsentiert (wobei R²³ Wasserstoff, C₁₋₈-Alkyl, Cycloalkyl, substituiertes oder unsubstituiertes Aryl, eine substituierte oder unsubstituierte heterocyclische aromatische Gruppe oder Aralkyl repräsentiert), es sei denn, R¹ und R² repräsentieren gleichzeitig substituiertes oder unsubstituiertes C₁₋₈-Alkyl, Cycloalkyl, Polycycloalkyl, C₂₋₈-Alkenyl oder Cycloalkenyl mit der obigen Definition, oder es sei denn, R¹ und R² sind kombiniert, um zusammen mit einem daran angrenzenden Kohlenstoffatom einen gesättigten Kohlenstoffring zu bilden, und R⁵ repräsentiert substituiertes oder unsubstituiertes Phenyl oder substituiertes oder unsubstituiertes Pyridyl}, (ii) -C(R^{19a})=Y- repräsentiert {wobei R^{19a} Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Hydroxyl, substituiertes oder unsubstituiertes C₁₋₈₋Alkoxyl, C₂₋₉-Alkanoyloxy, C₂₋₉-Alkanoyl, Cycloalkanoyl, C₁₋₈-Alkoxycarbonyl oder Cyan repräsentiert; und Y -C(R²⁴)-Z- repräsentiert (wobei R²⁴ Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, C₂₋₉-Alkanoyl, Cycloalkanoyl, C₁₋₈-Alkoxycarbonyl oder Cyan repräsentiert; oder R²⁴ und R^{19a} kombiniert sind, um eine Einfachbindung zu bilden; und Z CONH, CONHCH₂ oder eine Bindung repräsentiert) oder N repräsentiert), oder (iii) eine Bindung repräsentiert, es sei denn, R⁵ repräsentiert substituiertes oder unsubstituiertes Phenyl, Cyan oder C₁₋₈₋Alkoxycarbonyl,
oder eines pharmazeutisch verwendbaren Salzes davon, fakultativ auf einer Form von Addukten mit Wasser oder Lösungsmitteln zur Herstellung eines Phosphodiesterase-IV-Inhibitors.

9. Verwendung nach Anspruch 8 der sauerstoffhaltigen heterocyclischen Verbindung nach einem der Ansprüche 1 bis 7, oder eines pharmazeutisch verwendbaren Salzes davon, fakultativ auf einer Form von Addukten mit Wasser oder Lösungsmitteln.

10. Verwendung einer sauerstoffhaltigen heterocyclischen Verbindung, die durch die folgende Formel (ZA) repräsentiert wird: 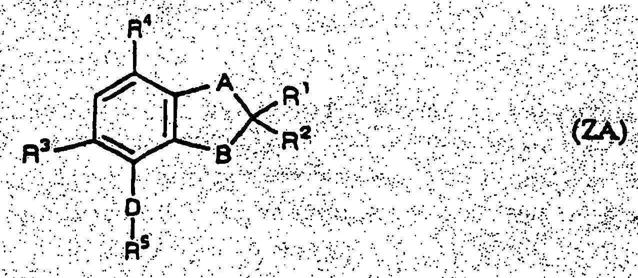 wobei R¹ und R² unabhängig voneinander Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Aralkyl, Cyan oder -(CH₂)ₙ-E¹-CO-G¹ repräsentieren {wobei E¹ eine Bindung, O oder NH repräsentiert; und G¹ Wasserstoff, substituiertes oder unsubstituiertes C₁₋₈-Alkyl, Cycloalkyl, Polycycloalkyl, substituiertes oder unsubstituiertes Aryl, eine substituierte oder unsubstituierte heterocyclische aromatische Gruppe, Aralkyl, OR⁶ (wobei R⁶ Wasserstoff, C₁₋₈-Alkyl, Cycloalkyl, Polycycloalkyl, substituiertes oder unsubstituiertes Aryl, eine substituierte oder unsubstituierte heterocyclische aromatische Gruppe oder Aralkyl repräsentiert), oder NR⁷R⁸ repräsentiert (wobei R⁷ und R⁸ unabhängig voneinander Wasserstoff, C₁₋₈-Alkyl, Cycloalkyl, Polycycloalkyl, substituiertes oder unsubstituiertes Aryl, eine substituierte oder unsubstituierte heterocyclische aromatische Gruppe, substituiertes oder unsubstituiertes Aralkyl oder Heteroarylalkyl repräsentieren; oder R⁷ und R⁸ kombiniert sind, um eine substituierte oder unsubstituierte heterocyclische Gruppe zu repräsentieren, die ein daran angrenzendes Stickstoffatom enthält); und n eine ganze Zahl von 0 bis 4 repräsentiert}; R¹ und R² kombiniert sind, um zusammen mit einem daran angrenzenden Kohlenstoffatom einen gesättigten Kohlenstoffring zu repräsentieren; oder R² und R¹³, die nachstehend beschrieben werden, kombiniert sind, um eine Einfachbindung zu bilden;
R³ Wasserstoff, Phenyl oder Halogen repräsentiert;
R⁴ Hydroxyl oder substituiertes oder unsubstituiertes C₁₋₈-Alkoxyl repräsentiert;
R⁵ substituiertes oder unsubstituiertes Aryl, eine substituierte oder unsubstituierte heterocyclische aromatische Gruppe (die substituierte heterocyclische aromatische Gruppe hat 1 bis 3 Substituenten), Cycloalkyl, Pyridin-N-oxid, Cyan oder C₁₋₈-Alkoxycarbonyl repräsentiert;
A -C(R⁹)(R¹⁰)- (wobei R⁹ und R¹⁰ unabhängig voneinander Wasserstoff, substituiertes oder unsubstituiertes C₁₋₈-Alkyl, Cycloalkyl oder Polycycloalkyl repräsentieren) oder O repräsentiert;
B O, -C(R¹²)(R¹³)- {wobei R¹² und R¹³ unabhängig voneinander Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Aralkyl, Cyan oder -(CH₂)ₚ-E³⁻CO-G³ repräsentieren (wobei E³, G³ und p jeweils die gleichen Bedeutungen haben wie die vorstehend beschriebenen E¹, G¹ und n); R¹³ und R² kombiniert sind, um eine Einfachbindung zu bilden; oder R¹³ und R² kombiniert sind, um zusammen mit zwei daran angrenzenden Kohlenstoffatomen einen gesättigten Kohlenstoffring zu bilden}; oder -C(R¹⁴)(R¹⁵)-C(R¹⁶)(R¹⁷)-repräsentiert {wobei R¹⁴ und R¹⁵ unabhängig voneinander Wasserstoff, substituiertes oder unsubstituiertes C₁₋₈-Alkyl, Cycloalkyl, Aralkyl, substituiertes oder unsubstituiertes Aryl oder eine substituierte oder unsubstituierte heterocyclische aromatische Gruppe repräsentieren; oder R¹⁴ und R¹⁵ zu O kombiniert sind; und R¹⁶ und R¹⁷ unabhängig voneinander Wasserstoff, substituiertes oder unsubstituiertes C₁₋₈-Alkyl, Cycloalkyl, Aralkyl, substituiertes oder unsubstituiertes Aryl oder eine substituierte oder unsubstituierte heterocyclische aromatische Gruppe repräsentieren; oder R¹⁷ und R¹⁵ kombiniert sind, um eine Einfachbindung zu bilden; oder R¹⁷ und R¹⁵ kombiniert sind, um zusammen mit zwei daran angrenzenden Kohlenstoffatomen einen gesättigten Kohlenstoffring zu bilden}, und D (i) -C(R¹⁸)(R¹⁹)-X- repräsentiert {wobei R¹⁸ Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Hydroxyl, substituiertes oder unsubstituiertes C₁₋₈-Alkoxyl oder C₂₋₉-Alkanoyloxy repräsentiert; und R¹⁹ Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Hydroxyl, substituiertes oder unsubstituiertes C₁₋₈-Alkoxyl, C₂₋₉-Alkanoyloxy, C₂₋₉-Alkanoyl, Cycloalkanoyl, C₁₋₈₋Alkoxycarbonyl oder Cyan repräsentiert; oder R¹⁸ und R¹⁹ kombiniert sind, um O, S oder NR²⁰ zu bilden (wobei R²⁰ Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Hydroxyl, substituiertes oder unsubstituiertes C₁₋₈-Alkoxyl oder C₂₋₉-Alkanoyloxy repräsentiert); X (a) -C(R²¹)(R²²)- repräsentiert (wobei R²¹ und R²² unabhängig voneinander Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, C₂₋₉-Alkanoyl, Cycloalkanoyl, C₁₋₈-Alkoxycarbonyl oder Cyan repräsentieren), oder (b) S repräsentiert; oder X (c) NR²³ repräsentiert (wobei R²³ Wasserstoff, C₁₋₈-Alkyl, Cycloalkyl, substituiertes oder unsubstituiertes Aryl, eine substituierte oder unsubstituierte heterocyclische aromatische Gruppe oder Aralkyl repräsentiert), es sei denn, R¹ und R² repräsentieren gleichzeitig substituiertes oder unsubstituiertes C₁₋₈-Alkyl, Cycloalkyl, Polycycloalkyl, C₂₋₈-Alkenyl oder Cycloalkenyl mit der obigen Definition, oder es sei denn, R¹ und R² sind kombiniert, um zusammen mit einem daran angrenzenden Kohlenstoffatom einen gesättigten Kohlenstoffring zu bilden, und R⁵ repräsentiert substituiertes oder unsubstituiertes Phenyl oder substituiertes oder unsubstituiertes Pyridyl}, oder (ii) -C(R^{19a})=Y- repräsentiert {wobei R^{19a} Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, Hydroxyl, substituiertes oder unsubstituiertes C₁₋₈₋Alkoxyl, C₂₋₉-Alkanoyloxy, C₂₋₉-Alkanoyl, Cycloalkanoyl, C₁₋₈-Alkoxycarbonyl oder Cyan repräsentiert; und Y -C(R²⁴)-Z- repräsentiert (wobei R²⁴ Wasserstoff, substituiertes oder unsubstituiertes C₁₋₈-Alkyl, Cycloalkyl, Polycycloalkyl, C₂₋₈-Alkenyl, Cycloalkenyl, substituiertes oder unsubstituiertes Aryl, eine substituierte oder unsubstituierte heterocyclische aromatische Gruppe, C₂₋₉-Alkanoyl, Cycloalkanoyl, C₁₋₈-Alkoxycarbonyl oder Cyan repräsentiert; oder R²⁴ und R^{19a} kombiniert sind, um eine Einfachbindung zu bilden; und Z CONH, CONHCH₂ oder eine Bindung repräsentiert) oder N repräsentiert}, oder D (iii) eine Bindung repräsentiert, es sei denn, R⁵ repräsentiert substituiertes oder unsubstituiertes Phenyl, Cyan oder C₁₋₈₋Alkoxycarbonyl,
oder eines pharmazeutisch verwendbaren Salzes davon, fakultativ auf einer Form von Addukten mit Wasser oder Lösungsmitteln, zur Herstellung einer pharmazeutischen Zusammensetzung, die zur Behandlung von entzündlichen allergischen Erkrankungen nützlich ist.

11. Verwendung nach Anspruch 10 der sauerstoffhaltigen heterocyclischen Verbindung nach einem der Ansprüche 1 bis 7 oder eines pharmazeutisch verwendbaren Salzes davon, fakultativ auf einer Form von Addukten mit Wasser oder Lösungsmitteln.
